# EUROPEAN PATENT SPECIFICATION

(11) **EP 3 911 645 B1**
(45) Date of publication and mention of the grant of the patent: **07.12.2022**
(21) Application number: 20701927.4
(22) Date of filing: 16.01.2020
(51) Int. Cl.: C07D 403/12, C07D 231/38, C07D 401/12, C07D 403/04, C07D 403/06, C07D 405/12, C07D 413/12, C07D 249/04, C07D 271/06, A61P 35/00, A61K 31/4192, A61K 31/4245, A61K 31/415, A61K 31/4439

(54) **5-MEMBERED HETEROARYL COMPOUNDS CONTAINING A HYDROXAMATE MOIETY AND THEIR USE**
5-GLIEDRIGE HETEROARYLVERBINDUNGEN MIT EINEM HYDROXAMATREST UND DEREN VERWENDUNG
COMPOSÉS HÉTÉROARYLES À 5 CHAÎNONS CONTENANT UNE FRACTION D'HYDROXAMATE ET LEUR UTILISATION

(30) Priority: 17.01.2019 EP 19305058
(43) Date of publication of application: 24.11.2021
(73) Proprietor: Université de Lille, 59000 Lille (FR); INSERM (Institut National de la Santé et de la Recherche Médicale), 75013 Paris (FR); Institut Pasteur de Lille, 59800 Lille (FR); Centre National de la Recherche Scientifique - CNRS, 75016 Paris (FR); UNIVERSITE PARIS DESCARTES, 75006 Paris (FR)
(72) Inventor: DEPREZ POULAIN, Rebecca, 59000 Lille (FR); DEPREZ, Benoit, 59000 Lille (FR); LEROUX, Florence, 59175 Templemars (FR); BOSC, Damien, 59320 Haubourdin (FR); GEALAGEAS, Ronan, 59800 Lille (FR); JAKHLAL, Jouda, 08170 Fumay (FR); VAN ENDERT, Peter, 92160 Antony (FR); HENNUYER, Nathalie, 62112 Corbehem (FR); STAELS, Bart, 7850 Petit-Enghien (BE)
(74) Representative: Plasseraud IP
(86) International application number: PCT/EP2020/051058
(87) International publication number: WO 2020/148403

(56) References cited:
- YAO YIWU ET AL: "Design, synthesis, and biological evaluation of 1, 3-disubstituted-pyrazole derivatives as new class I and IIb histone deacetylase inhibitors", EUROPEAN JOURNAL OF MEDICINAL CHEMISTRY, EDITIONS SCIENTIFIQUE ELSEVIER, PARIS, FR, vol. 86, 8 September 2014 (2014-09-08), pages 639-652, XP029048609, ISSN: 0223-5234, DOI: 10.1016/J.EJMECH.2014.09.024
- CHEN P C ET AL: "Synthesis and structure-activity relationship of histone deacetylase (HDAC) inhibitors with triazole-linked cap group", BIOORGANIC & MEDICINAL CHEMISTRY, PERGAMON, GB, vol. 16, no. 9, 1 May 2008 (2008-05-01), pages 4839-4853, XP022647279, ISSN: 0968-0896, DOI: 10.1016/J.BMC.2008.03.050 [retrieved on 2008-03-23]
- SUN QIAO ET AL: "Design, synthesis, and biological evaluation of novel histone deacetylase 1 inhibitors through click chemistry", BIOORGANIC & MEDICINAL CHEMISTRY LETTERS, PERGAMON, AMSTERDAM, NL, vol. 23, no. 11, 4 April 2013 (2013-04-04) , pages 3295-3299, XP028535157, ISSN: 0960-894X, DOI: 10.1016/J.BMCL.2013.03.102
- DEPREZ-POULAIN ET AL.: "Catalytic site inhibition of insulin-degrading enzyme by a small molecule induces glucose intolerance in mice", NATURE COMMUNICATIONS, vol. 6, no. 8250, 23 September 2015 (2015-09-23), pages 1-13, XP002790380,

## Description

The present invention relates to novel di-substituted 5-membered heteroaryl compounds containing a hydroxamate moiety including their pharmaceutically acceptable salts and solvates which are useful as sensitizers for chemotherapy of malignant tumors, particularly in multiple myeloma, cervical cancer and breast cancer, and are useful as therapeutic compounds, particularly in the treatment and/or prevention of cancers that may be treated by cytotoxic agents, such as proteasome inhibitors, histone deacetylase (HDAC) inhibitors and/or protein glycosylation inhibitors.

### BACKGROUND OF THE INVENTION

Chemotherapy is a type of treatment that includes a medication to treat cancer. The goal of chemotherapy is to stop or slow the growth of cancer cells. Chemotherapy medications attack rapidly growing cancer cells, but they can also affect healthy cells that grow rapidly.

The effect of these medications on normal cells often causes side effects.

Histone deacetylase (HDAC) is a kind of metalloprotease, which plays a key role in chromosome structure modification and gene expression modulation. In recent years, it has been found that inhibiting HDACs activity can effectively inhibit cancer cell proliferation, induce cell cycle arrest and promote cell apoptosis, and therefore, HDACs have become a new target of anticancer drug design, and using HDAC inhibitor is considered as an effective strategy for cancer therapy. Particularly, hydroxamic acid compounds have been paid much attention and demonstrated a good anti-tumor activity, both *in vitro* and *in vivo.*

In 2006, vorinostat was the first hydroxamic acid compound approved by the United States FDA and is used for treating skin T cell lymphoma. Other examples of hydroxamic acid compounds having an activity as HDAC inhibitors and their use for the treatment of cancer are namely disclosed in WO 2010/151318 A1, WO 2012/098132 A1, CN 107445896 A, US 2016/176879 A1, EUR. J. OF MED. CHEM. 2014, vol. 86, p. 639-652, BIOORG. & MED. CHEM. 2008, vol. 16, no. 9, p. 4839-4853 and BIOORG. & MED. CHEM. LETT. vol. 23, no. 11, p. 3295-3299.

Proteasomes are protein complexes which degrade unneeded or damaged proteins by proteolysis, a chemical reaction that breaks peptide bonds. Proteasome inhibitors are drugs that block the action of proteasomes, and are being studied in the treatment of cancer; and three are approved for use in treating multiple myeloma. Multiple mechanisms are likely to be involved, but proteasome inhibition may prevent degradation of pro-apoptotic factors such as the p53 protein, permitting activation of programmed cell death in neoplastic cells dependent upon suppression of pro-apoptotic pathways. For example, bortezomib causes a rapid and dramatic change in the levels of intracellular peptides.

Most of anticancer agents are used in combination. Synergistic effect is searched for a better efficacy and lowering of resistance. Also, sensitizers are used to effectively increase the sensitivity of tumor cells to treatments under a lower dose.

US 9,126,952 B2 discloses morpholinylquinazoline compounds and their use for the sensitization of cancer cells to anticancer agents.

However, there is still a need for new compounds having the ability to sensitize cancer cells to various anticancer drugs, and that may be of therapeutic value in the treatment of cancers that may be treated by cytotoxic agents, such as proteasome inhibitors, HDAC inhibitors or protein glycosylation inhibitors.

### SUMMARY OF THE INVENTION

Any references to methods of treatment or the use of the compounds as drugs or for a treatment in the subsequent paragraphs of this description are to be interpreted as references to the compounds, pharmaceutical compositions and medicaments of the present invention for use in a method for treatment of the human (or animal) body by therapy.

The inventors have now succeeded in developing novel compounds based on a 5-membered heteroaryl scaffold bearing two side groups, one of which carries a hydroxamic acid moiety. These compounds have the advantage of increasing the cytotoxicity of various anticancer drugs.

The invention therefore relates to compounds of general Formula I, their pharmaceutically acceptable salts and solvates as well as methods of use of such compounds or compositions comprising such compounds as sensitizers for chemotherapy of malignant tumors.

In a general aspect, the invention provides compounds of general Formula I: 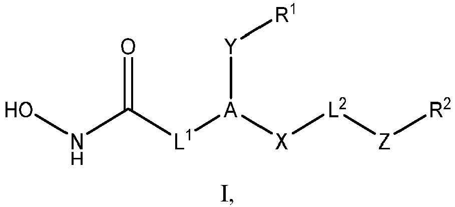 and pharmaceutically acceptable salts and solvates thereof,
wherein
**L¹** is inexistent or is selected from -CH₂- and -CH(OH)-;
**A** is CH or N;
**Y** is -CH₂- or -S-;
**R¹** is selected from aryl, heteroaryl, arylalkyl and arylalkenyl wherein said aryl, heteroaryl, arylalkyl or arylalkenyl is optionally substituted by one or more substituents independently selected from C1-C4-alkyl, halogen, C1-C4-haloalkyl, hydroxy-C1-C4-alkyl, acetylamino, acetylamino-C1-C4-alkyl and C1-C4-alkylaminocarboxyl;
**R²** is selected from aryl, heteroaryl, arylalkyl, cycloalkyl, heterocycloalkyl, C1-C6-alkyl, C1-C4-alkyloxycarbonyl, C1-C4-alkoxy, arylamino, wherein said aryl, arylalkyl, cycloalkyl or heterocycloalkyl is optionally substituted by one or more substituents independently selected from C1-C4-alkoxy, halogen, C1-C4-haloalkyl, C1-C4-alkyl, C1-C2-alkoxy-C1-C2-alkoxy, C1-C4-alkyloxycarbonyl, acetylamino, di(C1-C4-alkyl)amino-C1-C4-alkyl and hydroxycarbamoyl;
**X** is selected from 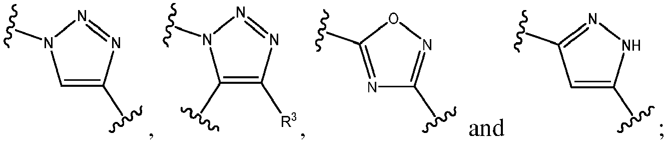
**R³** is selected from H and C(O)OR⁴;
**R⁴** is C1-C4-alkyl;
**L²** is linear or branched C1-C6-alkyl,optionally substituted by a group **R⁵;**
**R⁵** is selected from linear or branched C1-C4-alkyl, CH₂-OH, CHOH-CH₃, CH₂-C(O)NH₂, CH₂-CH₂-C(O)NH₂, CH₂-COOH, CH₂-CH₂-COOH, (CH₂)₄-NH₂, (CH₂)₄-NH-C(NH₂⁺)-NH₂, CH₂-Imidazolyl, CH₂-Indolyl, CH₂-SH, CH₂-CH₂-S-CH₃, CH₂-Ph, CH₂-Ph-OH, CH₂-OR⁶, CH₂-COOR⁶, CH₂-CH₂-COOR⁶, CH₂-SR⁶ and CH₂-Ph-OR⁶;
**R⁶** is selected from Me, Bn, Ph and Ac;
**Z** is selected from -NR⁷(CO)-, -NHSO₂-, -CH₂-CH₂-, -NH-, -NH-CH(C1-C6-alkyl)-, -O-CH₂-, -C(O)NH- and -O-; and
**R⁷** is selected from **H,** linear or branched C1-C6-alkyl and (1-methylimidazol-2-yl)-C1-C2-alkyl, or
**R⁷** and **L²** form together with the nitrogen atom they are attached to a 5- or 6-membered heterocyclyl group;
with the proviso that the compound of Formula I is none of the following:
   N-[1-((R)-2-hydroxycarbamoyl-1-naphthalen-2-ylmethyl-ethyl)-1H-[1,2,3]triazol-4-ylmethyl]-benzamide;
   N-[1-((R)-2-hydroxycarbamoyl-1-naphthalen-2-ylmethyl-ethyl)-1H-[1,2,3]triazol-4-ylmethyl]-4-methoxy-benzamide;
   4-fluoro-N-[1-(R-2-hydroxycarbamoyl-1-naphthalen-2-ylmethyl-ethyl)-1H-[1,2,3]triazol-4-ylmethyl] -benzamide;
   (R)-3-{4-[(4-fluoro-benzenesulfonylamino)-methyl]-[1,2,3]triazol-1-yl} -N-hydroxy-4-naphthalen-2-yl-butyramide;
   (R)-N-hydroxy-4-naphthalen-2-yl-3-{4-[(2-p-tolyl-acetylamino)-methyl]-[1,2,3]triazol-1-yl}-butyramide;
   (R)-N-hydroxy-4-naphthalen-2-yl-3-{4-[(3-phenyl-propionylamino)-methyl]-[1,2,3]triazol-1-yl}-butyramide;
   (R)-4-fluoro-N-[3-(1-hydroxycarbamoylmethyl-2-naphthalen-2-yl-ethyl)-3H-[1,2,3]triazol-4-ylmethyl] -benzamide;
   (R)-cyclohexanecarboxylic acid [3-(1-hydroxycarbamoylmethyl-2-naphthalen-2-yl-ethyl)-3H-[1,2,3]triazol-4-ylmethyl]-amide;
   (R)-N-hydroxy-4-naphthalen-2-yl-3-[4-(phenylacetylamino-methyl)-[1,2,3]triazol-1-yl]-butyramide;
   (R)-N-hydroxy-4-naphthalen-2-yl-3-{5-[(3-phenyl-propionylamino)-methyl]-[1,2,3]triazol-1-yl}-butyramide;
   4-fluoro-N-[5-(1-hydroxycarbamoylmethyl-2-naphthalen-2-yl-ethyl)-[1,2,4]oxadiazol-3-ylmethyl]-benzamide;
   N-[5-(1-hydroxycarbamoylmethyl-2-naphthalen-2-yl-ethyl)-[1,2,4]oxadiazol-3-ylmethyl]-benzamide;
   (S)-4-fluoro-N-[1-(2-hydroxycarbamoyl-1-naphthalen-2-ylmethyl-ethyl)-1H-[1,2,3]triazol-4-ylmethyl] -benzamide;
   (R)-4-fluoro-N-[1-(1-hydroxycarbamoyl-2-naphthalen-2-yl-ethyl)-1H-[1,2,3]triazol-4-ylmethyl]-benzamide;
   N-[1-(1-hydroxycarbamoylmethyl-2-phenyl-ethyl)-1H-[1,2,3]triazol-4-ylmethyl]-4-methyl-benzamide;
   N-[1-((R)-2-hydroxycarbamoyl-1-naphthalen-2-ylmethyl-ethyl)-1H-[1,2,3]triazol-4-ylmethyl] -4-methyl-benzamide;
   cyclohexanecarboxylic acid [1-(R-2-hydroxycarbamoyl-1-naphthalen-2-ylmethyl-ethyl)-1H-[1,2,3]triazol-4-ylmethyl]-amide; and
   N-[1-(1-hydroxycarbamoylmethyl-2-phenyl-ethyl)-1H-[1,2,3]triazol-4-ylmethyl]-4-fluoro-benzamide.

In another aspect, the present invention provides a pharmaceutical composition comprising at least one compound according to the invention or a pharmaceutically acceptable salt or solvate thereof and at least one pharmaceutically acceptable carrier, diluent, excipient and/or adjuvant.

The invention also relates to the use of compounds of Formula I or their pharmaceutically acceptable salts and solvates for the treatment and/or prevention of cancer, particularly as sensitizers for chemotherapy of malignant tumors. Thus, the compound of the invention is useful for the treatment and/or prevention of cancers that may be treated by cytotoxic agents, such as proteasome inhibitors, HDAC inhibitors or protein glycosylation inhibitors.

### DETAILED DESCRIPTION OF THE INVENTION

As detailed above, the invention relates to compounds of Formula I, as well as their pharmaceutically acceptable salts and solvates.

Preferred compounds of Formula I and pharmaceutically acceptable salts and solvates thereof are those wherein one or more of **L¹, L², X, Y, Z, R¹, R², R³, R⁴, R⁵, R⁶** and **R⁷** are defined as follows:
**L¹** is -CH₂- or -CH(OH)-; more preferably **L¹** is -CH₂-;
**A** is CH or N; preferably **A** is CH;
**Y** is -CH₂- or -S-; preferably **Y** is -CH₂-;
**R¹** is selected from naphthalen-2-yl, phenyl, biphenyl, 4-pyrimidin-5-yl-phenyl, tetraisoquinolinyl, 3-indolyl, 5-indolyl, 6-indolyl, 3-quinolinyl, 4-quinolinyl, 7-quinolinyl, dihydroquinolin-2-on-6-yl, 5,6,7,8-tetrahydronaphthalen-2-yl, (2H-1,2,3,4-tetrazol-5-yl)phenyl, benzyl, styryl, 5-benzothiophenyl, 6-benzothiophenyl, 3-benzothiophenyl, 2-benzothiophenyl, 6-(1,3-benzothiazolyl and 6-benzodioxinyl, wherein said naphthalen-2-yl, phenyl, biphenyl, 4-pyrimidin-5-yl-phenyl, tetraisoquinolinyl, 3-indolyl, 5-indolyl, 6-indolyl, 3-quinolinyl, 4-quinolinyl, 7-quinolinyl, dihydroquinolin-2-on-6-yl, 5,6,7,8-tetrahydronaphthalen-2-yl, (2H-1,2,3,4-tetrazol-5-yl)phenyl, benzyl, styryl, 5-benzothiophenyl, 6-benzothiophenyl, 3-benzothiophenyl, 2-benzothiophenyl, 6-(1,3-benzothiazolyl or 6-benzodioxinyl is optionally substituted by one or more substituents selected from C1-C4-alkyl, halo, preferably bromo or chloro, more preferably bromo, C1-C4-haloalkyl, preferably C1-C4-fluroalkyl, hydroxy-C1-C4-alkyl, preferably hydroxymethyl, acetylamino, acetylamino-C1-C4-alkyl, preferably acetylaminomethyl, and C1-C4-alkylaminocarboxyl, preferably methylaminocarboxyl; preferably **R¹** is selected from naphthalen-2-yl, phenyl, biphenyl, 4-pyrimidin-5-yl-phenyl, tetraisoquinolinyl, 3-indolyl, 3-quinolinyl, 4-quinolinyl, 5,6,7,8-tetrahydronaphthalen-2-yl, (2H-1,2,3,4-tetrazol-5-yl)phenyl, benzyl and styryl, wherein said naphthalen-2-yl, phenyl, biphenyl, tetraisoquinolinyl, 3-indolyl, 3-quinolinyl, 4-quinolinyl, 5,6,7,8-tetrahydronaphthalen-2-yl, (2H-1,2,3,4-tetrazol-5-yl)phenyl, benzyl or styryl is optionally substituted by one substituent selected from C1-C4-alkyl, halo, preferably bromo or chloro, more preferably bromo, C1-C4-haloalkyl, preferably C1-C4-fluroalkyl, hydroxy-C1-C4-alkyl, preferably hydroxymethyl, acetylamino, acetylamino-C1-C4-alkyl, preferably acetylaminomethyl, and C1-C4-alkylaminocarboxyl, preferably methylaminocarboxyl; more preferably **R¹** is selected from naphthalen-2-yl, phenyl, biphenyl, 4-pyrimidin-5-yl-phenyl, tetraisoquinolinyl, 3-indolyl, 3-quinolinyl, 4-quinolinyl, 5,6,7,8-tetrahydronaphthalen-2-yl, (2H-1,2,3,4-tetrazol-5-yl)phenyl, benzyl and styryl wherein said naphthalen-2-yl, phenyl, biphenyl or (2H-1,2,3,4-tetrazol-5-yl)phenyl is optionally substituted by one substituent selected from halo, preferably bromo or chloro, more preferably bromo, C1-C4-haloalkyl, preferably C1-C4-fluroalkyl, more preferably trifluoromethyl, hydroxy-C1-C4-alkyl, preferably hydroxymethyl, acetylamino, acetylamino-C1-C4-alkyl, preferably acetylaminomethyl, and C1-C4-alkylaminocarboxyl, preferably methylaminocarboxyl;
**R²** is selected from phenyl, 1,4-imidazolyl, pyrimidinyl, pyridinyl, phenyl-C1-C2-alkyl, cyclohexyl, piperidinyl, morpholinyl, 1,3-benzodioxolyl, C1-C5-alkyl, *t*-butyloxycarbonyl, *t*-butyloxy, phenylamino, wherein said phenyl, 1,4-imidazolyl, phenyl-C1-C2-alkyl, piperidinyl, is optionally substituted by one or more substituents independently selected from C1-C4-alkoxy, preferably methoxy, halogen, preferably fluoro and chloro, C1-C4-haloalkyl, preferably trifluoromethyl, C1-C4-alkyl, preferably methyl, C1-C2-alkoxy-C1-C2-alkoxy, preferably methoxyethoxy, C1-C4-alkyloxycarbonyl, preferably *t-*butyloxycarbonyl, acetylamino, di(C1-C4-alkyl)amino-C1-C4-alkyl, preferably dimethylaminomethyl, and hydroxycarbamoyl.
X is selected from 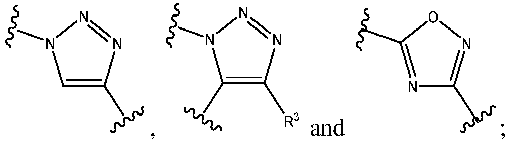 more preferably **X** is 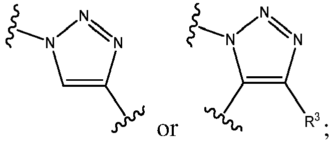 even more preferably **X** is 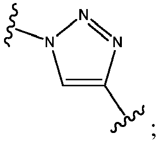
**R³** is H or C(O)OR⁴; preferably **R³** is H;
**R⁴** is C1-C2-alkyl; more preferably **R⁴** is ethyl;
**L²** is selected from -CH₂-, -CH₂-CH₂-, -C(CH₃)₂- and -CH(R⁵)-; more preferably **L²** is selected from -CH₂-, -CH₂-CH₂- and -CH(R⁵)-; even more preferably **L²** is -CH₂-;
**R⁵** is selected from CH₃, CH(CH₃)-CH₃, CH₂-CH(CH₃)-CH₃, CH(CH₃)-CH₂-CH₃, CH₂-OH, CHOH-CH₃, CH₂-C(O)NH₂, CH₂-CH₂-C(O)NH₂, CH₂-COOH, CH₂-CH₂-COOH, (CH₂)₄-NH₂, (CH₂)₄-NH-C(NH₂⁺)-NH₂, CH₂-Imidazolyl, CH₂-Indolyl, CH₂-SH, CH₂-CH₂-S-CH₃, CH₂-Ph, CH₂-Ph-OH, CH₂-OR⁶, CH₂-COOR⁶, CH₂-CH₂-COOR⁶, CH₂-SR⁶ and CH₂-Ph-OR⁶; more preferably **R⁵** is selected from CH₃, CH₂-OH, CH₂-Ph-OH and CH₂-Ph-OR⁶; even more preferably **R⁵** is CH₃;
**R⁶** is selected from Me, Bn, Ph and Ac, more preferably **R⁶** is Bn;
**Z** is selected from -NR⁷(CO)-, -NHSO₂- and -C(O)NH-; more preferably **Z** is - NR⁷(CO)-; and
**R⁷** is selected from H, methyl, ethyl, isobutyl, isopentyl and (1-methylimidazol-2-yl)methyl; more preferably **R⁷** is H; or
**R⁷** and **L²** form together with the nitrogen atom they are attached to a 5- or 6-membered heterocyclyl group selected from pyrrolidinyl, morpholinyl, thiazolidinyl and piperidinyl; preferably **R⁷** and **L²** form together with the nitrogen atom they are attached to a pyrrolidinyl or a morpholinyl group.

In one embodiment, **A** is CH and the compound of formula I may therefore be in racemic or optically active form. Preferably, **A** is CH and the compound of formula I is in optically active form. More preferably, **A** is CH and the compound of formula I is in (*R*) form.

In one embodiment, the compounds of Formula I are those of Formula II: 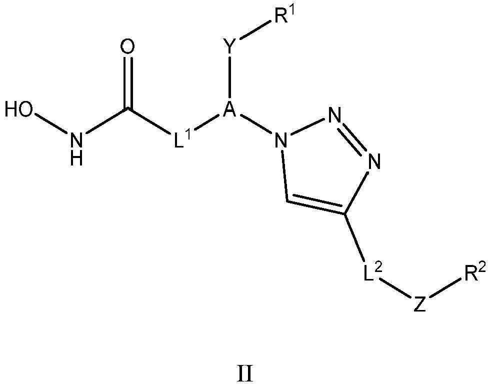 and pharmaceutically acceptable salts and solvates thereof,
wherein
**L¹, L², A, Y, Z, R¹, R², R⁵, R⁶** and **R⁷** are as defined above with respect to Formula I and any of its embodiments.

In one embodiment, preferred compounds of Formula II are those of Formula IIa: 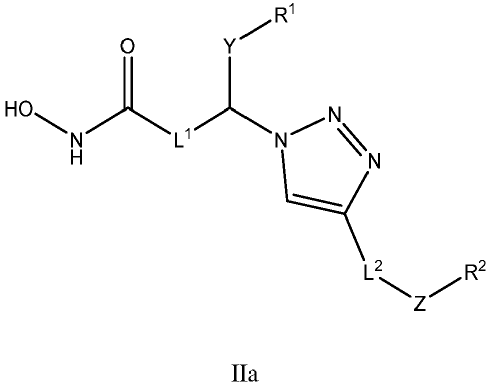 and pharmaceutically acceptable salts and solvates thereof,
wherein
**L¹, L², Y, Z, R¹, R², R⁵, R⁶** and **R⁷** are as defined above with respect to Formula I and any of its embodiments.

In one embodiment, the compounds of Formula II are those of Formula IIb: 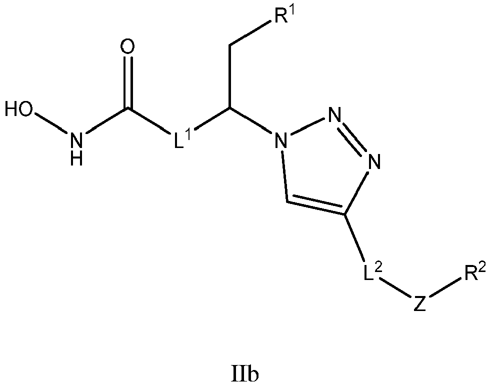 and pharmaceutically acceptable salts and solvates thereof,
wherein
**L¹, L², Z, R¹, R², R⁵, R⁶** and **R⁷** are as defined above with respect to Formula I and any of its embodiments.

In one embodiment, preferred compounds of Formula IIb are those of Formula IIc: 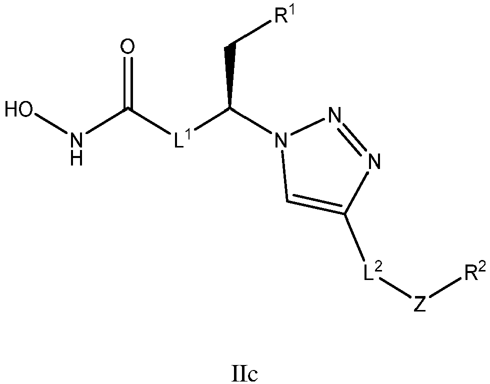 and pharmaceutically acceptable salts and solvates thereof,
wherein
**L¹, L², Z, R¹, R², R⁵, R⁶** and **R⁷** are as defined above with respect to Formula I and any of its embodiments.

In one embodiment, **Z** is -NR⁷(CO)- and **L¹, L², R¹, R², R⁵, R⁶** and **R⁷** are as defined above with respect to Formula I and any of its embodiments.

Examples of compounds according to this embodiment are those wherein one or more of **L¹, L², R¹, R², R⁵, R⁶** and **R⁷** are defined as follows:
**L¹** is -CH₂-;
**L²** is selected from -CH₂-, -CH₂-CH₂-, -C(CH₃)₂- and -CH(R⁵)-; more preferably **L²** is selected from -CH₂-, -CH₂-CH₂- and -CH(R⁵)-; even more preferably **L²** is -CH₂-;
**R¹** is selected from naphthalen-2-yl, phenyl, biphenyl, 4-pyrimidin-5-yl-phenyl, tetraisoquinolinyl, 3-indolyl, 5-indolyl, 6-indolyl, 3-quinolinyl, 4-quinolinyl, 7-quinolinyl, dihydroquinolin-2-on-6-yl, 5,6,7,8-tetrahydronaphthalen-2-yl, (2H-1,2,3,4-tetrazol-5-yl)phenyl, benzyl, styryl, 5-benzothiophenyl, 6-benzothiophenyl, 3-benzothiophenyl, 2-benzothiophenyl, 6-(1,3-benzothiazolyl and 6-benzodioxinyl, wherein said naphthalen-2-yl, phenyl, biphenyl, 4-pyrimidin-5-yl-phenyl, tetraisoquinolinyl, 3-indolyl, 5-indolyl, 6-indolyl, 3-quinolinyl, 4-quinolinyl, 7-quinolinyl, dihydroquinolin-2-on-6-yl, 5,6,7,8-tetrahydronaphthalen-2-yl, (2H-1,2,3,4-tetrazol-5-yl)phenyl, benzyl, styryl, 5-benzothiophenyl, 6-benzothiophenyl, 3-benzothiophenyl, 2-benzothiophenyl, 6-(1,3-benzothiazolyl or 6-benzodioxinyl is optionally substituted by one or more substituents selected from C1-C4-alkyl, preferably methyl, halo, preferably bromo or chloro, more preferably bromo, C1-C4-haloalkyl, preferably C1-C4-fluroalkyl, more preferably trifluoomethyl, hydroxy-C1-C4-alkyl, preferably hydroxymethyl, acetylamino, acetylamino-C1-C4-alkyl, preferably acetylaminomethyl, and C1-C4-alkylaminocarboxyl, preferably methylaminocarboxyl;
**R²** is selected from phenyl, 1,4-imidazolyl, pyrimidinyl, pyridinyl, phenyl-C1-C2-alkyl, cyclohexyl, piperidinyl, morpholinyl, 1,3-benzodioxolyl, C1-C5-alkyl, *t*-butyloxy, phenylamino, wherein said phenyl, 1,4-imidazolyl, phenyl-C1-C2-alkyl,piperidinyl, is optionally substituted by one or more substituents independently selected from C1-C4-alkoxy, preferably methoxy, halogen, preferably fluoro and chloro, more preferably fluoro, C1-C4-haloalkyl, preferably trifluoromethyl, C1-C4-alkyl, preferably methyl, C1-C2-alkoxy-C1-C2-alkoxy, preferably methoxyethoxy, C1-C4-alkyloxycarbonyl, preferably t-butyloxycarbonyl, acetylamino, di(C1-C4-alkyl)amino-C1-C4-alkyl, preferably dimethylaminomethyl, and hydroxycarbamoyl.
**R⁵** is selected from CH₃, CH₂-OH, CH₂-Ph-OH and CH₂-Ph-OR⁶;
**R⁶** is Bn;
**R⁷** is selected from H, methyl, ethyl, isobutyl, isopentyl and (1-methylimidazol-2-yl)methyl; or
**R⁷** and **L²** form together with the nitrogen atom they are attached to a 5- or 6-membered heterocyclyl group selected from pyrrolidinyl, morpholinyl, thiazolidinyl and piperidinyl. In one embodiment, **Z** is -NHSO₂- and **L¹** is -CH₂-; **R¹** is aryl, preferably naphthalen-2-yl; **L²** is -CH₂-; **R²** is aryl, preferably phenyl, optionally substituted by one or more substituents independently selected from halogen, preferably fluoro, and C1-C4-alkyl, preferably methyl.

In one embodiment, **Z** is -CH₂-CH₂- and **L¹** is -CH₂-; **R¹** is aryl, preferably naphthalen-2-yl; **L²** is -CH₂-; **R²** is aryl, preferably phenyl.

In one embodiment, **Z** is -NH- and **L¹** is -CH₂-; **R¹** is aryl, preferably naphthalen-2-yl; **L²** is -CH₂-; **R²** is aryl, preferably phenyl, optionally substituted by one or more substituents independently selected from halogen, preferably fluoro.

In one embodiment, **Z** is -NH-CH(C1-C6-alkyl)-, preferably Z is -NH-CH(C4-C6-alkyl)-, more preferably **Z** is -NH-CH(C5-alkyl)-, and **L¹** is -CH₂-; **R¹** is aryl, preferably naphthalen-2-yl; **L²** is -CH₂-; **R²** is C1-C4-alkyloxycarbonyl, preferably t-butyloxycarbonyl.

In one embodiment, **Z** is -O-CH₂- and **L¹** is -CH₂-; **R¹** is aryl, preferably naphthalen-2-yl; **L²** is -CH₂-; **R²** is aryl, preferably phenyl.

In one embodiment, **Z** is -C(O)NH- and **L¹** is -CH₂-; **R¹** is aryl, preferably naphthalen-2-yl; **L²** is -CH₂- or -CH₂-CH₂-; **R²** is aryl, preferably phenyl, optionally substituted by one or more substituents independently selected from halogen, preferably fluoro.

In one embodiment, **Z** is -O- and **L¹** is -CH₂-; **R¹** is aryl, preferably naphthalen-2-yl; **L²** is -CH₂-; **R²** is aryl, preferably phenyl, optionally substituted by acetylamino.

In one embodiment, preferred compounds of Formula IIc are those of Formula IId: 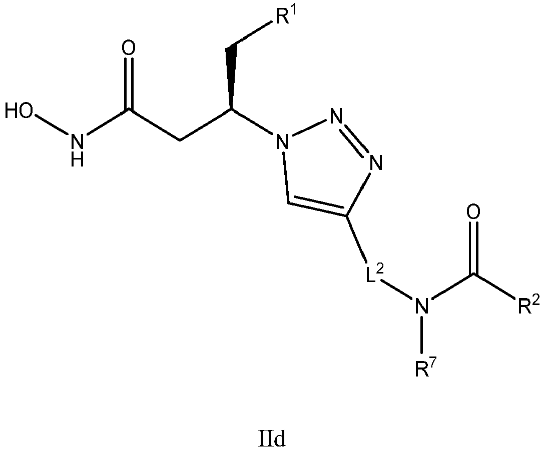 and pharmaceutically acceptable salts and solvates thereof,
wherein
**L², R¹, R², R⁵, R⁶** and **R⁷** are as defined above with respect to Formula I and any of its embodiments.

In one embodiment, the compounds of Formula II are those of Formula IIe: 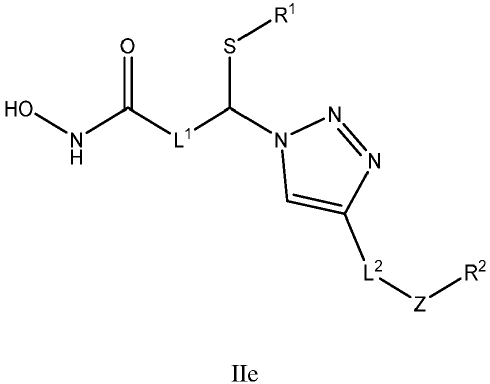 and pharmaceutically acceptable salts and solvates thereof,
wherein
**L¹, L², Z, R¹, R², R⁵, R⁶** and **R⁷** are as defined above with respect to Formula I and any of its embodiments.

In one embodiment, the compounds of Formula IIe are those wherein:
**L¹** is -CH₂-;
**R¹** is aryl optionally substituted by one or more substituents independently selected from C1-C4-alkyl, halogen and C1-C4-haloalkyl; preferably **R¹** is naphthalen-2-yl;
**L²** is -CH₂-;
Z is -NR⁷(CO)-;
**R⁷** is H; and
**R²** is aryl optionally substituted by one or more substituents independently selected from C1-C4-alkoxy, halogen, and C1-C4-haloalkyl; preferably **R²** is phenyl substituted by one or more substituents selected from halogen, preferably fluor.

In one embodiment, the compounds of Formula I are those of Formula III: 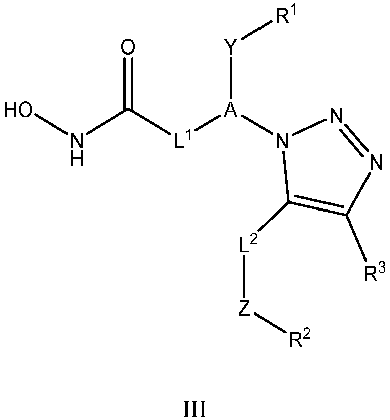 and pharmaceutically acceptable salts and solvates thereof,
wherein
**L¹, L², A, Y, Z, R¹, R², R³, R⁴, R⁵, R⁶** and **R⁷** are as defined above with respect to Formula I and any of its embodiments.

In one embodiment, preferred compounds of Formula III are those of Formula IIIa: 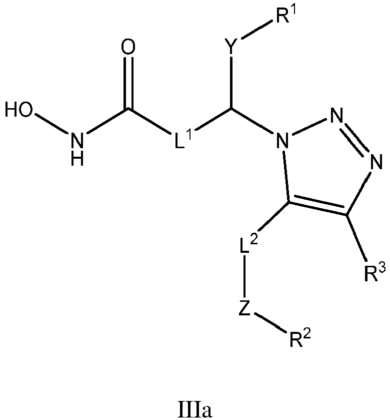 and pharmaceutically acceptable salts and solvates thereof,
wherein
**L¹, L², Y, Z, R¹, R², R³, R⁴, R⁵, R⁶** and **R⁷** are as defined above with respect to Formula I and any of its embodiments.

In one embodiment, preferred compounds of Formula III are those of Formula IIIb: 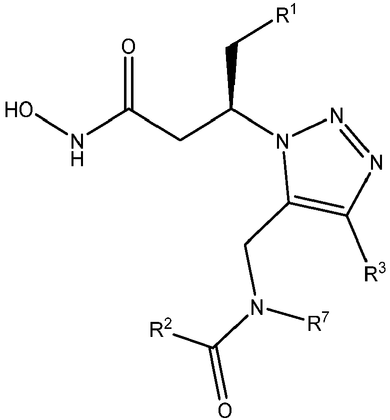 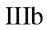 and pharmaceutically acceptable salts and solvates thereof,
wherein
R¹, R², R³, R⁴ and R⁷ are as defined above with respect to Formula I and any of its embodiments.

In one embodiment, the compounds of Formula IIIa are those wherein:
R¹ is aryl optionally substituted by one or more substituents independently selected from C1-C4-alkyl, halogen and C1-C4-haloalkyl; preferably R¹ is naphthalen-2-yl;
**R²** is selected from aryl, heteroaryl, arylalkyl, cycloalkyl, heterocycloalkyl and C1-C6-alkyl, wherein said aryl, arylalkyl, cycloalkyl or heterocycloalkyl is optionally substituted by one or more substituents independently selected from C1-C4-alkoxy, halogen, C1-C4-haloalkyl and C1-C4-alkyl; preferably **R²** is selected from aryl, arylalkyl, cycloalkyl and C1-C6-alkyl, wherein said aryl or arylalkyl is optionally substituted by one substituent selected from C1-C4-alkoxy and halogen; more preferably **R²** is selected from phenyl, cyclohexyl, phenylethyl, naphthalen-2-yl-CH(CH₃)- and methyl, wherein said phenyl or naphthalen-2-yl-CH(CH₃)- is optionally substituted by one substituent selected from C1-C4-alkoxy, preferably methoxy, and halogen, preferably fluor.
**R³** is selected from H and C(O)OR⁴; preferably **R³** is H;
**R⁴** is C1-C4-alkyl; preferably **R³** is ethyl;
**R⁷** is selected from H and C1-C6-alkyl; preferably **R⁷** is H.

In one embodiment, the compounds of Formula I are those of Formula IV: 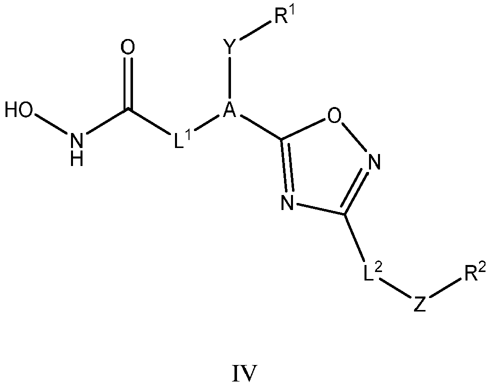 and pharmaceutically acceptable salts and solvates thereof,
wherein
**L¹, L², A, Y, Z, R¹, R², R⁵, R⁶** and **R⁷** are as defined above with respect to Formula I and any of its embodiments.

In one embodiment, preferred compounds of Formula IV are those of Formula IVa: 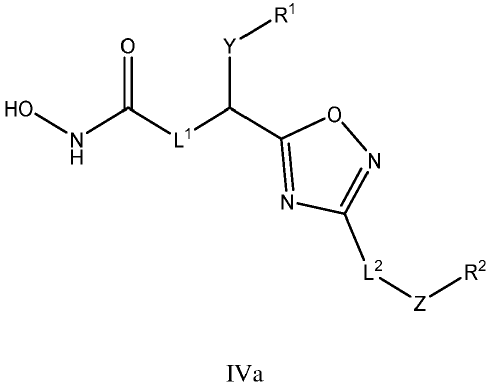 and pharmaceutically acceptable salts and solvates thereof,
wherein
**L¹, L², Y, Z, R¹, R², R⁵, R⁶** and **R⁷** are as defined above with respect to Formula I and any of its embodiments.

In one embodiment, the compounds of Formula IVa are those wherein:
**L¹** is -CH₂-;
**Y** is -CH₂-;
**L²** is -CH₂-;
**Z** is -NR⁷(CO)-;
**R⁷** is selected from H and C1-C6-alkyl; preferably **R⁷** is H;
**R¹** is aryl optionally substituted by one or more substituents independently selected from C1-C4-alkyl, halogen and C1-C4-haloalkyl; preferably **R¹** is naphthalen-2-yl;
**R²** is selected from aryl, heteroaryl, arylalkyl, cycloalkyl, heterocycloalkyl and C1-C6-alkyl, wherein said aryl, arylalkyl, cycloalkyl or heterocycloalkyl is optionally substituted by one or more substituents independently selected from C1-C4-alkoxy, halogen, C1-C4-haloalkyl and C1-C4-alkyl; preferably **R²** is aryl optionally substituted by one or more substituents independently selected from C1-C4-alkoxy, halogen, C1-C4-haloalkyl and C1-C4-alkyl; more preferably **R²** is aryl, optionally substituted by one substituent selected from halogen; still more preferably **R²** is phenyl, optionally substituted by one halogen, preferably fluor; even more preferably **R²** is phenyl substituted by one fluor.

In one embodiment, preferred compounds of Formula IV are those of Formula IVb: 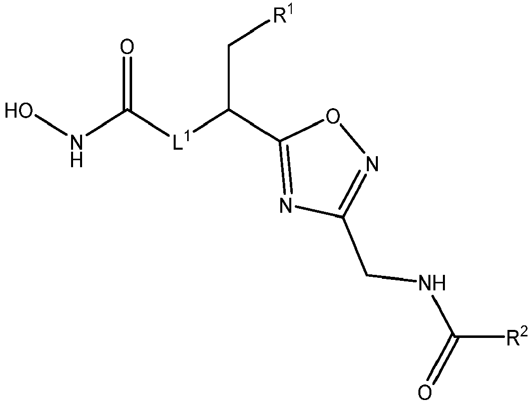 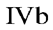 and pharmaceutically acceptable salts and solvates thereof,
wherein
**L¹, R¹** and **R²** are as defined above with respect to Formula I and any of its embodiments.

In one embodiment, the compounds of Formula IVb are those wherein:
**L¹** is inexistent or -CH₂-; preferably **L¹** is inexistent.
**R¹** is aryl optionally substituted by one or more substituents independently selected from C1-C4-alkyl, halogen and C1-C4-haloalkyl; preferably **R¹** is naphthalen-2-yl;
**R²** is selected from aryl, heteroaryl, arylalkyl, cycloalkyl, heterocycloalkyl and C1-C6-alkyl, wherein said aryl, arylalkyl, cycloalkyl or heterocycloalkyl is optionally substituted by one or more substituents independently selected from C1-C4-alkoxy, halogen, C1-C4-haloalkyl and C1-C4-alkyl; preferably **R²** is aryl optionally substituted by one or more substituents independently selected from C1-C4-alkoxy, halogen, C1-C4-haloalkyl and C1-C4-alkyl; more preferably **R²** is aryl, optionally substituted by one substituent selected from halogen; still more preferably **R²** is phenyl, optionally substituted by one halogen, preferably fluor; even more preferably **R²** is phenyl substituted by one fluor.

In one embodiment, the compounds of Formula I are those of Formula V: 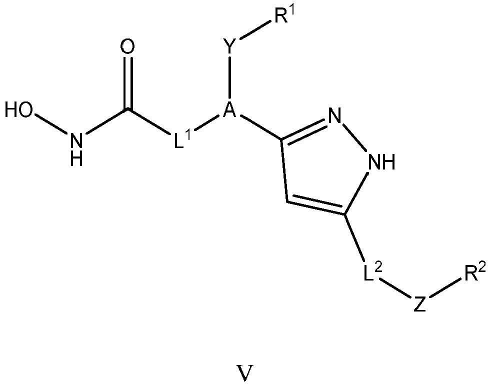 and pharmaceutically acceptable salts and solvates thereof,
wherein
**L¹, L², A, Y, Z, R¹, R², R⁵, R⁶** and **R⁷** are as defined above with respect to Formula I and any of its embodiments.

In one embodiment, preferred compounds of Formula V are those of Formula Va: 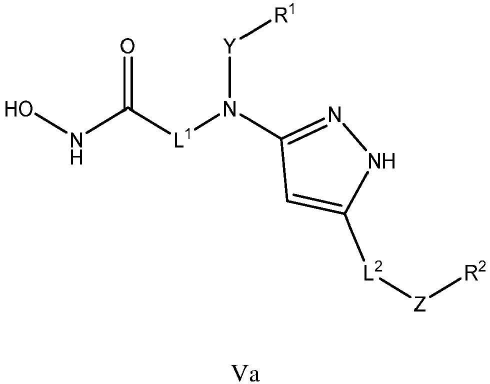 and pharmaceutically acceptable salts and solvates thereof,
wherein
**L¹, L², Y, Z, R¹, R², R⁵** and **R⁶** are as defined above with respect to Formula I and any of its embodiments.

In one embodiment, the compounds of Formula Va are those wherein:
**Z** is -NR⁶(CO)-;
**R⁶** is selected from H and C1-C6-alkyl; preferably **R⁶** is H;
**R¹** is aryl optionally substituted by one or more substituents independently selected from C1-C4-alkyl, halogen and C1-C4-haloalkyl; preferably **R¹** is naphthyl;
**R²** is selected from aryl, heteroaryl, arylalkyl, cycloalkyl, heterocycloalkyl and C1-C6-alkyl, wherein said aryl, arylalkyl, cycloalkyl or heterocycloalkyl is optionally substituted by one or more substituents independently selected from C1-C4-alkoxy, halogen, C1-C4-haloalkyl and C1-C4-alkyl; preferably **R²** is aryl optionally substituted by one or more substituents independently selected from C1-C4-alkoxy, halogen, C1-C4-haloalkyl and C1-C4-alkyl; more preferably **R²** is aryl, optionally substituted by one substituent selected from halogen; still more preferably **R²** is phenyl, optionally substituted by one halogen, preferably fluor; even more preferably **R²** is phenyl substituted by one fluor.

In one embodiment, preferred compounds of Formula V are those of Formula Vb: 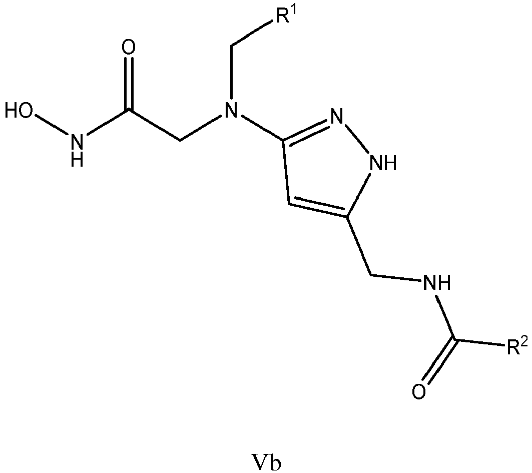 and pharmaceutically acceptable salts and solvates thereof,
wherein
**R¹** and **R²** are as defined above with respect to Formula I and any of its embodiments.

In one embodiment, the compounds of Formula Vb are those wherein:
**R¹** is aryl optionally substituted by one or more substituents independently selected from C1-C4-alkyl, halogen and C1-C4-haloalkyl; preferably **R¹** is naphthyl;
**R²** is selected from aryl, heteroaryl, arylalkyl, cycloalkyl, heterocycloalkyl and C1-C6-alkyl, wherein said aryl, arylalkyl, cycloalkyl or heterocycloalkyl is optionally substituted by one or more substituents independently selected from C1-C4-alkoxy, halogen, C1-C4-haloalkyl and C1-C4-alkyl; preferably **R²** is aryl optionally substituted by one or more substituents independently selected from C1-C4-alkoxy, halogen, C1-C4-haloalkyl and C1-C4-alkyl; more preferably **R²** is aryl, optionally substituted by one substituent selected from halogen; still more preferably **R²** is phenyl, optionally substituted by one halogen, preferably fluor; even more preferably **R²** is phenyl substituted by one fluor.

Particularly preferred compounds of the invention are those listed in Table 1 hereafter:

**Table 1**

| Compound | Structure | Name |
|---|---|---|
| 1 | 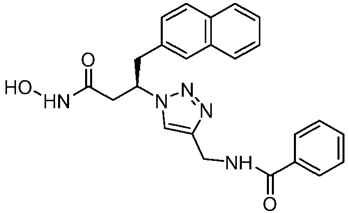 | N-[1-((R)-2-Hydroxycarbamoyl-1-naphthalen-2-ylmethyl-ethyl)-1H-[1,2,3]triazol-4-ylmethyl]-benzamide |
| 2 | 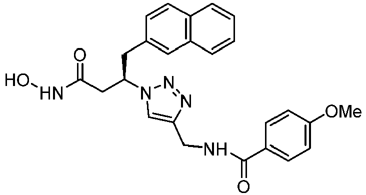 | N-[1-((R)-2-Hydroxycarbamoyl-1-naphthalen-2-ylmethyl-ethyl)-1H-[1,2,3]triazol-4-ylmethyl]-4-methoxybenzamide |
| 3 | 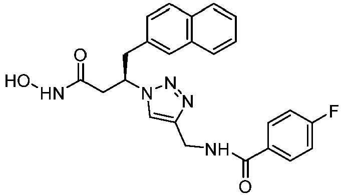 | 4-Fluoro-N-[1-(R-2-hydroxycarbamoyl-1-naphthalen-2-ylmethyl-ethyl)-1H-[1,2,3]triazol-4-ylmethyl]-benzamide |
| 4 | 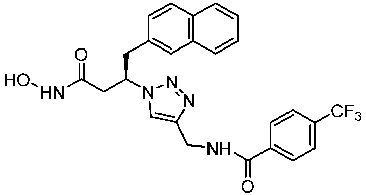 | N-[1-(R-2-Hydroxycarbamoyl-1-naphthalen-2-ylmethyl-ethyl)-1H-[1,2,3]triazol-4-ylmethyl] -4-trifluoromethyl-benzamide |
| 5 | 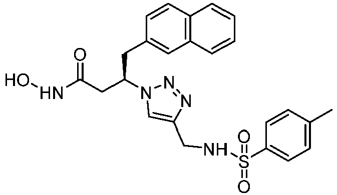 | (R)-N-Hydroxy-4-naphthalen-2-yl-3- { 4-[(toluene-4-sulfonylamino)-methyl]-[1,2,3]triazol-1-yl}-butyramide |
| 6 | 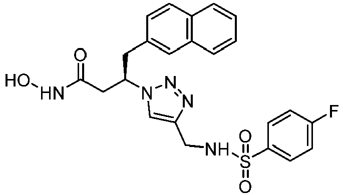 | (R)-3-{4-[(4-Fluorobenzenesulfonylamino)-methyl]-[1,2,3]triazol-1-yl}-N-hydroxy-4-naphthalen-2-yl-butyramide |
| 7 | 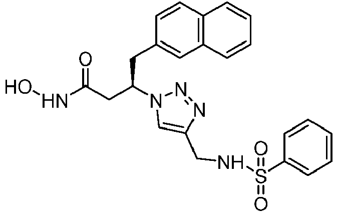 | (R)-3-[4-(Benzenesulfonylaminomethyl)-[1,2,3]triazol-1-yl] -N-hydroxy-4-naphthalen-2-yl-butyramide |
| 8 | 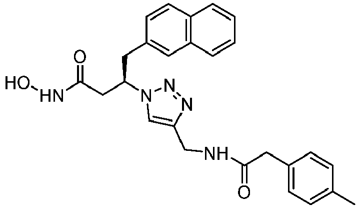 | (R)-N-Hydroxy-4-naphthalen-2-yl-3- { 4-[(2-p-tolyl-acetylamino)-methyl]-[1,2,3]triazol-1-yl}-butyramide |
| 9 | 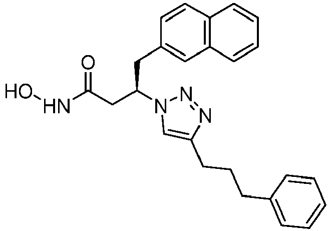 | (R)-3-{4-[(4-Fluoro-benzenesulfonylamino)-methyl]-[1,2,3]triazol-1-yl}-N-hydroxy-4-naphthalen-2-yl-butyramide |
| 10 | 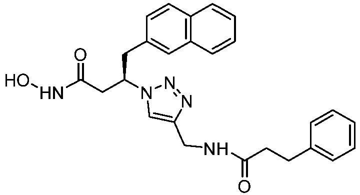 | (R)-N-Hydroxy-4-naphthalen-2-yl-3- { 4-[(3-phenyl-propionylamino)-methyl]-[1,2,3]triazol-1-yl}-butyramide |
| 11 | 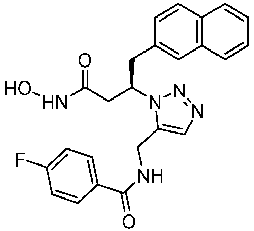 | (R)-4-Fluoro-N-[3-(1-hydroxycarbamoylmethyl-2-naphthalen-2-yl-ethyl)-3H-[1,2,3]triazol-4-ylmethyl]-benzamide |
| 12 | 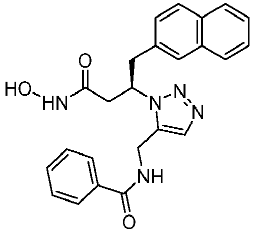 | (R)-N-[3-(1-Hydroxycarbamoylmethyl-2-naphthalen-2-yl-ethyl)-3H-[1,2,3]triazol-4- ylmethyl] -benzamide |
| 13 | 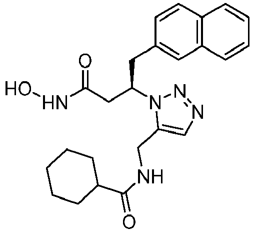 | (R)-Cyclohexanecarboxylic acid [3-(1-hydroxycarbamoylmethyl-2-naphthalen-2-yl-ethyl)-3H-[1,2,3]triazol-4-ylmethyl]-amide |
| 14 | 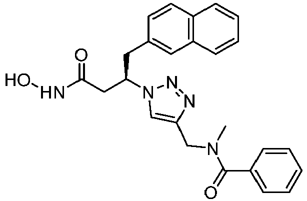 | (R)-N-[1-(2-hydroxycarbamoyl-1-naphthalen-2-ylmethyl-ethyl)-1H-[1,2,3]triazol-4-ylmethyl]-N-methyl-benzamide |
| 15 | 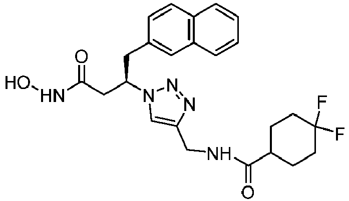 | (R)-4,4-difluoro-cyclohexanecarboxylic acid [1-(2-hydroxycarbamoyl-1-naphthalen-2-ylmethyl-ethyl)-1H-[1,2,3]triazol-4-ylmethyl]-amide |
| 16 | 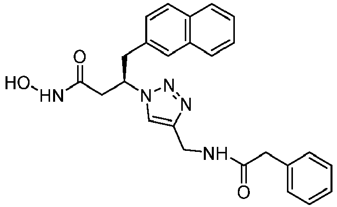 | (R)-N-hydroxy-4-naphthalen-2-yl-3-[4-(phenylacetylamino-methyl)-[1,2,3]triazol-1-yl]-butyramide |
| 17 | 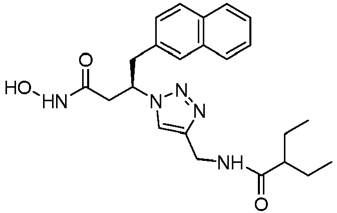 | (R)-3-{4-[(2-ethyl-butyrylamino)-methyl]-[1,2,3]triazol-1-yl}-N-hydroxy-4-naphthalen-2-yl-butyramide |
| 18 | 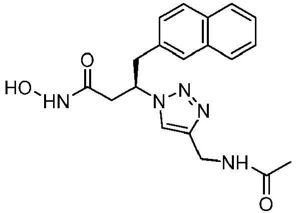 | (R)-3-[4-(acetylamino-methyl)-[1,2,3]triazol-1-yl]-N-hydroxy-4-naphthalen-2-yl-butyramide |
| 19 | 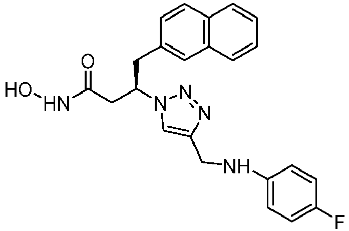 | (R)-3- {4- [(4-fluoro-phenylamino)-methyl]-[1,2,3]triazol-1-yl}-N-hydroxy-4-naphthalen-2-yl-butyramide |
| 20 | 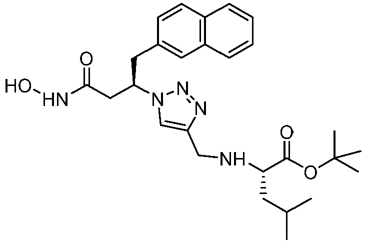 | tert-butyl ((1-((R)-4-(hydroxyamino)-1-(naphthalen-2-yl)-4-oxobutan-2-yl)-1H-1,2,3-triazol-4-yl)methyl)-L-leucinate |
| 21 | 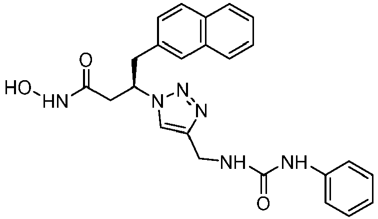 | (R)-N-hydroxy-4-naphthalen-2-yl-3- {4-[(3-phenyl-ureido)-methyl] - [1,2,3]triazol-1-yl}-butyramide |
| 22 | 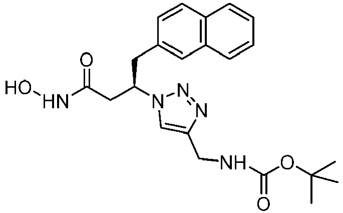 | (R)-[1-(2-hydroxycarbamoyl-1-naphthalen-2-ylmethyl-ethyl)-1H-[1,2,3]triazol-4-ylmethyl]-carbamic acid tert-butyl ester |
| 23 | 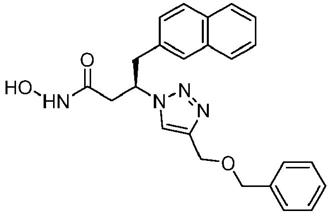 | (R)-3-(4-benzyloxymethyl-[1,2,3]triazol-1-yl)-N-hydroxy-4-naphthalen-2-yl-butyramide |
| 24 | 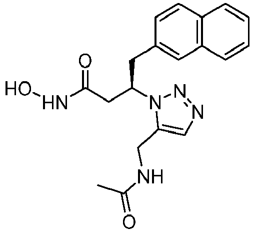 | (R)-3-[5-(Acetylamino-methyl)-[1,2,3]triazol-1-yl]-N-hydroxy-4-naphthalen-2-yl-butyramide |
| 25 | 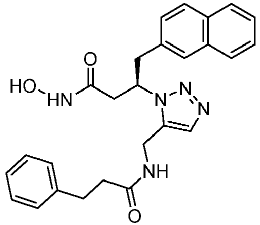 | (R)-N-hydroxy-4-naphthalen-2-yl-3-{ 5-[(3-phenyl-propionylamino)-methyl]-[1,2,3]triazol-1-yl}-butyramide |
| 26 | 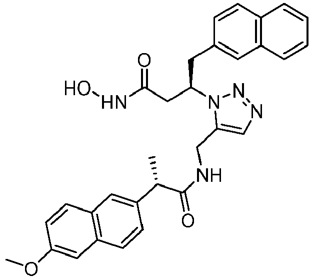 | (R,S)-N-hydroxy-3-(5-{ [2-(6-methoxy-naphthalen-2-yl)-propionylamino]-methyl}-[1,2,3]triazol-1-yl)-4-naphthalen-2-yl-butyramide |
| 27 | 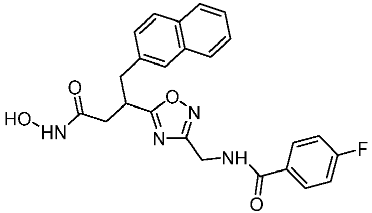 | 4-fluoro-N-[5-(1-hydroxycarbamoylmethyl-2-naphthalen-2-yl-ethyl)-[1,2,4]oxadiazol-3-ylmethyl]-benzamide |
| 28 | 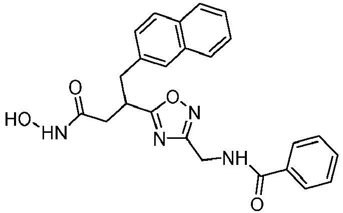 | N-[5-(1-hydroxycarbamoylmethyl-2-naphthalen-2-yl-ethyl)-[1,2,4]oxadiazol-3-ylmethyl]-benzamide |
| 29 | 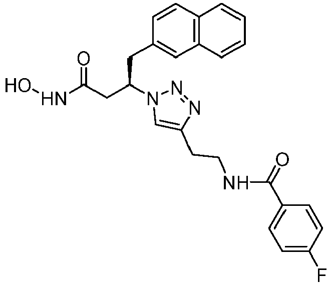 | (R)-4-Fluoro-N-{2-[1-(1-hydroxycarbamoyl-2-naphthalen-2-yl-ethyl)-1H-[1,2,3]triazol-4-yl]-ethyl}-benzamide |
| 30 | 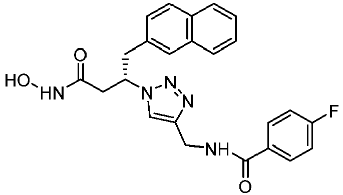 | (S)-4-fluoro-N-[1-(2-hydroxycarbamoyl-1-naphthalen-2-ylmethyl-ethyl)-1H-[1,2,3]triazol-4-ylmethyl]-benzamide |
| 31 | 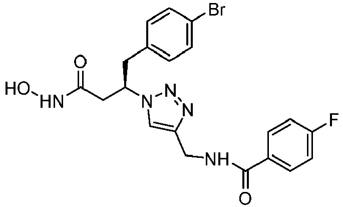 | (R)-N-{1-[1-(4-bromo-benzyl)-2-hydroxycarbamoyl-ethyl]-1H-[1,2,3]triazol-4-ylmethyl}-4-fluoro-benzamide |
| 32 | 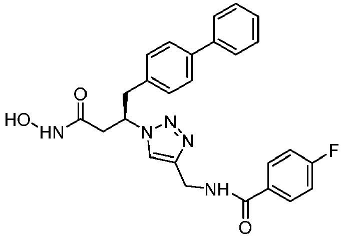 | (R)-N-[1-(1-biphenyl-4-ylmethyl-2-hydroxycarbamoyl-ethyl)-1H-[1,2,3]triazol-4-ylmethyl]-4-fluoro-benzamide |
| 33 | 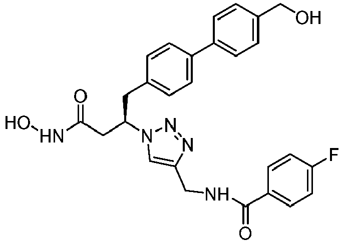 | (R)-4-fluoro-N-{1-[2-hydroxycarbamoyl-1-(4'-hydroxymethyl-biphenyl-4-ylmethyl)-ethyl]-1H-[1,2,3]triazol-4-ylmethyl}-benzamide |
| 34 | 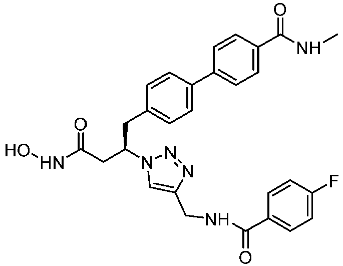 | (R)-4'-(2-{4-[(4-fluoro-benzoylamino)-methyl]-[1,2,3]triazol-1-yl}-3-hydroxycarbamoyl-propyl)-biphenyl-4-carboxylic acid methylamide |
| 35 | 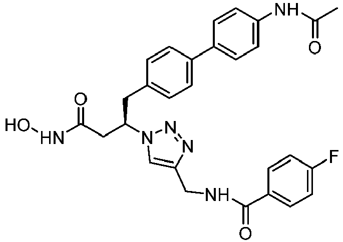 | (R)-N-{ 1-[1-(4'-acetylamino-biphenyl-4-ylmethyl)-2-hydroxycarbamoyl-ethyl]-1H-[1,2,3]triazol-4-ylmethyl}-4-fluoro-benzamide |
| 36 | 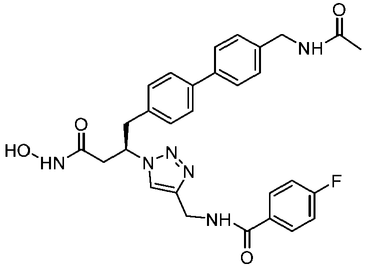 | (R)-N-(1-{1-[4'-(acetylamino-methyl)-biphenyl-4-ylmethyl] -2-hydroxycarbamoyl-ethyl}-1H-[1,2,3]triazol-4-ylmethyl)-4-fluoro-benzamide |
| 37 | 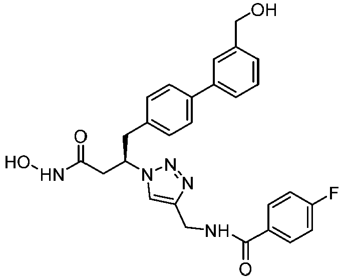 | (R)-4-fluoro-N-{1-[2-hydroxycarbamoyl-1-(3'-hydroxymethyl-biphenyl-4-ylmethyl)-ethyl]-1H-[1,2,3]triazol-4-ylmethyl}-benzamide |
| 38 | 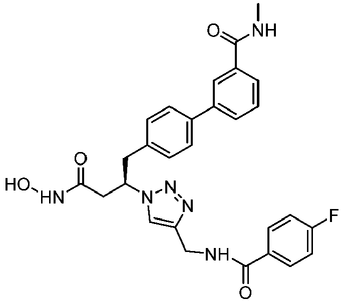 | (R)-4'-(2- {4- [(4-fluoro-benzoylamino)-methyl]-[1,2,3]triazol-1-yl}-3-hydroxycarbamoyl-propyl)-biphenyl-3-carboxylic acid methylamide |
| 39 | 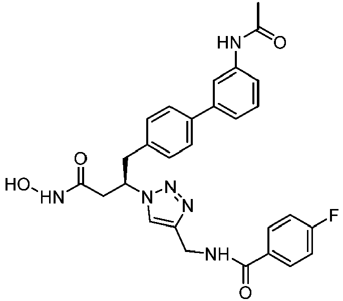 | (R)-N-{ 1-[1-(3'-acetylamino-biphenyl-4-ylmethyl)-2-hydroxycarbamoyl-ethyl]-1H-[1,2,3]triazol-4-ylmethyl}-4-fluoro-benzamide |
| 40 | 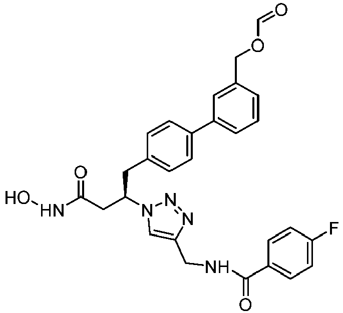 | (R)-Formic acid 4'-(2-{4-[(4-fluoro-benzoylamino)-methyl]-[1,2,3]triazol-1-yl}-3-hydroxycarbamoyl-propyl)-biphenyl-3-ylmethyl ester |
| 41 | 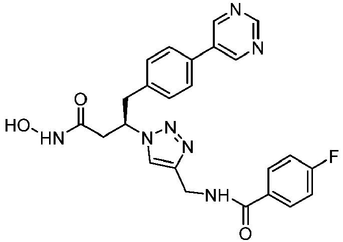 | (R)-4-fluoro-N-{1-[2-hydroxycarbamoyl-1-(4-pyrimidin-5-yl-benzyl)-ethyl]-1H-[1,2,3]triazol-4-ylmethyl}-benzamide |
| 42 | 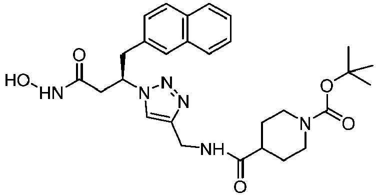 | 4-{[1-(2-Hydroxycarbamoyl-1-naphthalen-2-ylmethyl-ethyl)-1H-[1,2,3]triazol-4-ylmethyl]-carbamoyl}-piperidine-1-carboxylic acid *tert*-butyl ester |
| 43 | 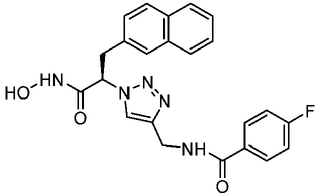 | (R)-4-Fluoro-N-[1-(1-hydroxycarbamoyl-2-naphthalen-2-yl-ethyl)-1H-[1,2,3]triazol-4-ylmethyl]-benzamide |
| 44 | 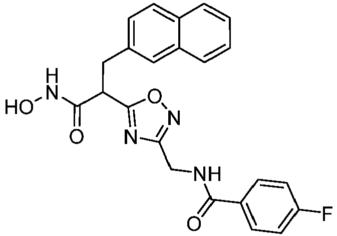 | 4-Fluoro-N-[5-(1-hydroxycarbamoyl-2-naphthalen-2-yl-ethyl)-[1,2,4] oxadiazol-3-ylmethyl]-benzamide |
| 45 | 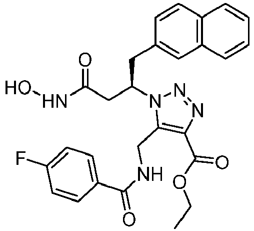 | Ethyl 5-[[(4-iluorobenzoyl)amino]methyl]-3-[(1R)-3-(hydroxyamino)-1-(2-naphthylmethyl)-3-oxo-propyl]triazole-4-carboxylate |
| 46 | 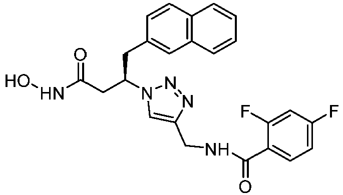 | 2,4-Difluoro-N-[1-(2-hydroxycarbamoyl-1-naphthalen-2-ylmethyl-ethyl)-1H-[1,2,3]triazol-4-ylmethyl]-benzamide |
| 47 | 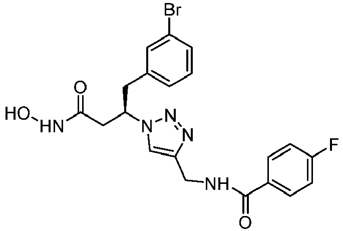 | (R)-N-{1-[1-(3-bromo-benzyl)-2-hydroxycarbamoyl-ethyl] -1H-[1,2,3]triazol-4-ylmethyl}-4-fluoro-benzamide |
| 48 | 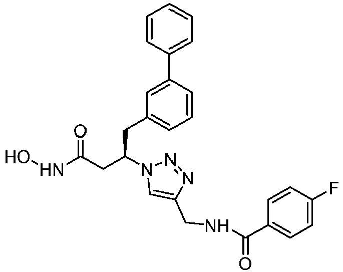 | (R)-N-[1-(1-biphenyl-3-ylmethyl-2-hydroxycarbamoyl-ethyl)-1H-[1,2,3]triazol-4-ylmethyl]-4-fluoro-benzamide |
| 49 | 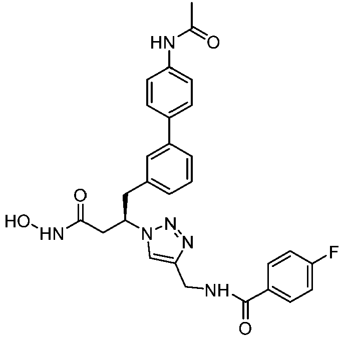 | (R)-N-{1-[1-(4'-acetylamino-biphenyl-3 - ylmethyl)-2-hydroxycarbamoyl-ethyl]-1H-[1,2,3]triazol-4-ylmethyl}-4-fluoro-benzamide |
| 50 | 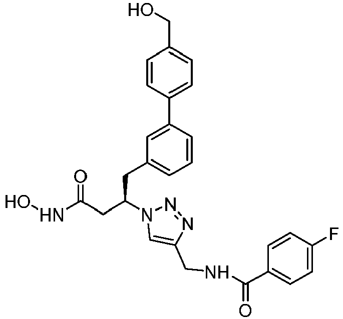 | (R)-4-fluoro-N-{1-[2-hydroxycarbamoyl-1-(4'-hydroxymethyl-biphenyl-3-ylmethyl)-ethyl]-1H-[1,2,3]triazol-4-ylmethyl}-benzamide |
| 51 | 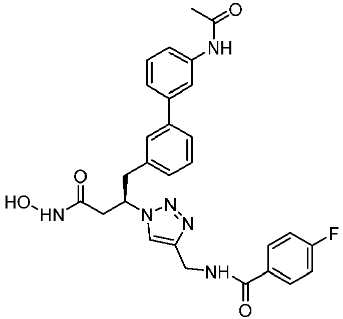 | (R)-N-{1-[1-(3'-acetylamino-biphenyl-3-ylmethyl)-2-hydroxycarbamoyl-ethyl]-1H-[1,2,3]triazol-4-ylmethyl}-4-fluoro-benzamide |
| 52 | 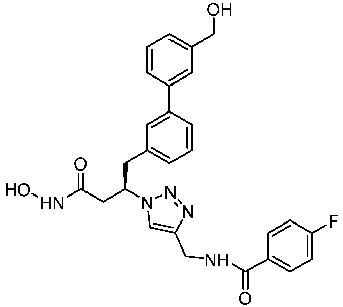 | (R)-4-fluoro-N-{1-[2-hydroxycarbamoyl-1-(3'-hydroxymethyl-biphenyl-3-ylmethyl)-ethyl]-1H-[1,2,3]triazol-4-ylmethyl}-benzamide |
| 53 | 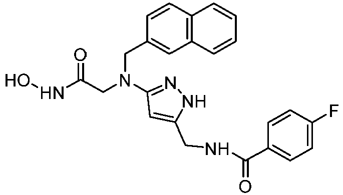 | 4-fluoro-N-((3-((2-(hydroxyamino)-2-oxoethyl)(naphthalen-2-ylmethyl)amino)-1H-pyrazol-5-yl)methyl)benzamide |
| 54 | 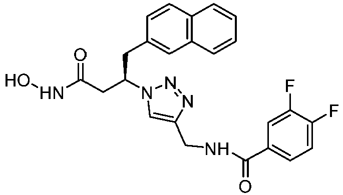 | 3,4-difluoro-N-[[1-[(1R)-3-(hydroxyamino)-1-(2-naphthylmethyl)-3-oxo-propyl]triazol-4-yl]methyl]benzamide |
| 55 | 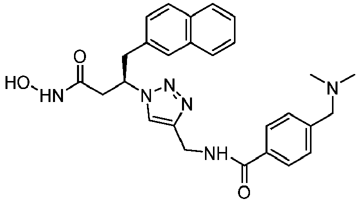 | 4-[(dimethylamino)methyl]-N-[[1-[(1R)-3-(hydroxyamino)-1-(2-naphthylmethyl)-3-oxo-propyl]triazol-4-yl]methyl]benzamide |
| 56 | 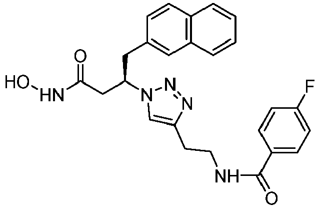 | 4-fluoro-N-[2-[1-[(1R)-3-(hydroxyamino)-1-(2-naphthylmethyl)-3-oxo-propyl]triazol-4-yl]ethyl]benzamide) |
| 57 | 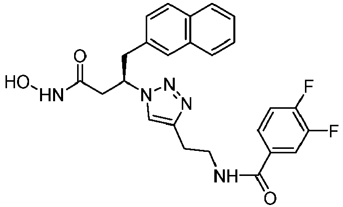 | 3,4-difluoro-N-[2-[1-[(1R)-3-(hydroxyamino)-1-(2-naphthylmethyl)-3-oxo-propyl]triazol-4-yl]ethyl]benzamide |
| 58 | 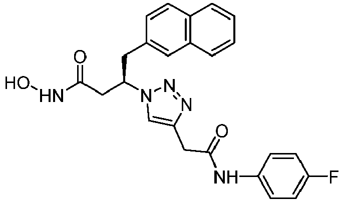 | 3-{4-[(4-Fluoro-phenylcarbamoyl)-methyl]-[1,2,3]triazol-1-yl} -N-hydroxy-4-naphthalen-2-yl-butyramide |
| 59 | 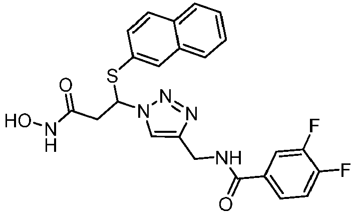 | 3 ,4-difluoro-N-((1-(3-(hydroxyamino)-1-(naphthalen-2-ylthio)-3-oxopropyl)-1H-1,2,3-triazol-4-yl)methyl)benzamide |
| 60 | 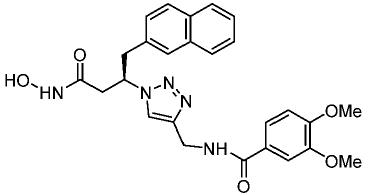 | N-[[1-[(1R)-3-(hydroxyamino)-1-(2-naphthylmethyl)-3-oxo-propyl]triazol-4-yl]methyl]-3,4-dimethoxy-benzamide |
| 61 | 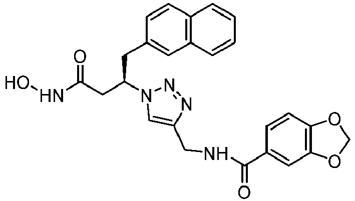 | N-[[1-[(1R)-3-(hydroxyamino)-1-(2-naphthylmethyl)-3-oxo-propyl]triazol-4-yl]methyl]-1,3-benzodioxole-5-carboxamide |
| 62 | 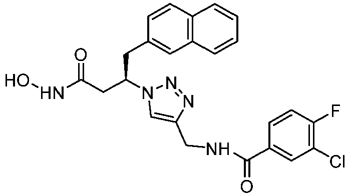 | 3-chloro-4-fluoro-N-[[1-[(1R)-3-(hydroxyamino)-1-(2-naphthylmethyl)-3-oxo-propyl]triazol-4-yl]methyl]benzamide |
| 63 | 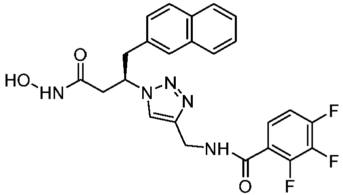 | 2,3,4-Trifluoro-N-[1-(1-hydroxycarbamoylmethyl-2-naphthalen-2-yl-ethyl)-1H-[1,2,3]triazol-4-ylmethyl]-benzamide |
| 64 | 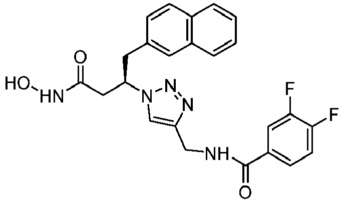 | methyl(3R)-3-[4-[[(3,4-difluorobenzoyl)amino]methyl]triazol-1- yl]-4-(1-naphthyl)butanoate |
| 65 | 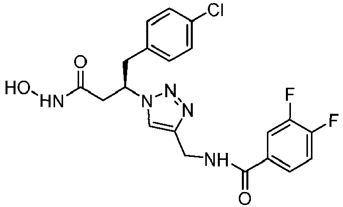 | N-[[1-[(1R)-1-[(4-chlorophenyl)methyl]-3-(hydroxyamino)-3-oxo-propyl]triazol-4-yl]methyl]-3,4-difluoro-benzamide |
| 66 | 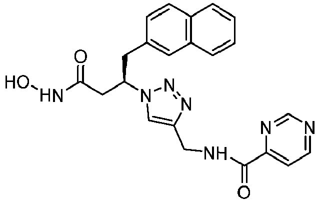 | N-[[1-[(1R)-3-(hydroxyamino)-1-(2-naphthylmethyl)-3-oxo-propyl]triazol-4-yl]methyl]pyrimidine-4-carboxamide |
| 67 | 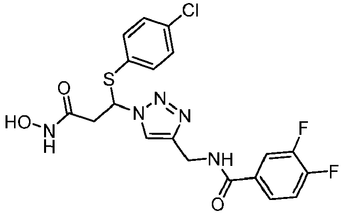 | N-[[1-[1-(4-chlorophenyl)sulfanyl-3-(hydroxyamino)-3-oxo-propyl]triazol-4-yl] methyl] - 3 ,4-difluoro-benzamide |
| 68 | 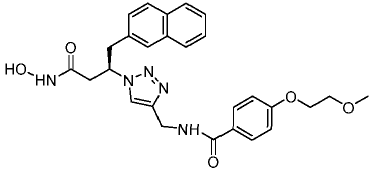 | N-[[1-[(1R)-3-(hydroxyamino)-1-(2-naphthylmethyl)-3-oxo-propyl]triazol-4-yl]methyl]-4-(2-methoxyethoxy)benzamide |
| 69 | 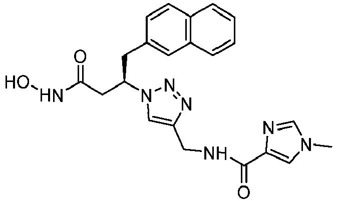 | N-[[1-[(1R)-3-(hydroxyamino)-1-(2-naphthylmethyl)- 3-oxo-propyl]triazol-4-yl] methyl] -1 -methyl-imidazole-4-carboxamide |
| 70 | 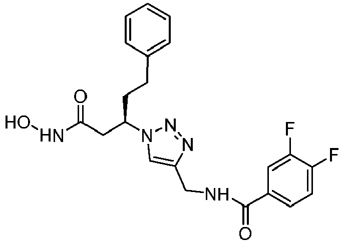 | 3,4-difluoro-N-[[1-[(1R)-3-(hydroxyamino)-3-oxo-1-(2-phenylethyl)propyl]triazol-4-yl]methyl]benzamide |
| 71 | 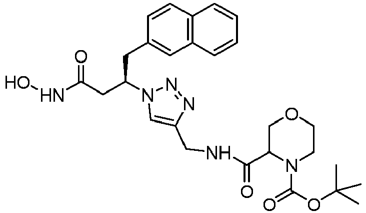 | tert-butyl3-[[1-[(1R)-3-(hydroxyamino)-1-(2-naphthylmethyl)-3-oxo-propyl]triazol-4-yl] methylcarbamoyl] morpholine-4-carboxylate |
| 72 | 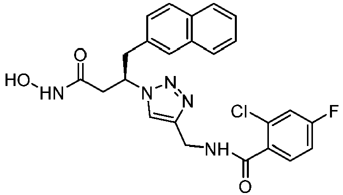 | 2-chloro-4-fluoro-N-[[1-[(1R)-3-(hydroxyamino)-1-(2-naphthylmethyl)-3-oxo-propyl]triazol-4-yl]methyl]benzamide |
| 73 | 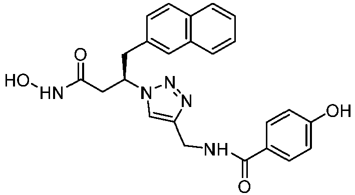 | 4-hydroxy-N-[[1-[(1R)-3-(hydroxyamino)-1-(2-naphthylmethyl)-3-oxo-propyl]triazol-4-yl]methyl]benzamide |
| 74 | 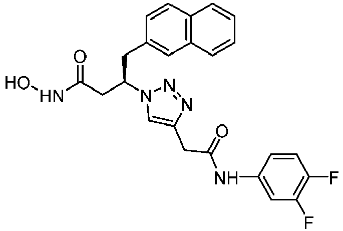 | 3-{ 4-[(3,4-Difluoro-phenylcarbamoyl)-methyl]-[1,2,3]triazol-1-yl}-N-hydroxy-4-naphthalen-2-yl-butyramide |
| 75 | 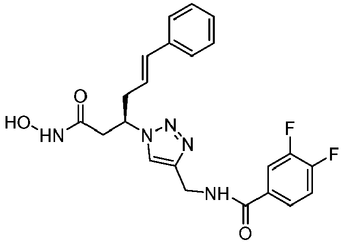 | 3,4-difluoro-N-[[1-[(E,1R)-1-[2-(hydroxyamino)-2-oxo-ethyl]-4-phenyl-but-3 -enyl] triazol-4-yl]methyl]benzamide |
| 76 | 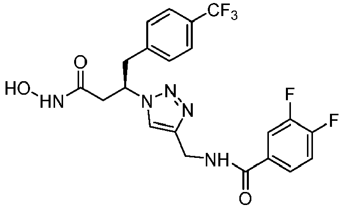 | 3,4-difluoro-N-[[1-[(1R)-3-(hydroxyamino)-3-oxo-1-[[4-(trifluoromethyl)phenyl]methyl]propyl]tr iazol-4-yl]methyl] benzamide |
| 77 | 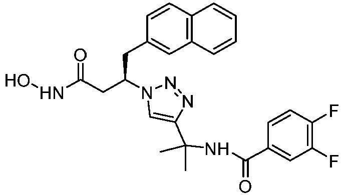 | 3,4-Difluoro-N-{1-[1-(1-hydroxycarbamoylmethyl-2-naphthalen-2-yl-ethyl)-1H-[1,2,3]triazol-4-yl]-1-methyl-ethyl} -benzamide |
| 78 | 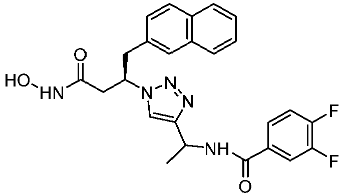 | 3,4-difluoro-N-[1-[1-[3-(hydroxyamino)-1-(2-naphthylmethyl)-3-oxo-propyl]triazol-4-yl]ethyl]benzamide |
| 79 | 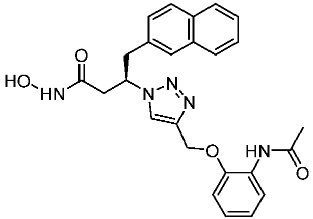 | 3-[4-(2-Acetylamino-phenoxymethyl)-[1,2,3]triazol-1-yl]-N-hydroxy-4-naphthalen-2-yl-butyramide |
| 80 | 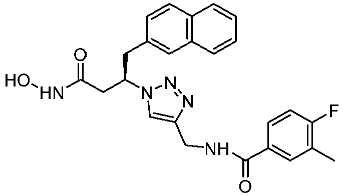 | 4-fluoro-N-[[1-[(1R)-3-(hydroxyamino)-1-(2-naphthylmethyl)-3-oxo-propyl]triazol-4-yl]methyl]-3-methyl-benzamide |
| 81 | 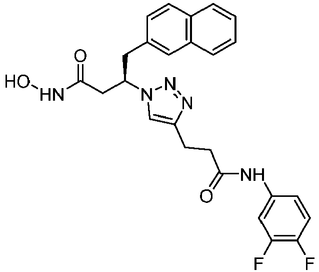 | N-(3,4-difluorophenyl)-3-[1-[3-(hydroxyamino)-1-(2-naphthylmethyl)-3-oxo-propyl] triazol-4-yl]propanamide |
| 82 | 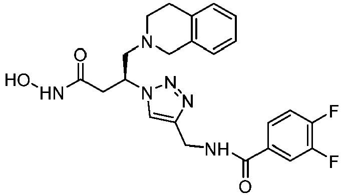 | N-[[1-[(1S)-1-(3,4-dihydro-1H-isoquinolin-2-ylmethyl)-3-(hydroxyamino)-3-oxo-propyl]triazol-4-yl] methyl] - 3 ,4-difluoro-benzamide |
| 83 | 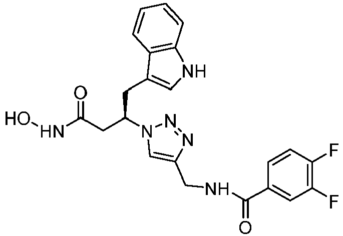 | 3,4-difluoro-N-[[1-[(1R)-3-(hydroxyamino)-1-(1H-indol-3-ylmethyl)-3-oxo-propyl]triazol-4-yl]methyl]benzamide |
| 84 | 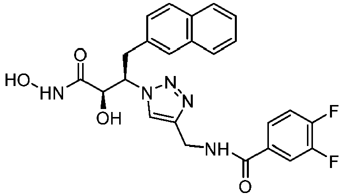 | 3,4-difluoro-N-[[1-[(1R,2S)-2-hydroxy-3-(hydroxyamino)-1-(2-naphthylmethyl)-3-oxo-propyl]triazol-4-yl]methyl]benzamide |
| 85 | 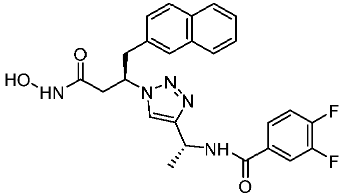 | 3,4-Difluoro-N-{1-[1-(1(R)-hydroxycarbamoylmethyl-2-naphthalen-2-yl-ethyl)-1H-[1,2,3]triazol-4-yl]-(S)-ethyl}-benzamide |
| 86 | 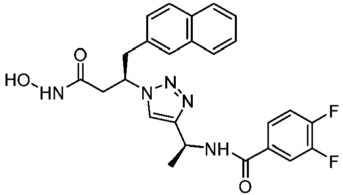 | 3,4-Difluoro-N-{1-[1-(1(R)-hydroxycarbamoylmethyl-2-naphthalen-2-yl-ethyl)-1H-[1,2,3]triazol-4-yl]-(R)-ethyl}-benzamide |
| 87 | 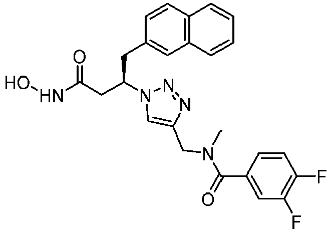 | Methyl (3R)-3-[4-[[(3,4-difluorobenzoyl)-methyl-amino]methyl]triazol-1-yl]-4-(2-naphthyl)butanoate |
| 88 | 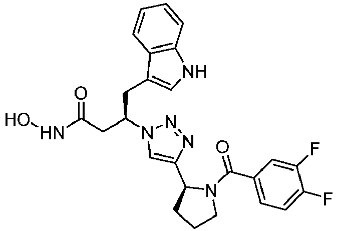 | (3R)-3-[4-[(2S)-1-(3,4-difluorobenzoyl)pyrrolidin-2-yl]triazol-1-yl]-4-(1H-indol-3-yl)butanehydroxamic acid |
| 89 | 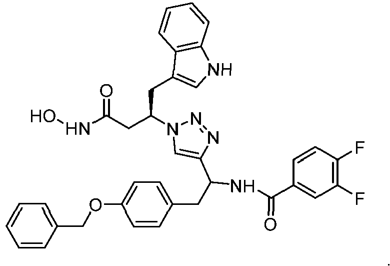 | N-[2-(4-benzyloxyphenyl)-1-[1-[(1R)-3-(hydroxyamino)-1-(1H-indol-3-ylmethyl)-3-oxo-propyl]triazol-4-yl] ethyl] - 3 ,4-difluoro-benzamide |
| 90 | 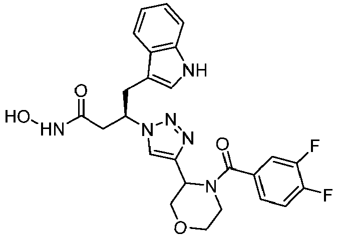 | (3R)-3-[4-[4-(3,4-difluorobenzoyl)morpholin-3-yl]triazol-1-yl]-4-(1H-indol-3-yl)butanehydroxamic acid |
| 91 | 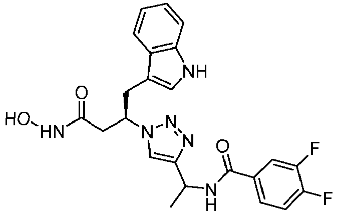 | 3,4-difluoro-N-[1-[1-[(1R)-3-(hydroxyamino)-1-(1H-indol-3-ylmethyl)-3-oxo-propyl]triazol-4-yl]ethyl]benzamide |
| 92 | 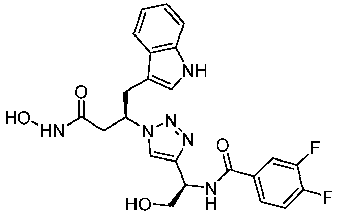 | 3,4-difluoro-N-[(1R)-2-hydroxy-1-[1-[(1R)-3-(hydroxyamino)-1-(1H-indol-3-ylmethyl)-3-oxo-propyl]triazol-4-yl]ethyl]benzamide |
| 93 | 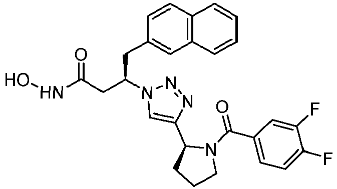 | (3R)-3-[4-[(2S)-1-(3,4-difluorobenzoyl)pyrrolidin-2-yl]triazol-1-yl]-4-(2-naphthyl)butanehydroxamic acid |
| 94 | 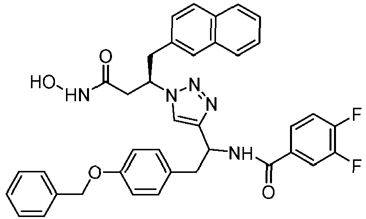 | N-[2-(4-benzyloxyphenyl)-1-[1-[(1R)-3-(hydroxyamino)-1-(2-naphthylmethyl)-3-oxo-propyl]triazol-4-yl]ethyl]-3,4-difluoro-benzamide |
| 95 | 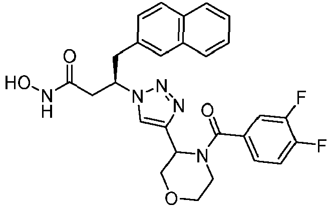 | (3R)-3-[4-[4-(3,4-difluorobenzoyl)morpholin-3-yl]triazol-1-yl]-4-(2-naphthyl)butanehydroxamic acid |
| 96 | 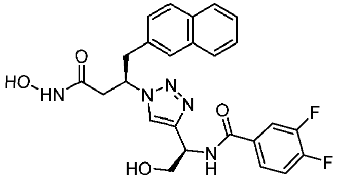 | 3,4-difluoro-N-[(1R)-2-hydroxy-1-[1-[(1R)-3-(hydroxyamino)-1-(2-naphthylmethyl)-3-oxo-propyl]triazol-4-yl]ethyl]benzamide |
| 97 | 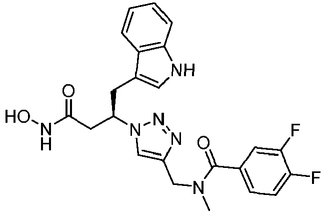 | 3,4-difluoro-N-[[1-[(1R)-3-(hydroxyamino)-1-(1H-indol-3-ylmethyl)-3-oxo-propyl]triazol-4-yl] methyl] -N-methyl-benzamide |
| 98 | 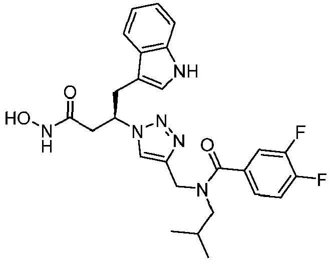 | 3,4-difluoro-N-[[1-[(1R)-3-(hydroxyamino)-1-(1H-indol-3-ylmethyl)- 3-oxo-propyl]triazol-4-yl] methyl] -N-isobutyl-benzamide |
| 99 | 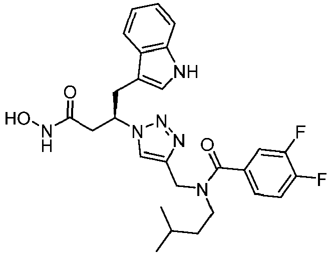 | 3,4-difluoro-N-[[1-[(1R)-3-(hydroxyamino)-1-(1H-indol-3-ylmethyl)-3-oxo-propyl]triazol-4-yl] methyl] -N-isopentyl-benzamide |
| 100 | 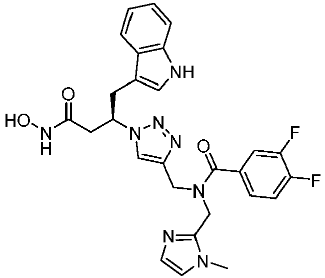 | 3,4-difluoro-N-[[1-[(1R)-3-(hydroxyamino)-1-(1H-indol-3-ylmethyl)-3-oxo-propyl]triazol-4-yl]methyl]-N-[(1-methylimidazol-2-yl)methyl]benzamide |
| 101 | 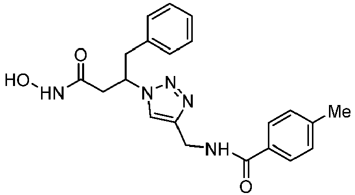 | N-[1-(1-Hydroxycarbamoylmethyl-2-phenyl-ethyl)-1H-[1,2,3]triazol-4-ylmethyl]-4-methyl-benzamide |
| 102 | 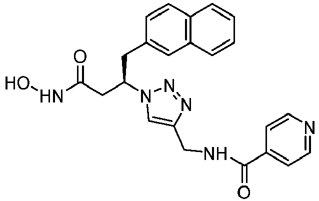 | methyl (3R)-4-(2-naphthyl)-3-[4-[(pyridine-4-carbonylamino)methyl]triazol-1-yl]butanoate |
| 103 | 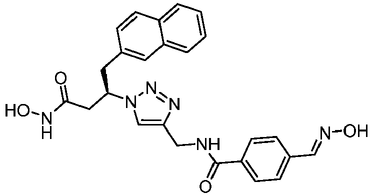 | N-[[1-[(1R)-3-(hydroxyamino)-1-(2-naphthylmethyl)-3-oxo-propyl]triazol-4-yl]methyl]-4-(hydroxycarbamoyl) benzamide |
| 104 | 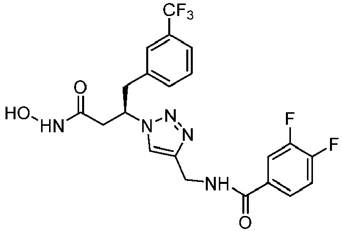 | 3,4-difluoro-N-[[1-[(1R)-3-(hydroxyamino)-3-oxo-1-[[3-(trifluoromethyl)phenyl]methyl]propyl]tr iazol-4-yl]methyl]benzamide |
| 105 | 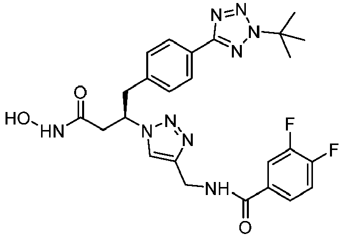 | N-({1-[(2R)-1-[4-(2-tert-butyl-2H-1,2,3,4-tetrazol-5-yl)phenyl]-3-(hydroxycarbamoyl)propan2-yl]-1H-1,2,3-triazol-4-yl}methyl)-3,4-difluorobenzamide |
| 143 | 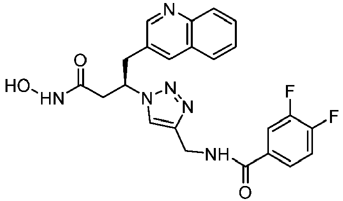 | (R)-3,4-difluoro-N-((1-(4-(hydroxyamino)-4-oxo-1-(quinolin-3-yl)butan-2-yl)-1H-1,2,3-triazol-4-yl)methyl)benzamide |
| 144 | 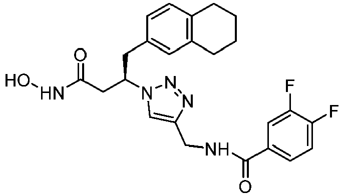 | (R)-3,4-difluoro-N-((1-(4-(hydroxyamino)-4-oxo-1-(5,6,7,8-tetrahydronaphthalen-2-yl)butan-2-yl)-1H-1,2,3-triazol-4-yl)methyl)benzamide |
| 146 | 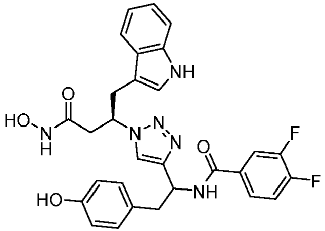 | 3,4-Difluoro-N-[1-[1-[(1R)-3-(hydroxyamino)-1-(1H-indol-3-ylmethyl)-3-oxo-propyl]triazol-4-yl] -2-(4-hydroxyphenyl)ethyl]benzamide |
| 164 | 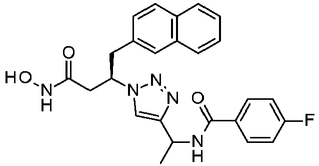 | 4-fluoro-N-(1-(1-((R)-4-(hydroxyamino)-1-(naphthalen-2-yl)-4-oxobutan-2-yl)-1H-1,2,3-triazol-4-yl)ethyl)benzamide |
| 165 | 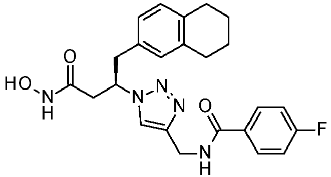 | (R)-4-fluoro-N-((1-(4-(hydroxyamino)-4-oxo-1-(5,6,7,8-tetrahydronaphthalen-2-yl)butan-2-yl)-1H-1,2,3 -triazol-4-yl)methyl)benzamide |
| 166 | 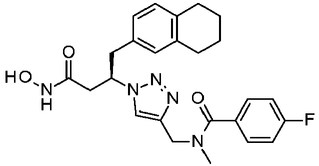 | (R)-4-fluoro-N-((1-(4-(hydroxyamino)-4-oxo-1-(5,6,7,8-tetrahydronaphthalen-2-yl)butan-2-yl)-1H-1,2,3 -triazol-4-yl)methyl)-N-methylbenzamide |
| 167 | 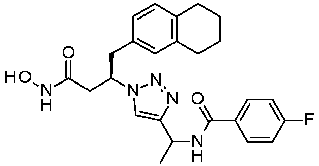 | 4-fluoro-N-(1-(1-((R)-4-(hydroxyamino)-4-oxo-1-(5,6,7,8-tetrahydronaphthalen-2-yl)butan-2-yl)-1H-1,2,3-triazol-4-yl)ethyl)benzamide |
| 168 | 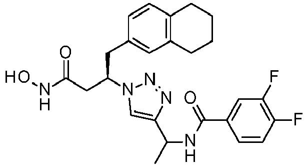 | 3,4-difluoro-N-(1-(1-((R)-4-(hydroxyamino)-4-oxo-1-(5,6,7,8-tetrahydronaphthalen-2-yl)butan-2-yl)-1H-1,2,3-triazol-4-yl)ethyl)benzamide |
| 169 | 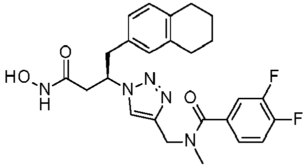 | (R)-3,4-difluoro-N-((1-(4-(hydroxyamino)-4-oxo-1-(5,6,7,8-tetrahydronaphthalen-2-yl)butan-2-yl)-1H-1,2,3-triazol-4-yl)methyl)-N-methylbenzamide |
| 170 | 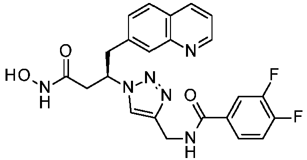 | (R)-3,4-difluoro-N-((1-(4-(hydroxyamino)-4-oxo-1-(quinolin-7-yl)butan-2-yl)-1H-1,2,3-triazol-4-yl)methyl)benzamide |
| 171 | 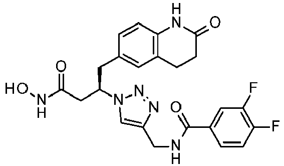 | (R)-3,4-difluoro-N-((1-(4-(hydroxyamino)-4-oxo-1-(2-oxo-1,2,3,4-tetrahydroquinolin-6-yl)butan-2-yl)-1H-1,2,3-triazol-4-yl)methyl)benzamide |
| 172 | 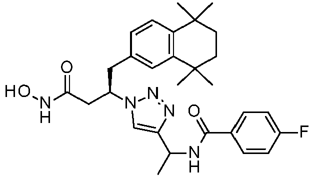 | 4-fluoro-N-(1-(1-((R)-4-(hydroxyamino)-4-oxo-1-(5,5,8,8-tetramethyl-5,6,7,8-tetrahydronaphthalen-2-yl)butan-2-yl)-1H-1,2,3-triazol-4-yl)ethyl)benzamide |
| 173 | 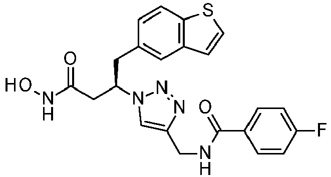 | (R)-N-((1-(1-(benzo[b]thiophen-5-yl)-4-(hydroxyamino)-4-oxobutan-2-yl)-1H-1,2,3-triazol-4-yl)methyl)-4-fluorobenzamide |
| 174 | 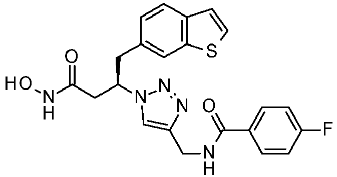 | (R)-N-((1-(1-(benzo[b]thiophen-6-yl)-4-(hydroxyamino)-4-oxobutan-2-yl)-1H-1,2,3-triazol-4-yl)methyl)-4-fluorobenzamide |
| 175 | 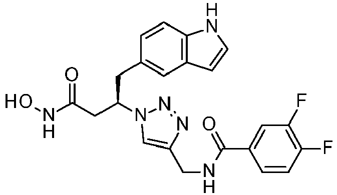 | (R)-3,4-difluoro-N-((1-(4-(hydroxyamino)-1-(1H-indol-5-yl)-4-oxobutan-2-yl)-1H-1,2,3-triazol-4-yl)methyl)benzamide |
| 176 | 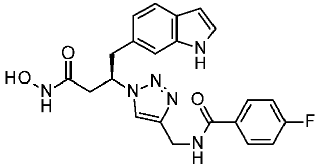 | (R)-4-fluoro-N-((1-(4-(hydroxyamino)-1-(1H-indol-6-yl)-4-oxobutan-2-yl)-1H-1,2,3-triazol-4-yl)methyl)benzamide |
| 177 | 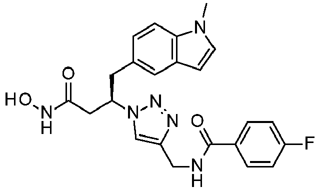 | (R)-4-fluoro-N-((1-(4-(hydroxyamino)-1-(1-methyl-1H-indol-5 -yl)-4-oxobutan-2-yl)-1H-1,2,3-triazol-4-yl)methyl)benzamide |
| 178 | 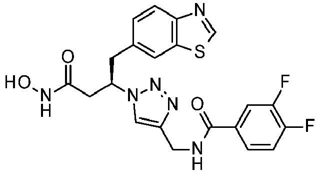 | (R)-N-((1-(1-(benzo[d]thiazol-6-yl)-4-(hydroxyamino)-4-oxobutan-2-yl)-1H-1,2,3-triazol-4-yl)methyl)-3,4-difluorobenzamide |
| 179 | 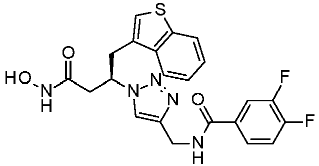 | (R)-N-((1-(1-(benzo[b]thiophen-3-yl)-4-(hydroxyamino)-4-oxobutan-2-yl)-1H-1,2,3-triazol-4-yl)methyl)-3,4-difluorobenzamide |
| 180 | 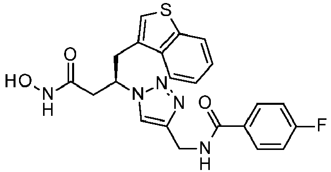 | (R)-N-((1-(1-(benzo[b]thiophen-3-yl)-4-(hydroxyamino)-4-oxobutan-2-yl)-1H-1,2,3-triazol-4-yl)methyl)-4-fluorobenzamide |
| 181 | 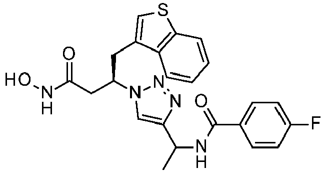 | N-(1-(1-((R)-1-(benzo[b]thiophen-3-yl)-4-(hydroxyamino)-4-oxobutan-2-yl)-1H-1,2,3-triazol-4-yl)ethyl)-4-fluorobenzamide |
| 182 | 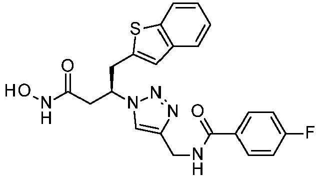 | (S)-N-((1-(1-(benzo[b]thiophen-2-yl)-4-(hydroxyamino)-4-oxobutan-2-yl)-1H-1,2,3-triazol-4-yl)methyl)-4-fluorobenzamide |
| 183 | 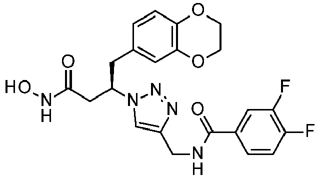 | (R)-N-((1-(1-(2,3-dihydrobenzo[b][1,4]dioxin-6-yl)-4-(hydroxyamino)-4-oxobutan-2-yl)-1H-1,2,3-triazol-4-yl)methyl)-3,4-difluorobenzamide |
| 184 | 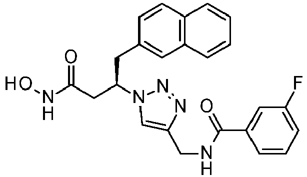 | (R)-3-fluoro-N-((1-(4-(hydroxyamino)-1-(naphthalen-2-yl)-4-oxobutan-2-yl)-1H-1,2,3-triazol-4-yl)methyl)benzamide |
| 185 | 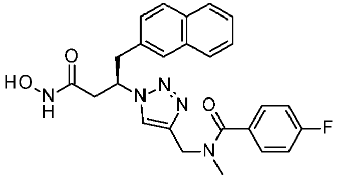 | (R)-4-fluoro-N-((1-(4-(hydroxyamino)-1-(naphthalen-2-yl)-4-oxobutan-2-yl)-1H-1,2,3-triazol-4-yl)methyl)-N-methylbenzamide |
| 186 | 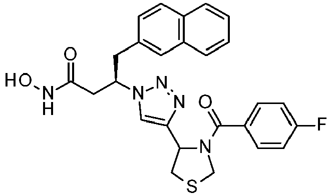 | (3R)-3-(4-(3-(4-fluorobenzoyl)thiazolidin-4-yl)-1H-1,2,3-triazol-1-yl)-N-hydroxy-4-(naphthalen-2-yl)butanamide |
| 187 | 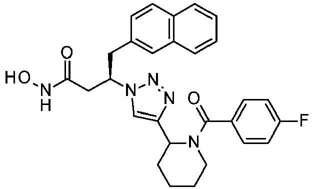 | (3R)-3-(4-(1-(4-fluorobenzoyl)piperidin-2-yl)-1H-1,2,3-triazol-1-yl)-N-hydroxy-4-(naphthalen-2-yl)butanamide |

The compounds of the invention can be prepared by different ways with reactions known by the person skilled in the art. Reaction schemes as described in the example section illustrate by way of example different possible approaches.

The compounds of Formula I are indeed capable of sensitizing cancer cells to various anticancer drugs. They further have the advantage of increasing the cytoxicity of cytotoxic agents, preferably proteasome inhibitors or HDAC inhibitors. The invention thus also provides the use of the compounds of Formula I or pharmaceutically acceptable salts, or solvates thereof as sensitizers for chemotherapy of malignant tumors.

Accordingly, in a particularly preferred embodiment, the invention relates to the use of compounds of Formula I and subformulae, in particular those of Table 1 above, or pharmaceutically acceptable salts and solvates thereof, for the treatment and/or prevention of cancer, particularly as sensitizers for chemotherapy of malignant tumors.

### APPLICATIONS

Unexpectedly, the inventors have discovered that the compounds of Formula I, including the 18 compounds listed in the proviso above, may be used as sensitizers for chemotherapy of malignant tumors.

As disclosed in the prior art, the 18 compounds listed in the proviso above may be of particular interest in animal models of chronic or degenerative diseases such as type-2 diabetes and Alzheimer's disease (R. Deprez-Poulain et al., Nature Communications, 2015, 6, article number:8250). However, the inventors have found that compounds of Formula I are capable of improving the effect of various cytotoxic agents for chemotherapy, and these compounds are therefore part of the use according to the invention.

The compounds for use according to the invention therefore include compounds of Formula I and subformulae II, IIa, IIb, IIc, IId, IIe, III, IIIa, IIIb, IV, IVa, IVb, V, Va and Vb as defined above, in particular compounds of Table 1 above.

The compounds of the invention are able to sensitize cancer cells to various anticancer agents in various cancers, and therefore to increase the cytotoxicity of those anticancer agents.

The compounds of Formula I, or any of its subformulae as defined above, can thus be used as sensitizers for chemotherapy of malignant tumors, aiming at improving the chemotherapy effect of the cancer treatment, preventing tolerance and decreasing toxicity and adverse effects.

The invention thus relates to a compound of Formula I: 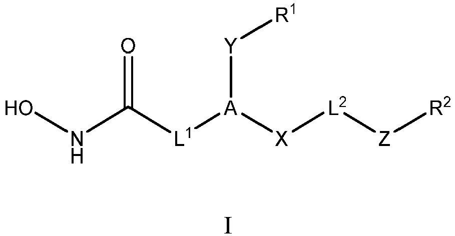 or a pharmaceutically acceptable salt or solvate thereof for use in treating and/or preventing cancer,
wherein
**L¹** is inexistent or is selected from -CH₂- and -CH(OH)-;
A is CH or N;
Y is -CH₂- or -S-;
**R¹** is selected from aryl, heteroaryl, arylalkyl and arylalkenyl wherein said aryl, heteroaryl, arylalkyl or arylalkenyl is optionally substituted by one or more substituents independently selected from C1-C4-alkyl, halogen, C1-C4-haloalkyl, hydroxy-C1-C4-alkyl, acetylamino, acetylamino-C1-C4-alkyl and C1-C4-alkylaminocarboxyl;
**R²** is selected from aryl, heteroaryl, arylalkyl, cycloalkyl, heterocycloalkyl, C1-C6-alkyl, C1-C4-alkyloxycarbonyl, C1-C4-alkoxy, arylamino, wherein said aryl, arylalkyl, cycloalkyl or heterocycloalkyl is optionally substituted by one or more substituents independently selected from C1-C4-alkoxy, halogen, C1-C4-haloalkyl, C1-C4-alkyl, C1-C2-alkoxy-C1-C2-alkoxy, C1-C4-alkyloxycarbonyl, acetylamino, di(C1-C4-alkyl)amino-C1-C4-alkyl and hydroxycarbamoyl;
**X** is selected from 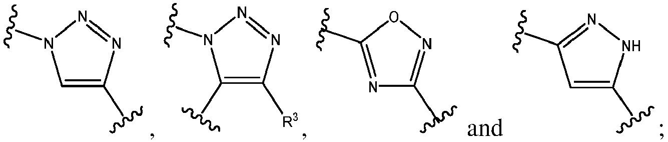
**R³** is selected from H and C(O)OR⁴;
**R⁴** is C1-C4-alkyl;
**L²** is linear or branched C1-C6-alkyl, optionally substituted by a group **R⁵**;
**R⁵** is selected from linear or branched C1-C4-alkyl, CH₂-OH, CHOH-CH₃, CH₂-C(O)NH₂, CH₂-CH₂-C(O)NH₂, CH₂-COOH, CH₂-CH₂-COOH, (CH₂)₄-NH₂, (CH₂)₄-NH-C(NH₂⁺)-NH₂, CH₂-Imidazolyl, CH₂-Indolyl, CH₂-SH, CH₂-CH₂-S-CH₃, CH₂-Ph, CH₂-Ph-OH, CH₂-OR⁶, CH₂-COOR⁶, CH₂-CH₂-COOR⁶, CH₂-SR⁶ and CH₂-Ph-OR⁶;
**R⁶** is selected from Me, Bn, Ph and Ac;
**Z** is selected from -NR⁷(CO)-, -NHSO₂-, -CH₂-CH₂-, -NH-, -NH-CH(C1-C6-alkyl)-, -O-CH₂-, -C(O)NH- and -O-; and
**R⁷** is selected from H, linear or branched C1-C6-alkyl and (1-methylimidazol-2-yl)-C1-C2-alkyl, or
**R⁷** and **L²** form together with the nitrogen atom they are attached to a 5- or 6-membered heterocyclyl group, or to a compound of any of the subformulae of Formula I as defined above, in particular a compound of Table 1 above, or a pharmaceutically acceptable salt or solvate thereof, for use in treating and/or preventing cancer, particularly as sensitizers for chemotherapy of malignant tumors.

Anticancer agents, or cytotoxic agents, within the meaning of the present invention include, but are not limited to, proteasome inhibitors, HDAC inhibitors and protein glycosylation inhibitors. Preferred proteasome inhibitors are epoxomycin, carfilzomib, bortezomib, oprozomib, ixazomib and nelfinavir. More preferred proteasome inhibitors are epoxomycin, carfilzomib and nelfinavir. Preferred HDAC inhibitors are panobinostat, quisinostat and N-[4-[3-[[[7-(hydroxyamino)-7-oxoheptyl]amino]carbonyl]-5-isoxazolyl]phenyl]-1,1-dimethylethyl ester, carbamic acid (CAS#: 1045792-66-2). A more preferred HDAC inhibitor is panobinostat. A preferred protein glycosylation inhibitor is tunicamycin.

Thus, in one embodiment, there is provided a compound of Formula I, or any of its subformulae as defined above, for use as sensitizer for chemotherapy of malignant tumors involving a proteasome inhibitor, a HDAC inhibitor or a protein glycosylation inhibitor. The compounds of the invention are therefore useful in the prevention and/or treatment of cancers, and particularly cancers that may be treated by proteasome inhibitors, HDAC inhibitors or protein glycosylation inhibitors.

The invention thus also relates to a compound of the invention or a pharmaceutically acceptable salt or solvate thereof for use in the treatment and/or prevention of cancer, in particular cancers that may be treated by proteasome inhibitors, HDAC inhibitors or protein glycosylation inhibitors. Or in other terms, the invention also relates to a method of treating and/or preventing cancer, in particular cancers that may be treated by proteasome inhibitors, HDAC inhibitors or protein glycosylation inhibitors, comprising the administration of a therapeutically effective amount of a compound of Formula I or a pharmaceutically acceptable salt or solvate, to a patient in need thereof. Preferably the patient is a warm-blooded animal, more preferably a human.

Cancers that may be treated by proteasome inhibitors, HDAC inhibitors or protein glycosylation inhibitors, within the meaning of the present invention include, but are not limited to, multiple myeloma, cervical cancer and breast cancer.

The invention further provides the use of a compound of the invention or a pharmaceutically acceptable salt or solvates thereof for the manufacture of a medicament for use in treating and/or preventing cancer, in particular cancers that may be treated by cytotoxic agents, preferably by proteasome inhibitors, HDAC inhibitors or protein glycosylation inhibitors. Preferably the patient is a warm-blooded animal, more preferably a human. The cancers that may be treated by proteasome inhibitors, HDAC inhibitors or protein glycosylation inhibitors are preferably those defined above.

According to a further feature of the present invention, there is provided a compound of Formula I or a pharmaceutically acceptable salt or solvate for use in sensitizing cancer cells to anticancer drugs, preferably proteasome inhibitors, HDAC inhibitors or protein glycosylation inhibitors, in a patient in need of such treatment, comprising administering to said patient an effective amount of a compound of the invention, or a pharmaceutically acceptable salt or solvate thereof. In other terms, the invention also provides a method for sensitizing cancer cells to anticancer drugs, preferably proteasome inhibitors, HDAC inhibitors or protein glycosylation inhibitors, in a patient in need of such treatment, which comprises administering to said patient an effective amount of a compound of the invention, or a pharmaceutically acceptable salt or solvate thereof. Preferably, the patient is a warm blooded animal, and even more preferably a human.

According to one embodiment, the compounds of the invention, their pharmaceutical acceptable salts or solvates may be administered as part of a combination therapy. Thus, are included within the scope of the present disclosure embodiments comprising coadministration of, and compositions and medicaments which contain, in addition to a compound of the present invention, a pharmaceutically acceptable salt or solvate thereof as active ingredient, additional therapeutic agents and/or active ingredients. Such multiple drug regimens, often referred to as combination therapy, may be used in the treatment and/or prevention of any cancer, particularly those defined above.

Thus, the methods of treatment and pharmaceutical compositions of the present disclosure may employ the compounds of the invention or their pharmaceutical acceptable salts or solvates thereof in the form of monotherapy, but said methods and compositions may also be used in the form of multiple therapy in which one or more compounds of the invention or their pharmaceutically acceptable salts or solvates are co-administered in combination with one or more other therapeutic agents. Such additional therapeutic agents include, but are not limited to, proteasome inhibitors, HDAC inhibitors and protein glycosylation inhibitors. Preferred proteasome inhibitors are epoxomycin, carfilzomib, bortezomib, oprozomib, ixazomib and nelfinavir. More preferred proteasome inhibitors are epoxomycin, carfilzomib and nelfinavir. Preferred HDAC inhibitors are panobinostat, quisinostat and N-[4-[3-[[[7-(hydroxyamino)-7-oxoheptyl]amino]carbonyl]-5-isoxazolyl]phenyl]-1,1-dimethylethyl ester, carbamic acid (CAS#: 1045792-66-2). A more preferred HDAC inhibitor is panobinostat. A preferred protein glycosylation inhibitor is tunicamycin.

In one embodiment, the methods of treatment and pharmaceutical compositions of the present disclosure may employ the compounds of Formula I, or any of its subformulae as defined above, or their pharmaceutical acceptable salts or solvates thereof, in combination with radiation therapy. According to this embodiment, the compounds of the invention, their pharmaceutical acceptable salts or solvates may be administered in combination with radiation therapy. Thus, there is provided a compound of Formula I, or any of its subformulae as defined above, for use in the treatment and/or prevention of cancers as defined above in combination with radiation therapy. Such radiation therapies include, but are not limited to, external beam radiation therapy, brachytherapy and systemic radioisotope therapy.

The invention also provides pharmaceutical compositions comprising a compound of the invention or a pharmaceutically acceptable salt or solvate thereof and at least one pharmaceutically acceptable carrier, diluent, excipient and/or adjuvant. As indicated above, the invention also covers pharmaceutical compositions which contain, in addition to a compound of the present invention, a pharmaceutically acceptable salt or solvate thereof as active ingredient, additional therapeutic agents and/or active ingredients.

Another object of this invention is a medicament comprising at least one compound of the invention, or a pharmaceutically acceptable salt or solvate thereof, as active ingredient.

Generally, for pharmaceutical use, the compounds of the invention may be formulated as a pharmaceutical preparation comprising at least one compound of the invention and at least one pharmaceutically acceptable carrier, diluent, excipient and/or adjuvant, and optionally one or more further pharmaceutically active compounds.

By means of non-limiting examples, such a formulation may be in a form suitable for oral administration, for parenteral administration (such as by intravenous, intramuscular or subcutaneous injection or intravenous infusion), for topical administration (including ocular), cerebral administration, for administration by inhalation, by a skin patch, by an implant, by a suppository, etc. Such suitable administration forms - which may be solid, semi-solid or liquid, depending on the manner of administration - as well as methods and carriers, diluents and excipients for use in the preparation thereof, will be clear to the skilled person; reference is made to the latest edition of Remington's Pharmaceutical Sciences.

### DEFINITIONS

The definitions and explanations below are for the terms as used throughout the entire application, including both the specification and the claims.

Unless otherwise stated any reference to compounds of the invention herein, means the compounds as such as well as their pharmaceutically acceptable salts and solvates.

When describing the compounds of the invention, the terms used are to be construed in accordance with the following definitions, unless indicated otherwise.

The term "unsubstituted" as used herein means that a radical, a group or a residue carries no substituents. The term "substituted" means that a radical, a group or a residue carries one or more substituents.

The term "halo" or "halogen" refers to the atoms of the group 17 of the periodic table (halogens) and includes in particular fluorine, chlorine, bromine and iodine atom. Preferred halo groups are fluoro and chloro, fluoro being particularly preferred.

The term "alkyl" by itself or as part of another substituent refers to a hydrocarbyl radical of Formula CₙH₂ₙ₊₁ wherein n is a number greater than or equal to 1. Alkyl groups may be linear or branched. Examples of alkyl groups include but are not limited to methyl (abbreviation: Me), ethyl (abbreviation: Et), *n*-propyl (abbreviation: *n*-Pr), isopropyl (abbreviation: *i*-Pr), *n*-butyl (abbreviation: *n*-Bu), isobutyl (abbreviation: *i*-Bu), *tert-butyl* (abbreviation: *t*-Bu), *sec*-butyl (abbreviation: *s*-Bu), *n*-pentyl, isopentyl, hexyl and isohexyl.

The term "haloalkyl" alone or in combination, refers to an alkyl radical having the meaning as defined above wherein one or more hydrogens are replaced with a halogen as defined above. Non-limiting examples of such haloalkyl radicals include chloromethyl, 1-bromoethyl, fluoromethyl, difluoromethyl, trifluoromethyl, 1,1,1-trifluoroethyl and the like.

The term "cycloalkyl" as used herein is a monovalent, saturated, or unsaturated monocyclic or bicyclic hydrocarbyl group. Cycloalkyl groups may comprise 3 or more carbon atoms in the ring and generally, according to this invention comprise from 3 to 10, more preferably from 3 to 8 carbon atoms still more preferably from 3 to 6 carbon atoms. Examples of cycloalkyl groups include but are not limited to cyclopropyl, cyclobutyl, cyclopentyl, and cyclohexyl.

The term "heteroatom" as used herein refers to any atom that is not carbon or hydrogen. Non-limiting examples of such heteroatoms include nitrogen, oxygen, sulfur, and phosphorus. Preferred heteroatoms according to the invention are nitrogen, oxygen and sulfur.

The terms "heterocyclyl", "heterocycloalkyl" or "heterocyclo" as used herein by itself or as part of another group refer to non-aromatic, fully saturated or partially unsaturated cyclic groups (for example, 3 to 7 member monocyclic, 7 to 11 member bicyclic, or containing a total of 3 to 10 ring atoms) which have at least one heteroatom in at least one carbon atom-containing ring. Each ring of the heterocyclic group containing a heteroatom may have 1, 2, 3 or 4 heteroatoms selected from nitrogen, oxygen and/or sulfur atoms, where the nitrogen and sulfur heteroatoms may optionally be oxidized and the nitrogen heteroatoms may optionally be quaternized. The heterocyclic group may be attached at any heteroatom or carbon atom of the ring or ring system, where valence allows. Examples of heterocyclyl groups include but are not limited to aziridinyl, azetidinyl, pyrrolidinyl, piperidinyl, azepanyl, piperazinyl, morpholinyl. Particular heterocyclyl groups according to the invention are pyrrolidinyl, morpholinyl, piperidinyl and thiazolidinyl. Preferred heterocyclyl groups according to the invention are pyrrolidinyl and morpholinyl.

The term "aryl" as used herein refers to a polyunsaturated, aromatic hydrocarbyl group having a single ring (i.e. phenyl) or multiple aromatic rings fused together (e.g. naphthyl), typically containing 5 to 12 atoms; preferably 6 to 10, wherein at least one ring is aromatic. Examples of aryl groups include but are not limited to phenyl (abbreviation: Ph), biphenyl, 1-naphthyl (or naphthalene-1-yl), 2-naphthyl (or naphthalene-2-yl), anthracenyl, indanyl, indenyl, 1,2,3,4-tetrahydronaphthyl. Preferred aryl groups according to the invention are phenyl, biphenyl and 2-naphthyl.

The term "heteroaryl" as used herein by itself or as part of another group refers but is not limited to 5 to 12 carbon-atom aromatic rings or ring systems containing 1 to 2 rings which are fused together, typically containing 5 to 6 atoms; at least one of which is aromatic, in which one or more carbon atoms in one or more of these rings is replaced by oxygen, nitrogen and/or sulfur atoms where the nitrogen and sulfur heteroatoms may optionally be oxidized and the nitrogen heteroatoms may optionally be quaternized. Examples of heteroaryl groups include but are not limited to pyridyl, pyrazinyl, pyrimidinyl, pyridazinyl, triazinyl, quinolinyl, quinoxalinyl, quinazolinyl, furanyl, benzofuranyl, pyrrolyl, indolyl, thiophenyl, benzothiophenyl, imidazolyl, benzimidazolyl, pyrazolyl, indazolyl, oxazolyl, benzoxazolyl, isoxazolyl, benzisoxazolyl, thiazolyl, and benzothiazolyl, triazolyl, oxadiazolyl, thiadiazolyl, dioxazolyl, dithiazolyl and tetrazolyl. Particular heteroaryl groups according to the invention are triazolyl, oxadiazolyl, pyrazolyl, imidazolyl, tetrazolyl, quinolinyl, indolyl, benzothiophenyl, dihydroquinolinonyl, benzodioxolyl and benzodioxinyl. Prefered heteroaryl groups according to the invention are triazolyl, oxadiazolyl, pyrazolyl, imidazolyl, tetrazolyl, quinolinyl and indolyl.

The term "arylalkyl" or "aralkyl" as used herein refers to a group -alkyl-aryl, wherein alkyl and aryl are as herein defined. Examples of arylalkyl groups include but are not limited to benzyl (abbreviation: Bn).

The term "arylalkenyl" as used herein refers to a group -alkenyl-aryl, wherein alkenyl and aryl are as herein defined. Examples of arylalkenyl groups include but are not limited to styryl.

The term "alkoxy" as used herein refers to a group -O-alkyl, wherein alkyl is as herein defined. Examples of alkoxy groups include but are not limited to methoxy, ethoxy, *n-*propyloxy, isopropyloxy, *n*-butyloxy, *t*-butyloxy, *sec*-butyloxy and *n*-pentyloxy.

The term "acetyl" or its abbreviation "Ac" as used herein refers to a group -C(O)CH₃.

The compounds of the invention containing a basic functional group may be in the form of pharmaceutically acceptable salts. Pharmaceutically acceptable salts of the compounds of the invention containing one or more basic functional groups include in particular the acid addition salts thereof. Suitable acid addition salts are formed from acids which form nontoxic salts. Examples include the acetate, adipate, aspartate, benzoate, besylate, bicarbonate/carbonate, bisulphate/sulphate, borate, camsylate, cinnamate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Pharmaceutically acceptable salts of compounds of Formula I and subformulae may for example be prepared as follows:
(i) reacting the compound of Formula I or any of its subformulae with the desired acid; or
(ii) converting one salt of the compound of Formula I or any of its subformulae to another by reaction with an appropriate acid or by means of a suitable ion exchange column.

All these reactions are typically carried out in solution. The salt, may precipitate from solution and be collected by filtration or may be recovered by evaporation of the solvent. The degree of ionization in the salt may vary from completely ionized to almost nonionized.

The term "solvate" is used herein to describe a molecular complex comprising the compound of the invention and one or more pharmaceutically acceptable solvent molecules, for example, ethanol. The term "hydrate" is employed when said solvent is water.

The compounds of the invention include compounds of the invention as hereinbefore defined, including all polymorphs and crystal habits thereof, prodrugs and isomers thereof (including optical, geometric and tautomeric isomers) and isotopically-labeled compounds of the invention.

In addition, although generally, with respect to the salts of the compounds of the invention, pharmaceutically acceptable salts are preferred, it should be noted that the invention in its broadest sense also includes non-pharmaceutically acceptable salts, which may for example be used in the isolation and/or purification of the compounds of the invention. For example, salts formed with optically active acids or bases may be used to form diastereoisomeric salts that can facilitate the separation of optically active isomers of the compounds of the invention.

The term "patient" refers to a warm-blooded animal, more preferably a human, who/which is awaiting or receiving medical care or is or will be the object of a medical procedure.

The term "human" refers to subjects of both genders and at any stage of development (i.e. neonate, infant, juvenile, adolescent, adult). In one embodiment, the human is an adolescent or adult, preferably an adult.

The terms "treat", "treating" and "treatment", as used herein, are meant to include alleviating or abrogating a condition or disease and/or its attendant symptoms.

The terms "prevent", "preventing" and "prevention", as used herein, refer to a method of delaying or precluding the onset of a condition or disease and/or its attendant symptoms, barring a patient from acquiring a condition or disease, or reducing a patient's risk of acquiring a condition or disease.

The term "therapeutically effective amount" (or more simply an "effective amount") as used herein means the amount of active agent or active ingredient which is sufficient to achieve the desired therapeutic or prophylactic effect in the individual to which it is administered.

The term "administration", or a variant thereof (e.g.,"administering"), means providing the active agent or active ingredient, alone or as part of a pharmaceutically acceptable composition, to the patient in whom/which the condition, symptom, or disease is to be treated or prevented.

By "pharmaceutically acceptable" is meant that the ingredients of a pharmaceutical composition are compatible with each other and not deleterious to the patient thereof.

The term "agonist" as used herein means a ligand that activates an intracellular response when it binds to a receptor.

The term "pharmaceutical vehicle" as used herein means a carrier or inert medium used as solvent or diluent in which the pharmaceutically active agent is formulated and/or administered. Non-limiting examples of pharmaceutical vehicles include creams, gels, lotions, solutions, and liposomes.

The present invention will be better understood with reference to the following examples and figures. These examples are intended to representative of specific embodiments of the invention, and are not intended as limiting the scope of the invention.

### FIGURES

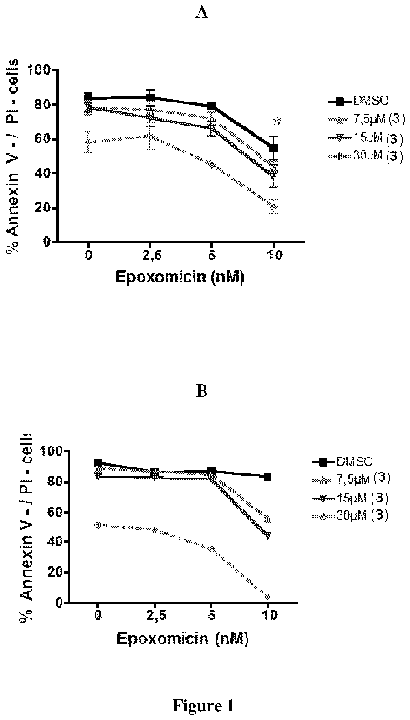

**Figure 1****:** EPX cytotoxicity in combination with compound **3** of the invention on different multiple myeloma cell lines. **A:** RPMI 8226; **B:** MOPC 315.BM.

### EXAMPLES

### CHEMISTRY EXAMPLES

All reagents, solvents and starting materials were purchased from commercial suppliers and used without further purification.

Melting points were determined using a Büchi B-540 melting point apparatus and are uncorrected.

¹H NMR spectra were recorded on a Brucker Avance 300 MHz spectrometer with methanol-d4, CDCl₃, DMSO-d6 or acetone-d6 as the solvent. ¹³C NMR spectra are recorded at 100 MHz. All coupling constants are measured in hertz (Hz) and the chemical shifts (δ) are quoted in parts per million (ppm).

Liquid chromatography mass spectroscopy analyses (LC-MS) were performed using LCMS-MS triple-quadrupole system (Waters) with a C₁₈ TSK-GEL Super ODS (2 µm particle size column, 50 × 4.6 mm). LCMS gradient starting from 98% H₂O / 0.1% formic acid and reaching 2% H₂O / 98% MeOH within 5 min (method A) at a flow rate of 2 mL/min or starting from 100% H₂O / 0.1% formic acid and reaching 5% H₂O / 95% MeOH within 10 min (method B) at a flow rate of 1 mL/min was used. Purity (%) was determined by Reversed Phase HPLC, using UV detection (215 nM).

High resolution mass spectroscopy (HRMS) were carried out on an Waters LCT Premier XE (TOF), ESI ionization mode, with a Waters XBridge C₁₈ (150 × 4.6 mm, 3.5 µm particle size). LCMS gradient starting from 98% ammonium formate buffer 5 mM (pH 9.2) and reaching 95% CH₃CN / 5% ammonium formate buffer 5 mM (pH 9.2) within 15 min at a flow rate of 1 mL/min was used.

Solvents, reagents and starting materials were purchased from well known chemical suppliers such as for example Sigma Aldrich, Acros Organics, Fluorochem, Eurisotop, VWR International, Sopachem and Polymer labs and the following abbreviations are used:
ACN: Acetonitrile,
AUC: Area under curve,
CC₅₀: 50% cytotoxic concentration,
CDI: 1,1'-Carbonyldiimidazole,
DCE: Dichloroethane,
DCM: Dichloromethane,
DMF: N,N-dimethylformamide,
DMSO: Dimethylsulfoxyde,
EDCI: 1-Ethyl-3-(3-dimethylaminopropyl)carbodiimide,
EDTA: Ethylenediaminetetraacetic acid,
eq or equiv: Equivalent,
EtOH: Ethanol,
KCN: Potassium cyanide,
HOBt: Hydroxybenzotriazole,
HRMS: High resolution mass spectrometry,
LC: Liquid chromatography,
LCMS: Liquid chromatography-mass spectrometry,
MeOH: Methanol,
Mp: Melting point,
MS: Mass spectrometry,
MW: Molecular weight,
NMM: N-methylmorpholine
NMR: Nuclear magnetic resonance,
PE: Petroleum ether,
RT or rt: Room temperature,
THF: tetrahydrofurane,
TLC: Thin layer chromatography,
TFA: Trifluoroacetic acid,
t_{R}: Retention time,
CFZ: carfilzomib,
TUN: tunicamycin,
BTZ: bortezomib,
OZ: oprozomib,
PAN: panobinostat,
NLF: nelfinavir,

EPX: epoxomycin.

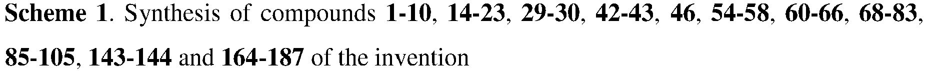

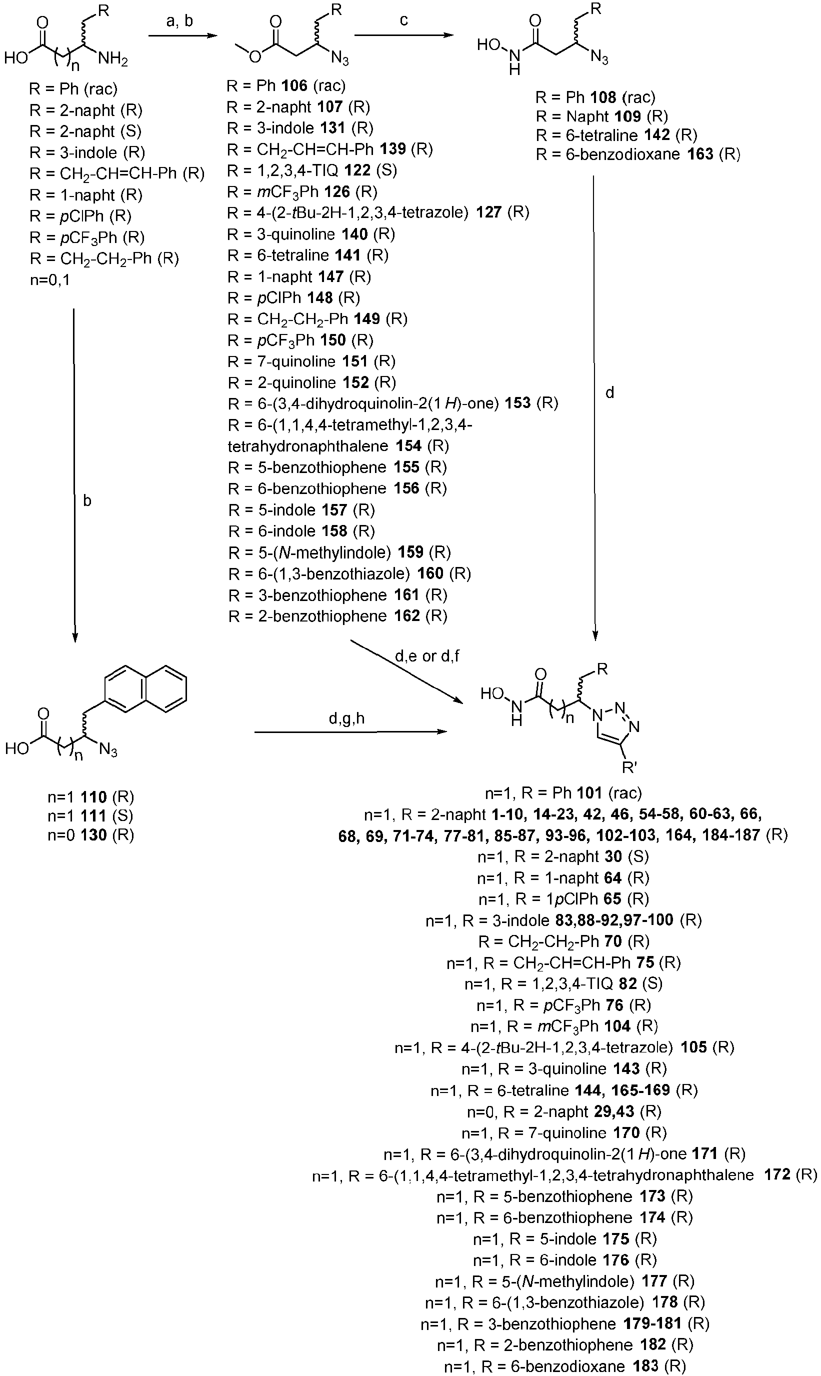

Reagents and conditions: (a) SOCl₂, MeOH, 0°C to rt, 16 h; (b) CuSO₄, K₂CO₃, imidazole-1-sulfonyl azide hydrochloride, MeOH, rt, 16 h; (c) NH₂OH.HCl, KOH, MeOH, 0°C to rt, 16 h. (d) alkyne, CuSO₄.5H₂O, sodium ascorbate, H₂O/DMF or H₂O/dioxane or DMSO/H₂O/tBuOH or CH₃CN/H₂O/dioxane or dioxane, rt, 16 h; (e) NH₂OH, KOH, KCN, Na₂SO₄, H₂O, MeOH, 0°C to rt, 16 h; (f) NH₂OH, KCN, H₂O, MeOH, rt, 16 h; (g) O-tritylhydroxylamine, EDCI, HOBt, N-methylmorpholine, DMF, rt, 16 h; (h) TFA, TIS, CH₂Cl₂, rt, 30 min.

### 1. General Protocoles

### (a) General procedure A for azide synthesis:

Imidazole-1-sulfonyl azide hydrochloride (1.2 equiv) was added to the β-amino ester (1 equiv), K₂CO₃ (1 equiv) and CuSO₄.5H₂O (0.01 equiv) in methanol and the mixture was stirred at room temperature overnight. The mixture was concentrated, diluted with H₂O, acidified with concentrated HCl and extracted with ethyl acetate (3 times). The combined organic layers were washed with brine, dried (MgSO₄), filtered and concentrated to give the azide which was purified if needed.

### (b) General procedure A' for azide synthesis:

The starting β-amino ester (1.0 equiv) was dissolved in dry MeOH under argon, and cooled down to 0°C before ZnCl₂ (0.06 equiv), potassium carbonate (1.0 equiv) and DIPEA (1.0 equiv) were added. In a separate flask, cooled DIPEA (1.15 equiv) was added to a solution of N-diazoimidazole-1-sulfonamide (as a bisulfate salt, 1.05 equiv) in MeOH at 0°C. The resulting solution was immediately added dropwise to the β-amino ester solution, and the resulting mixture was further stirred overnight in the melting ice bath. The reaction was cooled back down to 0°C, diluted with water and carefully acidified to pH = 2 with 1M KHSO₄ and extracted with ethyl acetate. Organic layers were mixed and concentrated to dryness *in vacuo.* Purification over silica gel column was performed if needed.

### (c) General procedure A" for azide synthesis:

The starting β-amino ester (1.0 equiv) was dissolved in dry MeOH under argon, and cooled down to 0°C before ZnCl₂ (0.06 equiv), potassium carbonate (1.0 equiv) and DIPEA (1.0 equiv) were added. In a separate flask, cooled DIPEA (1.15 equiv) was added to a solution of N-diazoimidazole-1-sulfonamide (as a bisulfate salt, 1.05 equiv) in MeOH at 0°C. The resulting solution was immediately added dropwise to the β-amino ester solution, and the resulting mixture was further stirred overnight in the melting ice bath. The reaction was cooled back down to 0°C, diluted with water and extracted with ethyl acetate. Organic layers were mixed and concentrated to dryness *in vacuo.* Purification over silica gel column was performed if needed.

### (d) General procedure B for 1,4-disubstituted 1,2,3-triazole synthesis:

The azide (1 equiv) and the alkyne (1 equiv) were dissolved separately in DMSO (100-150 µL) then added to a mixture of tBuOH/water (1:1 (v/v)) or directly solubilised in a mixture of DMF/water (1:1 (v/v)). CuSO₄:5H₂O (0.1 equiv) and sodium ascorbate (1 equiv) were added. After 16 h of stirring at room temperature, the media was filtered. In most cases, concentration of the filtrate, followed by precipitation in water, filtration and washing with ethyl acetate gave a pure product. If necessary, the final triazole was purified.

### (e) General procedure C for 1,4-disubstituted 1,2,3-triazole synthesis:

Azide (1.0 equiv) and alkyne (1.2 equiv) were added in a 1:1 (v/v) mixture of water and DMF or dioxane or a 2:1:1 (v/v) mixture of CH₃CN, dioxane and water. Sodium ascorbate (0.3 equiv) was added, followed by copper(II) sulfate pentahydrate (0.06 equiv). The mixture was stirred overnight. Solvents were evaporated under reduced pressure and the residue was diluted with water and extracted three times with EtOAc. The combined organic layers were dried with MgSO₄, filtered and concentrated under reduced pressure to give the triazole which was purified if needed.

### (f) General procedure C' for 1,4-disubstituted 1,2,3-triazole synthesis:

Azide (1.0 equiv) and alkyne (1.05 equiv) were dissolved in DMF or dioxane. A solution of copper(II) sulfate pentahydrate (0.1 equiv) in distilled water was added, quickly followed by sodium ascorbate (0.5 equiv). The mixture was stirred overnight before it was diluted with water and extracted three times with EtOAc. The combined organic layers were dried over MgSO₄, filtered and concentrated under reduced pressure to give the triazole which was purified if needed.

### (g) General procedure D for hydroxamic acid synthesis:

The carboxylic acid (1.0 equiv) was dissolved in DMF. EDCI (1.2 equiv), HOBt (1.5 equiv), N-methylmorpholine (6.0 equiv) were added. The mixture was stirred at room temperature for 5 min, and O-tritylhydroxylamine (1.2 equiv) was added. The mixture was stirred at room temperature overnight. The solvent was evaporated under reduced pressure and the residue was dissolved in CH₂Cl₂ and washed three times with a 5% NaHCO₃ (aq) solution and once with water. The organic layer was dried with MgSO₄, filtered and concentrated under reduced. The crude product was purified by flash chromatography on silica gel (CH₂Cl₂/MeOH). The obtained O-trityl hydroxamate intermediate was dissolved in TFA 5%/CH₂Cl₂ and triisopropylsilane was added. The mixture was stirred at room temperature for 30 min. Solvents were removed under reduced pressure and the residue was triturated with diethyl ether and petroleum ether to give a residue which was purified if needed to afford the hydroxamic acid.

### (h) General procedure E for hydroxamic acid synthesis:

The azido ester (1 equiv) was dissolved in MeOH. HONH₂.HCl (7.2 equiv) was dissolved in MeOH. KOH (11.4 equiv) was dissolved in MeOH. The KOH solution was poured into the HONH₂.HCl solution, and the resulting mixture was cooled to 0°C for 1 h. The KOH/HONH₂ solution was then filtered into the solution of the ester, and the reaction mixture was stirred at room temperature until completion. After removal of the solvent, the mixture was dissolved in ethyl acetate and washed successively with a 1N solution of HCl and brine. Removal of the solvent gave a brown oil which was purified if needed.

### (i) General procedure F for hydroxamic acid synthesis:

A solution of hydroxylamine in water (50 % in water, 1 equiv) and MeOH was dried with an excess of Na₂SO₄ and filtered. The filtrate was added to a flask containing the ester (1 equiv) and KCN (1 equiv). The mixture was stirred until completion of the reaction. The reaction mixture was diluted with CH₃CN. The unsoluble solids were filtered. The filtrate was evaporated to dryness and purified if needed to give the desired hydroxamic acid.

### (j) General procedure G for hydroxamic acid synthesis:

To a solution of the ester (1 equiv) in a 1:1 (v/v) mixture of MeOH and NH₂OH (50% in water) was added KCN (0.1 equiv). The mixture was stirred at room temperature for 16 h. The mixture was diluted with water and EtOAc. The aqueous phase was extracted twice with EtOAc. The combined organic layers were dried over Na₂SO₄, filtered, and concentrated under reduced pressure to afford the desired compounds, which was purified if needed.

### (k) General procedure G' for hydroxamic acid synthesis:

To a solution of the ester (1 equiv) in a 1:1 (v/v) mixture of MeOH and NH₂OH (50% in water) was added KCN (0.1-0.5 equiv). The mixture was stirred at room temperature for 4-16 h. All solvents were evaporated *in vacuo* before purification.

### (l) General procedure H for carboxylic acid methylation:

To a stirred solution of the corresponding amino acid (1 equiv) in methanol (107 equiv) was added dropwise thionyl chloride (15 equiv) at 0°C. After stirring at room temperature overnight, the solvent was evaporated and the crude material precipitated in ether to give the methyl ester.

### (m) General procedure I for amine acylation:

The amine (1 equiv) was dissolved in dioxane and an aqueous saturated solution of K₂CO₃. The biphasic mixture was cooled to 0°C. A solution of acyl chloride (1 equiv) in dioxane was slowly added to the amino acid solution and the reaction was allowed to slowly warm to room temperature. After 3 hours, the reaction was dissolved in water and extracted with diethyl ether (twice). The resulting aqueous layer was acidified to pH 1 with 3 M HCl (aq) and extracted with ethyl acetate (3 times). The combined organic layers were dried with MgSO₄, filtered and concentrated under reduced pressure to give the desired compound after purification if needed.

### (n) General procedure J for Negishi coupling:

Zinc dust (3 equiv) was added to a dry round bottom 100 mL flask under argon. Dry DMF was added via a syringe followed by a catalytic amount of iodine (0.15 equiv). Compound **123** (1 equiv) was added immediately followed by a catalytic amount of iodine (0.15 equiv). The solution was stirred at room temperature and gave a noticeable exotherm. When the solution had cooled Pd₂dba₃ (0.025 equiv), SPhos (0.05 equiv) and the aromatic bromide or iodide (1.3 equiv) were added to the flask and left to stir at room temperature overnight, under argon. The crude reaction mixture was purified by flash chromatography to afford the desired compound.

### (o) General procedure K for Negishi coupling:

Zinc dust (6 equiv) was added to a dry round-bottom flask under argon. Dry DMF and TMSCl (1.3 equiv) were added via a syringe. The reaction mixture was sonicated then stirred at room temperature for 5 min. The zinc was allowed to settle and the supernatant solution was removed via a syringe, followed by drying of the zinc under vacuum using heat from a heat-gun. Compound **123** (1 equiv) was dissolved in DMF (1.42 mL) and flushed under argon then transferred to the zinc via a syringe. The solution was stirred at room temperature 5 min then Pd₂(dba)₃ (0.03 equiv), SPhos (0.06 equiv) and the aromatic bromide or iodide (1.3 equiv) were added to the flask. The reaction mixture was stirred at room temperature overnight. The DMF was evaporated under reduced pressure. The crude reaction mixture was purified by flash chromatography to afford the desired compound.

### (p) General procedure L for Negishi coupling:

Zinc dust (3-5 equiv) was added to a dry sealable tube under argon. A solution of iodine (0.25 equiv) in dry DMF was added. After the brown-purple colour disappeared, TMSCl (0.25 equiv) was added. After 2 min, stirring was stopped and all insolubles were allowed to settle for 10 min. The supernatant was removed via a syringe, followed by drying of the zinc under vacuum using heat from a heat-gun. The tube was cooled down to 0°C before a solution of compound **123** (1 equiv) and a crystal of iodine in dry DMF was added dropwise. After 15 min, Pd₂(dba)₃ (0.025 equiv), SPhos (0.05 equiv) and the aromatic bromide or iodide (1.2 equiv) were added and the resulting mixture was stirred at room temperature overnight. The resulting mixture was filtered, diluted with water and extracted thrice with EtOAc. The collected organic phases were dried over MgSO₄, filtered and concentrated under reduced pressure to give the desired compound. Purification was performed if needed.

### (q) General procedure M for Negishi coupling:

Zinc dust (3-5 equiv) was added to a dry sealable tube under argon. A solution of iodine (0.25 equiv) in dry DMF was added. After the brown-purple colour disappeared, TMSCl (0.25 equiv) was added. After 2 min, stirring was stopped and all insolubles were allowed to settle for 10 min. The supernatant was removed via a syringe, followed by drying of the zinc under vacuum using heat from a heat-gun. A solution of iodine (1-3 crystals) in dry DMF was added to the zinc, followed by Pd₂(dba)₃ (0.025 equiv), SPhos (0.05 equiv) and the aromatic bromide or iodide (1.2 equiv) and the resulting mixture was cooled down to 0°C. A solution of the compound **123** (1 equiv) in dry DMF was added dropwise over 5 h at 0°C. The resulting mixture was stirred at room temperature overnight. The resulting mixture was filtered, diluted with water and extracted thrice with EtOAc. The collected organic phases were dried over MgSO₄, filtered and concentrated under reduced pressure to give the desired compound. Purification was performed if needed.

### (r) General procedure N for amine deprotection:

N-Boc protected compound (1 equiv) was dissolved in CH₂Cl₂ and TFA. The mixture was stirred at room temperature until completion. Solvents were removed under reduced pressure to give the desired compound. Purification was performed if needed.

### (s) General procedure O for amine deprotection:

To a solution of N-Boc protected compound (1 equiv) in MeOH at 0°C was added a solution of 4N HCl in dioxane. The resulting mixture was stirred overnight at room temperatire. Solvents were eremoved under reduced pressure. The residue was partitioned between aq. 0.5N HCl and CH₂Cl₂. The aqueous phase was basified by careful addition of solid NaHCO₃. The basic aqueous layer was extracted thrice with CH₂Cl₂. The collected organic phases were dried over MgSO₄, filtered and concentrated under reduced pressure to give the desired compound. Purification was performed if needed.

### (t) General procedure O' for amine deprotection:

The starting Boc-protected β-amino-ester was put in solution in DCM and 2,6-lutidine (10 equiv). TMSOTf (5 equiv) was added dropwise and the mixture was stirred overnight at room temperature. The reaction was quenched by addition of MeOH. It was washed with 5% NaHCO₃ and the aqueous phase was further extracted with DCM twice. Organic layers were combined and concentrated to dryness i*n vacuo.* The product was purified through silica gel column when needed.

### (u) General procedure P for saponification:

To a solution the ester (1 equiv) in THF/EtOH (2:1 (v/v)) was added 2 M NaOH. The mixture was stirred at room temperature overnight. 1 N HCl was added and the mixture was diluted with water and CH₂Cl₂. The aqueous layer was extracted twice with CH₂Cl₂. The collected organic phases were dried over MgSO₄, filtered and concentrated under reduced pressure to give a white amorphous solid, which was purified if needed.

### (v) General procedure Q for Suzuki-Miyaura coupling:

To a suspension of Na₂CO₃ (3 equiv), the boronic acid (2.5 equiv), and the bromo-compound (1 equiv) in a mixture of toluene and H₂O was added Pd(dppf)₂Cl₂.CH₂Cl₂ (0.2 equiv) and the suspension was heated under microwaves irradiation at 90°C for 15 min or at 100°C for 45 min. The mixture was filtered through a pad of Celite and washed with EtOAc. The residue on Celite was solubilized in MeOH and the filtrate was concentrated under reduced pressure. The residue was dissolved in CH₂Cl₂ and 1N HCl solution resulting to a precipitate which was filtered to give the desired compound, which was purified if needed.

### (w) General procedure R for thio-Michael addition:

To a solution of **134** (0.186 mmol, 1 equiv) in methanol was added a solution of arylthiol (01.2 equiv) and DIPEA (1.5 equiv) in methanol dropwise at 0°C. The solution was stirred at 0°C for 30 min. Then the reaction mixture was diluted with EtOAc, washed twice with aq. K₂CO₃ solution, twice with saturated aq. NH₄Cl solution, then with brine, dried over Na₂SO₄, evaporated under reduced pressure to afford the desired thiol, which was purified if necessary.

### (x) General procedure S for the synthesis of N-hydroxy amidine:

Aminoacetonitrile hydrochloride (1 equiv) was placed in a flask where pyridine was carefully added to obtain a solution, and to this was added acyl chloride (1.05 eq) dropwise over 20 min. After stirring overnight at room temperature, water was carefully added; pyridinium hydrochloride dissolved while the product precipitated as a white solid. The precipitate was collected by filtration and washed with water. If no precipitation was observed, the mixture was extracted three times with dichloromethane. The combined organic layers were dried over MgSO₄, filtered and concentrated under reduced pressure to give the cyanomethylbenzamide as a solid. To a solution of the obtained cyanomethylbenzamide (1.0 equiv) in methanol cooled to 0°C were added hydroxylamine hydrochloride (1.0 equiv) and triethylamine (1.0 equiv), and the mixture was stirred at room temperature or heated to reflux overnight. The mixture was concentrated under reduced pressure. Water was added to the residue and the solid was collected by filtration, washed with water and dried to give the N-hydroxy amidine product, which was purified if necessary.

### (y) General procedure T for the synthesis of oxadiazole:

To a solution of the ester (1 equiv) in CH₂Cl₂ was added carbonyldiimidazole (2.2 equiv). The reaction mixture was stirred at room temperature for 10 min. N-hydroxy amidine (1 equiv) was added and the solution was stirred at room temperature for 16 h. The solvent was removed by evaporation in vacuo, and the residue was dissolved in EtOAc and washed twice with water. The organic layer was dried (MgSO₄) and the solvent was removed by evaporation in vacuo. The residue was solubilized in DMF and the solution was heated to reflux for 4 h. The reaction mixture was cooled to room temperature, the solvent was removed by evaporation in vacuo, and the residue was dissolved in EtOAc and washed twice with water. The organic layer was dried (MgSO₄) and the solvent was removed by evaporation in vacuo to afford theoxadiazole, which was purified if necessary.

### 2. Synthesis of azide precursors

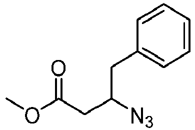

**methyl 3-azido-4-phenylbutanoate (106)**. Compound **106** was obtained according to general procedure H followed by general procedure A as a colorless oil (1.01 g, 71%). Purity: 100%, LC t_{R}= 5.83 min (method B), MS (ESI+): m/z = 192 (M-N₂+H)⁺. ¹H NMR (CDCl₃) δ (ppm): 7.37-7.22 (m, 5H), 4.12-4.03 (m, 1H), 3.71 (s, 3H), 2.90 (dd, J = 7.4 and 13.7 Hz, 1H), 2.84 (dd, J = 6.6 and 13.7 Hz, 1H), 2.55 (dd, J = 5.3 and 16.1 Hz, 1H) 2.47 (dd, J = 8.2 and J = 16.1 Hz, 1H). 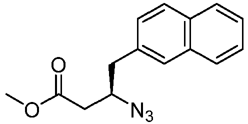 **methyl (R)-3-azido-4-(naphthalen-2-yl)butanoate (107)** Compound **107** was obtained according to general procedure H followed by general procedure A as an orange oil (622 mg, 74%), Purity: 100%, LC t_{R}= 6.54 min (method B), MS (ESI-): m/z = 242 (M-N₂-H)⁻. ¹H NMR (CDCl₃) δ (ppm): 7.86-7.80 (m, 3H), 7.70 (sl, 1H), 7.52-7.45 (m, 2H), 7.37 (dd, J = 1.7 and 8.4 Hz, 1H), 4.24-4.15 (m, 1H), 3.71 (s, 3H), 3.08 (dd, J = 13.7 and 7.3 Hz, 1H), 3.01 (dd, J = 13.7 and 6.6 Hz, 1H), 2.59 (dd, J = 16.1 and 5.4 Hz, 1H), 2.52 (dd, J = 16.1 and 7.9 Hz, 1H).

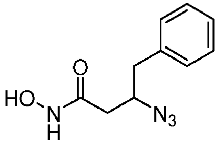

**3-azido-N-hydroxy-4-phenylbutanamide (108).** Compound **108** was obtained according to general procedure E from azido ester **106** as an orange oil (75 mg, 25%), Purity: 100%, LC t_{R}= 4.04 min (method B), MS (ESI-): m/z = 219 (M-H)-, 1H NMR (CD3OD) δ (ppm): 7.38-7.24 (m, 5H), 4.11-4.02 (m, 1H), 2.91 (dd, J = 5.3 Hz and J = 13.8 Hz, 1H), 2.79 (dd, J = 8.4 Hz and J = 13.8 Hz, 1H), 2.34 (dd, J = 4.7 Hz and J = 14.5 Hz, 1H), 2.22 (dd, J = 9.1 Hz and J = 14.5 Hz, 1H). ¹³C NMR (CD₃OD) δ (ppm): 168.2, 137.3, 129.1, 128.2, 126.5, 60.8, 40.3, 37.2.

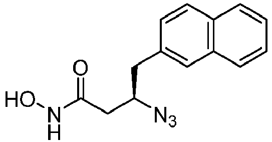

**(3R)-3-azido-N-hydroxy-4-naphthylbutanamide (109).** Compound **109** was obtained according to general procedure E from azido ester **107** as an orange oil (144 mg, 29%), Purity: 90%, LC tR= 5.25 min (method B), MS (ESI+): m/z = 271 [M + H]⁺. 1H NMR (CD3OD) δ (ppm): 7.84-7.80 (m, 3H), 7.74 (sl, 1H), 7.47-7.40 (m, 3H), 4.20 (dddd, J = 4.7, 5.3, 8.2 Hz and 9.1 Hz, 1H), 3.08 (dd, J = 5.3 Hz and J = 13.7 Hz, 1H), 2.98 (dd, J = 8.2 Hz and J = 13.7 Hz, 1H), 2.38 (dd, J = 4.7 Hz and J = 14.6 Hz, 1H) 2.28 (dd, J = 9.1 Hz and J = 14.6 Hz, 1H). ¹³C NMR (CD₃OD) δ (ppm): 168.3, 134.8, 133.6, 132.5, 127.9, 127.8, 127.3, 127.2, 125.8, 125.4, 60.7, 40.4, 37.3.

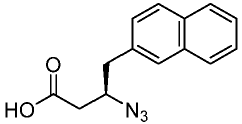

**(R)-3-azido-4-(naphthalen-2-yl)butanoic acid (110)**. Compound **110** was obtained according to general procedure A as a yellow oil (2.1 g, quantitative yield), LC t_{R}= 2.84 min (method A), MS (ESI-): m/z = 254 (M-N₂+H)⁺. ¹H NMR (CDCl₃) δ (ppm): 7.85-7.80 (m, 3H), 7.69 (s, 1H), 7.52-7.45 (m, 2H), 7.37 (dd, J = 1.5 and 8.4 Hz, 1H), 4.22-4.13 (m, 1H), 3.11 (dd, J = 7.2 and 13.8 Hz, 1H), 7.37 (dd, J = 6.8 and 13.5 Hz, 1H), 2.66-2.51 (m, 2H). ¹³C NMR (CDCl₃) δ (ppm): 175.7, 134.0, 133.5, 132.5, 128.5, 128.2, 127.7, 127.6, 127.3, 126.3, 125.9, 59.8, 40.7, 38.5.

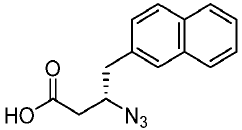

**(S)-3-azido-4-(naphthalen-2-yl)butanoic acid (111).** Compound **111** was obtained according to general procedure A as a yellow solid (902 mg, quantitative yield), LC t_{R}= 3.12 min (method A), MS (ESI-): m/z = 254 (M-H)⁻. ¹H NMR (CDCl₃) δ (ppm): 7.85-7.81 (m, 3H), 7.69 (s, 1H), 7.52-7.45 (m, 2H), 7.37 (dd, J = 1.8 and 8.4 Hz, 1H), 4.22-4.13 (m, 1H), 3.11 (dd, J = 7.2 and 13.5 Hz, 1H), 3.02 (dd, J = 6.6 and 13.5 Hz, 1H), 2.61-2.57 (m, 2H). ¹³C NMR (CDCl₃) δ (ppm): 176.3, 134.0, 133.5, 132.5, 128.5, 128.2, 127.7, 127.6, 127.3, 126.3, 125.9, 59.8, 40.7, 38.6.

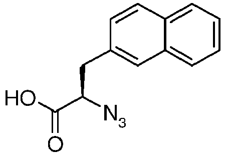

**(2R)-2-Azido-3-naphthalen-2-yl-propionic acid (130)**. Compound **130** was obtained according to general procedure A as a brown solid (764 mg, 79%), Purity: 100%, LC t_{R} = 2.39 min (method A), MS (ESI-): m/z = 240 [M-H]⁻. ¹H NMR (CDCl₃) δ (ppm): 7.83-7.81 (m, 3H), 7.72 (s, 1H), 7.51-7.47 (m, 2H), 7.39-7.37 (dd, J = 1.5 and 8.4 Hz, 1H), 4.25 (dd, J = 4.8 and 8.7 Hz, 1H), 3.40 (dd, J = 4.5 and 13.8 Hz, 1H), 3.19 (dd, J = 9 and 14.1 Hz, 1H). ¹³C NMR (CDCl₃) δ (ppm): 133.5, 133.1, 132.6, 128.6, 128.2, 127.8, 127.1, 126.4, 126.0, 57.0, 37.7.

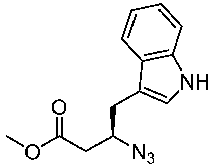

**Methyl (R)-3-azido-4-(1H-indol-3-yl)butanoate (131)**. Compound **131** was obtained according to general procedure H followed by general procedure A as a yellow oil (74 mg, 77%), Purity: 94%, LC t_{R} = 2.83 min (method A), MS (ESI+): m/z = 231 [M-N₂+H]⁺. ¹H NMR (CDCl₃) δ (ppm): 8.09 (br s, 1H), 7.60 (d, J = 8.0 Hz, 1H), 7.37 (d, J = 8.0 Hz, 1H), 7.17 (m, 3H), 4.16 (m, 1H), 3.67 (s, 3H), 3.04 (m, 2H), 2.54 (m, 2H). ¹³C NMR (DMSO-*d₆*) δ (ppm): 172.0, 135.9, 126.5, 123.4, 120.8, 117.7, 118.3, 111.1, 100.2, 51.1, 48.8, 31.7, 21.1. 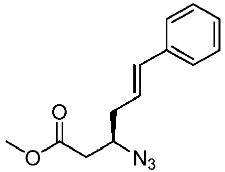 **Methyl (R,E)-3-azido-6-phenylhex-5-enoate (139)**. Compound **139** was obtained according to general procedure H followed by general procedure A as a colorless oil (104 mg, 39%), Purity: 90%, LC t_{R} = 3.23 min (method A), MS (ESI+): m/z = 218 [M-N₂+H]⁺. ¹H NMR (MeOD-*d₄*) δ (ppm): 7.39-7.17 (m, 5H), 6.52 (d, *J* = 15.7 Hz, 1H), 6.23 (dt, *J* = 7.3 and 15.7 Hz, 1H), 4.00-3.91 (m, 1H), 3.68 (s, 3H), 2.63 (dd, J = 4.6 and 16.1 Hz, 1H), 2.52-2.44 (m, 3H).

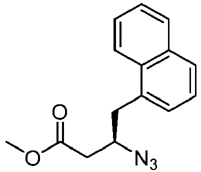

**Methyl (R)-3-azido-4-(naphthalen-1-yl)butanoate (147).** Compound **147** was obtained according to general procedure H followed by general procedure A as a yellow powder (275 mg, quant. yield), Purity: 96%, LC t_{R} = 1.90 min (method A), MS (ESI+): m/z = 242 [M-N₂+H]⁺. ¹H NMR (MeOD-*d₄*) δ (ppm): 8.14 (d, *J* = 8.1 Hz, 1H), 7.90 (d, *J* = 8.1 Hz, 1H), 7.80 (dt, *J* = 4.8 Hz, 1H), 7.59-7.47 (m, 2H), 7.42 (d, *J* = 4.8 Hz, 2H), 4.19 (dddd, *J* = 5.4, 8.5, 10.5 and 13.8 Hz, 1H), 3.67 (s, 3H), 3.40 (dd, *J* = 8.5 Hz, *J* = 13.9 Hz, 2H), 2.60 (m, 2H). ¹³C NMR (MeOD-*d₄*) δ (ppm): 172.9, 135.5, 134.7, 133.3, 129.9, 129.1, 128.9, 127.3, 126.7, 126.4, 124.5, 61.3, 52.3, 40.0, 38.7.

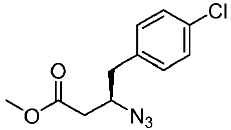

**Methyl (R)-3-azido-4-(4-chlorophenyl)butanoate (148).** Compound **148** was obtained according to general procedure H followed by general procedure A as a yellow powder (59 mg, 93%), Purity: 80%, LC t_{R} = 3.13 min (method A), MS (ESI+): m/z = 226 [M-N₂+H]⁺. 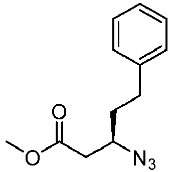 **Methyl (R)-3-azido-5-phenylpentanoate (149).** Compound **149** was obtained according to general procedure H followed by general procedure A as a yellow powder (81 mg, 54%), Purity: 95%, LC t_{R} = 3.18 min (method A), MS (ESI+): m/z = 206 [M-N₂+H]⁺.

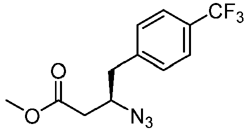

**Methyl (R)-3-azido-5-phenylpentanoate (150).** Compound **150** was obtained according to general procedure H followed by general procedure A as a yellow powder (55 mg, quant. yield), Purity: 90%, LC t_{R} = 3.18 min (method A), MS (ESI+): m/z = 260 [M-N₂+H]⁺.

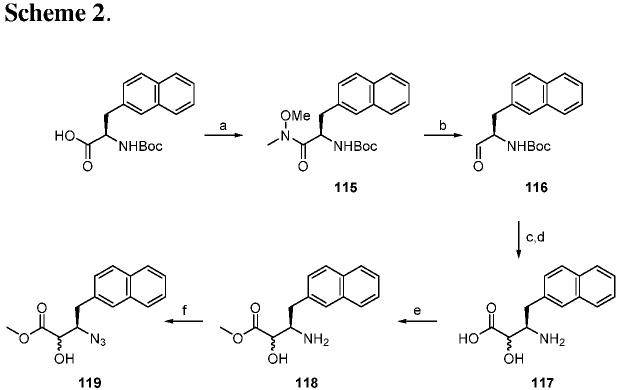

Reagents and conditions: (a) MeNHOMe.HCl, NMM, EDC.HCl, DCM, -15°C to rt, 12h; (b) LiAlH₄, Et₂O, -33 °C to 0°C, 45 min; (c) 1) NaHSO₃, THF, 0°C, 16h, 2) KCN, r. t. ,3h; (d) HCl, dioxane, reflux; (e) SOCl₂, MeOH, rt, 12h; (f) CuSO₄, K₂CO₃, imidazole-1-sulfonyl azide hydrochloride, MeOH, rt, 16 h.

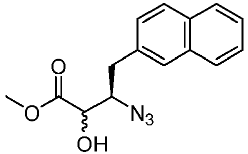

**Methyl (3R)-3-azido-2-hydroxy-4-(naphthalen-2-yl)butanoate (119)**. (2R)-2-(tert-butoxycarbonylamino)-3-(2-naphthyl)propanoic acid (0.960 g, 3.04 mmol) was dissolved with dry CH₂Cl₂ (7 mL, 0.42 M) and the solution was cooled to -15°C. N,O-Dimethylhydroxylamine hydrochloride (0.306 g, 3.13 mmol) was added, followed by N-methylmorpholine (0.34 mL, 3.06 mmol)). After 5 min, 1-(3-methylaminopropyl-3-ethylcarbodiimide hydrochloride (0.578 g, 3.06 mmol)) was added in five portions over 30 min. The reaction was warmed to room temperature overnight. Water (1 mL) was added and the solution was extracted with CH₂Cl₂ (3 × 2.5 mL). The combined organic phases were washed with brine (2.5 mL), dried on Na₂SO₄, and concentrated. The residue was purified by flash chromatography (cyclohexane/AcOEt (100/0 to 70/30) to give **115** as a white solid (1.08 g, 95%). Purity: 100%, LC t_{R} = 3.10 min, MS (ESI+): m/z = 359 [M+H]⁺.¹H NMR (CDCl₃) δ (ppm): 1.36 (s, 9H), 3.00-3.26 (m, 5H), 3.65 (s, 3H), 5.03 (br s, 1H), 5.20 (br s, 1H, NH), 7.31 (d, J = 8.5 Hz, 1H), 7.42-7.46 (m, 2H), 7.62 (s, 1H), 7.76-7.81 (m, 3H). ¹³C NMR (75 MHz, CDCl₃) δ (ppm): 28.2, 28.5, 32.3, 39.2, 51.7, 61.8, 79.8, 125.7, 126.2, 127.8, 127.8, 128.1, 128.3, 132.6, 133.7, 134.3, 172.6, 207.1. In inert atmosphere *tert*-butyl N-[(1R)-2-[methoxy(methyl)amino]-1-(2-naphthylmethyl)-2-oxoethyl]carbamate **(115)** (0.750 g, 2.092 mmol) in anhydrous Et₂O (8.05 mL). The solution was cooled to -33°C and LiAlH₄ (2.5 mL, 2.5 mmol, 1M in THF) was added dropwise, and the reaction mixture was warmed to 0°C. After 45 min, the solution was cooled to - 33°C and 10% aq KHSO₄ (4.03 mL) was added to quench the reaction. After warming to room temperature, 1N HCl (4.03 mL) was added, and the organic phases were separated, the aqueous phase was extracted with EtOAc (2 × 4 mL), and the combined organic phases were dried over MgSO₄, filtered, and concentrated. The residue was purified by flash chromatography on silica gel (cyclohexane/AcOEt: 100/0 to 0/100 during 60 min) to yield quantitatively the aldehyde **116** (0.626 g) as a colorless solid. Purity: 100%, LC t_{R} = 2.95 min and 3.45 min, MS (ESI+): m/z = 300 [M+H]⁺. ¹H NMR (CDCl₃) δ (ppm): 1.43 (s, 9H), 3.29 (d, J = 6.5 Hz, 2H), 4.50 (q, J = 6.5 Hz, J = 13.0 Hz, 1H), 5.06 (br s, 1H, NH), 7.30 (dd, J = 1.8 Hz, J = 8.4 Hz, 1H), 7.47 (ddd, J = 1.9 Hz, J = 5.7 Hz, J = 9.5 Hz, 2H), 7.62 (br s, 1H), 7.76-7.84 (m, 3H).¹³C NMR (CDCl₃) δ (ppm): 28.4, 35.8, 60.9, 126.0, 126.5, 127.4, 127.7, 127.8, 128.2, 128.7, 132.6, 133.4, 133.6, 155.4, 199.6. To a solution of *tert-*butyl N-[(1R)-1-formyl-2-(2-naphthyl)ethyl]carbamate 116 (361 mg, 1.21 mmol) in THF (3.65 mL) at 0°C was added aqueous solution of NaHSO₃ (150.6 mg, 1.447 mmol, in 4.385 ml of water) The mixture was stirred at 0°C overnight. KCN (78.5 mg, 1.21 mmol) was added to the mixture and was stirred for 3h at room temperature. The mixture was extracted with EtOAc (2 × 20 mL) and the collected organic layer were dried over Na₂SO₄, filtered and evaporated to afford the desired compound (468 mg). To a solution of the obtained compound (0.393 mg, 1.206 mmol) in dioxane (4.5 mL) was added a concentrated solution of HCl (6N, 8.9 mL). The solution was gently refluxed and stirred overnight. Then the solution was cold to room temperature and concentrated in vacuo. The crude was purified by preparative HPLC (MeCN/H₂O, 0.1% HCO₂H, gradient from 2% to 100% MeCN over 30 min) to afford the diastereoisomer **117** as a white solid (0.218 g, 74%). LC t_{R} = 1.75 and 1.68 min, MS (ESI+): m/z = 246 [M+H]⁺. **118** was obtained according to general procedure H from **117**. White powder. LC t_{R} = 1.85 and 1.95 min, MS (ESI+): m/z = 260 [M+H]⁺. ¹H NMR (MeOD) δ (ppm): 2.88 (dd, J = 7.9 Hz, J = 13.8 Hz, 1H), 2.96 (dd, J = 7.0 Hz, J = 13.2 Hz, 1H), 3.05 (m, 2H), 3.54 (s, 3H), 3.51-3.57 (m, 1H), 3.60-3.67 (m, 1H), 3.71 (s, 3H), 4.06 (d, J = 2.8 Hz, 1H), 4.28 (d, J = 3.6 Hz, 1H), 7.37-7.50 (m, 6H), 7.72 (s, 1H), 7.73 (s, 1H), 7.79-7.85 (m, 6H). Azide **119** was obtained according to general procedure A from **118.** Transparent oil. LC t_{R} = 3.15 and 3.25 min. ¹H NMR (MeOD) δ (ppm): 3.11 (dd, J = 2.6 Hz, 1H), 3.13 (d, J = 4.0 Hz, 1H), 3.30 (d, J = 2.6 Hz, 2.8H), 3.74 (s, 2.8H), 3.80 (s, 4.4 H), 3.87 (td, J = 2.0 Hz, J = 7.8 Hz, 1.5 H), 3.99 (ddd, J = 3.0 Hz, J = 6.7 Hz , J = 8.0 Hz, 1H), 4.15 (d, J = 1.9 Hz, 1.4H), 4.39 (d, J = 2.2 Hz, 1H), 7.37 (dd, J = 1.7 Hz, J = 8.5 Hz, 1H), 7.42 (dd, J = 1.8 Hz, J = 8.5 Hz, 1.7H), 7.47-7.52 (m, 4H), 7.70 (br s, 1H), 7.77(br s, 1.5H), 7.80-7.85 (m, 6H).

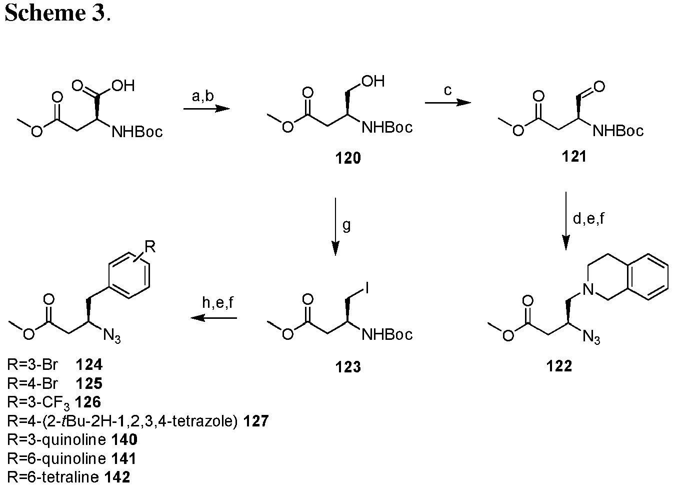

Reagent and conditions: (a) NHS, DCC, EtOAc, 0°C to rt, 16 h; (b) NaBH₄, THF, H₂O, 0°C, 15 min; (c) DMP, DCM, rt, 1.25h; (d) 1,2,3,4-tetrahydroisoquinoline, NaBH(OAc)₃, DCE, 16h, rt; (e) TFA, CH₂Cl₂, rt, 2 h or 4N HCl in dioxane, MeOH, rt, 16h; (f) CuSO₄, K₂CO₃, imidazole-1-sulfonyl azide hydrochloride, MeOH, rt, 16 h; (g) I₂, PPh₃, imidazole, CH₂Cl₂, rt, 1.5 h; (h) *(i)* Zn, iodine, DMF, rt, 30 min, *(ii)* Pd₂(dba)₃, SPhos, aromatic bromide or iodide, rt, 16 h.

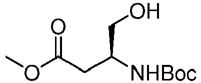

**(S)-3-tert-Butoxycarbonylamino-4-hydroxy-butyric acid methyl ester (120).** To a stirred solution of N-Boc aspartic acid β-methyl ester (4.15 g, 16.8 mmol, 1 equiv) in ethyl acetate (33 mL) was added solid N-hydroxysuccinimide (2.07 g, 18.0 mmol, 1.07 equiv) at 0°C. A solution of dicyclohexylcarbodiimide (3.52 g, 17.1 mmol, 1.02 equiv) in ethyl acetate (33 mL) was added slowly. The reaction was allowed to attain room temperature and stirred overnight. The precipitate of dicyclohexylurea was filtered off, and the filtrate was washed successively with saturated aqueous sodium hydrogen carbonate and brine, dried, and evaporated under reduced pressure to give crude succinimide ester (5.78 g, quantitative yield). Purity: 83%, LC t_{R} = 2.39 min, MS (ESI+): m/z = 345 [M+H]⁺. Sodium borohydride (1.03 g, 27.4 mmol, 1.6 equiv) was dissolved in water (7 mL) and THF (50 mL) at 0°C. A solution of the obtained succinimide ester (5.78 g, 16.8 mmol, 1 equiv) in THF (8 mL) was added dropwise. The reaction was stirred for 15 min. Saturated aqueous ammonium chloride was added to quench the reaction. Extraction with ethyl acetate followed by washing of the combined organic extracts with brine, drying, and evaporation to dryness, afforded the crude product, which was purified by flash chromatography on silica gel (petroleum ether/EtOAc 1:0 to 1:1) to give the desired compound **120** as a colorless oil (3.14 g, 40%). Purity: 50%, LC t_{R} = 1.97 min, MS (ESI+): m/z = 256 [M+Na]⁺. ¹H NMR (CDCl₃) δ (ppm): 5.27 (br s, 1H), 3.99-3.93 (m, 1H), 3.69-3.68 (m, 5H), 2.77 (br s, 1H), 2.63 (d, J = 6.0 Hz, 2H), 1.43 (s, 9H).

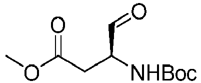

**Methyl (3S)-3-(*tert*-butoxycarbonylamino)-4-oxo-butanoate (121).** A solution of Dess-Martin periodinane (1.15 equiv) in anhydrous CH₂Cl₂ was added at room temperature to a solution of **120** (1 equiv) in anhydrous CH₂Cl₂. The mixture was stirred during 1h15. The mixture was diluted with CH₂Cl₂ and the excess reagent was quenched with a saturated aqueous solution of NaHCO₃ containing Na₂S₂O₃. The phases were separated and the organic layer washed with saturated aqueous solution of NaHCO₃, H₂O, dried over MgSO₄, filtered and evaporated to give the crude product, which was purified by flash chromatography on silica gel (cyclohexante/EtOAc 100/0 to 70/30). Purity: 100%, LC t_{R} = 2.03 min, MS (ESI+): m/z = 232 [M+H]⁺. ¹H NMR (CDCl₃) δ (ppm): 1.45 (s, 9H), 2.82 (dd, J = 4.9 Hz, J = 17.4 Hz, 1H), 2.99 (dd, J = 4.7 Hz, J = 17.4 Hz, 1H), 3.69 (s, 3H), 4.35 (ddd, J = 4.7 Hz, J = 4.9 Hz, J = 6.7 Hz, 1H), 5.62 (d, J = 6.7 Hz), 9.64 (s, 1H). ¹³C NMR (CDCl₃; 75 MHz), δ (ppm): 28.4, 34.4, 52.3, 56.0, 80.7, 155.6, 171.7, 199.3.

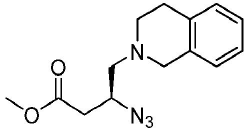

**Methyl (S)-3-azido-4-(3,4-dihydroisoquinolin-2(1H)-yl)butanoate (122).** Compound **121** (1.5 equiv), 1,2,3,4-tetrahydroisoquinoline (1 equiv) and 3A molecular sieves were added in DCE. After stirring 15 min, sodium triacetoxyborohydride (2.2 equiv) was added in small portions. The mixture was stirred at room temperature under argon overnight. The reaction was diluted with CH₂Cl₂ and washed with water. The organic layer was extracted with CH₂Cl₂. Then the combined organic layer was washed with brine, dried over Na₂SO₄, filtered and evaporated to give the crude product, which was purified by flash chromatography on silica gel (cyclohexane/EtOAc 100/0 to 10/90). Azide **122** was obtained according to general procedure N from the obtained product followed by general procedure A. LC t_{R} = 1.77 min, MS (ESI+): m/z = 275 [M+H]⁺. This compound was directly engaged in the next reaction.

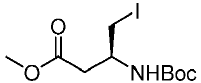

**(R)-3-tert-butoxycarbonylamino-4-iodo-butyric acid methyl ester (123)** Triphenylphosphine (6.02 g, 23.0 mmol, 1.2 equiv), imidazole (1.56 g, 23.0 mmol, 1.2 equiv), and iodine (5.83 g, 23.0 mmol, 1.2 equiv) were added to dry dichloromethane (57 mL) with stirring. Alcohol **29** (4.46 g, 19.1 mmol, 1 equiv) was dissolved in dry dichloromethane (19 mL) under argon and transferred to the reaction mixture via syringe. The reaction was stirred at room temperature for 1.5 h. The mixture was filtered before washing with aqueous sodium thiosulfate solution (1M, 25 mL) and brine (25 mL) and drying (MgSO₄). The dichloromethane was evaporated under reduced pressure, and then the residue was slurried in diethyl ether and filtered through a bed of silica, with washing with additional ether. The filtrate was concentrated under vacuum giving yellow oil. The residue was purified by flash chromatography on silica gel (cyclohexane/EtOAc 1:0 to 1:1) giving a yellow solid (1.64 g, 25%). Purity: 98%, LC t_{R} = 3.05 min, MS (ESI+): m/z = 344 [M + H]⁺. ¹H NMR (CDCl₃) δ (ppm): 1.45 (s, 9H), 2.64 (dd, 1H, J = 6.3 and 16.5 Hz), 2.76 (dd, 1H, J = 5.4 and 16.5 Hz), 3.43 (m, 2H), 3.71 (s, 3H), 3.92 (m, 1H), 5.08 (br d, 1H, J = 6.6).

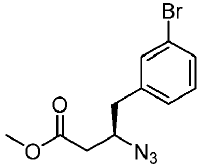

**(R)-3-azido-4-(3-bromo-phenyl)-butyric acid (124).** Compound **124** was obtained according to general procedure J from **123** and 1-bromo-3-iodo-benzene followed by general procedure N and then general procedure A as an orange oil (510 mg, 32% over 3 steps). LC t_{R}= 3.40 min. ¹H NMR (CDCl₃) δ (ppm): 7.43-7.40 (m, 2H), 7.24-7.16 (m, 2H), 4.11-4.02 (m, 1H), 3.73 (s, 3H), 2.83 (d, J = 6.9 Hz, 2H), 2.53-2.51 (m, 2H).

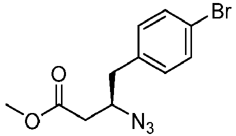

**(R)-3-azido-4-(4-bromo-phenyl)-butyric acid (125).** Compound **125** was obtained according to general procedure J from **123** and 1-bromo-4-iodo-benzene followed by general procedure N and then general procedure A as a yellow oil (3.44 g, 38% over 3 steps). LC t_{R} = 3.62 min. ¹H NMR (CDCl₃) δ (ppm): 7.48 (d, J = 8.1 Hz, 2H), 7.11 (d, J = 8.1 Hz, 2H), 4.05 (t, J = 6.8 Hz, 1H), 3.91 (s, 3H), 2.82 (d, J = 6.8 Hz, 2H), 2.52 (d, J = 6.8 Hz, 2H).

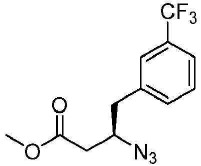

**Methyl (R)-3-azido-4-(3-(trifluoromethyl)phenyl)butanoate (126).** Compound **126** was obtained according to general procedure K from **123** and 1-iodo-3-(trifluoromethyl)benzene followed by general procedure N and then general procedure A as a colorless oil (41 mg, 15% over 3 steps). Purity: 99%, LC t_{R} = 3.20 min.

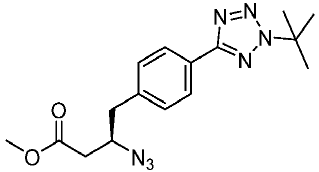

**Methyl (R)-3-azido-4-(4-(2-(tert-butyl)-2H-tetrazol-5-yl)phenyl)butanoate (127).** Compound **127** was obtained according to general procedure K from **123** and 2-tert-butyl-5-(4-iodophenyl)-2H-1,2,3,4-tetrazole followed by general procedure N and then general procedure A as a colorless oil (41 mg, 26% over 3 steps). Purity: 99%, LC t_{R} = 3.06 min. MS (ESI+): m/z = 344 [M + H]⁺.

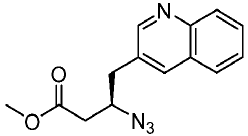

**Methyl (3R)-3-azido-4-(3-quinolyl)butanoate (140).** Compound **140** was obtained according according to general procedure M from **123** and 3-bromoquinoline followed by general procedure O and then general procedure A as a colorless solid (69 mg, 19% over 3 steps). Purity: 92%, LC t_{R} = 2.48 min, MS (ESI+): m/z = 271 [M+H]⁺.

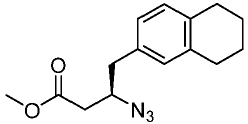

**Methyl (3R)-3-azido-4-tetralin-6-yl-butanoate (141).** Compound **141** was obtained according according to general procedure L from **123** and 6-bromotetraline followed by general procedure O and then general procedure A as a yellowish oil (44 mg, 12% over 3 steps). LC tᵣ = 3.42 min, MS (ESI+): m/z = 246 [M-N₂+H]⁺.

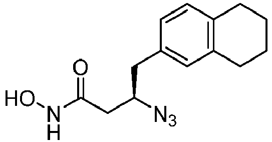

**(3R)-3-azido-4-(6-tetralinyl)butanehydroxamic acid (142).** Compound **142** was obtained according to general procedure L from cyclohexyl (3S)-3-(tert-butoxycarbonylamino)-4-iodo-butanoate and 6-bromotetraline followed by general procedure O and then general procedures G' and A' as a brownish wax (60 mg, 38% over 4 steps). LC tᵣ = 2.53 min, MS (ESI-): m/z = 273 [M-H]⁻. ¹H NMR, CDCl₃ + 1% TMS, δ (ppm) : 7.01-6.96 (m, 1H), 6.93-6.81 (m, 2H), 4.10-3.99 (m, 1H), 2.83-2.67 (m, 6H), 2.36 (dd, *J* = 14.7, 3.6Hz, 1H), 2.21 (dd, *J* = 14.7, 9.3Hz, 1H), 1.80-1.72 (m, 4H). ¹³C NMR, CDCl₃ + 1% TMS, δ (ppm) : 168.8, 137.5, 135.9, 133.5, 130.0, 129.4, 126.4, 60.3, 40.1, 37.5, 29.3, 29.0, 23.2, 23.1.

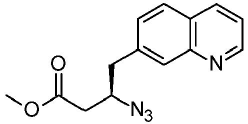

**Methyl (3R)-3-azido-4-(7-quinolyl)-butanoate (151).** Compound **151** was obtained according to general procedure L from **123** and 7-bromoquinoline followed by general procedure O and then general procedure A" as a colourless solid (23 mg, 2.7% over 3 steps). LC tᵣ = 2.40 min, MS (ESI+): m/z = 271 [M + H]⁺. ¹H NMR (CDCl₃+1% TMS) δ (ppm): 8.91 (s, 1H), 8.15 (br d, *J* = 8.4 Hz, 1H), 7.96 (s, 1H), 7.80 (br d, *J* = 8.4 Hz, 1H), 7.45 (dd, *J* = 8.4 and 1.7 Hz, 1H), 7.41-7.37 (m, 1H), 4.26-4.15 (m, 1H), 3.71 (s, 3H), 3.07 (dd, *J* = 11.2 and 7.1 Hz, 2H), 2.59-2.55 (m, 2H).

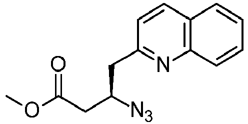

**Methyl (3R)-3-azido-4-(2-quinolyl)-butanoate (152).** Compound **152** was obtained according to general procedure L from **123** and 6-iodoquinoline followed by general procedure *O* and then general procedure A" as a dark red oil (71 mg, 29% over 3 steps). LC tᵣ = 2.62 min, MS (ESI+): m/z = 271 [M + H]⁺. ¹H NMR (CDCl₃ + 1% TMS) δ (ppm): 8.10 (d, *J* = 8.5Hz, 1H), 8.06 (ddd, *J* = 8.5 and 1.6 and 1.1 Hz, 1H), 7.79 (dd, *J* = 8.1 and 1.2 Hz, 1H), 7.70 (ddd, *J* = 8.4 and 6.9 and 1.5 Hz, 1H), 7.51 (ddd, *J* = 8.1 and 6.9 and 1.1 Hz, 1H), 7.32 (d, *J* = 8.4 Hz, 1H), 4.64-4.54 (m, 1H), 3.71 (s, 3H), 3.22 (d, *J* = 6.9 Hz, 2H), 2.71 (dd, *J* = 16.2 and 4.5 Hz, 1H), 2.60 (dd, *J* = 16.2 and 8.8 Hz, 1H). ¹³C NMR (CDCl₃ + 1% TMS) δ (ppm): 171.1, 157.6, 147.9, 136.6, 129.7, 129.1, 127.6, 127.0, 126.3, 121.9, 58.6, 51.9, 43.3, 39.1.

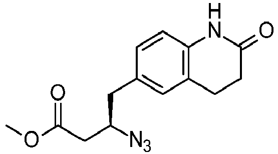

**Methyl (3R)-3-azido-4-(6-quinolyl)-butanoate (153).** Compound **153** was obtained according to general procedure L from **123** and 6-iodoquinoline followed by general procedure O' and then general procedure B as a light beige wax (29 mg, 19% over 3 steps). LC tᵣ = 2.25 min, MS (ESI+): m/z = 289 [M+H]⁺. ¹H NMR (CDCl₃ + 1% TMS) δ (ppm): 9.26 (br s, 1H), 7.04-7.00 (m, 2H), 6.81 (d, *J* = 8.5 Hz, 1H), 4.08-3.98 (m, 1H), 3.72 (s, 3H), 2.96 (dd, *J* = 8.0 and 7.0Hz, 2H), 2.80 (d, *J* = 7.0 Hz, 2H), 2.65 (d, *J* = 8.0 Hz, 1H), 2.63 (d, *J* = 9.0 Hz, 1H), 2.54 (dd, *J* = 16.1 and 5.4Hz, 1H), 2.47 (dd, *J* = 16.2 and 8.0Hz, 1H). ¹³C NMR (CDCl₃ + 1% TMS) δ (ppm): 172.2, 171.1, 136.4, 131.5, 128.9, 128.4, 123.9, 115.8, 60.2, 52.0, 40.0, 38.7, 30.6, 25.3.

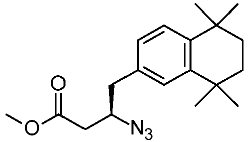

**Methyl (3R)-3-azido-4-(6-(1,1,4,4-tetramethyl-2,3-dihydronaphthyl)-butanoate (154).** Compound **154** was obtained according to general procedure L from **123** and 6-iodo-1,1,4,4-tetramethyl-1,2,3,4-tetrahydronaphthalene followed by general procedure S and then general procedure A' as a yellowish thick oil (159 mg, 56% over 3 steps). LC tᵣ = 3.65 min, MS (ESI+): m/z = 302 [M-N₂+H]⁺. ¹H NMR, (CDCl₃ + 1% TMS) δ (ppm): 7.24 (d, *J* = 8.1 Hz, 1H), 7.13 (d, *J* = 1.9 Hz, 1H), 6.96 (dd, *J* = 8.1 and 1.9 Hz, 1H), 4.08-3.99 (m, 1H), 3.69 (s, 3H), 2.85 (dd, *J* = 13.8 and 7.3 Hz, 1H), 2.76 (dd, *J* = 13.8 and 6.6 Hz, 1H), 2.53 (dd, *J* = 16.1 and 5.0 Hz, 1H), 2.45 (dd, *J* = 16.1 and 8.5 Hz, 1H), 1.67 (s, 4H), 1.27 (s, 6H), 1.26 (s, 6H). ¹³C NMR (CDCl₃ + 1% TMS) δ (ppm): 171.2, 145.1, 143.5, 133.5, 127.4, 126.8, 126.5, 60.1, 51.9, 40.3, 38.7, 35.1, 35.0, 34.2, 34.0, 31.8 (4C).

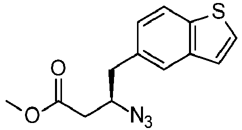

**Methyl (3R)-3-azido-4-(5-benzothiophene)-butanoate (155).** Compound **155** was obtained according to general procedure L from **123** and 5-iodo-benzothiophene followed by general procedure O' and then general procedure A' as a yellow thick oil (120 mg, 49% over 3 steps). LC tᵣ = 3.02 min, MS (ESI+): m/z = 248 [M-N₂+H]⁺. ¹H NMR (CDCl₃ + 1% TMS) δ (ppm): 7.82 (d, *J* = 8.3 Hz, 1H), 7.66 (d, *J* = 1.4 Hz, 1H), 7.44 (d, *J* = 5.4 Hz, 1H), 7.29 (d, *J* = 5.4 Hz, 1H), 7.20 (dd, *J* = 8.3 and 1.6 Hz, 1H), 4.17-4.07 (m, 1H), 3.69 (s, 3H), 3.01 (dd, *J* = 13.9 and 7.5 Hz, 1H), 2.94 (dd, *J* = 13.9 and 6.7 Hz, 1H), 2.55 (dd, *J* = 16.2 and 5.3 Hz, 1H), 2.48 (dd, *J* = 16.2 and 8.1 Hz, 1H). ¹³C NMR (CDCl₃ + 1% TMS) δ (ppm): 171.1, 140.0, 138.5, 132.8, 127.0, 125.7, 124.2, 123.6, 122.7, 60.3, 51.9, 40.5, 38.6.

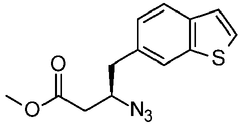

**Methyl (3R)-3-azido-4-(6-benzothiophene)-butanoate (156).** Compound **156** was obtained according to general procedure L from **123** and 6-iodo-benzothiophene followed by general procedure O' and then general procedure A' as a colourless oil (69 mg, 26% over 3 steps). LC tᵣ = 3.00 min, MS (ESI+): m/z = 248 [M-N₂+H]⁺. ¹H NMR (CDCl₃ + 1% TMS) δ (ppm): 7.75 (d, *J* = 8.2 Hz, 1H), 7.71-7.70 (m, 1H), 7.39 (d, *J* = 5.4 Hz, 1H), 7.29 (dd, *J* = 5.4 and 0.6 Hz, 1H), 7.21 (dd, *J* = 8.2 and 1.6 Hz, 1H), 4.16-4.06 (m, 1H), 3.68 (s, 3H), 2.99 (dd, *J* = 13.9 and 7.5 Hz, 1H), 2.92 (dd, *J* = 13.8 and 6.7 Hz, 1H), 2.54 (dd, *J* = 16.2 and 5.4 Hz, 1H), 2.47 (dd, *J* = 16.2 and 8.0 Hz, 1H). ¹³C NMR (CDCl₃ + 1% TMS) δ (ppm): 171.0, 140.2, 138.6, 132.9, 126.3, 125.7, 123.7, 123.6, 123.0, 60.3, 51.9, 40.6, 38.6.

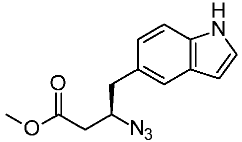

**Methyl (3R)-3-azido-4-(5-indole)-butanoate (157).** Compound **157** was obtained according to general procedure L from **123** and 5-iodoindole followed by general procedure O' and then general procedure A' as a brown solid (86 mg, 29% over 3 steps). LC tᵣ = 2.67 min, MS (ESI+): m/z = 231 [M-N₂+H]⁺. ¹H NMR (CDCl₃ + 1% TMS) δ (ppm): 8.23 (br s, 1H), 7.45 (br s, 1H), 7.27 (d, *J* = 8.3 Hz, 1H), 7.13 (t, *J* = 2.8 Hz, 1H), 7.00 (dd, *J* = 8.3 and 1.5 Hz, 1H), 6.49-6.47 (m, 1H), 4.14-4.04 (m, 1H), 3.66 (s, 3H), 3.01 (dd, *J* = 13.7 and 7.1 Hz, 1H), 2.87 (dd, *J* = 13.7 and 7.1 Hz, 1H), 2.54 (dd, *J* = 16.1 and 4.6 Hz, 1H), 2.44 (dd, *J* = 16.2 and 8.9 Hz, 1H). ¹³C NMR (CDCl₃ + 1% TMS) δ (ppm): 171.5, 134.9, 128.2, 127.8, 124.8, 123.3, 121.2, 111.2, 102.2, 60.7, 51.9, 40.7, 38.5.

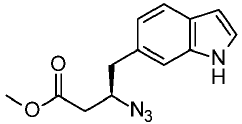

**Methyl (3R)-3-azido-4-(6-indole)-butanoate (158).** Compound **158** was obtained according to general procedure L from **123** and 6-iodoindole followed by general procedure O' and then general procedure A' as a brownish-red solid (67 mg, 24% over 3 steps). LC tᵣ = 2.72 min, MS (ESI+): m/z = 259 [M + H]⁺. ¹H NMR (CDCl₃ + 1% TMS) δ (ppm): 8.19 (br s, 1H), 7.57 (d, *J* = 8.1 Hz, 1H), 7.17 (br s, 1H), 7.12 (dd, *J* = 3.1 and 2.4 Hz, 1H), 6.95 (dd, *J* = 8.1 and 1.4 Hz, 1H), 6.51-6.49 (m, 1H), 4.14-4.05 (m, 1H), 3.66 (s, 3H), 3.01 (dd, *J* = 13.7 and 7.1 Hz, 1H), 2.88 (dd, *J* = 13.7 and 6.9 Hz, 1H), 2.53 (dd, *J* = 16.1 and 4.7 Hz, 1H), 2.44 (dd, *J* = 16.2 and 8.7 Hz, 1H). ¹³C NMR (CDCl₃ + 1% TMS) δ (ppm): 171.4, 136.1, 130.4, 128.9, 124.5, 121.3, 120.8, 111.7, 102.4, 60.6, 51.9, 40.8, 38.6.

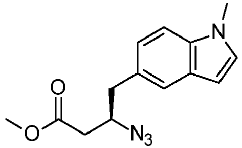

**Methyl (3R)-3-azido-4-(5-(N-methyl)indole)-butanoate (159).** Compound **159** was obtained according to general procedure L from **123** and 5-iodoindole followed by general procedure O' and then general procedure A' as a yellow thick oil (25 mg, 2.6% over 3 steps). LC tᵣ = 3.05 min, MS (ESI+): m/z = 273 [M + H]⁺.

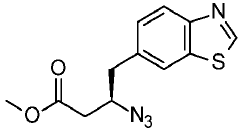

**Methyl (3R)-3-azido-4-(6-(1,3-benzothiazole))-butanoate (160).** Compound **160** was obtained according to general procedure L from **123** and 6-bromo-1,3-benzothiazole followed by general procedure O and then general procedure A' as a colourless wax (15 mg, 8.3% over 3 steps). LC tᵣ = 2.53 min, MS (ESI+): m/z = 277 [M + H]⁺. ¹H NMR (CDCl₃+1% TMS), δ (ppm): 8.98 (s, 1H), 8.10 (d, *J* = 8.4 Hz, 1H), 7.84 (s, 1H), 7.39 (d, *J* = 8.4 Hz, 1H), 4.19-4.09 (m, 1H), 3.71 (s, 3H), 3.01 (d, *J* = 7.0 Hz, 2H), 2.55 (d, *J* = 7.6 Hz, 2H).

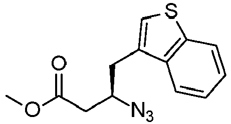

**Methyl (3R)-3-azido-4-(3-benzothiophene)-butanoate (161).** Compound **161** was obtained according to general procedure L from **123** and 3-iodobenzothiophene followed by general procedure O and then general procedure A' as a light-brown thick wax (91 mg, 15% over 3 steps). LC tᵣ = 3.03 min, MS (ESI+): m/z = 248 [M-N₂+H]⁺. ¹H NMR (CDCl₃ + 1% TMS) δ (ppm): 7.88-7.84 (m, 1H), 7.79-7.75 (m, 1H), 7.43-7.32 (m, 2H), 7.25 (s, 1H), 4.20 (quintet, *J* = 6.9 Hz, 1H), 3.69 (s, 3H), 3.10 (dd, *J* = 6.9 and 0.7 Hz, 2H), 2.56 (d, *J* = 6.9 Hz, 2H). ¹³C NMR (CDCl₃ + 1% TMS) δ (ppm) : 171.0, 140.4, 138.5, 131.3, 124.5, 124.4, 124.2, 123.0, 121.4, 58.5, 52.0, 38.9, 33.3.

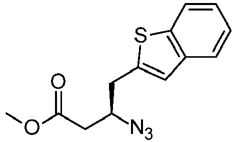

**Methyl (3R)-3-azido-4-(2-benzothiophene)-butanoate (162).** Compound **162** was obtained according to general procedure L from **123** and 2-iodobenzothiophene followed by general procedure O' and then general procedure A' as a yellowish thick oil (17 mg, 7.0% over 3 steps). LC tᵣ = 3.05 min, MS (ESI+): m/z = 248 [M-N₂+H]⁺. ¹H NMR (CDCl₃ + 1% TMS) δ (ppm): 7.80-7.76 (m, 1H), 7.72-7.68 (m, 1H), 736-7.26 (m, 2H), 7.12 (d, *J* = 0.6 Hz, 1H), 4.23-4.14 (m, 1H), 3.72 (s, 3H), 3.22-3.08 (m, 2H), 2.63 (dd, *J* = 16.3 and 5.0 Hz, 1H), 2.54 (dd, *J* = 16.3 and 8.3 Hz, 1H). ¹³C NMR (CDCl₃ + 1% TMS) δ (ppm): 170.9, 139.8, 139.8, 139.6, 124.4, 124.1, 123.4, 123.2, 122.2, 59.5, 52.1, 38.6, 35.6. 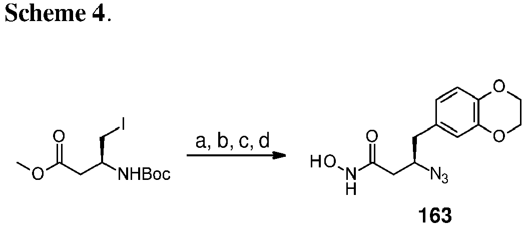 Reagent and conditions: (a) *(i)* Zn, iodine, TMSCl, DMF, rt, 2 min, *(ii)* Pd₂(dba)₃, SPhos, aromatic bromide or iodide, iodine, DMF, rt, 16 h; (b) 4N HCl in dioxane, MeOH, 0°C to rt, 16h; (c) NH₂OH, KCN, H₂O, MeOH, rt, 16h; (d) ZnCl₂, K₂CO₃, DIPEA, imidazole-1-sulfonyl azide hydrochloride, MeOH, 0°C to rt, 16 h.

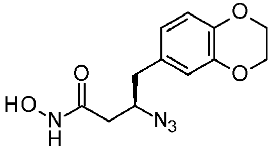

**(3R)-3-azido-4-(6-(1,4-benzodioxyl))butanehydroxamic acid (163).** Compound **163** was obtained according to general procedure L from **123** and 6-iodobenzodioxane followed by general procedure O and then general procedure G' and then general procedure A' as a yellowish wax (88 mg, 72% over 4 steps). LC tᵣ = 1.97 min, MS (ESI-): m/z = 277 [M-H]⁻.

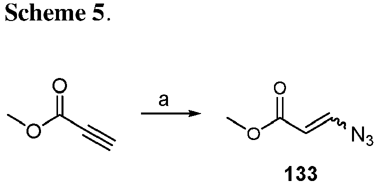

Reagent and conditions: (a) Borax, KH₂PO₄, rt, ultrasound, 1 h.

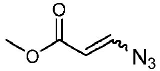

**Methyl 3-azidoacrylate (133).** A solution of sodium azide (50 mmol, 5 equiv), borax (15 mmol, 1.5 equiv) and KH₂PO₄ (15 mmol, 1.5 equiv) in water (70 mL) was prepared in a crystallizer. Methyl prop-2-ynoate (10 mmol, 1 equiv) was added and the crystallizer was put into the ultrasound and sonicated at room temperature for 1 h. Then the mixture was extracted with Et₂O, dried with Na₂SO₄ and concentrated at 25°C under reduced pressure to give the desired compound as white crystals.

### 3. Triazole formation

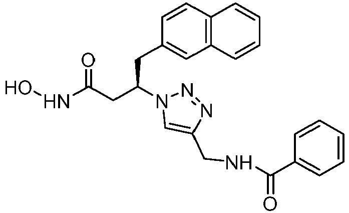

**N-[1-((R)-2-Hydroxycarbamoyl-1-naphthalen-2-ylmethyl-ethyl)-1H-[1,2,3]triazol-4-ylmethyl]-benzamide (1)**. Compound **1** was obtained according to general procedure B from azide **109** and N-(prop-2-yn-1-yl)benzamide as a beige solid (24 mg, 51%), Purity: 95%, mp 192.8-194.0 °C, LC t_{R} = 3.10 min, MS (ESI+): m/z = 430 [M + H]⁺. ¹H NMR (DMSO-*d₆*) δ (ppm): 10.51 (s, 1H), 8.95 (t, J = 5.9 Hz, 0.8H), 8.79 (s, 0.5H), 7.88-7.67 (m, 5H), 7.56-7.35 (m, 7H), 7.15 (dd, J = 1.3 and 8.6 Hz, 1H), 5.28-5.26 (m, 1H), 4.42 (d, J = 5.6 Hz, 2H), 3.32 (m, 2H), 2.77 (dd, J = 8.8 and 15.0 Hz, 1H), 2.65 (dd, J = 5.4 and 15.0 Hz, 1H). ¹³C NMR (DMSO-*d₆*) δ (ppm): 166.5, 166.0, 145.1, 135.0, 134.6, 133.3, 132.3, 131.7 (2C), 128.7 (2C), 128.2, 127.9 (2C), 127.7 (3C), 126.5, 126.0, 122.9, 59.2, 41.4, 37.6, 35.2. HRMS m/z calculated for C₂₄H₂₄N₅O₃ [M + H]⁺ 430.1879, found 430.1884.

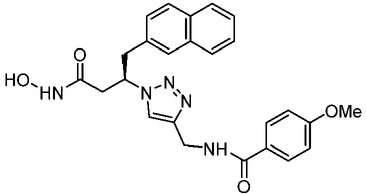

**N-[1-((R)-2-Hydroxycarbamoyl-1-naphthalen-2-ylmethyl-ethyl)-1H-[1,2,3]triazol-4-ylmethyl]-4-methoxy-benzamide (2).** Compound **2** was obtained according to general procedure B from azide **109** and 4-methoxy-N-(prop-2-yn-1-yl)benzamide as a beige solid (43 mg, 68%), Purity: 96%, mp 178.3-179.6°C, LC t_{R} = 2.33 min, MS (ESI+): m/z = 460 [M + H]⁺. ¹H NMR (DMSO-*d₆*) δ (ppm): 10.50 (s, 1H), 8.80 (m, 2H), 7.86-7.69 (m, 6H), 7.47-7.39 (m, 3H), 7.15 (d, J = 8.6 Hz, 1H), 6.98 (d, J = 8.6 Hz, 2H), 5.26 (m, 1H), 4.40 (d, J = 5.3 Hz, 2H), 3.81 (s, 3H), 3.31 (m, 2H), 2.77 (dd, J = 9.5 and 15.1 Hz, 1H), 2.65 (dd, J = 5.2 and 15.1 Hz, 1H). ¹³C NMR (DMSO-*d₆*) δ (ppm): 166.0 (2C), 162.1, 145.2, 135.0, 133.3, 132.3, 129.6 (2C), 128.2 (2C), 127.9 (2C), 127.8, 126.8, 126.5, 126.0, 123.0, 113.9 (2C), 59.2, 55.8, 41.4, 37.6, 35.1. HRMS m/z calculated for C₂₅H₂₆N₅O₄ [M + H]⁺ 460.1985, found 460.1977.

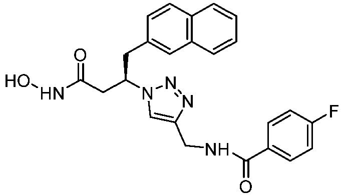

**4-Fluoro-N-[1-(R-2-hydroxycarbamoyl-1-naphthalen-2-ylmethyl-ethyl)-1H-[1,2,3]triazol-4-ylmethyl]-benzamide (3).** Compound **3** was obtained according to general procedure B from azide **109** and 4-fluoro-N-(prop-2-yn-1-yl)benzamide as a beige solid (36 mg, 57%), Purity: 96%, mp 183.6-184.1°C, LC t_{R} = 2.42 min, MS (ESI+): m/z = 448 [M + H]⁺. ¹H NMR (DMSO-*d₆*) δ (ppm): 10.50 (s, 1H), 8.99 (t, 0.9H, J = 5.4 Hz, 1H), 8.80 (s, 1H), 7.93-7.87 (m, 3H), 7.78-7.68 (m, 3H), 7.45-7.36 (m, 3H), 7.29 (t, J = 8.8 Hz, 2H), 7.14 (d, J = 8.9 Hz, 1H), 5.28-5.24 (m, 1H), 4.41 (d, J = 5.4 Hz, 2H), 3.31 (m, 2H), 2.77 (dd, J = 8.7 and 14.9 Hz, 1H), 2.66 (dd, J = 5.4 and 14.9 Hz, 1H). ¹³C NMR (DMSO-*d₆*) δ (ppm): 165.5, 165.0, 163.9 (d, J_{C-F} = 247 Hz), 144.5, 135.0, 132.9, 132.3, 131.8, 130.7, 130.6, 130.0, 129.9, 127.7, 127.4 (2C), 127.3, 126.0, 125.6, 118.0, 115.2 (d, J_{C-F} = 22 Hz), 58.8, 40.9, 37.1, 34.8. ¹⁹F NMR (DMSO-*d₆*) δ (ppm): -109.99. HRMS m/z calculated for C₂₄H₂₃FN₅O₃ [M + H]⁺ 448.1785, found 448.1801.

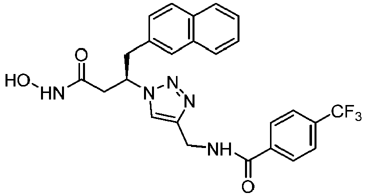

**N-[1-(R-2-Hydroxycarbamoyl-1-naphthalen-2-ylmethyl-ethyl)-1H-[1,2,3]triazol-4-ylmethyl]-4-trifluoromethyl-benzamide (4).** Compound **4** was obtained following general procedure B from azide **109** and N-(prop-2-yn-1-yl)-4-(trifluoromethyl)benzamide as a beige solid (36 mg, 40%), Purity: 95%, mp 198.9-200.4°C, LC t_{R} = 2.62 min, MS (ESI+): m/z = 498 [M + H]⁺. ¹H NMR (DMSO-*d₆*) δ (ppm): 10.51 (s, 1H), 9.20 (s, 1H), 8.80 (s, 1H), 8.02 (m, 2H), 7.88-7.83 (m, 3H), 7.76-7.65 (m, 3H), 7.46-7.38 (m, 3H), 7.12 (d, J = 8.8 Hz, 1H), 5.27 (m, 1H), 4.44 (d, J = 3.9 Hz, 2H), 3.32 (m, 2H), 2.82-2.64 (m, 2H). ¹³C NMR (DMSO-*d₆*) δ (ppm): 166.0, 165.3, 144.7, 138.3, 134.9, 133.3, 132.2, 131.6 (d, J_{C-F} = 30 Hz), 128.7, 128.2, 127.8, 127.7, 126.0, 125.8, 124.5 (q, J_{C-F} = 271 Hz), 123.1, 59.3, 41.4, 37.6, 35.3. HRMS m/z calculated for C₂₅H₂₂O₃ N₅F₃ [M + H]⁺ 498.1753, found 498.1749.

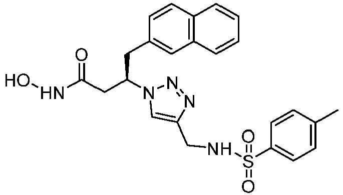

**(R)-N-Hydroxy-4-naphthalen-2-yl-3-{4-[(toluene-4-sulfonylamino)-methyl]-[1,2,3]triazol-1-yl}-butyramide (5).** Compound **5** was obtained according to general procedure B from azide **109** and 4-methyl-N-(prop-2-yn-1-yl)benzenesulfonamide as a beige solid (20 mg, 35%), Purity: 95%, mp 115.8-117.2°C, LC t_{R} = 2.55 min, MS (ESI+): m/z = 480 [M + H]⁺. ¹H NMR (DMSO-*d₆*) δ (ppm): 10.52 (s, 1H), 8.81 (s, 1H), 8.00 (t, J = 6.0 Hz, 1H), 7.85-7.75 (m, 4H), 7.65 (d, J = 8.2 Hz, 2H), 7.47-7.42 (m, 3H), 7.33 (d, J = 8.2 Hz, 2H), 7.14 (d, J = 7.4 Hz, 1H), 5.25-5.21 (m, 1H), 3.92 (d, J = 5.8 Hz, 2H), 3.27 (m, 2H), 2.71 (dd, J = 8.9 and 15.2 Hz, 1H), 2.69 (dd, J = 5.7 and 15.2 Hz, 1H), 2.34 (s, 3H). ¹³C NMR (DMSO-*d₆*) δ (ppm): 165.9, 143.4, 143.1, 137.9, 135.0, 133.4, 132.3, 130.0, 128.3, 127.9, 127.7, 127.1, 126.6, 126.1123.2, 59.4, 41.3, 38.5, 37.6, 21.4. HRMS m/z calculated for C₂₄H₂₅N₅O₄S [M + H]⁺ 480.1706, found 480.1696.

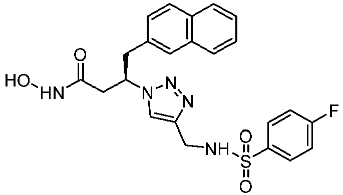

**(R)-3-[4-[(4-Fluoro-benzenesulfonylamino)-methyl]-[1,2,3]triazol-1-yl}-N-hydroxy-4-naphthalen-2-yl-butyramide (6).** Compound **6** was obtained according to general procedure B from azide **109** and 4-fluoro-N-(prop-2-yn-1-yl)benzenesulfonamide as a beige solid (40 mg, 60%), Purity: 95%, mp 157.1-158.7°C, LC t_{R} = 2.54 min, MS (ESI+): m/z = 484 [M + H]⁺. ¹H NMR (DMSO-*d₆*) δ (ppm): 7.83-7.75 (m, 6H), 7.54-7.33 (m, 5H), 7.15 (d, J = 8.7 Hz, 1H), 5.27-5.23 (m, 1H), 3.95 (s, 2H), 3.28-3.26 (m, 2H), 2.75 (dd, J = 9.1 and14.9 Hz, 1H), 2.63 (dd, J = 5.6 and 14.9 Hz, 1H). ¹³C NMR (DMSO-*d₆*) δ (ppm): 166.0, 164.5 (d, J_{C-F} = 247 Hz), 137.1, 135.0, 133.4, 132.3, 130.1(2C), 130.0, 128.3, 127.9 (3C), 127.7, 126.5, 126.1, 123.3, 116.7 (d, J_{C-F} = 22 Hz, 2C), 59.3, 41.3, 38.4, 37.5. HRMS m/z calculated for C₂₃H₂₃FN₅O₄S [M + H]⁺ 484.1455, found 484.1457.

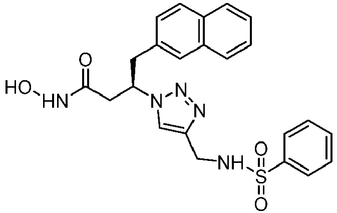

**(R)-3-[4-(Benzenesulfonylamino-methyl)-[1,2,3]triazol-1-yl]-N-hydroxy-4-naphthalen-2-yl-butyramide** (7). Compound 7 was obtained according to general procedure B from azide **109** and N-(prop-2-yn-1-yl)benzenesulfonamide as a beige solid (25 mg, 47%), Purity: 96%, mp 137.4-138.8°C, LC t_{R} = 2.47 min, MS (ESI+): m/z = 466 [M + H]⁺. ¹H NMR (DMSO-*d₆*) δ (ppm): 10.51 (s, 1H), 8.80 (s, 1H), 8.09 (m, 1H), 7.82-7.76 (m, 6H), 7.57-7.46 (m, 6H), 7.14 (d, J = 8.1 Hz, 1H), 5.24 (m, 1H), 3.93 (m, 2H), 3.28 (3, 2H), 2.70-2.66 (m, 2H). ¹³C NMR (DMSO-*d₆*) δ (ppm): 165.9, 140.7, 135.0, 133.4, 132.9, 132.3, 129.6, 128.3, 127.9, 127.7, 127.0, 126.6, 126.1, 59.3, 41.3, 38.6, 37.6. HRMS m/z calculated for C₂₃H₂₃N₅O₄S [M + H]⁺ 466.1549, found 466.1550.

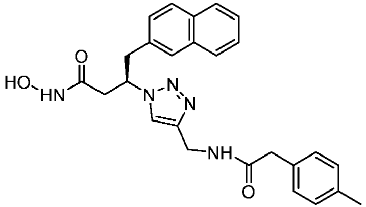

**(R)-N-Hydroxy-4-naphthalen-2-yl-3-{4-[(2-p-tolyl-acetylamino)-methyl]-[1,2,3]triazol-1-yl}-butyramide (8).** Compound **8** was obtained according to general procedure B from azide **109** and N-(prop-2-yn-1-yl)-2-(p-tolyl)acetamide as a yellow oil (21 mg, 55%), Purity: 90%, LC t_{R} = 2.52 min, MS (ESI+): m/z = 458 [M + H]⁺.¹H NMR (DMSO-*d₆*) δ (ppm): 10.52 (s, 1H), 8.81 (s, 1H), 8.41 (t, J = 5.0 Hz, 1H), 7.85-7.74 (m, 4H), 7.49-7.44 (m, 3H), 7.15-7.04 (m, 5H), 5.26 (m, 1H), 4.20 (d, J = 5.2 Hz, 2H), 3.31-3.25 (m, 4H), 2.76 (dd, J = 9.3 and 15.3 Hz, 1H), 2.65 (dd, J = 5.4 and 15.3 Hz, 1H), 2.24 (s, 3H). ¹³C NMR (DMSO-*d₆*) δ (ppm): 170.7, 166.1, 144.8, 135.9, 135.1, 133.7, 133.4, 132.4, 129.4, 129.3 (2C), 128.3, 127.9 (3C), 127.7 (2C), 126.6, 126.2, 122.9, 59.3, 42.2, 41.6, 37.8, 34.7, 21.1. HRMS m/z calculated for C₂₆H₂₈N₅O₃ [M + H]⁺ 458.2192, found 458.2212.

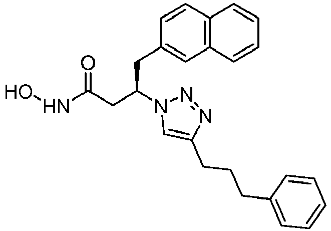

**(R)-3-{4-[(4-Fluoro-benzenesulfonylamino)-methyl]-[1,2,3]triazol-1-yl}-N-hydroxy-4-naphthalen-2-yl-butyramide (9).** Compound **9** was obtained according to general procedure B from azide **109** and pent-4-yn-1-ylbenzene as a beige solid (40 mg, 60%), Purity: 95%, mp 157.1-158.7°C, LC t_{R} = 2.54 min, MS (ESI+): m/z = 484 [M + H]⁺. ¹H NMR (DMSO-*d₆*) δ (ppm): 7.83-7.75 (m, 6H), 7.54-7.33 (m, 5H), 7.15 (d, J = 8.7 Hz, 1H), 5.27-5.23 (m, 1H), 3.95 (s, 2H), 3.28-3.26 (m, 2H), 2.75 (dd, J = 9.1 and 14.9 Hz, 1H), 2.63 (dd, J = 5.6 and 14.9 Hz, 1H). ¹³C NMR (DMSO-*d₆*) δ (ppm): 166.0, 164.5 (d, J_{C-F} = 247 Hz), 137.1, 135.0, 133.4, 132.3, 130.1, 130.0, 128.3, 127.9, 127.7, 126.5, 126.1, 116.7 (d, J_{C-F} = 22 Hz), 59.3, 41.3, 38.4, 37.5. HRMS m/z calculated for C₂₃H₂₃FN₅O₄S [M + H]⁺ 484.1455, found 484.1457.

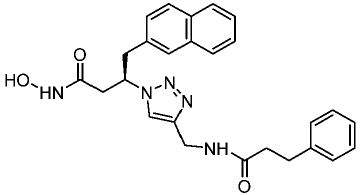

**(R)-N-Hydroxy-4-naphthalen-2-yl-3-{4-[(3-phenyl-propionylamino)-methyl]-[1,2,3]triazol-1-yl}-butyramide (10).** Compound **10** was obtained according to general procedure B from azide **109** and 3-phenyl-N-(prop-2-yn-1-yl)propanamide as a beige solid (43 mg, 82%), Purity: 95%, mp 150.0-151.4°C, LC t_{R} = 2.50 min, MS (ESI+): m/z = 457 [M + H]⁺. ¹H NMR (DMSO-*d₆*) δ (ppm): 10.52 (s, 1H), 8.81 (s, 1H), 8.27 (m, 1H), 7.83-7.67 (m, 4H), 7.50-7.42 (m, 3H), 7.28-7.16 (m, 6H), 5.25 (m, 1H), 4.20 (m, 2H), 3.32 (m, 2H), 2.80-2.73 (m, 2H), 2.78 (t, J = 7.5 Hz, 2H), 2.35 (t, J = 7.8 Hz, 2H). ¹³C NMR (DMSO-*d₆*) δ (ppm): 171.6, 166.0, 141.8, 135.3, 135.1, 133.4, 132.3, 128.7 (2C), 128.6 (2C), 128.3, 127.9 (3C), 127.7, 126.5, 126.3 (2C), 126.1, 59.2, 41.4, 37.7, 37.3, 34.5, 31.5. HRMS m/z calculated for C₂₆H₂₈N₅O₃ [M + H]⁺ 458.2192, found 458.2191.

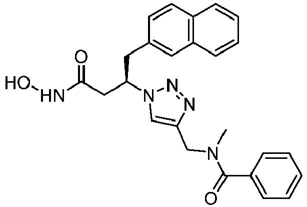

**(R)-N-[1-(2-hydroxycarbamoyl-1-naphthalen-2-ylmethyl-ethyl)-1H-[1,2,3]triazol-4-ylmethyl]-N-methyl-benzamide (14).** Compound **14** was obtained according to general procedure C from azide **110** and N-methyl-N-(prop-2-yn-1-yl)benzamide followed by general procedure D as a white solid (0.049 g, 37%). Purity: 100%, LC t_{R} = 2.32 min (method A), MS (ESI+): m/z = 444 [M + H]⁺. ¹H NMR (DMSO-d₆) δ (ppm): 10.54 (s, 0.9H), 8.81 (m, 0.8H), 7.87-7.70 (m, 4H), 7.46-7.15 (m, 10H), 5.33-5.24 (m, 1H), 4.48 (q, J = 15.3 Hz, 1H), 4.30 (s, 1H), 3.41-3.16 (m, 5H), 2.78 (d, J = 5.7 Hz, 1H), 2.68 (s, 1H). ¹³C NMR (CD₃CN) δ (ppm): 171.1, 166.7, 142.8, 134.6, 133.3, 132.3, 129.6, 128.3, 127.9, 127.7, 127.5, 127.4, 127.2, 126.8, 126.1, 125.7, 59.7, 41.1, 37.6, 36.5, 31.9. HRMS m/z calculated for C₂₅H₂₆N₅O₃ [M + H]⁺ 444.2036, found 444.2036.

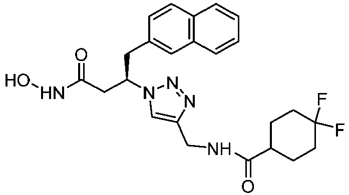

**(R)-4,4-difluoro-cyclohexanecarboxylic acid [1-(2-hydroxycarbamoyl-1-naphthalen-2-ylmethyl-ethyl)-1H-[1,2,3]triazol-4-ylmethyl]-amide (15).** Compound **15** was obtained according to general procedure C from azide **110** and 4,4-difluoro-N-(prop-2-yn-1-yl)cyclohexane-1-carboxamide followed by general procedure D as a white solid (0.016 g, 10%). Purity: 96%, LC t_{R} = 2.35 min (method A), MS (ESI+): m/z = 472 [M + H]⁺. ¹H NMR (CD₃CN) δ (ppm): 8.98 (m, 1H), 7.85-7.75 (m, 3H), 7.49-7.46 (m, 3H), 7.41 (s, 1H), 7.87 (d, J = 8.4 Hz, 1H), 6.95 (s, 1H), 6.75 (s, 1H), 5.29-5.19 (m, 1H), 4.25-4.28 (m, 2H), 3.41-3.38 (m, 2H), 2.82-2.78 (m, 2H), 1.57-1.83 (m, 6H). ¹³C NMR (MeOD-*d₄*) δ (ppm): 175.8, 167.1, 144.2, 134.1, 133.5, 132.5, 129.1, 127.9, 127.4, 127.2, 126.6, 125.8, 125.4, 123.3, 122.5, 60.0, 42.0, 41.0, 37.6, 33.9, 32.4 (t, J_{C-F} = 24.1 Hz), 25.4 (dd, J_{C-F} = 6.0 and 9.2 Hz). HRMS m/z calculated for C₂₄H₂₈N₅O₃F₂ [M + H]⁺ 472.2160, found 472.2180.

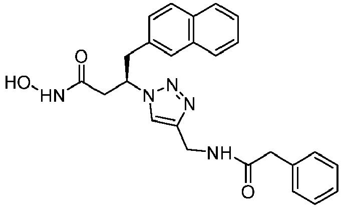

**(R)-N-hydroxy-4-naphthalen-2-yl-3-[4-(phenylacetylamino-methyl)-[1,2,3]triazol-1-yl]-butyramide (16).** Compound **16** was obtained according to general procedure C from azide **110** and 2-phenyl-N-(prop-2-yn-1-yl)acetamide followed by general procedure D as a white solid (36 mg, 30%). Purity: 95%, LC t_{R} = 2.34 min, MS (ESI+): m/z = 444 [M + H]⁺. ¹H NMR (CD₃CN) δ (ppm): 9.08 (s, 1H), 7.85-7.74 (m, 3H), 7.48-7.42 (m, 4H), 7.34 (m, 6H), 6.88 (s, 1H), 5.28-5.17 (m, 1H), 4.25 (d, J = 5.7 Hz, 2H), 3.42-3.35 (m, 4H), 2.81-2.76 (m, 2H). ¹³C NMR (CD₃CN) δ (ppm): 170.9, 166.4, 144.5, 135.9, 134.6, 134.5, 133.4, 132.3, 129.2, 128.5 (2C), 128.0, 127.7, 127.5 (2C), 127.2, 126.7, 126.2, 125.8, 122.4, 59.5, 42.6, 40.9, 37.7, 34.7. HRMS m/z calculated for C₂₅H₂₆N₅O₃ [M + H]⁺ 444.2036, found 444.2024.

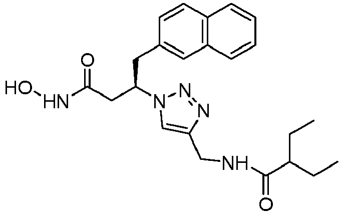

**(R)-3-{4-[(2-ethyl-butyrylamino)-methyl]-[1,2,3]triazol-1-yl}-N-hydroxy-4-naphthalen-2-yl-butyramide (17).** Compound **17** was obtained according to general procedure C from azide **110** and 2-ethyl-N-(prop-2-yn-1-yl)butanamide followed by general procedure D as a white solid (0.009 g, 7%). Purity: 98%, LC t_{R} = 2.37 min (method A), MS (ESI+): m/z = 424 [M + H]⁺. ¹H NMR (CD₃CN) δ (ppm): 9.01 (s, 0.8H), 7.84-7.74 (m, 3H), 7.52-7.45 (m, 4H), 7.20 (dd, J = 1.8 and 8.4 Hz, 1H), 6.82 (s, 0.9H), 5.31-5.21 (m, 1H), 4.28 (dd, J = 2.1 and 5.7 Hz, 2H), 3.40 (d, J = 7.5 Hz, 2H), 1.83-1.91 (m, 1H), 2.81-2.76 (m, 2H), 1.46-1.38 (m, 4H), 0.77 (q, J = 7.5 Hz, 6H). ¹³C NMR (CD₃CN) δ (ppm): 175.6, 166.4, 144.8, 134.7, 134.0, 133.4, 132.3, 128.0, 127.7, 127.5, 127.2, 126.1, 125.7, 122.5, 59.5, 50.1, 40.8, 37.8, 34.3, 25.4, 11.3. HRMS m/z calculated for C₂₃H₃₀N₅O₃ [M + H]⁺ 424.2349, found 424.2359.

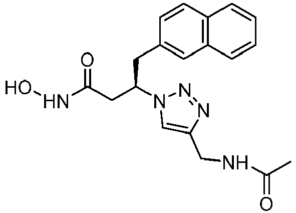

**(R)-3-[4-(acetylamino-methyl)-[1,2,3]triazol-1-yl]-N-hydroxy-4-naphthalen-2-yl-butyramide (18).** Compound **18** was obtained according to general procedure C from azide **110** and N-(prop-2-yn-1-yl)acetamide followed by general procedure D as a white solid (0.035 g, 14%). Purity: 100%, LC t_{R} = 1.99 min (method A), MS (ESI+): m/z = 368 [M + H]⁺. ¹H NMR (CD₃CN) δ (ppm): 9.22 (s, 1H), 7.85-7.77 (m, 3H), 7.49-7.45 (m, 4H), 7.20 (dd, J = 1.5 and 8.4 Hz, 1H), 6.90 (s, 1H), 5.29-5.19 (m, 1H), 4.24 (d, J = 5.7 Hz, 2H), 3.40-3.37 (m, 2H), 2.82-2.77 (m, 2H), 1.81 (s, 3H). ¹³C NMR (CD₃CN) δ (ppm): 170.4, 166.7, 144.6, 134.6, 133.3, 132.3, 128.0, 127.7, 127.5, 127.3, 126.2, 125.8, 122.7, 59.6, 40.9, 37.6, 34.5, 21.9. HRMS m/z calculated for C₁₉H₂₂N₅O₃ [M + H]⁺ 368.1723, found 368.1737.

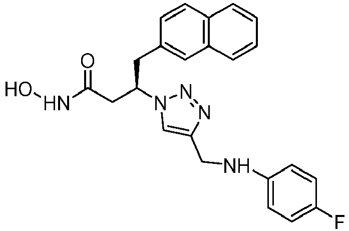

**(R)-3-{4-[(4-fluoro-phenylamino)-methyl]-[1,2,3]triazol-1-yl}-N-hydroxy-4-naphthalen-2-yl-butyramide (19).** Compound **9** was obtained according to general procedure C from azide **110** and 4-fluoro-N-(prop-2-yn-1-yl)aniline followed by general procedure D as a white solid (0.010 g, 8%). Purity: 100%, LC t_{R} = 2.52 min (method A), MS (ESI+): m/z = 420 [M + H]⁺. ¹H NMR (MeOD-*d₄*) δ (ppm): 7.78-7.75 (m, 1H), 7.67-7.61 (m, 2H), 7.48-7.41 (m, 4H), 7.06 (d, J = 8.1 Hz, 1H), 6.71 (t, J = 8.7 Hz, 1.8H), 6.48-6.43 (m, 1.6H), 5.27-5.23 (m, 1H), 4.22 (s, 1.4H), 3.43-3.36 (m, 2H), 3.05 (m, 2H). ¹³C NMR (MeOD-*d₄*) δ (ppm): 134.0, 133.4, 132.5, 127.8, 127.4, 127.2, 126.5, 125.8, 125.4, 115.0, 114.7, 113.7, 60.1, 48.9, 41.0, 37.5. HRMS m/z calculated for C₂₃H₂₃N₅O₂F [M + H]⁺ 420.1836, found 420.1828.

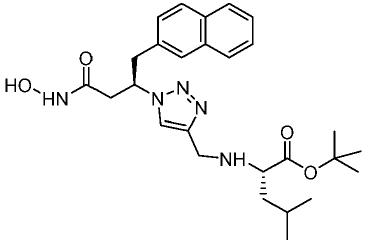

**Tert-butyl ((1-((R)-4-(hydroxyamino)-1-(naphthalen-2-yl)-4-oxobutan-2-yl)-1H-1,2,3-triazol-4-yl)methyl)-L-leucinate (20).** Compound **20** was obtained according to general procedure C from azide **110** and tert-butyl prop-2-yn-1-yl-L-leucinate followed by general procedure D as a white solid (0.017 g, 11%). Purity: 97%, LC t_{R} = 2.67 min (method A), MS (ESI+): m/z = 496 [M + H]⁺. ¹H NMR (MeOD-*d₄*) δ (ppm): 7.79-7.65 (m, 4H), 7.50-7.41 (m, 3H), 7.21 (d, J = 8.1 Hz, 1H), 5.38-5.28 (m, 1H), 3.83 (dd, J = 27.6 and 13.8 Hz, 2H), 3.45 (d, J = 7.2 Hz, 2H), 2.97-2.84 (m, 2H), 1.70-1.29 (m, 13H), 0.88 (dd, J = 10.2 and 6.3 Hz, 6H). ¹³C NMR (MeOD-*d₄*) δ (ppm): 172.5, 166.9, 142.5, 134.0, 133.5, 132.5, 127.9, 127.5, 127.2, 126.6, 125.8, 125.4, 124.1, 82.2, 60.2, 59.1, 41.3, 40.9, 40.8, 37.6, 26.9, 24.7, 21.7, 21.0. HRMS m/z calculated for C₂₇H₃₈N₅O₄ [M + H]⁺ 496.2924, found 496.2941.

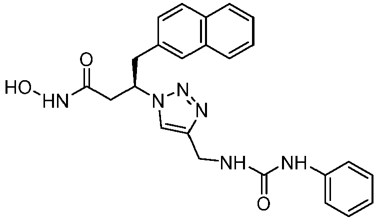

**(R)-N-hydroxy-4-naphthalen-2-yl-3-{4-[(3-phenyl-ureido)-methyl]-[1,2,3]triazol-1-yl}-butyramide (21).** Compound **21** was obtained according to general procedure C from azide **110** and 1-phenyl-3-(prop-2-yn-1-yl)urea followed by general procedure D as a white solid (0.051 g, 38%). Purity: 100%, LC t_{R} = 2.34 min (method A), MS (ESI+): m/z = 445 [M + H]⁺. ¹H NMR (DMSO-d6) δ (ppm): 10.51 (s, 0.8H), 8.80 (s, 0.8H), 8.52 (s, 1H), 7.88 (s, 1H), 7.75-7.72 (m, 3H), 7.50-7.13 (m, 9H), 6.90 (t, J = 7.2 Hz, 1H), 6.49 (t, J = 5.4 Hz, 1H), 5.32-5.23 (m, 1H), 4.24 (d, J = 5.4 Hz, 2H), 3.33 (m, 2H+H₂O), 2.82-2.63 (m, 2H). ¹³C NMR (DMSO-d6) δ (ppm): 166.0, 155.4, 145.4, 140.9, 135.1, 133.4, 132.3, 129.5, 129.1, 128.8, 128.3, 127.9, 127.7, 126.5, 126.1, 122.7, 121.6, 118.1, 59.2, 41.4, 37.7, 35.2. HRMS m/z calculated for C₂₄H₂₅N₆O₃ [M + H]⁺ 445.1988, found 445.1966.

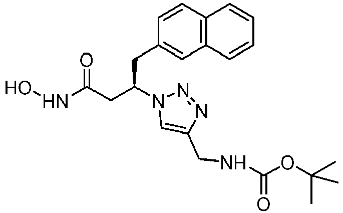

**(R)-[1-(2-hydroxycarbamoyl-1-naphthalen-2-ylmethyl-ethyl)-1H-[1,2,3]triazol-4-ylmethyl]-carbamic acid tert-butyl ester (22).** Compound **22** was obtained according to general procedure C from azide **110** and tert-butyl prop-2-yn-1-ylcarbamate followed by general procedure D as an off-white oil (0.021 g, 13%). Purity: 100%, LC t_{R} = 2.44 min (method A), MS (ESI+): m/z = 426 [M + H]⁺. ¹H NMR (CD₃CN) δ (ppm): 9.07 (s, 1H), 7.85-7.75 (m, 3H), 7.49-7.46 (m, 4H), 7.19 (d, J = 8.4 Hz, 1H), 7.03 (s, 0.6H), 5.62 (s, 0.8H), 5.31-5.21 (m, 1H), 4.16 (d, J = 6.0 Hz, 2H), 3.40-3.37 (m, 2H), 2.84-2.81 (m, 2H), 2.81 (m, 9H). ¹³C NMR (CD₃CN) δ (ppm): 134.6, 133.4, 132.3, 128.0, 127.7, 127.5, 127.2, 126.2, 125.8, 122.1, 59.4, 41.0, 37.6, 35.7, 27.6. HRMS m/z calculated for C₂₂H₂₈N₅O₄ [M + H]⁺ 426.2141, found 426.2144.

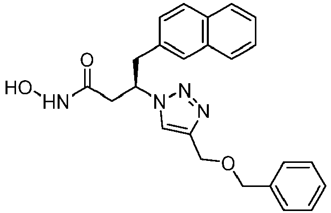

**(R)-3-(4-benzyloxymethyl-[1,2,3]triazol-1-yl)-N-hydroxy-4-naphthalen-2-yl-butyramide (23).** Compound **23** was obtained according to general procedure C from azide **110** and ((prop-2-yn-1-yloxy)methyl)benzene followed by general procedure D as a white solid (0.066 g, 27%). Purity: 100%, LC t_{R} = 2.57 min (method A), MS (ESI+): m/z = 417 [M + H]⁺. ¹H NMR (CD₃CN) δ (ppm): 9.22 (s, 1H), 7.81-7.70 (m, 3H), 7.49-7.41 (m, 4H), 7.435-7.29 (m, 3H), 7.21 (m, 3H), 5.33-5.23 (m, 1H), 4.48 (s, 2H), 4.33 (dd, J = 12.0 and 14.7 Hz, 2H), 3.44-3.33 (m, 2H), 2.94-2.79 (m, 2H). ¹³C NMR (CD₃CN) δ (ppm): 166.6, 143.7, 138.3, 134.6, 133.3, 132.3, 128.3, 128.0, 127.8, 127.7, 127.5, 127.2, 126.2, 125.8, 123.6, 71.3, 62.8, 59.5, 41.0, 37.6. HRMS m/z calculated for C₂₄H₂₅N₄O₃ [M + H]⁺ 417.1927, found 417.1932.

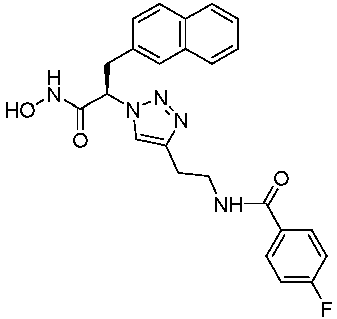

**(R)-4-Fluoro-N-{2-[1-(1-hydroxycarbamoyl-2-naphthalen-2-yl-ethyl)-1H-[1,2,3]triazol-4-yl]-ethyl}-benzamide (29).** Compound **29** was obtained according to general procedure C from azide **130** and N-(but-3-yn-1-yl)-4-fluorobenzamide followed by general procedure D as a white solid (70 mg, 3%), Purity: 97%, LC t_{R} = 2.73 min, MS (ESI+): m/z = 448 [M+1]⁺. ¹H NMR (DMSO) δ (ppm): 8.64 (br s, 1H), 8.22 (s, 1H), 7.95-7.90 (m, 2H), 7.83-780 (m, 1H), 7.75-7.69 (m, 2H), 7.63 (s, 1H), 7.46-7.44 (m, 2H), 7.33-7.26 (m, 3H), 5.42 (t, J = 7.9 Hz, 1H), 3.52-3.49 (m, 2H), 2.89-2.84 (m, 2H). ¹³C NMR (DMSO) δ (ppm): 165.6, 164.5, 164.3 (J_{C-F} = 247 Hz), 144.7, 134.3, 133.3, 132.4, 131.4, 130.3 (J_{C-F} = 9 Hz), 129.5, 128.8, 128.2, 127.9, 127.8, 126.5, 126.1, 122.0, 115.6 (J_{C-F} = 22 Hz), 62.3, 39.5, 38.1, 25.9. HRMS m/z calculated for C₂₄H₂₃N₅O₃F [M + H]⁺ 448.1785, found 448.1793.

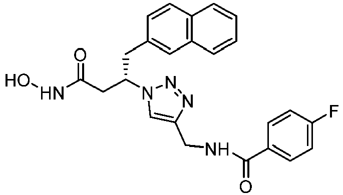

**(S)-4-fluoro-N-[1-(2-hydroxycarbamoyl-1-naphthalen-2-ylmethyl-ethyl)-1H-[1,2,3]triazol-4-ylmethyl]-benzamide (30)** Compound **30** was obtained according to general procedure C from azide **111** and 4-fluoro-N-(prop-2-yn-1-yl)benzamide followed by general procedure D as a white solid (0.255 g, 46%). Purity: 96%, LC t_{R} = 2.75 min, MS (ESI+): m/z = 448 [M+H]⁺. ¹H NMR (DMSO-d6) δ (ppm): 10.51 (s, 0.9H), 8.99 (br t, J = 5.7 Hz, 1H), 8.79 (br s, 0.9H), 7.93-7.88 (m, 3H), 7.78-7.70 (m, 3H), 7.47-7.40 (m, 3H), 7.30 (t, J = 8.7 Hz, 2H), 7.14 (d, J = 8.4 Hz, 1H), 5.31-5.22 (m, 1H), 4.42 (d, J = 5.7 Hz, 2H), 3.32-3.28 (m, H₂O+2H), 2.78 (dd, J = 8.7 and 15.0 Hz, 1H), 2.66 (dd, J = 5.4 and 15.0 Hz, 1H). ¹³C NMR (DMSO-d6) δ (ppm): 166.0, 165.4, 164.3 (d, J_{C-F} = 246 Hz), 144.9, 135.0, 133.3, 132.3, 131.1, 130.4 (2C), 130.3, 128.2, 127.8 (2C), 127.7, 126.5, 126.0, 123.0, 115.7 (d, J_{C-F} = 21 Hz, 2C), 59.2, 41.4, 37.6, 35.2. Mp = 190 °C. HRMS m/z calculated for C₂₄H₂₃N₅O₃F [M+H]⁺ 448.1785, found 448.1761.

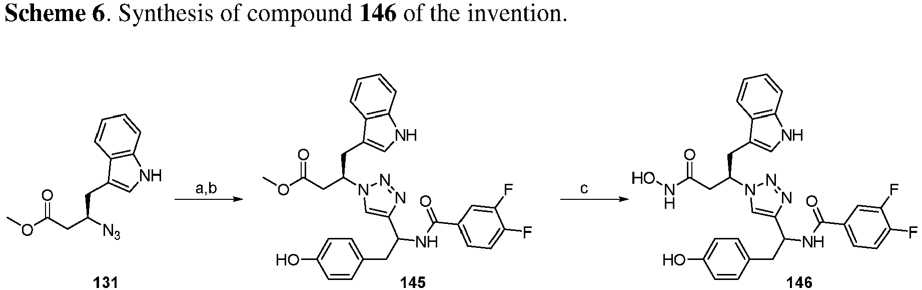

Reagents and conditions: (a) alkyne, CuSO₄.5H₂O, sodium ascorbate, H₂O/dioxane, rt, 16 h; (b) BBr₃, DCM, -78°C to -20°C, 1 h; (c) NH₂OH, KCN, H₂O, MeOH, rt, 16 h.

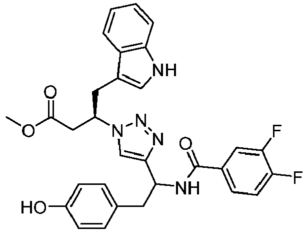

**Methyl (3R)-3-[4-[1-[(3,4-difluorobenzoyl)amino]-2-(4-hydroxyphenyl)ethyl]triazol-1-yl]-4-(1H-indol-3-yl)butanoate (145).** The benzylated compound, obtained according to general procedure C from azide **131** and N-(1-(4-(benzyloxy)phenyl)but-3-yn-2-yl)-3,4-difluorobenzamide, (85 mg, 0.13 mmol) was put in solution in CH₂Cl₂ and cooled down to -78°C before a 1.0 M solution of boron tribromide in CH₂Cl₂ (485 µL, 0.39 mmol, 3.0 equiv) was added dropwise. The resulting mixture was further stirred for 1 h before the reaction was quenched with iced water. The mixture was further diluted with water and layers were separated. The aqueous layer was further extracted twice with CH₂Cl₂. The collected organic phases were dried over MgSO₄, filtered and concentrated under reduced pressure to give the desired compound. The residue was purified by flash chromatography on silica gel (CH₂Cl₂/MeOH 10/0 to 7/3) to afford the desired compound **145** as an off-white solid (72 mg, 98%). LC t_{R} = 2.60 min, MS (ESI+): m/z = 560 [M+H]⁺. ¹H NMR (CDCl₃ + 1% TMS), mixture of diastereoisomers, δ (ppm): 8.49 (dd, J = 13.6 and 1.7 Hz, 1H), 8.43 (br s, 0.5H), 8.31 (br s, 0.5H), 7.70-7.61 (m, 1H), 7.54-7.39 (m, 3H), 7.25-7.22 (m, 1H), 7.15-6.99 (m, 3H), 6.95 (s, 0.5H), 6.81 (s, 0.5H), 6.72 (d, J = 8.4 Hz, 1H), 6.66-6.59 (m, 3H), 6.45 (dd, J = 10.6 and 2.3 Hz, 1H), 5.46-5.34 (m, 1H), 5.14-4.95 (m, 1H), 3.58 (s, 3H), 3.34-3.21 (m, 2H), 3.18-2.88 (m, 4H). ¹³C NMR (CDCl₃ + 1% TMS), mixture of diastereoisomers, δ (ppm): 170.7, 170.6, 164.9, 164.9, 155.2, 155.2, 152.6 (dd, J = 154.8 and 12.6 Hz), 150.1 (dd, J = 249.7 and 13.2 Hz), 145.9, 145.4, 136.1, 136.1, 130.8 (dd, J = 6.6 and 4. 2 Hz), 130.6, 130.6, 127.9, 127.8, 126.9, 126.8, 123.8 (dd, J = 6.6 and 3.6 Hz), 123.3, 123.3, 122.9 (br), 122.2, 119.7, 118.1, 118.8, 117.4 (d, J = 18.0 Hz), 116.9 (d, J = 18.6 Hz), 115.5, 115.4, 111.5, 109.7, 109.6, 59.2, 59.0, 52.1, 47.7, 47.7, 40.7, 40.7, 38.9, 38.8, 31.5, 31.2.

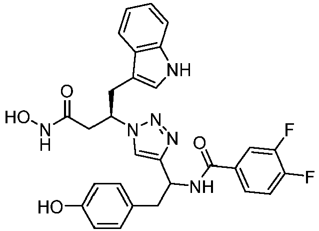

**3,4-Difluoro-N-[1-[1-[(1R)-3-(hydroxyamino)-1-(1H-indol-3-ylmethyl)-3-oxopropyl]triazol-4-yl]-2-(4-hydroxyphenyl)ethyl]benzamide (146).** Compound **146** was obtained from ester **145** according to general procedure G as a white solid (44 mg, 61%). Purity 94%, LC t_{R} = 2.25 min, MS (ESI+): m/z = 561 [M+H]⁺. ¹H NMR (MeOD-*d₄*), mixture of diastereoisomers, δ (ppm): 7.63-7.55 (m, 1H), 7.55-7.48 (m, 1H), 7.45 (s, 0.5H), 7.39 (s, 0.5H), 7.38-7.34 (m, 1H), 7.33-7.24 (m, 2H), 7.07-7.00 (m, 1H), 6.98-6.91 (m, 3H), 6.78 (d, J = 6.7 Hz, 1H), 6.66-6.61 (m, 2H), 5.33 (td, J = 13.8 and 6.3 Hz, 1H), 5.27-5.16 (m, 1H), 3.39 (dd, J = 14.4 and 5.4 Hz, 1H), 3.36-3.27 (m, 1H), 3.08-2.82 (m, 4H). ¹³C NMR (MeOD-*d₄*), mixture of diastereoisomers, δ (ppm): 168.8, 167.2, 157.1, 153.6 (dd, J = 251.8 and 12.7 Hz), 151.2 (dd, J = 247.5 and 13.3 Hz), 148.7, 148.6, 137.9, 137.9, 137.2 (br), 133.0-132.9 (m), 131.3 (2C), 129.7, 129.7, 128.5, 128.4, 125.6-125.4 (m), 124.6, 124.6, 124.1, 124.5, 120.0, 118.9, 118.4 (d, J = 18.0 Hz), 117.9 (d, J = 19.0 Hz), 116.1 (2C), 112.3, 110.6, 61.2, 61.1, 49.6, 49.6, 40.9, 40.8, 38.7 (br), 32.5. HRMS m/z calculated for C₂₉H₂₆F₂N₆O₄ [M+H]⁺ 561.2062, found 561.2062.

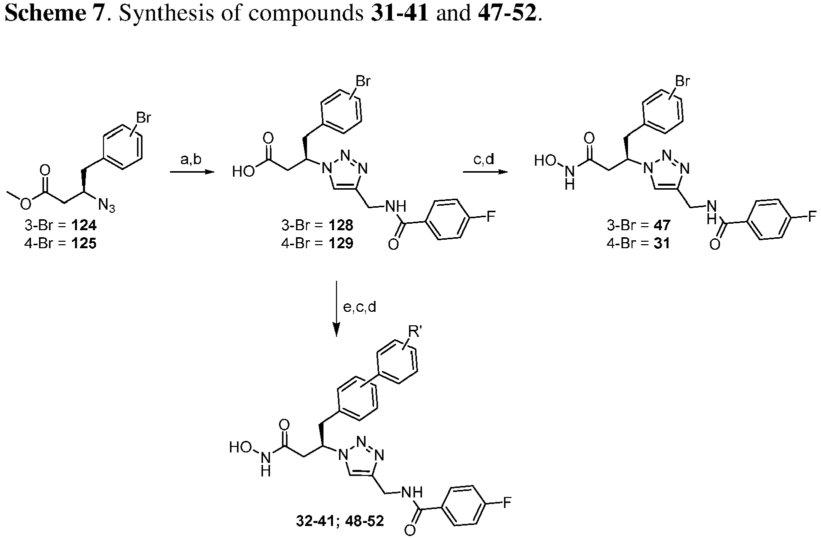

Reagents and conditions: (a) alkyne, CuSO₄.5H₂O, sodium ascorbate, H₂O/DMF, rt, 16 h; (b) (i) 2M NaOH, EtOH/EtOH, rt, 16 h, (ii) 1N HCl; (c) O-tritylhydroxylamine, EDCI, HOBt, N-methylmorpholine, DMF, rt, 16 h; (d) TFA, TIS, CH₂Cl₂, rt, 30 min; (e) boronic acid, Na₂CO₃, Pd(dppf)₂Cl₂.CH₂Cl₂, toluene, H₂O, MW, 90°C, 15 min or 100°C, 45 min.

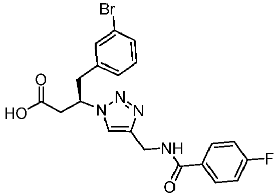

**(R)-4-(3-bromo-phenyl)-3-{4-[(4-fluoro-benzoylamino)-methyl]-[1,2,3]triazol-1-yl}-butyric acid (128).** Compound **128** was obtained according to general procedure C from azide **124** and 4-fluoro-N-(prop-2-yn-1-yl)benzamide followed by general procedure P as a white solid (366 mg, 50%). Purity: 85%, LC t_{R} = 2.52 min, MS (ESI+): m/z = 459/461 [M+H]+. ¹H NMR (CDCl₃) δ (ppm): 7.77-7.70 (m, 2H), 7.56-7.53 (m, 1H), 7.28-7.26 (m, 1H + CDCl₃), 7.13 (s, 1H), 7.05 (t, J = 7.5 Hz, 3H), 6.89 (d, J = 8.4 Hz, 1H), 5.07-5.02 (m, 1H), 4.53-4.48 (m, 2H), 3.23-3.16 (m, 3H), 3.01-2.94 (m, 1H). ¹³C NMR (CDCl₃) δ (ppm): 172.5, 166.6, 164.9 (d, J_{C-F} = 250 Hz), 138.2, 131.9, 130.4 (d, J_{C-F} = 11 Hz), 129.7, 129.6, 127.6, 124.0, 122.7, 115.6 (d, J_{C-F}= 22 Hz), 59.9, 41.0, 38.8, 34.6.

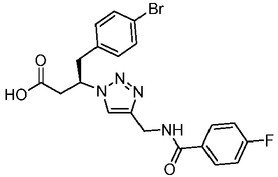

**(R)-4-(4-bromo-phenyl)-3-{4-[(4-fluoro-benzoylamino)-methyl]-[1,2,3]triazol-1-yl}-butyric acid (129).** Compound **129** was obtained according to general procedure C from azide **125** and 4-fluoro-N-(prop-2-yn-1-yl)benzamide followed by general procedure P as a white solid (3.56 g, 33%). Purity: 100%, LC t_{R} = 2.82 min, MS (ESI+): m/z = 463/461 [M+H]+. ¹H NMR (DMSO-d₆) δ (ppm): 12.38 (br s, 0.9H), 9.01 (br s, 1H), 7.96-7.91 (m, 3H), 7.35-7.27 (m, 4H), 6.94 (d, J = 8.1 Hz, 2H), 5.12-5.02 (m, 1H), 4.44 (d, J = 5.7 Hz, 2H), 3.12-2.85 (m, 4H). ¹³C NMR (DMSO-d₆) δ (ppm): 166.0, 165.4, 162.7, 136.8, 131.7, 131.6, 131.1, 130.4, 130.3, 123.0, 120.3, 115.8, 115.5 (d, J_{C-F}= 22 Hz), 35.3.

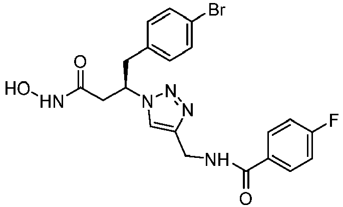

**(R)-N-{1-[1-(4-bromo-benzyl)-2-hydroxycarbamoyl-ethyl]-1H-[1,2,3]triazol-4-ylmethyl}-4-fluoro-benzamide (31).** Compound **31** was obtained according to general procedure D from carboxylic acid **129** as a white solid (0.044 g, 17%). Purity: 100%, LC t_{R} = 2.76 min, MS (ESI+): m/z = 478/476 [M+H]⁺. ¹H NMR (acetone-d₆+MeOD-d₄) δ (ppm): 7.90-7.86 (m, 2H), 7.63 (s, 1H), 7.28 (d, J = 8.1 Hz, 2H), 7.20 (t, J = 8.7 Hz, 2H), 6.89 (d, J = 8.1 Hz, 2H), 5.21-5.12 (m, 1H), 4.54 (s, 2H), 3.22-3.19 (m, 2H), 2.90 (dd, J = 8.1 and 14.7 Hz, 1H), 2.80 (dd, J = 5.4 and 14.7 Hz, 1H). ¹³C NMR (acetone-d₆+MeOD-d₄) δ (ppm): 167.4, 167.0, 164.9 (d, J_{C-F} = 249 Hz), 144.4, 135.8, 131.2, 130.6, 129.7, 129.6, 123.4, 120.4, 115.0 (d, J_{C-F} = 22 Hz), 59.8, 40.2, 37.4, 34.6. HRMS m/z calculated for C₂₀H₂₀N₅O₃BrF [M+H]⁺ 476.0734, found 476.0739.

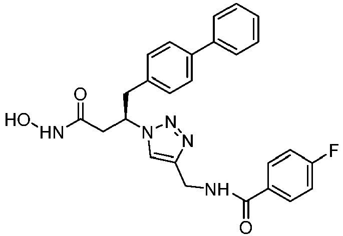

**(R)-N-[1-(1-biphenyl-4-ylmethyl-2-hydroxycarbamoyl-ethyl)-1H-[1,2,3]triazol-4-ylmethyl]-4-fluoro-benzamide (32).** Compound **32** was obtained according to general procedure Q from bromo-compound **129** followed by general procedure D as a white solid (0.044 g, 43%). Purity: 100%, LC t_{R} = 2.85 min, MS (ESI+): m/z = 474 [M+H]⁺. ¹H NMR (dmso-d₆) δ (ppm): 10.51 (br s, 0.9H), 9.01 (br t, J = 5.7 Hz, 1H), 8.79 (br s, 0.9H), 7.93-7.89 (m, 2H), 7.84 (s, 1H), 7.53-7.21 (m, 9H), 7.03 (d, J = 8.1 Hz, 2H), 5.23-5.14 (m, 1H), 4.44 (d, J = 5.7 Hz, 2H), 3.16 (d, J = 7.2 Hz, 2H), 2.75 (dd, J = 8.7 and 15.6 Hz, 1H), 2.80 (dd, J = 5.4 and 15.3 Hz, 1H). ¹³C NMR (DMSO-d6) δ (ppm): 166.0, 164.1 (d, J_{C-F}= 204 Hz), 144.9, 140.2, 138.8, 136.6, 131.1, 130.4 (d, J_{C-F} = 9 Hz), 130.0, 129.3, 127.8, 126.9, 123.2, 115.7 (d, J_{C-F} = 22 Hz), 59.8, 40.2, 37.5, 35.3. HRMS m/z calculated for C₂₆H₂₅N₅O₃F [M+H]⁺ 474.1941, found 474.1970.

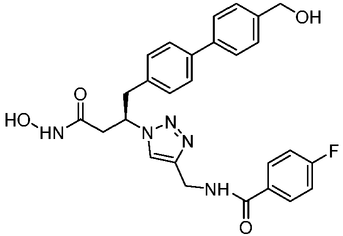

**(R)-4-fluoro-N-{1-[2-hydroxycarbamoyl-1-(4'-hydroxymethyl-biphenyl-4-ylmethyl)-ethyl]-1H-[1,2,3]triazol-4-ylmethyl}-benzamide (33).** Compound **33** was obtained according to general procedure Q from bromo-compound **129** followed by general procedure D as a white solid (0.017 g, 4%). Purity: 100%, LC t_{R} = 2.57 min, MS (ESI+): m/z = 504 [M+H]⁺. ¹H NMR (DMSO-d₆) δ (ppm): 9.01 (br t, J = 5.7 Hz, 1H), 8.79 (br s, 0.8H), 7.94-7.89 (m, 2H), 7.86 (s, 1H), 7.49 (d, J = 8.4 Hz, 2H), 7.43 (d, J = 8.1 Hz, 2H), 7.35 (d, J = 8.1 Hz, 2H), 7.25 (t, J = 8.7 Hz, 2H), 7.03 (d, J = 8.4 Hz, 2H), 5.21-5.17 (m, 2H), 4.52 (d, J = 5.7 Hz, 2H), 4.44 (d, J = 5.7 Hz, 2H), 3.16 (d, J = 7.2 Hz, 2H), 2.75 (dd, J = 8.7 and 15.0 Hz, 1H), 2.65 (dd, J = 5.4 and 15.0 Hz, 1H). ¹³C NMR (DMSO-d₆) δ (ppm): 166.0, 165.4, 144.9, 142.1, 138.7, 138.5, 136.4, 131.0, 130.3 (d, J_{C-F}= 9 Hz), 130.0, 127.4, 126.8, 126.6, 123.1, 115.6 (d, J_{C-F}= 22 Hz), 63.0, 59.2, 40.2, 37.5, 35.3. HRMS m/z calculated for C₂₇H₂₇N₅O₄F [M+H]⁺ 504.2047, found 504.2039.

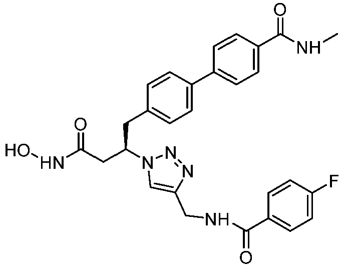

**(R)-4'-(2-{4-[(4-fluoro-benzoylamino)-methyl]-[1,2,3]triazol-1-yl}-3-hydroxycarbamoyl-propyl)-biphenyl-4-carboxylic acid methylamide (34).** Compound **34** was obtained according to general procedure Q from bromo-compound **129** followed by general procedure D as a white solid (0.130 g, 53%). Purity: 97%, LC t_{R} = 2.48 min, MS (ESI+): m/z = 531 [M+H]⁺. ¹H NMR (DMSO-d₆) δ (ppm): 10.54 (br s, 0.7H), 9.03 (br t, J = 5.4 Hz, 1H), 8.47 (d, J = 4.2 Hz, 1H), 8.20 (br s, 1H), 7.94-7.86 (m, 5H), 7.62 (d, J = 8.1 Hz, 2H), 7.50 (d, J = 8.1 Hz, 2H), 7.24 (t, J = 8.7 Hz, 2H), 7.06 (d, J = 7.8 Hz, 2H), 5.24-5.15 (m, 1H), 4.45 (d, J = 5.4 Hz, 2H), 3.18 (d, J = 6.9 Hz, 2H), 2.80-2.61 (m, 4H), 2.65 (dd, J = 5.4 and 15.3 Hz, 1H). ¹³C NMR (DMSO-d₆) δ (ppm): 166.7, 165.9, 165.4, 163.5 (d, J_{C-F} = 113 Hz), 145.0, 142.5, 137.8, 137.3, 133.6, 131.1, 130.4 (d, J_{C-F} = 9 Hz), 130.1, 128.1, 127.1, 126.7, 123.1, 115.6 (d, J_{C-F}= 22 Hz), 59.2, 40.2, 39.1, 35.3, 26.7. HRMS m/z calculated for C₂₈H₂₈N₆O₄F [M+H]⁺ 531.2156, found 531.2144.

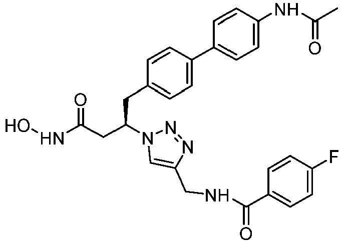

**(R)-N-{1-[1-(4'-acetylamino-biphenyl-4-ylmethyl)-2-hydroxycarbamoyl-ethyl]-1H-[1,2,3]triazol-4-ylmethyl}-4-fluoro-benzamide (35).** Compound **35** was obtained according to general procedure Q from bromo-compound **129** followed by general procedure D as a pale brown solid (0.132 g, 48%). Purity: 100%, LC t_{R} = 2.52 min, MS (ESI+): m/z = 531 [M+H]⁺. ¹H NMR (DMSO-d₆) δ (ppm): 10.51 (br s, 0.8H), 10.00 (br s, 1H), 9.01 (t, J = 5.7 Hz, 1H), 8.17 (br s, 0.7H), 7.94-7.89 (m, 2H), 7.85 (s, 1H), 7.62 (d, J = 8.7 Hz, 2H), 7.47 (d, J = 8.7 Hz, 2H), 7.41 (d, J = 8.4 Hz, 2H), 7.25 (t, J = 8.7 Hz, 2H), 7.01 (d, J = 8.4 Hz, 2H), 5.22-5.13 (m, 1H), 4.43 (d, J = 5.7 Hz, 2H), 3.15 (d, J = 8.4 Hz, 2H), 2.75 (dd, J = 8.7 and 15.0 Hz, 1H), 2.64 (dd, J = 5.4 and 15.0 Hz, 1H). ¹³C NMR (DMSO-d₆) δ (ppm): 168.8, 166.0, 165.4, 144.9, 139.2, 138.4, 136.1, 134.6, 131.1, 130.4, 130.3, 130.0, 127.1, 126.4, 119.7, 115.7 (d, J_{C-F} = 21 Hz), 59.3, 40.2, 37.5, 35.3, 24.5. HRMS m/z calculated for C₂₈H₂₈N₆O₄F [M+H]⁺ 531.2156, found 531.2165.

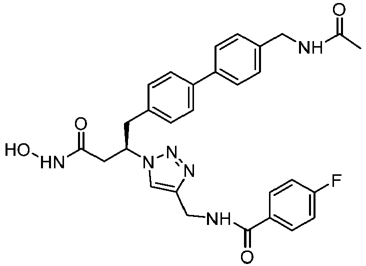

**(R)-N-(1-[1-[4'-(acetylamino-methyl)-biphenyl-4-ylmethyl]-2-hydroxycarbamoyl-ethyl}-1H-[1,2,3]triazol-4-ylmethyl)-4-fluoro-benzamide (36).** Compound **36** was obtained according to general procedure Q from bromo-compound **129** followed by general procedure D as a pale brown solid (0.130 g, 41%). Purity: 95%, LC t_{R} = 2.54 min, MS (ESI+): m/z = 545 [M+H]⁺. ¹H NMR (DMSO-d₆) δ (ppm): 10.51 (br s, 0.8H), 9.01 (br s, 1H), 8.35 (s, 0.9H), 8.20 (br s, 1H), 7.94-7.86 (m, 3H), 7.48-7.41 (m, 4H), 7.29-7.22 (m, 4H), 7.03 (d, J = 7.2 Hz, 2H), 5.24-5.14 (m, 1H), 4.45 (d, J = 3.9 Hz, 2H), 4.27 (d, J = 4.5 Hz, 2H), 3.16 (d, J = 6.3 Hz, 2H), 2.75 (dd, J = 8.4 and 14.4 Hz, 1H), 2.67 (dd, J = 4.8 and 14.4 Hz, 1H), 1.88 (s, 3H). ¹³C NMR (DMSO-d₆) δ (ppm): 169.7, 166.0, 165.4 162.7, 145.0, 139.2, 138.7, 138.6, 136.5, 131.1, 130.3 (d, J_{C-F}= 9 Hz), 130.0, 128.3, 126.8, 123.1, 115.6 (d, J_{C-F} = 22 Hz), 59.2, 42.3, 40.9, 37.5, 35.3, 23.0. HRMS m/z calculated for C₂₉H₃₀N₆O₄F [M+H]⁺ 545.2313, found 545.2298.

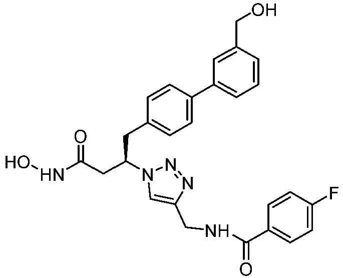

**(R)-4-fluoro-N-{1-[2-hydroxycarbamoyl-1-(3'-hydroxymethyl-biphenyl-4-ylmethyl)-ethyl]-1H-[1,2,3]triazol-4-ylmethyl}-benzamide (37).** Compound **37** was obtained according to general procedure Q from bromo-compound **129** followed by general procedure D as a white solid (0.025 g, 8%). Purity: 100%, LC t_{R} = 2.50 min, MS (ESI+): m/z = 504 [M+H]⁺. ¹H NMR (DMSO-d₆) δ (ppm): 10.51 (br s, 0.7H), 9.03 (br t, J = 5.7 Hz, 1H), 8.80 (br s, 0.8H), 7.94-7.89 (m, 2H), 7.86 (s, 1H), 7.51(s, 1H), 7.45-7.21 (m, 7H), 7.04 (d, J = 8.1 Hz, 2H), 5.23-5.19 (m, 2H), 4.54 (d, J = 5.7 Hz, 2H), 4.44 (d, J = 5.7 Hz, 2H), 3.16 (d, J = 7.2 Hz, 2H), 2.75 (dd, J = 9.0 and 15.3 Hz, 1H), 2.64 (dd, J = 5.7 and 15.3 Hz, 1H). ¹³C NMR (DMSO-d₆) δ (ppm): 166.0, 165.4, 162.7, 144.9, 143.6, 139.9, 139.0, 136.5, 131.1, 131.0, 130.4 (d, J_{C-F} = 22 Hz), 130.0, 129.1, 126.9, 125.9, 125.2, 125.0, 123.1, 115.6 (d, J_{C-F} = 21 Hz), 63.3, 59.2, 41.2, 37.5, 35.3. HRMS m/z calculated for C₂₇H₂₇N₅O₄F [M+H]⁺ 504.2047, found 504.2042.

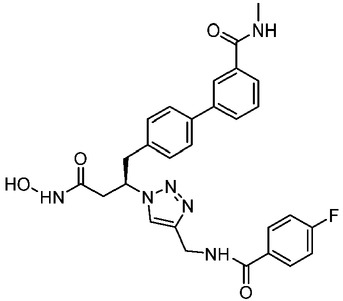

**(R)-4'-(2-{4-[(4-fluoro-benzoylamino)-methyl]-[1,2,3]triazol-1-yl}-3-hydroxycarbamoyl-propyl)-biphenyl-3-carboxylic acid methylamide (38).** Compound **38** was obtained according to general procedure Q from bromo-compound **129** followed by general procedure D as a white solid (0.130 g, 25%). Purity: 93%, LC t_{R} = 2.58 min, MS (ESI+): m/z = 531 [M+H]⁺. ¹H NMR (DMSO-d₆) δ (ppm): 10.52 (br s, 0.8H), 9.03 (br t, J = 5.4 Hz, 1H), 8.53 (br m, 1H), 8.15 (s, 1H), 8.03 (br s, 1H), 7.93-7.86 (m, 3H), 7.79 (d, J = 7.8 Hz, 1H), 7.67 (d, J = 7.8 Hz, 1H), 7.53-7.49 (m, 3H), 7.23 (t, J = 8.7 Hz, 2H), 7.08 (d, J = 8.1 Hz, 2H), 5.25-5.16 (m, 1H), 4.44 (d, J = 5.7 Hz, 2H), 3.19 (d, J = 6.9 Hz, 2H), 2.80 (d, J = 4.5 Hz, 3H), 2.76 (dd, J = 8.7 and 15.3 Hz, 1H), 2.65 (dd, J = 5.4 and 15.3 Hz, 1H). ¹³C NMR (DMSO-d₆) δ (ppm): 166.9, 166.0, 165.4, 163.7, 145.0, 140.2, 138.2, 137.0, 135.6, 131.1, 130.4, 130.3, 130.1, 129.4, 127.1, 126.6, 125.5, 123.1, 115.7 (d, J_{C-F} = 22 Hz), 59.2, 40.2, 37.5, 35.3, 26.7. HRMS m/z calculated for C₂₈H₂₈N₆O₄F [M+H]⁺ 531.2156, found 531.2151.

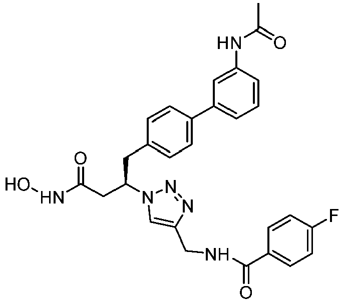

**(R)-N-{1-[1-(3'-acetylamino-biphenyl-4-ylmethyl)-2-hydroxycarbamoyl-ethyl]-1H-[1,2,3]triazol-4-ylmethyl}-4-fluoro-benzamide (39).** Compound **39** was obtained according to general procedure Q from bromo-compound **129** followed by general procedure D as a white solid (0.156 g, 62%). Purity: 100%, LC t_{R} = 2.63 min, MS (ESI+): m/z = 531 [M+H]⁺. ¹H NMR (DMSO-d₆) δ (ppm): 10.53 (br s, 0.9H), 10.00 (br s, 1H), 9.03 (br t, J = 5.7 Hz, 1H), 8.81 (br s, 0.8H), 7.93-7.84 (m, 4H), 7.51 (d, J = 7.8 Hz, 1H), 7.40-7.16 (m, 6H), 7.04 (d, J = 7.8 Hz, 2H), 5.25-5.15 (m, 1H), 4.45 (d, J = 5.7 Hz, 2H), 3.17 (d, J = 6.6 Hz, 2H), 2.77 (dd, J = 8.4 and 15.0 Hz, 1H), 2.65 (dd, J = 5.1 and 15.0 Hz, 1H). ¹³C NMR (DMSO-d₆) δ (ppm): 168.9, 166.0, 165.5, 162.7, 145.0, 140.7, 140.3, 138.9, 136.7, 131.1, 130.3 (d, J_{C-F}= 9 Hz), 130.1, 129.7, 126.9, 123.1, 121.6, 118.4, 117.5, 115.6 (d, J_{C-F} = 22 Hz), 59.2, 40.2, 37.5, 35.3, 24.5. HRMS m/z calculated for C₂₈H₂₈N₆O₄F [M+H]⁺ 531.2156, found 531.2137.

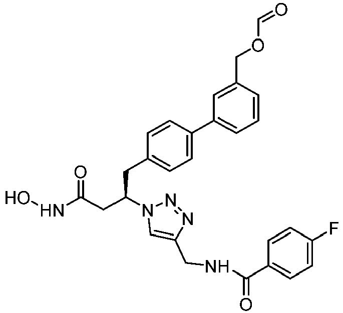

**(R)-Formic acid 4'-(2-{4-[(4-fluoro-benzoylamino)-methyl]-[1,2,3]triazol-1-yl}-3-hydroxycarbamoyl-propyl)-biphenyl-3-ylmethyl ester (40).** Compound **40** was obtained according to general procedure Q from bromo-compound **129** followed by general procedure D as a white solid (0.026 g, 7%). Purity: 100%, LC t_{R} = 2.75 min, MS (ESI+): m/z = 532 [M+H]⁺. ¹H NMR (DMSO-d₆) δ (ppm): 10.51 (br s, 0.7H), 9.01 (br t, J = 5.7 Hz, 1H), 8.80 (br s, 0.8H), 8.34 (s, 1H), 7.93-7.88 (m, 2H), 7.85 (s, 1H), 7.58 (s, 1H), 7.49-7.34 (m, 5H), 7.23 (t, J = 9.0 Hz, 2H), 7.05 (d, J = 8.1 Hz, 2H), 5.21-5.14 (m, 3H), 4.44 (d, J = 5.7 Hz, 2H), 3.16 (d, J = 7.2 Hz, 2H), 2.76 (dd, J = 8.1 and 15.6 Hz, 1H), 2.64 (dd, J = 5.4 and 15.6 Hz, 1H). ¹³C NMR (DMSO-d₆) δ (ppm): 166.0, 165.4, 162.7, 162.5, 144.9, 140.4, 138.4, 136.8, 136.8, 131.0, 130.3 (d, J_{C-F}= 9 Hz), 130.0, 129.5, 127.5, 127.0, 126.8, 126.7, 123.1, 115.6 (d, J_{C-F} = 22 Hz), 65.3, 59.2, 40.2, 37.5, 35.3. HRMS m/z calculated for C₂₇H₂₇N₅O₄F [M-COH+H]+ 504.2047, found 504.2056.

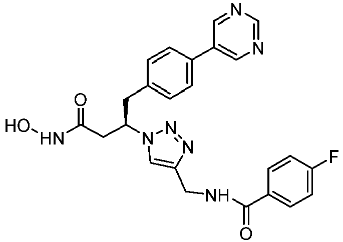

**(R)-4-fluoro-N-{1-[2-hydroxycarbamoyl-1-(4-pyrimidin-5-yl-benzyl)-ethyl]-1H-[1,2,3]triazol-4-ylmethyl}-benzamide (41).** Compound **17** was obtained according to general procedure Q from bromo-compound **129** followed by general procedure D as a yellow oil (0.109 g, 42%). Purity: 100%, LC t_{R} = 2.30 min, MS (ESI+): m/z = 476 [M+H]+. ¹H NMR (DMSO-d₆) δ (ppm): 10.54 (br s, 0.8H), 9.15 (br s, 1H), 9.02 (s, 3H), 7.92-7.87 (m, 2H), 7.83 (s, 1H), 7.60 (d, J = 8.1 Hz, 2H), 7.23 (t, J = 9.0 Hz, 2H), 7.13 (d, J = 8.1 Hz, 2H), 5.27-5.17 (m, 1H), 4.44 (d, J = 6.0 Hz, 2H), 3.21 (d, J = 5.7 Hz, 2H), 2.79 (dd, J = 9.0 and 15.3 Hz, 1H), 2.66 (dd, J = 5.4 and 15.3 Hz, 1H). ¹³C NMR (DMSO-d₆) δ (ppm): 165.4, 164.2 (d, J_{C-F} = 252 Hz), 163.5, 157.6, 154.9, 145.0, 138.3, 133.2, 132.4, 131.0, 130.4, 130.3 (d, J_{C-F} = 9 Hz), 127.2, 123.2, 115.6 (d, J_{C-F} = 22 Hz), 59.1, 40.2, 37.5, 35.2. HRMS m/z calculated for C₂₄H₂₃N₇O₃F [M+H]⁺ 476.1846, found 476.1846.

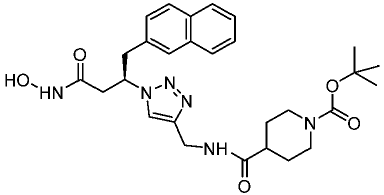

**4-{[1-(2-Hydroxycarbamoyl-1-naphthalen-2-ylmethyl-ethyl)-1H-[1,2,3]triazol-4-ylmethyl]-carbamoyl}-piperidine-1-carboxylic acid *tert*-butyl ester (42).** Compound **42** was obtained according to general procedure C from azide **107** and tert-butyl 4-(prop-2-yn-1-ylcarbamoyl)piperidine-1-carboxylate followed by general procedure E as a white foam (130 mg, 18%). Purity: 100%, LC t_{R} = 2.73 min, MS (ESI+): m/z = 439 [M + H]⁺. ¹H-NMR (DMSO-d₆) δ (ppm): 2.14 (d, 2H, J = 6.4 Hz), 2.82-2.96 (m, 2H), 3.71-3.85 (m, 2H), 4.14-4.25 (m, 1H), 6.22 (d, 1H, J = 8.3 Hz), 6.30 (t, 1H, J = 5.0 Hz), 7.13 (t, 2H, J = 8.8 Hz), 7.38 (d, 1H, J = 8.0 Hz), 7.42-7.49 (m , 2H), 7.58-7.62 (m, 2H), 7.69 (br s, 1H), 7.81-7.87 (m, 3H), 8.79 (s, 1H), 9.95 (s, 1H), 10.42 (s, 1H). ¹³C-NMR (DMSO-d₆) δ (ppm): 169.3, 167.7, 158.4 (d, J_{C-F} = 238.1 Hz), 157.9, 137.0, 135.8, 133.5, 132.2, 128.5, 128.0, 127.9, 126.4, 125.8, 121.2 (d, J_{C-F} = 7.6 Hz), 115.8 (d, J_{C-F} = 22.0 Hz), 48.9, 43.8, 37.7.

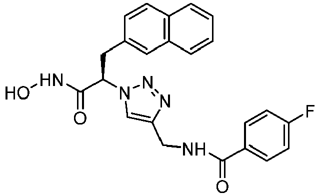

**(R)-4-Fluoro-N-[1-(1-hydroxycarbamoyl-2-naphthalen-2-yl-ethyl)-1H-[1,2,3]triazol-4-ylmethyl]-benzamide (43).** Compound **43** was obtained according to general procedure C from azide **130** and 4-fluoro-N-(prop-2-yn-1-yl)benzamide followed by general procedure D as a white solid (65 mg, 7%). Purity: 96%, LC t_{R} = 2.77 min, MS (ESI+): m/z = 434 [M+1]⁺. ¹H NMR (DMSO) δ (ppm): 9.08 (t, J = 5.0 Hz, 1H), 8.22 (s, 1H), 8.00-7.95 (m, 2H), 7.85-7.77 (m, 2H), 7.73-7.70 (m, 1H), 7.64 (s, 1H), 7.47-7.40 (m, 2H), 7.38-7.29 (m, 3H), 5.45 (t, J = 8.0 Hz,, 1H), 4.49 (d, J = 5.4 Hz, 2H), 3.54-3.52 (m, 2H). ¹³C NMR (DMSO) δ (ppm): 165.5, 164.4 (J_{C-F} = 247 Hz), 164.3, 145.5, 134.2, 133.3, 132.4, 131.1 (J_{C-F} = 3 Hz), 130.4 (J_{C-F} = 9 Hz), 128.3, 128.0, 127.9, 127.7, 126.5, 126.2, 122.5, 115.7 (J_{C-F} = 22 Hz), 62.3, 38.0, 35.3. HRMS m/z calculated for C₂₃H₂₁N₅O₃F [M + H]⁺ 434.1628, found 448.1588.

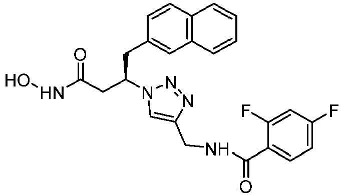

**2,4-Difluoro-N-[1-(2-hydroxycarbamoyl-1-naphthalen-2-ylmethyl-ethyl)-1H-[1,2,3]triazol-4-ylmethyl]-benzamide (46).** Compound **17** was obtained according to general procedure C from azide **107** and 2,4-difluoro-N-(prop-2-yn-1-yl)benzamide followed by general procedure E as a white foam (87 mg, 54%). Purity: 100%, LC t_{R} = 2.73 min, MS (ESI+): m/z = 4439 [M + H]⁺. ¹H-NMR (DMSO-d6) δ (ppm): 2.14 (d, 2H, J = 6.4 Hz), 2.82-2.96 (m, 2H), 3.71-3.85 (m, 2H), 4.14-4.25 (m, 1H), 6.22 (d, 1H, J = 8.3 Hz), 6.30 (t, 1H, J = 5.0 Hz), 7.13 (t, 2H, J = 8.8 Hz), 7.38 (d, 1H, J = 8.0 Hz), 7.42-7.49 (m , 2H), 7.58-7.62 (m , 2H), 7.69 (br s, 1H), 7.81-7.87 (m, 3H), 8.79 (s, 1H), 9.95 (s, 1H), 10.42 (s, 1H). ¹³C-NMR (DMSO-d6) δ (ppm): 169.3, 167.7, 158.4 (d, J_{C-F} = 238.1 Hz), 157.9, 137.0, 135.8, 133.5, 132.2, 128.5, 128.0, 127.9, 126.4, 125.8, 121.2 (d, J_{C-F} = 7.6 Hz), 115.8 (d, J_{C-F} = 22.0 Hz), 48.9, 43.8, 37.7. HRMS m/z calculated for C₂₄H₂₁F₂N₅O₃ [M+H]⁺ 466.1691, found 466.1692.

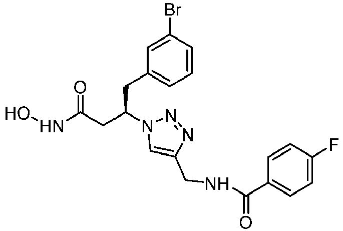

**(R)-N-{1-[1-(3-bromo-benzyl)-2-hydroxycarbamoyl-ethyl]-1H-[1,2,3]triazol-4-ylmethyl}-4-fluoro-benzamide (47).** Compound **47** was obtained according to general procedure D from carboxylic acid **128** as a colorless oil (0.042 g, 30%). Purity: 90%, LC t_{R} = 2.38 min, MS (ESI+): m/z = 478/476 [M+H]⁺. ¹H NMR (DMSO-d₆) δ (ppm): 10.52 (br s, 0.9H), 9.03 (br t, J = 5.7 Hz, 1H), 8.80 (br s, 0.8H), 7.97-7.92 (m, 2H), 7.86 (s, 1H), 7.34-7.25 (m, 4H), 7.12 (t, J = 7.5 Hz, 1H), 6.96 (d, J = 7.5 Hz, 1H), 5.22-5.13 (m, 1H), 4.45 (d, J = 5.7 Hz, 2H), 3.15-3.12 (m, 2H), 2.75 (dd, J = 9.3 and 15.3 Hz, 1H), 2.60 (dd, J = 5.4 and 15.3 Hz, 1H). ¹³C NMR (acetone-d₆) δ (ppm): 166.4, 165.7, 164.5 (d, J_{C-F} = 248 Hz), 144.5, 139.8, 132.0, 130.9, 130.2, 129.9, (d, J_{C-F} = 9 Hz), 129.7, 128.0, 123.2, 121.9, 115.1 (d, J_{C-F} = 22 Hz), 59.4, 40.5, 37.4, 34.8. HRMS m/z calculated for C₂₀H₂₀N₅O₃BrF [M+H]⁺ 476.0734, found 476.0732.

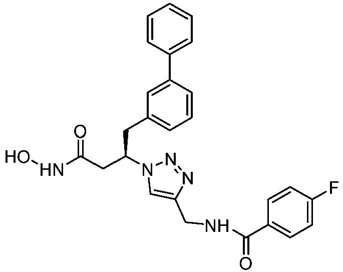

**(R)-N-[1-(1-biphenyl-3-ylmethyl-2-hydroxycarbamoyl-ethyl)-1H-[1,2,3]triazol-4-ylmethyl]-4-fluoro-benzamide (48).** Compound **48** was obtained according to general procedure Q from bromo-compound **128** followed by general procedure D as a colorless oil (0.034 g, 24%). Purity: 96%, LC t_{R} = 2.53 min, MS (ESI+): m/z = 474 [M+H]⁺. ¹H NMR (acetone-d₆) δ (ppm): 8.14 (s, 1H), 7.97-7.76 (m, 3H), 7.54-7.15 (m, 9H), 7.04 (d, J = 7.5 Hz, 1H), 5.35-5.31 (m, 1H), 4.60-4.46 (m, 2H), 3.34 (d, J = 9.6 Hz, 2H), 3.02-2.83 (m, 2H). ¹³C NMR (acetone-d₆) δ (ppm): 166.5, 165.6, 164.5 (d, J_{C-F} = 248 Hz), 144.4, 141.0, 140.7, 137.7, 130.8, 129.9 (d, J_{C-F} = 9 Hz), 128.9, 128.8, 128.1, 127.7, 127.3, 126.8, 125.3, 123.3, 115.1 (d, J_{C-F} = 22 Hz), 59.7, 41.0, 37.5, 34.9. HRMS m/z calculated for C₂₆H₂₅N₅O₃F [M+H]⁺ 474.1941, found 474.1934.

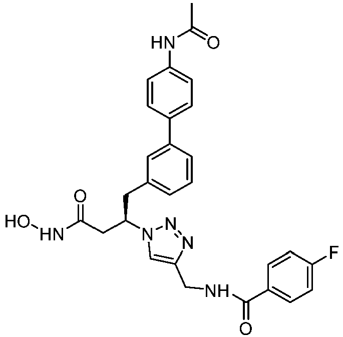

**(R)-N-{1-[1-(4'-acetylamino-biphenyl-3-ylmethyl)-2-hydroxycarbamoyl-ethyl]-1H-[1,2,3]triazol-4-ylmethyl}-4-fluoro-benzamide (49).** Compound **49** was obtained according to general procedure Q from bromo-compound **128** followed by general procedure D as a colorless oil (0.060 g, 38%). Purity: 95%, LC t_{R} = 2.15 min, MS (ESI+): m/z = 531 [M+H]⁺. ¹H NMR (acetone-d₆) δ (ppm): 9.36 (br s, 0.5H), 8.46 (br s, 0.5H), 8.14 (s, 1H), 7.96-7.14 (m, 11H), 6.99 (d, J = 7.2 Hz, 1H), 5.33-5.30 (m, 1H), 4.55-4.49 (m, 2H), 3.33-3.31 (m, 2H), 3.05-2.82 (m, 2H), 2.11 (s, 3H). ¹³C NMR (acetone-d₆) δ (ppm): 168.4, 166.6, 165.7, 164.5 (d, J_{C-F} = 248 Hz), 144.4, 140.5, 138.9, 137.6, 135.4, 129.9 (d, J_{C-F} = 9 Hz), 128.9, 127.6, 127.2, 127.0, 124.9, 123.3, 119.5, 119.4, 115.1 (d, J_{C-F} = 22 Hz), 59.7, 41.1, 37.4, 34.9, 23.4. HRMS m/z calculated for C₂₈H₂₈N₆O₄F [M+H]⁺ 531.2156, found 531.2167.

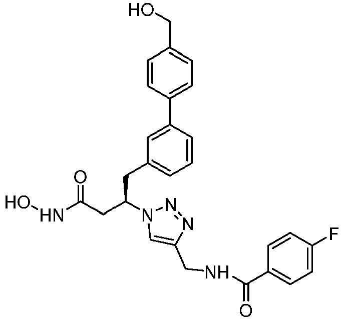

**(R)-4-fluoro-N-{1-[2-hydroxycarbamoyl-1-(4'-hydroxymethyl-biphenyl-3-ylmethyl)-ethyl]-1H-[1,2,3]triazol-4-ylmethyl}-benzamide (50).** Compound **50** was obtained according to general procedure Q from bromo-compound **128** followed by general procedure D as a white solid (0.013 g, 22%). Purity: 97%, LC t_{R} = 2.18 min, MS (ESI+): m/z = 504 [M+H]⁺. ¹H NMR (DMSO-d₆) δ (ppm): 10.53 (br s, 0.9H), 9.03 (br t, J = 5.7 Hz, 1H), 8.80 (br s, 0.9H), 7.95-7.89 (m, 3H), 7.51-7.23 (m, 9H), 6.96 (d, J = 7.8 Hz, 1H), 5.24-5.20 (m, 2H), 4.52 (d, J = 5.7 Hz, 2H), 4.45 (d, J = 5.7 Hz, 2H), 3.20 (d, J = 7.2 Hz, 2H), 2.78 (dd, J = 8.7 and 15.3 Hz, 1H), 2.65 (dd, J = 5.4 and 15.3 Hz, 1H). ¹³C NMR (DMSO-d₆) δ (ppm): 166.0, 165.4, 164.1 (d, J_{C-F} = 204 Hz), 144.9, 142.2, 140.5, 138.8, 138.0, 131.1, 130.4 (d, J_{C-F} = 9 Hz), 129.3, 128.3, 127.6, 127.4, 126.8, 125.4, 123.2, 115.7 (d, J_{C-F} = 22 Hz), 63.0, 59.2, 41.3, 37.4, 35.3. HRMS m/z calculated for C₂₇H₂₇N₅O₄F [M+H]⁺ 504.2047, found 504.2033.

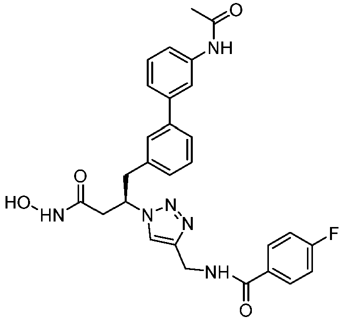

**(R)-N-{1-[1-(3'-acetylamino-biphenyl-3-ylmethyl)-2-hydroxycarbamoyl-ethyl]-1H-[1,2,3]triazol-4-ylmethyl}-4-fluoro-benzamide (51).** Compound **51** was obtained according to general procedure Q from bromo-compound **128** followed by general procedure D as a colorless oil (0.069 g, 41%). Purity: 100%, LC t_{R} = 2.22 min, MS (ESI+): m/z = 531 [M+H]⁺. ¹H NMR (DMSO-d₆) δ (ppm): 10.53 (br s, 0.8H), 10.04 (br s, 1H), 9.03 (br t, J = 5.7 Hz, 1H), 7.94-7.89 (m, 3H), 7.79 (s, 1H), 7.56 (d, J = 7.8 Hz, 1H), 7.37-7.18 (m, 7H), 6.95 (d, J = 7.8 Hz, 1H), 5.27-5.17 (m, 0.9H), 4.45 (d, J = 5.7 Hz, 2H), 3.20 (d, J = 6.9 Hz, 2H), 2.78 (dd, J = 8.7 and 15.0 Hz, 1H), 2.65 (dd, J = 5.7 and 15.0 Hz, 0.9H). ¹³C NMR (DMSO-d₆) δ (ppm): 168.9, 165.5, 164.3 (d, J_{C-F} = 245 Hz), 145.0, 141.0, 140.7, 140.2, 138.1, 131.1, 131.0, 130.4 (d, J_{C-F}= 9 Hz), 129.7, 129.4, 128.6, 127.7, 125.4, 123.1, 121.9, 118.6, 117.7, 115.7 (d, J_{C-F} = 22 Hz), 59.3, 41.3, 37.5, 35.3, 24.5. HRMS m/z calculated for C₂₈H₂₈N₆O₄F [M+H]⁺ 531.2156, found 531.2172.

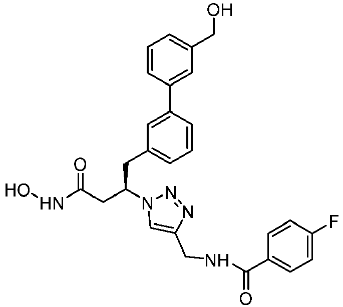

**(R)-4-fluoro-N-{1-[2-hydroxycarbamoyl-1-(3'-hydroxymethyl-biphenyl-3-ylmethyl)-ethyl]-1H-[1,2,3]triazol-4-ylmethyl}-benzamide (52).** Compound **52** was obtained according to general procedure Q from bromo-compound **128** followed by general procedure D as a white solid (0.040 g, 32%). Purity: 100%, LC t_{R} = 2.27 min, MS (ESI+): m/z = 504 [M+H]⁺. ¹H NMR (DMSO-d₆) δ (ppm): 10.53 (br s, 0.9H), 9.02 (br t, J = 5.7 Hz, 1H), 8.81 (br s, 0.8H), 7.94-7.88 (m, 3H), 7.50-7.23 (m, 9H), 6.96 (d, J = 7.8 Hz, 1H), 5.28-5.20 (m, 2H), 4.56 (d, J = 6.3 Hz, 2H), 4.46 (d, J = 5.7 Hz, 2H), 3.21 (d, J = 6.3 Hz, 2H), 2.79 (dd, J = 9.0 and 15.0 Hz, 1H), 2.65 (dd, J = 5.4 and 15.0 Hz, 1H). ¹³C NMR (DMSO-d₆) δ (ppm): 166.0, 165.5, 164.1 (d, J_{C-F} = 206 Hz), 144.9, 143.6, 140.8, 140.3, 138.1, 131.1, 131.0, 130.4 (d, J_{C-F}= 9 Hz), 129.3, 129.1, 128.5, 127.8, 126.0, 125.5, 125.2, 123.1, 115.6 (d, J_{C-F} = 22 Hz), 63.4, 59.2, 41.3, 37.4, 35.3. HRMS m/z calculated for C₂₇H₂₇N₅O₄F [M+H]⁺ 504.2047, found 504.2043.

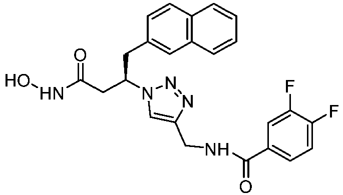

**3,4-difluoro-N-[[1-[(1R)-3-(hydroxyamino)-1-(2-naphthylmethyl)-3-oxopropyl]triazol-4-yl]methyl]benzamide (54).** Compound **54** was obtained according to general procedure C from azide **107** and 3,4-difluoro-N-(prop-2-yn-1-yl)benzamide followed by general procedure E as a white foam (62 mg, 45%), Purity: 100%, LC t_{R} = 2.47 min, MS (ESI+): m/z = 466 [M + H]⁺. ¹H NMR (MeOD) δ (ppm): 8.56 (s, 1H), 7.71-7.60 (m, 4H), 7.70-7.51 (m, 1H), 7.47 (s, 1H), 7.45 (s, 1H), 7.42-7.25 (m, 2H), 7.25-7.19 (m, 1H), 7.14-7.10 (dd, J = 1.8 Hz, J = 10.2 Hz, 1H), 5.35-5.22 (m, 1H), 4.46 (s, 2H), 3.39 (d, J = 7.5 Hz, 2H), 3.31 (dd, J = 1.5 Hz, J = 3.3 Hz, 1H), 2.85 (dd, J = 1.5 Hz, J = 3.3 Hz, 1H). ¹³C NMR (MeOD) δ (ppm): 168.6, 167.4, 145.5, 135.4, 134.0, 133.0, 129.2, 128.8, 128.7, 128.5 (2C), 128.0, 127.1, 126.7, 125.5, 125.0, 118.6, 118.4, 118.0, 117.8, 61.5, 42.5, 38.9, 36.0.¹⁹F NMR (MeOD) δ (ppm): -136.3 (d, J = 19.7 Hz), -140.0 (d, J = 19.7 Hz). HRMS m/z calculated for C₂₄H₂₂FN₅O₃ [M + H]⁺ 466.1691, found 466.1688.

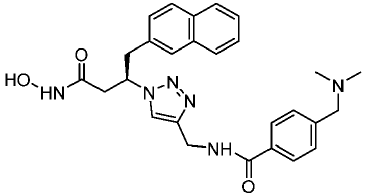

**4-[(dimethylamino)methyl]-N-[[1-[(1R)-3-(hydroxyamino)-1-(2-naphthylmethyl)-3-oxo-propyl]triazol-4-yl]methyl]benzamide (55).** Compound **55** was obtained according to general procedure C from azide **107** and 4-((dimethylamino)methyl)-N-(prop-2-yn-1-yl)benzamide followed by general procedure E as a white solid (13 mg, 16%). Purity: 100%, LC t_{R} = 1.88 min, MS (ESI+): m/z = 487 [M + H]⁺. ¹H NMR (MeOD) δ (ppm): 8.49 (s, 1H), 7.80 (d, J = 8.1 Hz, 2H), 7.71-7.63 (m, 4H), 7.51 (d, J = 8.1 Hz, 2H), 7.44 (s, 1H), 7.39-7.32 (m, 2H), 7.12 (dd, J = 1.5 Hz, J = 8.4 Hz, 1H), 5.32-5.28 (m, 1H), 4.50 (s, 2H), 4.03 (s, 2H), 3.42-3.39 (m, 2H), 2.95-2.84 (m, 2H), 2.62 (s, 6H). ¹³C NMR (MeOD) δ (ppm): 169.2, 168.5, 145.6, 137.9, 135.9, 135.4, 134.8, 134.0, 133.9, 131.5 (2C), 129.2, 129.0 (2C), 128.8, 128.6, 128.5, 128.0, 127.1, 126.7, 124.8, 62.7, 61.5, 43.9, 42.4, 38.8, 36.1. HRMS m/z calculated for C₂₇H₃₁N₆O₃ [M + H]⁺ 487.2458, found 487.2458.

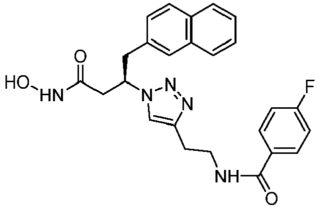

**4-fluoro-N-[2-[1-[(1R)-3-(hydroxyamino)-1-(2-naphthylmethyl)-3-oxo-propyl]triazol-4-yl]ethyl]benzamide) (56).** Compound **56** was obtained according to general procedure C from azide **107** and N-(but-3-yn-1-yl)-4-fluorobenzamide followed by general procedure F as a white foam (27 mg, 54%), Purity: 100%, LC t_{R} = 2.10 min, MS (ESI+): m/z = 462 [M + H]⁺. ¹H NMR (MeOD) δ (ppm): 8.55 (s, 1H), 7.79-7.71 (m, 3H), 7.67 (d, J = 8.1 Hz, 2H), 7.52 (s, 1H, H₅), 7.44-7.36 (m, 3H), 7.17-7.11 (m, 3H), 5.29-5.27 (m, 1H), 3.50 (t, J = 4.8 Hz, 2H), 3.39 (t, J = 4.8 Hz, 2H), 2.98-2.81 (m, 4H, H₂). ¹³C NMR (MeOD) δ (ppm): 167.7, 167.2, 166.4, 163.1, 144.2, 134.1, 133.4, 132.5, 130.5, 129.5, 127.8, 127.4 (2C), 127.1, 126.6, 125.8, 125.4, 122.8, 115.1, 114.8, 60.0, 41.0, 39.3, 37.6, 24.9. ¹⁹F NMR (MeOD) δ (ppm): -111.2. HRMS m/z calculated for C₂₅H₂₅FN₅O₃ [M + H]⁺ 462.1942, found 462.1953.

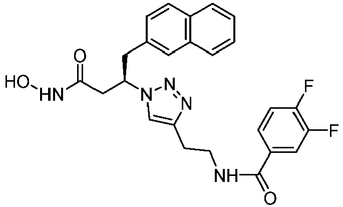

**3,4-difluoro-N-[2-[1-[(1R)-3-(hydroxyamino)-1-(2-naphthylmethyl)-3-oxo-propyl]triazol-4-yl]ethyl]benzamide (57).** Compound **57** was obtained according to general procedure C from azide **107** and N-(but-3-yn-1-yl)-3,4-difluorobenzamide followed by general procedure F as a white foam (27 mg, 89%). Purity: 100%, LC t_{R} = 2.50 min, MS (ESI+): m/z = 480 [M + H]⁺. ¹H NMR (MeOD) δ (ppm): 8.54 (s, 1H), 7.74-7.64 (m, 4H), 7.59-7.40 (m, 1H), 7.39 (s, 1H), 7.39-7.14 (m, 4H), 7.11 (dd, J = 1.5 Hz, J = 8.4 Hz , 1H), 5.36-5.16 (m, 1H), 3.52-3.41 (td, J = 3.3 Hz, J = 7.2 Hz, 2H), 3.35 (t, J = 7.2 Hz, 2H), 2.93-2.83 (m, 4H, H₂). ¹³C NMR (MeOD) δ (ppm): 167.2, 166.3, 154.0, 151.6, 150.7, 148.3, 144.1, 134.1, 133.4, 132.5, 131.5, 127.8, 127.2, 126.6, 125.8, 125.4, 124.1, 122.9, 117.2, 116.5, 60.0, 41.0, 39.4, 37.6, 24.9. ¹⁹F NMR (MeOD) δ(ppm): -136.5 (d, J = 17.8 Hz), -140.0 (d, J = 20.6 Hz). HRMS m/z calculated for C₂₅H₂₄F₂N₅O₃ [M + H]⁺ 480.1847, found 480.1856.

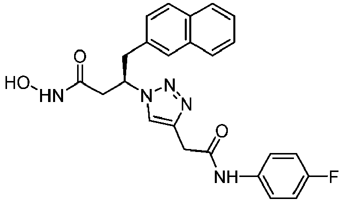

**3-{4-[(4-Fluoro-phenylcarbamoyl)-methyl]-[1,2,3]triazol-1-yl}-N-hydroxy-4-naphthalen-2-yl-butyramide (58).** Compound **58** was obtained according to general procedure C from azide **107** and N-(4-fluorophenyl)but-3-ynamide followed by general procedure G as a white foam (3 mg, 1.2% over 2 steps). Purity: 100%, LC t_{R} = 2.47 min, MS (ESI+): m/z = 448 [M + H]⁺. ¹H NMR (MeOD) δ (ppm): 2.86 (dd, J = 5.7 Hz, J = 14.9 Hz, 1H), 2.96 (dd, J = 8.9 Hz, J = 14.9 Hz, 1H), 3.40-3.43 (m, 2H), 4.74 (s, 2H), 3.68 (s, 2H), 5.25-5.35 (m, 1H), 7.03 (t, J = 8.8 Hz, 2H), 7.35-7.39 (m, 2H), 7.45-7.51 (m, 3H), 7.65-7.74 (m, 4H). ¹³C NMR (MeOD) δ (ppm): 34.5, 38.9, 42.4, 61.6, 116.2 (d, J = 22.9 Hz, 2C), 123.1 (d, J = 8.0 Hz, 2C), 125.1, 126.7, 127.1, 128.0, 128.5, 128.6, 128.9, 129.2, 133.9, 134.8, 135.4, 135.9, 142.0, 160.7 (d, J = 241.5 Hz), 168.6, 169.9. ¹⁹F NMR (MeOD) δ (ppm): -120.9. HRMS m/z calculated for C₂₄H₂₃FN₅O₃ [M + H]⁺ 448.1785, found 448.1809.

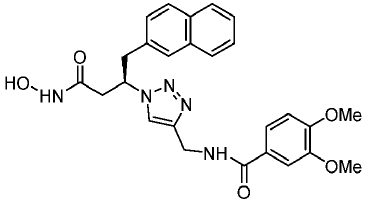

**N-[[1-[(1R)-3-(hydroxyamino)-1-(2-naphthylmethyl)-3-oxo-propyl]triazol-4-yl]methyl]-3,4-dimethoxy-benzamide (60).** Compound **17** was obtained according to general procedure C from azide **107** and 3,4-dimethoxy-N-(prop-2-yn-1-yl)benzamide followed by general procedure F as a white solid (25 mg, 66%). Purity: 100%, LC t_{R} = 3.22 min, MS (ESI+): m/z = 444 [M + H]⁺. ¹H NMR (MeOD-*d₄*) δ (ppm): 7.93-7.79 (m, 1H), 7.80-7.60 (m, 3H), 7.43-7.29 (m, 5H), 7.12 (d, J = 7.2 Hz, 1H), 6.99 (d, J = 7.2 Hz, 1H), 5.38-5.26 (m, 1H), 4.55-4.42 (m, 2H), 3.83 (s, 3H), 3.84 (s, 3H), 3.50-3.37 (m, 2H), 3.20 (dd, J = 14.7 and 7.5 Hz, 1H), 2.81-3.02 (m, 1H). HRMS m/z calculated for C₂₄H₂₃FN₅O₃ [M + H]⁺ 448.1785, found 448.1801.

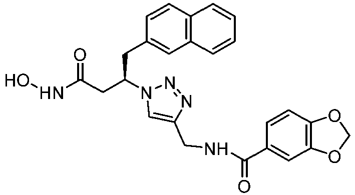

**N-[[1-[(1R)-3-(hydroxyamino)-1-(2-naphthylmethyl)-3-oxo-propyl]triazol-4-yl]methyl]-1,3-benzodioxole-5-carboxamide (61).** Compound **61** was obtained according to general procedure C from azide **107** and N-(prop-2-yn-1-yl)benzo[d][1,3]dioxole-5-carboxamide followed by general procedure F as a white solid (22 mg, 45%). Purity: 100%, LC t_{R} = 2.65 min, MS (ESI+): m/z = 474 [M + H]⁺. ¹H NMR (MeOD-*d₄*) δ (ppm): 7.67-7.59 (m, 4H), 7.42-7.30 (m, 4H), 7.21 (d, J = 1.8 Hz, 1H), 7.12 (dd, J = 8.1 and 1.8 Hz, 1H), 6.85 (d, J = 8.1 Hz, 1H), 6.04 (s, 2H), 5.31-5.26 (m, 1H), 4.46 (s, 2H), 3.43-3.37 (m, 2H), 2.95 (dd, J = 15.0 and 8.7 Hz, 1H), 2.86 (dd, J = 15.0 and 5.7 Hz, 1H). HRMS m/z calculated for C₂₅H₂₃N₅O₅ [M+H]⁺ 474.1777 found 474.1772.

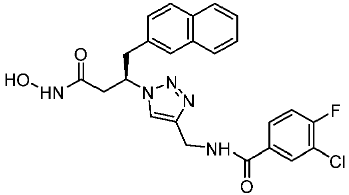

**3-chloro-4-fluoro-N-[[1-[(1R)-3-(hydroxyamino)-1-(2-naphthylmethyl)-3-oxo-propyl]triazol-4-yl]methyl]benzamide (62).** Compound **62** was obtained according to general procedure C from azide **107** and 3-chloro-4-fluoro-N-(prop-2-yn-1-yl)benzamide followed by general procedure F as a white solid (25 mg, 47%). Purity: 100%, LC t_{R} = 2.87 min, MS (ESI+): m/z = 482 [M + H]⁺. ¹H NMR (DMSO-*d₆*) δ (ppm): 10.52 (br s, 1H), 9.11 (t, J = 5.7 Hz, 1H), 8.80 (br s, 1H), 8.05 (dd, J = 4.8 and 2.1 Hz, 1H), 7.90-7.85 (m, 2H), 7.78-7.67 (m, 3H), 7.54 (t, J = 8.7 Hz, 1H), 7.46-7.35 (m, 3H), 7.13 (dd, J = 8.4 and 1.5 Hz, 1H), 5.32-5.22 (m, 1H), 4.42 (d, J = 5.7 Hz, 2H), 3.34-3.26 (m, 2H + DMSO), 2.78 (dd, J = 15.0 and 8.7 Hz, 1H), 2.67 (dd, J = 15.0 and 5.7 Hz, 1H). ¹⁹F NMR (MeOD-*d₄*) δ (ppm): -113.7. HRMS m/z calculated for C₂₄H₂₁ClFN₅O₃ [M+H]⁺ 482.1395, found 482.1393.

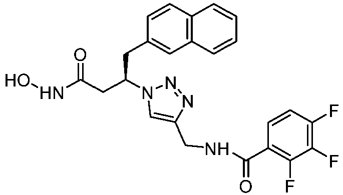

**2,3,4-Trifluoro-N-[1-(1-hydroxycarbamoylmethyl-2-naphthalen-2-yl-ethyl)-1H-[1,2,3]triazol-4-ylmethyl]-benzamide (63).** Compound **63** was obtained according to general procedure C from azide **107** and 2,3,4-trifluoro-N-(prop-2-yn-1-yl)benzamide followed by general procedure G as a white foam (15 mg, 10% over 2 steps). Purity: 100%, LC t_{R} = 2.50 min, MS (ESI+): m/z = 484 [M + H]⁺. ¹H NMR (MeOD) δ (ppm): 2.88 (dd, J = 5.7 Hz, J = 14.9 Hz, 1H), 2.97 (dd, J = 9.0 Hz, J = 14.9 Hz, 1H), 3.38-3.45 (m, 2H), 4.48 (br s, 1H), 5.25-5.34 (m, 1H), 7.58 (s, 1H), 7.64-7.71 (m, 3H). ¹³C NMR (MeOD) δ (ppm): 36.0, 38.9, 42.4, 61.5, 113.7 (dd, J = 18.6 Hz, 2C), 118.5 (d, J = 18.0 Hz), 124.8, 125.5 (dd, J = 3.8, J = 7.3 Hz), 126.3 (dd, J = 3.5, J = 7.2 Hz), 125.6 (d, J = 3.7 Hz, J = 18.1 Hz), 122.0 (d, J = 11.0 Hz), 124.9, 130.8 (2C), 125.8 (br s), 126.7, 127.0, 128.0, 128.5 (2C), 128.53, 128.8, 129.2, 133.9, 134.8, 135.4, 141.0 (td, J = 15.9 Hz, J = 250.3 Hz), 150.7 (ddd, J = 3.3 Hz, J = 11.2 Hz, J = 254.4 Hz), 154.0 (ddd, J = 2.9 Hz, J = 9.9 Hz, J = 253.5 Hz), 164.6, 168.5. ¹⁹F NMR (MeOD) δ (ppm): -133.6 (dd, J = 9.7 Hz, J = 20.0 Hz), -137.6 (dd, J = 9.7 Hz, J = 20.0 Hz), -163.3 (t, J = 20.0 Hz). HRMS m/z calculated for C₂₄H₂₁F₃N₅O₃ [M + H]⁺ 484.1596, found 484.1644.

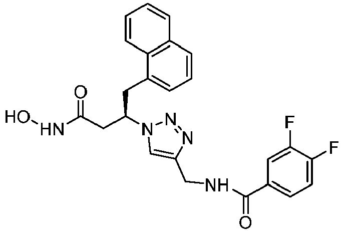

**Methyl (3R)-3-[4-[[(3,4-difluorobenzoyl)aminolmethyl]triazol-1-yl]-4-(1-naphthyl)butanoate (64)** Compound **64** was obtained according to general procedure C from azide **147** and 3,4-difluoro-N-(prop-2-yn-1-yl)benzamide followed by general procedure G as a white foam (45 mg, 58% over 2 steps). Purity: 95%, LC t_{R} = 2.45 min, MS (ESI+): m/z = 466 [M + H]⁺. ¹H NMR (MeOD) δ (ppm) (ppm): 2.98-3.01 (m, 2H), 3.53 (dd, J = 10.2 Hz, J = 14.0 Hz, 1H), 3.82 (dd, J = 4.5 Hz, J = 14.0 Hz, 1H) 4.43 (d, J = 2.5 Hz, 2H), 5.38-5.28 (m, 1H), 6.94 (d, J = 7.0 Hz, 1H), 7.14 (t, J = 8.0 Hz, 1H), 7.32-7.54 (m, 4H), 7.59-7.73 (m, 3H), 7.77 (d, J = 8.0 Hz, 1H), 8.05 (d, J = 8.0 Hz, 1H). ¹³C NMR (MeOD) δ (ppm): 35.9, 38.9, 39.5, 60.8, 117.9 (d, J = 19.0 Hz), 118.4 (d, J = 17.9 Hz), 124.9, 124.9, 125.6 (dd, J = 3.7 Hz, J = 7.3 Hz,), 126.2, 126.8, 127.5, 128.6, 128.9, 129.9, 132.6, 133.3, 133.8, 135.3, 145.4, 150.9 (dd, J = 13.3 Hz; J = 176.0 Hz), 154.2 (dd, J = 12.9 Hz; J = 186.8 Hz), 167.4, 168.6. ¹⁹F NMR (MeOD) δ (ppm): -136.5 (d, J = 17.8 Hz), - 140.0 (d, J = 20.6 Hz). HRMS m/z calculated for C₂₄H₂₂F₂N₅O₃ [M + H]⁺ 466.1691, found 466.1718.

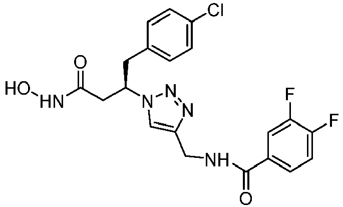

**N-[[1-[(1R)-1-[(4-chlorophenyl)methyl]-3-(hydroxyamino)-3-oxo-propyl]triazol-4-yl]methyl]-3,4-difluoro-benzamide (65).** Compound **65** was obtained according to general procedure C from azide **148** and 3,4-difluoro-N-(prop-2-yn-1-yl)benzamide followed by general procedure G as a white foam (55 mg, 28% over 4 steps). Purity: 100%, LC t_{R} = 2.40 min, MS (ESI+): m/z = 450 [M + H]⁺. ¹H NMR (MeOD) δ (ppm): 2.81 (dd, J = 5.7 Hz, J = 14.8 Hz, 2H), 2.90 (dd, J = 8.9 Hz, J = 14.8 Hz, 2H), 3.19-3.26 (m, 2H), 4.52 (s, 2H), 5.10-5.20 (m, 1H), 6.94 (d, J = 8.3 Hz, 2H), 7.12 (d, J = 8.3 Hz, 2H), 7.37 (td, J = 8.3 Hz, J = 10.2 Hz, 1H), 7.61 (s, 1H), 7.65-7.69 (m, 1H), 7.75 (ddd, J = 2.1 Hz, J = 7.7 Hz, J = 11.1 Hz, 1H). ¹³C NMR (MeOD) δ (ppm): 36.1, 38.7, 41.5, 61.3, 117.9 (d, J = 18.8 Hz), 118.5 (d, J = 18.0 Hz), 124.9, 125.5 (dd, J = 3.7 Hz, J = 7.3 Hz), 129.6 (2C), 131.6 (2C), 132.6, 133.8, 136.7, 145.6, 151.4 (dd, J = 13.1 Hz, J = 248.4 Hz), 153.8 (dd, J = 12.6 Hz, J = 252.2 Hz), 167.5, 168.4. ¹⁹F NMR (MeOD) δ (ppm): -136.3 (d, J = 20.5 Hz), -140.0 (d, J = 20.5 Hz). HRMS m/z calculated for C₂₀H₁₉ClF₂N₅O₃ [M + H]⁺ 450.1144, found 450.1172.

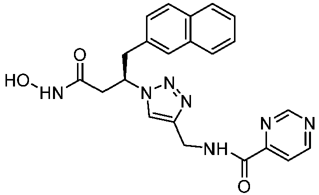

**N-[[1-[(1R)-3-(hydroxyamino)-1-(2-naphthylmethyl)-3-oxo-propyl]triazol-4-yl]methyl]pyrimidine-4-carboxamide (66).** Compound **65** was obtained according to general procedure C from azide **107** and N-(prop-2-yn-1-yl)pyrimidine-4-carboxamide followed by general procedure G. White foam (40 mg, 20% over 2 steps). Purity: 100%, LC t_{R} = 2.05 min, MS (ESI+): m/z = 432 [M + H]⁺. ¹H NMR (MeOD) δ (ppm): 2.86 (dd, J = 5.6 Hz, J = 14.9 Hz), 2.97 (dd, J = 9.6 Hz, J = 14.9 Hz), 3.39-3.35 (m, 2H), 4.53 (s, 2H), 5.33-5.23 (m, 1H), 7.11 (dd, J = 1.5 Hz, J = 8.4 Hz, 1H), 7.32-7.27 (m, 2H), 7.37 (s, 1H), 7.65-7.56 (m, 4H), 7.97 (dd, J = 1.1 Hz, J = 5.1 Hz, 1H), 8.98 (d, J = 5.1 Hz, 1H), 9.21 (d, J = 1.1 Hz, 1H). ¹³C NMR (MeOD) δ (ppm): 35.5, 38.8, 42.5, 61.5, 119.8, 125.0, 126.7, 127.0, 128.0, 128.4, 128.5, 128.8, 129.2, 133.8, 134.8, 135.3, 145.1, 157.7, 159.0, 160.4, 164.7, 168.5. HRMS m/z calculated for C₂₂H₂₁N₇O₃ [M + H]⁺ 432.1784, found 432.1783.

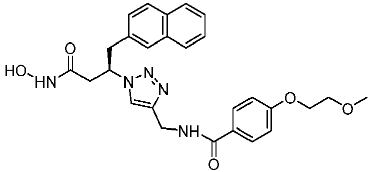

**N-[[1-[(1R)-3-(hydroxyamino)-1-(2-naphthylmethyl)-3-oxo-propyl]triazol-4-yl]methyl]-4-(2-methoxyethoxy)benzamide (68).** Compound **68** was obtained according to general procedure C from azide **107** and 4-(2-methoxyethoxy)-N-(prop-2-yn-1-yl)benzamide followed by general procedure G as a white foam (75 mg, 42% over 2 steps). Purity: 100%, LC t_{R} = 2.33 min, MS (ESI+): m/z = 504 [M + H]⁺. ¹H NMR (MeOD) δ (ppm): 2.97-2.81 (m, 2H), 3.39-7.30 (m, 2H), 3.42 (s, 3H), 3.75 (td, J = 3.1 Hz, J = 6.2 Hz, 2H), 4.16 (td, J = 3.1 Hz, J = 6.2 Hz, 2H), 4.47 (s, 1H), 5.33-5.24 (m, 1H), 6.97 (d, J = 8.9 Hz, 2H), 7.11 (dd, J = 1.4 Hz, J = 8.4 Hz , 1H), 7.36-7.32(m, 2H), 7.41 (s, 1H), 7.61 (s, 1H), 7.73-7.62 (m, 5H). ¹³C NMR (MeOD) δ (ppm): 35.9, 38.9, 42.4, 59.3, 61.5, 68.6, 72.0, 115.3 (2C), 124.7, 126.7, 127.1, 127.5, 128.0, 128.5 (2C), 128.8, 129.2, 130.3 (2C), 133.8, 134.8, 135.3, 146.0, 163.1, 168.5, 169.4. HRMS m/z calculated for C₂₇H₃₀N₅O₅ [M + H]⁺ 504.2247, found 504.2224.

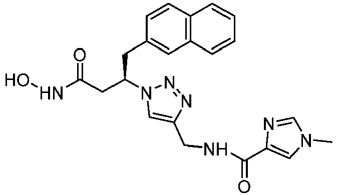

**N-[[1-[(1R)-3-(hydroxyamino)-1-(2-naphthylmethyl)-3-oxo-propyl]triazol-4-yl]methyl]-1-methyl-imidazole-4-carboxamide (69).** Compound **69** was obtained according to general procedure C from azide **107** and 1-methyl-N-(prop-2-yn-1-yl)-1H-imidazole-4-carboxamide followed by general procedure G as a white foam (23.2 mg, 29% over 2 steps). Purity: 100%, LC t_{R} = 2.00 min, MS (ESI+): m/z = 434 [M + H]⁺. ¹H NMR (MeOD) δ: 2.83 (dd, J = 5.7 Hz, J = 14.8 Hz, 1H), 2.93 (dd, J = 8.9 Hz, J = 14.8 Hz, 1H), 3.37-3.40 (m, 2H), 3.34 (s, 3H), 4.78 (s, 2H), 5.23-5.33 (m, 1H), 7.11 (dd, J = 1.7 Hz, J = 8.4 Hz, 1H), 7.32-7.38 (m, 2H), 7.42 (s, 1H), 7.57 (d, J = 6.7 Hz, 2H), 7.56-7.71 (m, 4H). ¹³C NMR (MeOD) δ (ppm): 34.0, 35.1, 38.9, 42.4, 61.5, 124.7, 125.1, 126.7, 127.0, 128.0, 128.5, 128.6, 128.8, 129.2, 133.9, 134.8, 135.4, 137.1, 139.8, 145.7, 164.8, 168.5. HRMS m/z calculated for C₂₂H₂₄N₇O₃ [M + H]⁺ 434.1941, found 434.1946.

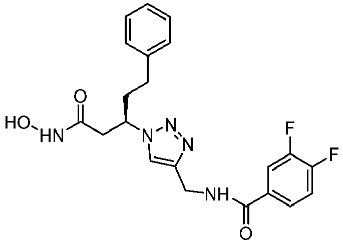

**3,4-difluoro-N-[[1-[(1R)-3-(hydroxyamino)-3-oxo-1-(2-phenylethyl)propyl]triazol-4-yl]methyl]benzamide (70).** Compound **70** was obtained according to general procedure C from azide **149** and 3,4-difluoro-N-(prop-2-yn-1-yl)benzamide followed by general procedure G as a white foam (41 mg, 18% over 4 steps). Purity: 100%, LC t_{R} = 2.37 min, MS (ESI+): m/z = 430 [M + H]⁺. ¹H NMR (MeOD) δ (ppm): 2.15-2.39 (m, 2H), 2.41-2.49 (m, 2H), 4.63 (s, 2H), 4.90-4.98 (m, 1H), 7.08-7.25 (m, 5H), 7.36 (ddd, J = 8.0 Hz, J = 8.5 Hz, J = 10.3 Hz, 1H), 7.72 (dddd, J = 1.4 Hz, J = 2.2 Hz, J = 4.3 Hz, J = 8.5 Hz, 1H), 7.80 (ddd, J = 2.2 Hz, J = 7.7 Hz, J = 11.3 Hz, 1H), 7.87 (s, 1H). ¹³C NMR (MeOD) δ (ppm): 32.8, 36.3, 37.5, 39.7, 59.5, 117.9 (d, J = 19.0 Hz), 118.5 (d, J = 17.9 Hz), 124.5, 125.6, 125.6 (dd, J = 3.7, J = 7.3 Hz), 127.2, 129.4 (2C), 129.5 (2C), 132.7, 141.7, 145.8, 154.8 (dd, J = 13.0 Hz, J = 248.0 Hz), 155.7 (dd, J = 12.7 Hz, J = 252.0 Hz), 167.7, 168.5. ¹⁹F NMR (MeOD) δ (ppm): -136.2 (d, J = 20.5 Hz), -140.0 (d, J = 20.5 Hz). HRMS m/z calculated for C₂₁H₂₂F₂N₅O₃ [M + H]⁺ 430.1691, found 430.1670.

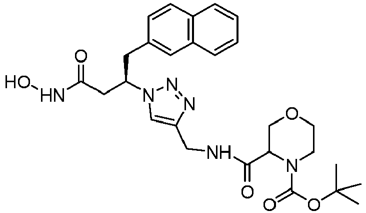

***Tert-butyl* 3-[[1-[(1R)-3-(hydroxyamino)-1-(2-naphthylmethyl)-3-oxo-propyl]triazol-4-yl]methylcarbamoyl]morpholine-4-carboxylate (71).** Compound **71** was obtained according to general procedure C from azide **107** and tert-butyl 3-(prop-2-yn-1-ylcarbamoyl)morpholine-4-carboxylate followed by general procedure G as a white foam (59 mg, 17% over 2 steps). Purity: 100%, LC t_{R} = 2.43 min, MS (ESI+): m/z = 539 [M + H]⁺. ¹H NMR (MeOD) δ (ppm): 1.41 (s, 9H3 (Boc)), 2.80-3.94 (m, 4H), 3.38-3.43 (m, 3H), 3.54-3.60 (m, 1H), 3.65-3.79 (m, 2H), 4.11-4.21 (m, 1H), 4.29-4.38 (m, 3H), 5.28-5.32 (m, 1H), 7.13-7.17 (2xt, J = 2.0 Hz, 1H), 7.40-7.43 (m, 2H), 7.50 and 7.58 (2xbrs, 2H), 7.71-7.79 (m, 3H). ¹³C NMR (MeOD) δ (ppm): 28.5 (3C), 35.7, 39.1, 42.3, 61.4 (2C), 61.5, 67.4 (2C), 68.7, 82.0, 124.5, 126.8, 127.2, 128.0, 128.6 (2C), 128.8, 129.3, 133.9, 134.9, 135.4 (Cdia), 135.5 (Cdia), 145.8, 168.5, 172.3.

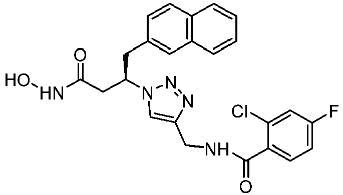

**2-chloro-4-fluoro-N-[[1-[(1R)-3-(hydroxyamino)-1-(2-naphthylmethyl)-3-oxopropyl]triazol-4-yl]methyl]benzamide (72).** Compound **72** was obtained according to general procedure C from azide **107** and 2-chloro-4-fluoro-N-(prop-2-yn-1-yl)benzamide followed by general procedure G as a white foam (28 mg, 22% over 2 steps). Purity: 100%, LC t_{R} = 2.43 min, MS (ESI+): m/z = 482 [M + H]⁺. ¹H NMR (MeOD) δ (ppm): 2.87 (dd, J = 5.7 Hz, J = 14.9 Hz, 1H), 2.96 (dd, J = 8.8 Hz, J = 14.9 Hz, 1H), 3.41-3.44 (m, 2H), 4.48 (d, J = 1.9 Hz, 1H), 5.28-5.37 (m, 1H), 7.08 (td, J = 2.5 Hz, J = 8.4 Hz, 1H), 7.14 (dd, J = 1.7 Hz, J = 8.4 Hz, 1H), 7.24 (dd, J = 2.5 Hz, J = 8.8 Hz, 1H), 7.30 (dd, J = 6.0 Hz, J = 8.6 Hz, 1H), 7.38-7.41 (m, 2H), 7.48 (s, 1H), 7.64 (s, 1H), 7.68-7.77 (m, 3H). ¹³C NMR (MeOD) δ (ppm): 35.9, 39.0, 42.3, 61.5, 115.3 (d, J = 21.8 Hz), 118.3 (d, J = 25.3 Hz), 124.9, 126.8, 127.1, 128.0, 128.6 (2C), 128.8, 129.9, 131.7, 131.8, 133.5 (dd, J = 3.4 Hz, J = 7.6 Hz), 133.9, 134.9, 135.4, 145.2, 164.4 (d, J = 251 Hz), 168.5, 168.9. ¹⁹F NMR (MeOD) δ (ppm): -111.3. HRMS m/z calculated for C₂₄H₂₂FClN₅O₃ [M + H]⁺ 482.1395, found 482.1436.

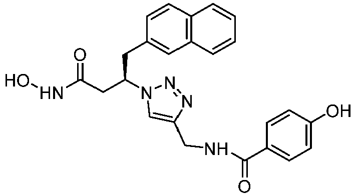

**4-hydroxy-N-[[1-[(1R)-3-(hydroxyamino)-1-(2-naphthylmethyl)-3-oxo-propyl]triazol-4-yl]methyl]benzamide4-hydroxy-N-[[1-[(1R)-3-(hydroxyamino)-1-(2-naphthylmethyl)-3-oxo-propyl]triazol-4-yl]methyl]benzamide (73).** Compound **73** was obtained according to general procedure C from azide **107** and 4-hydroxy-N-(prop-2-yn-1-yl)benzamide followed by general procedure G as a white foam (16 mg, 9% over 2 steps). Purity: 100%, LC t_{R} = 2.10 min, MS (ESI+): m/z = 446 [M + H]⁺. ¹H NMR (MeOD) δ (ppm): 2.84 (dd, J = 5.7 Hz, J = 14.9 Hz, 1H), 2.94 (dd, J = 8.9 Hz, J = 14.9 Hz, 1H), 3.43-3.37 (m, 2H), 4.74 (s, 2H), 5.33-5.24 (m, 1H), 6.80 (d, J = 8.8 Hz, 2H), 7.11 (dd, J = 1.7 Hz, J = 8.4 Hz , 1H), 7.40-7.31 (m, 2H), 7.42 (s, 1H), 7.70-7.60 (m, 6H). ¹³C NMR (MeOD) δ (ppm): 35.9, 38.9, 42.4, 61.5, 116.1 (2C), 124.7, 126.0, 126.7, 127.1, 128.0, 128.5 (2C), 128.8, 129.2, 130.4 (2C), 133.8, 134.8, 135.3, 146.1, 162.2, 168.5, 169.7. HRMS m/z calculated for C₂₄H₂₃N₅O₄ [M + H]⁺ 446.1828, found 446.1832.

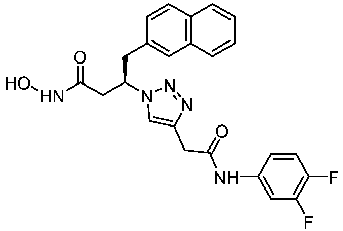

**3-{4-[(3,4-Difluoro-phenylcarbamoyl)-methyl]-[1,2,3]triazol-1-yl}-N-hydroxy-4-naphthalen-2-yl-butyramide (74).** Compound **74** was obtained according to general procedure C from azide **107** and N-(3,4-difluorophenyl)but-3-ynamide followed by general procedure G as a white foam (22 mg, 14% over 2 steps). Purity: 100%, LC t_{R} = 2.57 min, MS (ESI+): m/z = 466 [M + H]⁺.¹H NMR (MeOD) δ (ppm): 2.86 (dd, 1H, J = 5.4 Hz, J = 14.8 Hz, 1H), 2.97 (dd, 1H, J = 8.9 Hz; J = 14.8 Hz, 1H), 3.39-3.45 (m, 2H), 3.67 (s, 2H), 5.25-5.35 (m, 1H), 7.15-7.19 (m, 3H), 7.32-7.40 (m, 2H), 7.43 (s, 1H), 7.60-7.73 (m, 5H). ¹³C NMR (MeOD) δ (ppm): 34.5, 38.9, 42.5, 61.5, 110.3 (d, J = 21.8 Hz), 116.9 (dd, J = 3.6 Hz, J = 5.6 Hz), 118.1 (dd, J = 1.0 Hz, J = 18.3 Hz), 125.2, 126.7, 127.1, 128.0, 128.5, 128.6, 128.9, 129.2, 133.9, 134.8, 135.4, 136.8 (d, J = 2.9 Hz), 141.8, 147.9 (dd, J = 12.7 Hz, J = 243.4 Hz), 151.1 (dd, J = 12.9 Hz, J = 244.5 Hz), 168.6, 169.9. ¹⁹F NMR (MeOD) δ (ppm): -139.6 (d, J = 21.2 Hz), -146.5 (d, J = 21.2 Hz). HRMS m/z calculated for C₂₄H₂₁F₂N₅O₃ [M + H]⁺ 466.1691, found 466.1686.

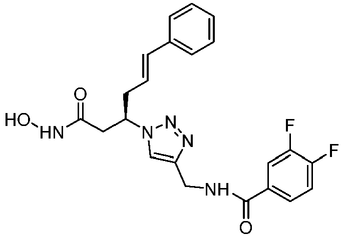

**3,4-difluoro-N-[[1-[(E,1R)-1-[2-(hydroxyamino)-2-oxo-ethyl]-4-phenyl-but-3-enyl]triazol-4-yl]methyl]benzamide (75).** Compound **75** was obtained according to general procedure C from azide **139** and 3,4-difluoro-N-(prop-2-yn-1-yl)benzamide followed by general procedure G as a white foam (117 mg, 60% over 2 steps) Purity: 100%, LC t_{R} = 2.43 min, MS (ESI+): m/z = 442 [M + H]⁺. ¹H NMR (MeOD) δ (ppm): 2.92-2.76 (m, 4H), 4.57 (s, 2H), 5.17-5.08 (m, 1H), 6.04 (td, J = 7.2 Hz, J = 115.9 Hz, 1H), 6.30 (d, J = 15.9 Hz, 1H), 7.22-7.10 (m, 5H), 7.32 (td, J = 8.2 Hz, J = 10.1 Hz, 1H), 7.63 (ddd, J = 1.5 Hz, J = 4.1 Hz, J = 8.5 Hz, 1H), 7.70 (ddd, J = 2.2 Hz, J = 7.7 Hz, J = 11.2 Hz, 1H), 7.90 (s, 1H). ¹³C NMR (MeOD) δ (ppm): 36.2, 38.7, 39.7, 59.9, 117.9 (d, J = 18.8 Hz), 118.5 (d, J = 18.1 Hz), 124.5, 125.0, 125.5 (dd, J = 3.7, J = 7.2 Hz), 127.1 (2C), 128.4, 129.4 (2C), 132.6, 135.2, 138.2, 145.7, 151.4 (dd, J = 13.0 Hz, J = 248 Hz), 153.8 (dd, J = 13.0 Hz, J = 252.0 Hz), 167.5, 168.6. ¹⁹F NMR (MeOD) δ (ppm): -136.3 (d, J = 20.5 Hz), - 139.9 (d, J = 20.5 Hz). HRMS m/z calculated for C₂₂H₂₂F₂N₅O₃ [M + H]⁺ 442.1691, found 442.1707.

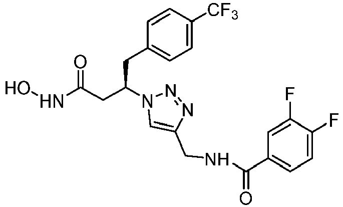

**3,4-difluoro-N-[[1-[(1R)-3-(hydroxyamino)-3-oxo-1-[[4-(trifluoromethyl)phenyl]methyl]propyl]triazol-4-yl]methyl]benzamide** (**76**). Compound **76** was obtained according to general procedure C from azide **150** and 3,4-difluoro-N-(prop-2-yn-1-yl)benzamide followed by general procedure G as a white foam (37 mg, 21% over 4 steps). Purity: 100%, LC t_{R} = 2.52 min, MS (ESI+): m/z = 484 [M + H]⁺. ¹H NMR (MeOD) δ (ppm): 2.97-2.80 (m, 2H), 3.35 (d, J = 7.6 Hz, 2H), 4.53 (s, 2H), 5.29-5.19 (m, 1H), 7.18 (d, J = 8.0 Hz, 2H), 7.36 (dt, J = 8.2 Hz, J = 10.1 Hz, 1H), 7.45 (d, J = 8.0 Hz, 1H), 7.69-7.65 (m, 2H), 7.76 (ddd, J = 2.0 Hz, J = 7.6 Hz, J = 10.3 Hz, 1H). ¹³C NMR (MeOD) δ (ppm): 36.1, 38.9, 41.8, 61.1, 117.9 (d, J = 18.6 Hz, 2C), 118.5 (d, J = 18.0 Hz), 124.8, 125.5 (dd, J = 3.8, J = 7.3 Hz), 126.3 (dd, J = 3.5, J = 7.2 Hz), 125.6 (d, J = 271 Hz), 130, 130.4, 130.8 (2C), 132.7, 142.6, 145.7, 151.4 (dd, J = 13.2 Hz, J = 250.0 Hz), 153.8 (dd, J = 12.8 Hz, J = 250.0 Hz), 167.5, 168.3. ¹⁹F NMR (MeOD) δ (ppm): -64.6 (s, 3F), -136.2 (d, J = 20.4 Hz), -140.0 (d, J = 20.4 Hz). HRMS m/z calculated for C₂₁H₁₉FN₇O₆ [M + H]⁺ 484.1408, found 484.1436.

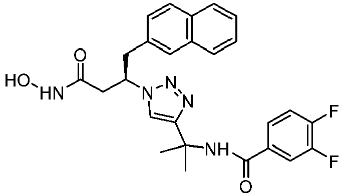

**3,4-Difluoro-N-[1-[1-(1-hydroxycarbamoylmethyl-2-naphthalen-2-yl-ethyl)-1H-[1,2,3]triazol-4-yl]-1-methyl-ethyl]-benzamide** (**77**). Compound **77** was obtained according to general procedure C from azide **107** and 3,4-difluoro-N-(2-methylbut-3-yn-2-yl)benzamide followed by general procedure G as a white foam (46 mg, 37% over 2 steps). Purity: 95%, LC t_{R} = 2.62 min, MS (ESI+): m/z = 494 [M + H]⁺. ¹H NMR (MeOD) δ (ppm): 1.60 (s, 3H), 1.64 (s, 3H), 2.86 (dd, J = 5.9 Hz, J = 14.8 Hz, 1H), 2.94 (dd, J = 8.6 Hz, J = 14.8 Hz, 1H), 4.60 (br s, 1H, OH), 5.20-5.29 (m, 1H), 7.26-7.34 (m, 1H), 7.35-7.40 (m,3H), 7.54-7.59 (m, 1H), 7.57 (s, 1H), 7.63-7.76 (m, 4H). ¹³C NMR (MeOD) δ (ppm): 28.2, 28.5, 38.8, 42.5, 52.5, 61.5, 118.0 (d, J = 19.5 Hz), 118.3 (d, J = 18.5 Hz), 123.2, 125.7 (dd, J = 5.6 Hz, J = 7.2 Hz), 126.7, 127.0, 128.2, 128.5, 128.6, 128.9, 129.2, 133.9, 134.8, 135.4, 151.2 (dd, J = 12.9 Hz, J = 248.5 Hz), 153.8, 153.5 (dd, J = 12.9 Hz, J = 250.0 Hz), 167.3, 168.6. ¹⁹F NMR (MeOD) δ (ppm): -136.8 (d, J = 20.4 Hz), -140.2 (d, J = 20.4 Hz). HRMS m/z calculated for C₂₆H₂₆F₂N₅O₃ [M + H]+ 494.2004, found 494.2005.

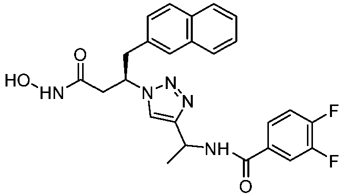

**3,4-difluoro-N-[1-[1-[3-(hydroxyamino)-1-(2-naphthylmethyl)-3-oxo-propyl]triazol-4-yl]ethyl]benzamide** (**78**). Compound **78** was obtained according to general procedure C from azide **107** and N-(but-3-yn-2-yl)-3,4-difluorobenzamide followed by general procedure G as a white foam (24 mg, 20% over 2 steps). Purity: 100%, LC t_{R} = 2.53 min, MS (ESI+): m/z = 480 [M + H]⁺. ¹H NMR (MeOD) δ (ppm): 1.44 and 1.48 (2d, J = 7.0 Hz, J =6.9 Hz, 3H), 2.87-3.03 (m, 2H), 3.37-3.42 (m, 2H), 5.22-5.28 (m, 2H), 5.38-5.28 (m, 1H), 7.13-7.15 (m, 1H), 7.31-7.41 (m, 3H), 7.53 -7.71 (m, 5H). ¹³C NMR (MeOD) δ (ppm): 20.3 (Cdia), 20.5 (Cdia), 38.8, 42.5, 43.4, 61.5, 117.9 (d, J = 18.8 Hz, Cdia), 118.4 (d, J = 17.8 Hz, Cdia), 123.8 (d, J = 24.8 Hz), 125.6 (br s), 126.7, 127.1, 128.0, 128.5 (3C), 128.8, 129.2, 132.8, 133.8, 134.8, 135.4, 150.4 (d, J = 12.2 Hz), 151.2 (dd, J = 13.0 Hz; J = 248.2 Hz), 153.7 (dd, J = 13.0 Hz; J = 252.0 Hz), 166.8, 168.6. ¹⁹F NMR (MeOD) δ(ppm): -136.4 (d, J = 20.2 Hz), -140.0 (d, J = 20.2 Hz, Fdia), -140.1 (d, J = 20.2 Hz, Fdia). HRMS m/z calculated for C₂₅H₂₄F₂N₅O₃ [M + H]⁺ 480.1837, found 480.1847.

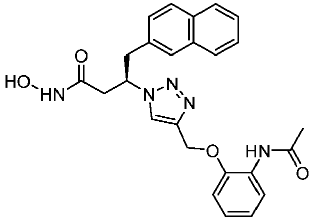

**3-[4-(2-Acetylamino-phenoxymethyl)-[1,2,3]triazol-1-yl]-N-hydroxy-4-naphthalen-2-yl-butyramide** (**79**). Compound **79** was obtained according to general procedure C from azide **107** and N-(2-(prop-2-yn-1-yloxy)phenyl)acetamide followed by general procedure G as a white foam (39 mg, 8% over 2 steps). Purity: 100%, LC t_{R} = 2.37 min, MS (ESI+): m/z = 460 [M + H]⁺. ¹H NMR (MeOD-d₄, 300 MHz) δ (ppm): 2.07 (s, 3H), 2.88 (dd, J = 5.6 and 15.0 Hz, 1H), 2.97 (dd, J = 8.9 and 15.0 Hz, 2H), 3.39-3.46 (m, 2H), 5.10 (s, 2H), 5.31 (dddd, J = 5.6, 5.7, 8.9 and 9.0 Hz, 1H), 6.88-6.95 (m, 1H), 6.98-7.01 (m, 2H), 7.06 (dd, J = 1.6 and 8.4 Hz, 1H), 7.38-7.41 (m, 2H), 7.43 (s br, 1H), 7.57-7.65 (m, 2H), 7.73-7.76 (m, 2H), 7.90 (d, J = 7.9 Hz). ¹³C NMR (MeOD) δ (ppm): 23.8, 38.9, 42.4, 61.7, 62.8, 113.8, 122.3, 123.9, 125.9, 126.1, 126.8, 127.2, 127.9, 128.6 (2C), 128.8, 129.3, 133.9, 134.9, 135.3 (2C), 144.0, 150.0, 168.5, 171.8. HRMS m/z calculated for C₂₅H₂₆N₅O₄ [M + H]⁺ 460.1985, found 460.1982.

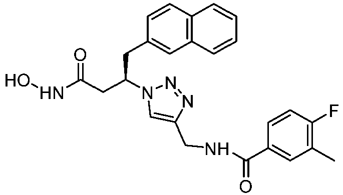

**4-fluoro-N-[[1-[(1R)-3-(hydroxyamino)-1-(2-naphthylmethyl)-3-oxo-propyl]triazol-4-yl]methyl]-3-methyl-benzamide** (**80**). Compound **80** was obtained according to general procedure C from azide **107** and 4-fluoro-3-methyl-N-(prop-2-yn-1-yl)benzamide followed by general procedure G as a white powder (20 mg, 16% over 2 steps). Purity: 100%, LC t_{R} = 2.48 min, MS (ESI+): m/z = 462 [M + H]⁺. ¹H NMR (MeOD): δ (ppm): 2.29 (d, J = 1.9 Hz, 3H), 2.90 (ddd, J = 5.6, J = 15.0, 2H), 3.37-3.41 (m, 2H), 4.47 (s, 2H), 5.26-5.32 (m, 1H), 7.05-7.14 (m, 2H), 7.29-7.38 (m, 2H), 7.42 (s, 1H), 7.56-7.69 (m, 6H). ¹³C NMR (MeOD): δ (ppm): 14.5 (d, J = 3.4 Hz), 35.9, 38.9, 42.4, 61.5, 116.0 (d, J = 22.5 Hz), 124.8, 126.1, 126.3, 126.7, 127.1, 128.0, 128.3 (d, J = 8.0 Hz), 128.5, 128.8, 129.2, 131.3, 132.2 (d, J = 8.0 Hz), 133.8, 134.8, 135.4, 145.8, 165.0 (d, J = 245.9 Hz), 168.5, 168.9. ¹⁹F NMR (MeOD): δ (ppm): -115.3. HRMS m/z calculated for C₂₅H₂₅F₂N₅O₃ [M + H]⁺ 462.1941, found 462.1980.

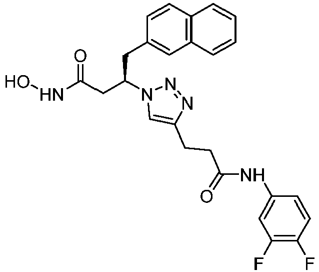

**N-(3,4-difluorophenyl)-3-[1-[3-(hydroxyamino)-1-(2-naphthylmethyl)-3-oxo-propyl]triazol-4-yl]propanamide** (**81**). Compound **81** was obtained according to general procedure C from azide **107** and N-(3,4-difluorophenyl)pent-4-ynamide followed by general procedure G as a white foam (28 mg, 10% over 2 steps). Purity: 100%, LC t_{R} = 2.57 min, MS (ESI+): m/z = 480 [M + H]⁺. ¹H NMR (MeOD) δ (ppm): 2.54-2.61 (m, 2H), 2.81-2.97 (m, 4H), 3.37-3.40 (m, 2H), 5.20-5.30 (m, 1H), 7.09 (dd, J = 1.6 Hz, J = 8.4 Hz, 1H), 7.11-7.18 (m, 2H), 7.40-7.31 (m, 2H), 7.36-7.39 (m, 2H), 7.43 (s, 1H), 7.47 (s, 1H), 7.59-7.71 (m, 4H). ¹³C NMR (MeOD) δ (ppm): 1.9, 36.9, 39.0, 42.4, 61.4, 110.3 (d, J = 22.0 Hz), 116.9 (dd, J = 3.5 Hz, J = 5.4 Hz), 118.1 d, J = 18.4 Hz), 124.0, 126.7, 127.1, 127.9, 128.5 (2C), 128.8, 129.2, 133.8, 134.8, 135.4, 136.8 (dd, J = 2.7 Hz, J = 9.0 Hz), 147.8 (dd, J = 12.6 Hz, J = 246.7 Hz), 151.0 (dd, J = 11.7 Hz, J = 246.7 Hz), 168.6, 172.6. ¹⁹F NMR (MeOD) δ (ppm): -139.7 (d, J = 21.5 Hz), -146.8 (d, J = 21.5 Hz). HRMS m/z calculated for C₂₅H₂₄F₂N₅O₃ [M + H]⁺ 460.1847, found 460.1877.

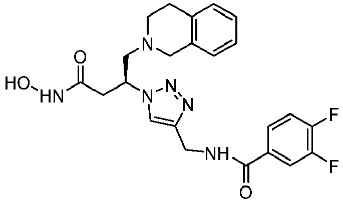

**N-[[1-[(1S)-1-(3,4-dihydro-1H-isoquinolin-2-ylmethyl)-3-(hydroxyamino)-3-oxo-propyl]triazol-4-yl]methyl]-3,4-difluoro-benzamide** (**82**) Compound **82** was obtained according to general procedure C from azide **122** and 3,4-difluoro-N-(prop-2-yn-1-yl)benzamide followed by general procedure G as a white foam (14 mg, 16% over 2 steps). Purity: 100%, LC t_{R} = 2.02 min, MS (ESI+): m/z = 471 [M + H]⁺. ¹H NMR (MeOD) δ (ppm): 2.58-2.63 (m, 1H), 2.72-2.85 (m, 5H), 2.94 (dd, J = 5.5 Hz, J = 13.2 Hz, 1H), 2.94 (dd, J = 5.4 Hz, J = 13.3 Hz, 1H), 3.06 (dd, J = 8.7 Hz, J = 13.3 Hz, 1H), 3.53 (d, J = 14.7 Hz, 1H), 3.68 (d, J = 14.7 Hz, 1H), 4.59 (s, 2H), 5.21-5.31 (m, 1H), 6.92-7.08 (m, 4H), 7.31 (td, J = 8.1 Hz, J = 10.3 Hz, J = 16.5 Hz, 1H), 7.65 (ddd, J = 1.5 Hz, J = 4.1 Hz, J = 8.5 Hz, 1H), 7.70 (ddd, J = 2.2 Hz, J = 7.7 Hz, J = 11.2 Hz, 1H), 7.94 (s, 1H). ¹³C NMR (MeOD) δ (ppm): 30.0, 36.2, 37.2, 52.1, 57.0, 57.9, 62.5, 117.9 (d, J = 18.8 Hz), 118.5 (d, J = 18.2 Hz), 125.0, 125.5 (dd, J = 3.5 Hz, J = 7.0 Hz), 126.7, 127.2, 127.4, 129.5, 132.6, 135.2, 135.5, 145.6, 151.3 (dd, J = 13.7 Hz, J = 248.0 Hz), 153.3 (dd, J = 11.9 Hz, J = 251.7 Hz), 167.5, 168.7. ¹⁹F NMR (MeOD) δ (ppm): -139.9 (d, J = 20.4 Hz), -136.2 (d, J = 20.4 Hz). HRMS m/z calculated for C₂₃H₂₅F₂N₆O₃ [M+H]⁺ 471.1956 found 471.1950.

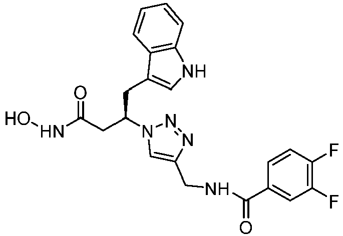

**3,4-difluoro-N-[[1-[(1R)-3-(hydroxyamino)-1-(1H-indol-3-ylmethyl)-3-oxopropyl]triazol-4-yl]methyl]benzamide** (**83**). Compound **83** was obtained according to general procedure C from azide **131** and 3,4-difluoro-N-(prop-2-yn-1-yl)benzamide followed by general procedure G as a white foam (61 mg, 54% over 2 steps). Purity: 100%, LC t_{R} = 2.22 min, MS (ESI+): m/z = 455 [M + H]⁺. ¹H NMR (DMSO-d₆) δ (ppm): 2.63 (dd, J = 5.7 Hz, J = 15.0 Hz, 1H), 2.74 (dd, J = 8.5 Hz, J = 15.0 Hz, 1H), 3.24 (d, J = 7.1 Hz, 2H), 4.45 (d, J = 5.7 Hz, 2H), 5.14-5.24 (m, 1H), 7.90 (d, J = 2.3 Hz, 1H), 7.94 (td, J = 1.0 Hz, J = 6.9 Hz, 1H), 7.02 (td, J = 1.1 Hz, J = 7.0 Hz, 1H), 7.29 (d, J = 8.0 Hz, 1H), 7.57 (d, J = 6.7 Hz, 1H), 7.73 (d, J = 7.8 Hz, 1H), 7.56 (ddd,J = 8.3 Hz, J = 8.5 Hz, J = 10.5 Hz, 1H), 7.74-7.79 (m, 1H), 7.91 (ddd, J = 2.0 Hz, J = 7.8 Hz, J = 11.6 Hz, 1H), 7.92 (s, 1H), 8.79 (br s, NHOH), 9.09 (t, J = 5.4 Hz, 1H, NH), 10.8 (br s, NH). ¹³C NMR (MeOD) δ (ppm): 32.4, 36.1, 38.7, 61.1, 110.6, 112.3, 118.0 (d, J = 18.9 Hz), 118.5 (d, J = 18.1 Hz), 118.9, 119.9, 122.5, 124.6, 124.7, 125.6 (dd, J = 3.5 Hz, J = 7.1 Hz), 128.4, 132.7, 137.9, 145.3, 151.3 (dd, J = 12.5 Hz, d, J = 247.4 Hz), 153.7 (dd, J = 13.1 Hz, d, J = 252.5 Hz), 167.5, 168.9. ¹⁹F NMR (MeOD) δ (ppm): -140.0 (d, J = 20.4), -136.3 (d, J = 20.4). HRMS m/z calculated for C₂₂H₂₁F₂N₆O₃ [M + H]⁺ 455.1643, found 455.1653.

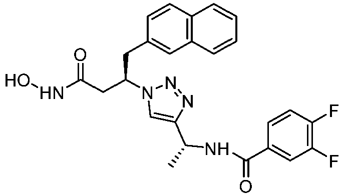

**3,4-Difluoro-N-{1-[1-(1(R)-hydroxycarbamoylmethyl-2-naphthalen-2-yl-ethyl)-1H-[1,2,3]triazol-4-yl]-(S)-ethyl}-benzamide** (**85**). Compound **85** was obtained according to general procedure C from azide **107** and (R)-N-(but-3-yn-2-yl)-3,4-difluorobenzamide followed by general procedure G as a white powder (32 mg, 30% over 2 steps). Diastereoisomeric yield (**dr**): 92%. Purity: 100%, LC t_{R} = 2.48 min, MS (ESI+): m/z = 4= 480 [M + H]. ¹H NMR (MeOD) δ (ppm): 1.48 (d, J = 7.0 Hz, 3H), 2.87 (dd, J = 5.8 Hz, J = 15.0 Hz, 2H), 2.97 (dd, J = 9.2 Hz, J = 15.0 Hz, 2H), 3.33-3.45 (m, 2H), 5.21-5.33 (m, 2H), 7.13 (dd, J = 1.6 Hz, J = 8.4 Hz, 1H), 7.27-7.39 (m, 3H), 7.40 (br s, 1H), 7.56(s, 1H), 7.57-7.70 (m, 5H). ¹³C NMR (MeOD) δ (ppm): 20.5, 38.8, 42.5, 43.4, 61.5, 117.9 (d, J = 19.1 Hz), 118.4 (d, J = 18.0 Hz), 123.6, 125.6 (dd, J = 3.5, J = 7.2 Hz), 126.7, 127.1, 128.0, 128.5 (2C), 128.8, 129.4, 132.8 (t, J = 4.3), 133.8, 134.8, 135.4, 150.2, 151.2 (dd, J = 13.0 Hz, J = 247.6 Hz), 153.3 (dd, J = 12.7 Hz, J = 252.4 Hz), 166.8, 168.6. ¹⁹F NMR (MeOD) δ (ppm): -140.1 (d, J = 20.2 Hz), -136.5 (d, J = 20.2 Hz). HRMS m/z calculated for C₂₅H₂₄F₂N₅O₃ [M + H]+ 480.1815, found 480.1847.

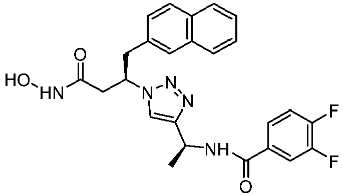

**3,4-Difluoro-N-{1-[1-(1(R)-hydroxycarbamoylmethyl-2-naphthalen-2-yl-ethyl)-1H-[1,2,3]triazol-4-yl]-(R)-ethyl}-benzamide** (**86**). Compound **86** was obtained according to general procedure C from azide **107** and (S)-N-(but-3-yn-2-yl)-3,4-difluorobenzamide followed by general procedure G as a white powder (27 mg, 21% over 2 steps), diastereoisomeric yield (**dr**): 83%, Purity: 100%, LC t_{R} = 2.55 min, MS (ESI+): m/z = 4= 480 [M + H]. ¹H NMR (MeOD) δ (ppm): 1.44 (d, J = 7.0 Hz, 3H), 2.87 (dd, J = 5.6 Hz, J = 15.0 Hz, 2H), 2.97 (dd, J = 9.0 Hz, J = 15.0 Hz, 2H), 3.35-3.43 (m, 2H), 5.21-5.31 (m, 2H), 7.13 (dd, J = 1.6 Hz, J = 8.4 Hz, 1H), 7.26-7.34 (m, 1H), 7.36-7.40 (m, 3H), 7.53 (s, 1H), 7.63-7.72 (m, 5H). ¹³C NMR (MeOD) δ (ppm): 20.5, 38.8, 42.5, 43.4, 61.5, 117.9 (d, J = 19.1 Hz), 118.4 (d, J = 18.0 Hz), 123.6, 125.6 (dd, J = 3.5, J = 7.2 Hz), 126.7, 127.1, 128.0, 128.5 (2C), 128.8, 129.4, 132.8 (t, J = 4.3), 133.8, 134.8, 135.4, 150.2, 151.2 (dd, J = 13.0 Hz, J = 247.6 Hz), 153.3 (dd, J = 12.7 Hz, J = 252.4 Hz), 166.8, 168.6. ¹⁹F NMR (MeOD) δ (ppm): -140.1 (d, J = 20.2), -136.5 (d, J = 20.2). HRMS m/z calculated for C₂₅H₂₄F₂N₅O₃ [M + H]⁺ 480.1876, found 480.1847.

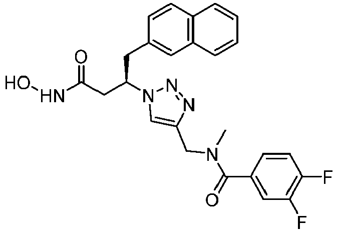

**Methyl (3R)-3-[4-[[(3,4-difluorobenzoyl)-methyl-amino]methyl]triazol-1-yl]-4-(2-naphthyl)butanoate** (**87**). Compound **87** was obtained according to general procedure C from azide **107** and 3,4-difluoro-N-methyl-N-(prop-2-yn-1-yl)benzamide followed by general procedure G as a white powder (65 mg, 46% over 2 steps). Purity: 100%, LC t_{R}: 2.46 min, MS (ESI+): m/z = 480 [M+H]⁺. ¹H NMR (DMSO-d₆) δ (ppm): 10.41 (br s, 1H), 8.81 (br s, 1H), 7.81 (m, 3H), 7.47 (m, 3H), 7.27 (s, 1H), 7.15 (m, 4H), 5.28 (m, 1H), 4.29 (s, 2H), 3.37 (dd, J = 5.1 and 13.8 Hz, 1H), 3.26 (m, 1H), 2.71 (m, 2H), 2.59 (s, 3H). HRMS m/z calculated for C₂₅H₂₄F₂N₅O₃ [M+H]⁺ 480.1847 found 480.1837.

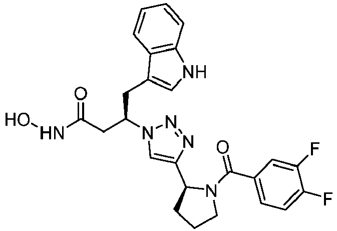

**(3R)-3-[4-[(2S)-1-(3,4-difluorobenzoyl)pyrrolidin-2-yl]triazol-1-yl]-4-(1H-indol-3-yl)butanehydroxamic acid** (**88**). Compound **88** was obtained according to general procedure C from azide **131** and (S)-(3,4-difluorophenyl)(2-ethynylpyrrolidin-1-yl)methanone followed by general procedure G as a white solid (S/R ratio 4.4/1.5 mg, 59% over 2 steps). Purity: 99%, LC t_{R}= 2.20 min, MS (ESI+): m/z = 495 [M + H]⁺. ¹H NMR (300 MHz, MeOD-d₄) δ (ppm): 7.46-7.21 (m, 5H), 7.16-6.82 (m, 4H), 6.61-6.57 (m, 0.3H), 5.27-5.10 (m, 2H), 3.70-3.54 (m, 1H), 3.49-3.22 (m, 3H), 3.02-2.83 (m, 2H), 2.29-2.10 (m, 1H), 2.08-1.94 (m, 1H), 1.91-1.57 (m, 3H). ¹³C NMR (75 MHz, MeOD-d₄) δ (ppm): 170.3, 169.5, 168.9, 168.7, 152.7 (dd, J = 250.8, 12.4 Hz), 151.2 (dd, J = 248.3, 12.7 Hz), 150.7, 149.7, 148.9, 145.4, 137.8, 134.9 (dd, J = 8.4, 4.2 Hz), 128.5, 125.4 (dd, J = 6.4, 4.3 Hz), 124.7, 124.6, 124.3, 124.1, 124.0, 122.6, 122.5, 120.0, 118.9, 118.6 (d, J = 18.0 Hz), 117.9 (d, J = 19.3 Hz), 117.0 (d, J = 19.3 Hz), 112.4, 112.3, 110.8, 110.7, 110.5, 61.4, 61.3, 61.1, 57.4, 54.9, 51.3, 51.0, 47.4, 38.8, 38.6, 34.7, 33.2, 33.2, 32.8, 32.5, 32.2, 30.8, 30.5, 25.8, 25.7, 23.7, 22.8. HRMS m/z calculated for C₂₅H₂₄F₂N₆O₃ [M+H]⁺ 495.1956 found 495.1957.

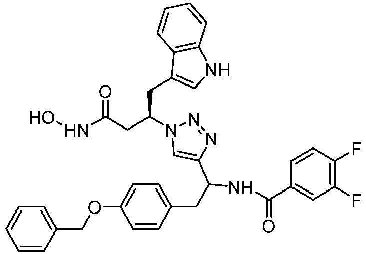

**N-[2-(4-benzyloxyphenyl)-1-[1-[(1R)-3-(hydroxyamino)-1-(1H-indol-3-ylmethyl)-3-oxo-propyl]triazol-4-yl]ethyl]-3,4-difluoro-benzamide** (**89**). Compound **89** was obtained according to general procedure C from azide **131** and N-(1-(4-(benzyloxy)phenyl)but-3-yn-2-yl)-3,4-difluorobenzamide followed by general procedure G as a white solid (71 mg, 63% over 2 steps). Purity: 99%, LC t_{R}= 2.80 min, MS (ESI+): m/z = 651 [M + H]⁺. ¹H NMR (300 MHz, MeOD-d₄) δ (ppm): 7.65-7.56 (m, 1H), 7.55-7.48 (m, 1H), 7.43-7.24 (m, 9H), 7.07-6.92 (m, 4H), 6.81-6.74 (m, 3H), 5.41-5.31 (m, 1H), 5.27-5.15 (m, 1H), 4.96 (s, 1H), 4.95 (s, 1H), 3.42-3.28 (m, 2H), 3.11-2.82 (m, 4H). ¹³C NMR (75 MHz, MeOD-d₄) δ (ppm): 168.8, 167.1, 158.9, 153.6 (dd, J = 252.4, 12.5 Hz), 151.2 (dd, J = 248.2, 13.9 Hz), 148.5, 148.3, 138.7, 137.9, 132.9 (dd, J = 4.8, 3.6 Hz), 131.4, 131.1, 129.4, 128.8, 128.55, 128.49, 128.44, 125.6-125.5 (m), 124.7, 124.6, 124.19, 124.17, 122.5, 120.0, 119.0, 118.4 (d, J = 17.8 Hz), 117.9 (d, J = 18.6 Hz), 115.8, 112.3, 110.6, 70.9, 61.1, 49.6, 49.5, 41.0, 40.8, 38.9, 38.8, 32.5, 32.4. HRMS m/z calculated for C₃₆H₃₂F₂N₆O₄ [M+H]⁺ 651.2531 found 651.2531.

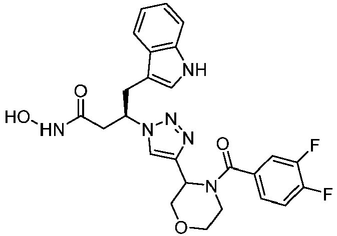

**(3R)-3-[4-[4-(3,4-difluorobenzoyl)morpholin-3-yl]triazol-1-yl]-4-(1H-indol-3-yl)butanehydroxamic acid** (**90**). Compound **90** was obtained according to general procedure C from azide **131** and (3,4-difluorophenyl)(3-ethynylmorpholino)methanone followed by general procedure G as an off-white solid (55 mg, 62% over 2 steps). Purity: 99%, LC tR= 2.15 min, MS (ESI+): m/z = 511 [M + H]⁺. ¹H NMR (300 MHz, MeOD-d₄) δ (ppm): 7.53-7.26 (m, 5H), 7.14-6.77 (m, 4H), 5.32-5.22 (m, 1H), 4.03 (br s, 2H), 3.77-3.68 (m, 1H), 3.52-3.25 (m, 3H), 3.09-2.90 (m, 2H), 2.79-2.69 (m, 0.5H), 2.51 (br s, 0.5H). ¹³C NMR (75 MHz, MeOD-d₄) δ (ppm): 170.3, 168.9, 152.6 (dd, J = 252.4, 13.0 Hz), 151.4 (dd, J = 248.9, 13.8 Hz), 145.5, 145.0, 144.6, 137.83, 137.80, 133.4 (dd, J = 9.4, 4.8 Hz), 128.5, 126.0, 125.5-125.2 (m), 125.4, 124.7, 122.5, 120.01, 119.97, 118.9, 118.7, 118.1 (d, J = 18.7 Hz), 118.0 (d, J = 18.7 Hz), 112.3, 110.7, 69.5 (br s2), 67.7, 61.3, 49.3, 38.6, 38.5, 32.5. HRMS m/z calculated for C₂₅H₂₄F₂N₆O₄ [M+H]⁺ 511.1905, found 511.1906.

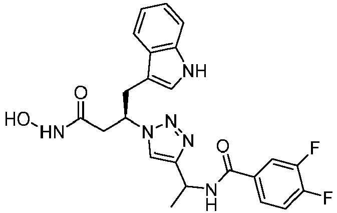

**3,4-difluoro-N-[1-[1-[(1R)-3-(hydroxyamino)-1-(1H-indol-3-ylmethyl)-3-oxopropyl]triazol-4-yl]ethyl]benzamide** (**91**). Compound **91** was obtained according to general procedure C from azide **131** and N-(but-3-yn-2-yl)-3,4-difluorobenzamide followed by general procedure G as an off-white solid (60 mg, 73% over 2 steps). Purity: 99%, LC tR= 2.18 min, MS (ESI+): m/z = 469 [M + H]⁺. ¹H NMR (300 MHz, MeOD-d₄) δ (ppm): 7.72 (ddd, J = 11.3, 7.7, 2.2 Hz, 1H), 7.66-7.60 (m, 1H), 7.49 (d, J = 3.0 Hz, 1H), 7.38-7.31 (m, 2H), 7.29-7.24 (m, 1H), 7.03 (dddd, J = 8.1, 6.9, 3.4, 1.2 Hz, 1H), 6.94 (dddd, J = 8.1, 6.9, 1.4, 1.2 Hz, 1H), 6.83 (d, J = 3.0 Hz, 1H), 5.29-5.16 (m, 2H), 3.43-3.28 (m, 2H), 3.00-2.82 (m, 2H), 1.49 (d, J = 7.1 Hz, 1.5H), 1.45 (d, J = 7.1 Hz, 1.5H). ¹³C NMR (75 MHz, MeOD-d₄) δ (ppm): 168.9, 166.9, 153.7 (dd, J = 252.2, 12.6 Hz), 151.3 (dd, J = 247.7, 12.6 Hz), 150.0, 137.9, 132.9 (dd, J = 5.4, 3.6 Hz), 128.5, 125.7 (dd, J = 7.2, 3.2 Hz), 124.7, 124.6, 123.8, 123.8, 122.5, 119.9, 118.9, 118.4 (d, J = 18.0 Hz), 118.0 (d, J = 18.6 Hz), 112.3, 110.7, 110.6, 61.2, 61.1, 43.5, 38.6, 32.5, 20.51, 20.46. HRMS m/z calculated for C₂₃H₂₂F₂N₆O₃ [M+H]⁺ 469.1800, found 469.1800.

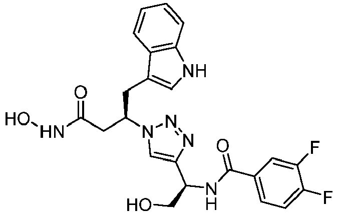

**3,4-difluoro-N-[(1R)-2-hydroxy-1-[1-[(1R)-3-(hydroxyammo)-1-(1H-indol-3-ylmethyl)-3-oxo-propyl]triazol-4-yl]ethyl]benzamide** (**92**). Compound **92** was obtained according to general procedure C from azide **131** and (R)-3,4-difluoro-N-(1-hydroxybut-3-yn-2-yl)benzamide followed by general procedure G as a white solid (31 mg, 35% over 2 steps). Purity: 98%, LC tR= 2.03 min, MS (ESI+): m/z = 485 [M + H]⁺. ¹H NMR (300 MHz, MeOD-d₄) δ (ppm): 7.77 (br dd, J = 10.8, 7.8 Hz, 1H), 7.69-7.63 (br m, 1H), 7.56 (br s, 1H), 7.38-7.22 (m, 3H), 7.03 (br t, J = 7.3 Hz, 1H), 6.94 (br t, J = 7.3 Hz, 1H), 6.83 (br s, 1H), 5.29 (br t, J = 5.9 Hz, 1H), 5.24-5.17 (m, 1H), 3.83-3.69 (m, 2H), 3.44-3.30 (m, 2H), 2.99-2.82 (m, 2H). ¹³C NMR (75 MHz, MeOD-d₄) δ (ppm): 168.8, 167.6, 153.7 (dd, J = 252.1, 12.9 Hz), 151.2 (dd, J = 247.6, 12.9 Hz), 146.5, 137.9, 132.8 (dd, J = 4.2, 3.6 Hz), 128.5, 125.7 (dd, J = 7.0, 3.6 Hz), 124.6, 124.5, 122.5, 119.9, 118.9, 118.4 (d, J = 18.3 Hz), 118.1 (d, J = 19.1 Hz), 112.3, 110.6, 64.5, 61.3, 50.2, 38.6, 32.4. HRMS m/z calculated for C₂₃H₂₂F₂N₆O₄ [M+H]⁺ 485.1749, found 485.1746.

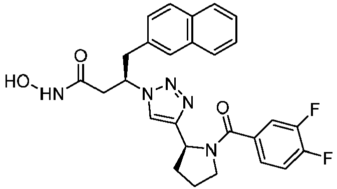

**(3R)-3-[4-[(2S)-1-(3,4-difluorobenzoyl)pyrrolidin-2-yl]triazol-1-yl]-4-(2-naphthyl)butanehydroxamic acid** (**93**). Compound **93** was obtained according to general procedure C from azide **107** and (S)-(3,4-difluorophenyl)(2-ethynylpyrrolidin-1-yl)methanone followed by general procedure G as a white solid (65 mg, 60% over 2 steps). Purity: 97%, LC t_{R}= 2.45 min, MS (ESI+): m/z = 506 [M + H]⁺. ¹H NMR (30 0 MHz, MeOD-d₄) δ (ppm): 7.80-7.58 (m, 3H), 7.52-7.26 (m, 6H), 7.21-7.12 (m, 2H), 6.89-6.80 (m, 0.4H), 6.64 (brs, 0.3H), 5.30-5.20 (m, 2H), 3.68-3.19 (m, 4H), 3.06-2.87 (m, 2H), 2.27-2.08 (m, 1H), 2.05-1.88 (m, 1H), 1.76-1.58 (m, 2H). ¹³C NMR (75 MHz, MeOD-d₄) δ (ppm): 168.8, 168.0, 167.2, 167.0, 151.3 (dd, J = 250.7, 12.3 Hz), 149.8 (dd, J = 248.9, 13.1 Hz), 148.4, 147.8, 147.6, 134.1, 133.6-133.3 (m), 133.4, 132.4, 127.8, 127.45, 127.37, 127.2, 126.7, 126.6, 125.9, 125.7, 125.6, 125.5, 125.4, 125.3, 124.0 (dd, J = 6.3, 3.4 Hz), 123.1, 122.8, 117.2 (dd, J = 18.0 Hz), 117.1 (d, J = 18.0 Hz), 116.5 (d, J = 18.6 Hz), 115.7 (d, J = 18.6 Hz), 60.3, 60.1, 56.0, 53.5, 53.4, 50.0, 49.5, 45.9, 41.3, 41.2, 41.0, 37.6, 37.5, 37.3, 33.4, 31.9, 31.6, 31.2, 29.2, 29.0, 24.2, 21.0. HRMS m/z calculated for C₂₇H₂₅F₂N₅O₃ [M+H]⁺ 506.2004, found 506.1997.

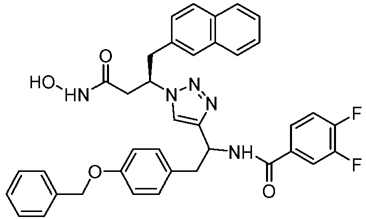

**N-[2-(4-benzyloxyphenyl)-1-[1-[(1R)-3-(hydroxyamino)-1-(2-naphthylmethyl)-3-oxopropyl]triazol-4-yl]ethyl]-3,4-difluoro-benzamide** (**94**). Compound **94** was obtained according to general procedure C from azide **107** and N-(1-(4-(benzyloxy)phenyl)but-3-yn-2-yl)-3,4-difluorobenzamide followed by general procedure G as a white solid (48 mg, 39% over 2 steps). Purity: 97%, LC t_{R}= 2.97 min, MS (ESI+): m/z = 662 [M + H]⁺. ¹H NMR (300 MHz, DMSO-d₆) δ (ppm): 10.56 (br s, 1H), 8.86 (t, J = 8.8 Hz, 1H), 8.85 (br s, 1H), 7.89 (s, 1H), 7.85-7.76 (m, 2H), 7.74-7.64 (m, 3H), 7.58-7.48 (m, 1H), 7.45-7.27 (m, 8H), 7.14-7.08 (m, 3H), 6.86-6.80 (m, 2H), 5.33-5.20 (m, 2H), 4.99 (s, 2H), 3.32-3.23 (m, 2H), 3.12-2.94 (m, 2H), 2.86-2.66 (m, 2H). ¹³C NMR (75 MHz, DMSO-d₆) δ (ppm): 165.5, 163.3, 156.8, 151.3 (dd, J = 250.0, 13.2 Hz), 149.0 (dd, J = 246.6, 13.4 Hz), 147.8, 147.7, 137.2, 134.60, 134.56, 132.9, 131.8, 131.7-131.6 (br s), 130.6, 130.5, 130.1, 128.4 (2C), 127.8, 127.7 (2C), 127.4 (2C), 127.3, 126.0, 125.6, 124.9-124.7 (m), 122.1, 121.9, 117.5 (d, J = 17.5 Hz), 116.7 (d, J = 18.3 Hz), 114.4 (2C), 69.1, 59.0, 58.8, 47.8, 41.1, 41.0, 37.2, 37.1, 29.0. HRMS m/z calculated for C₃₈H₃₃F₂N₅O₄ [M+H]⁺ 662.2579, found 662.2577.

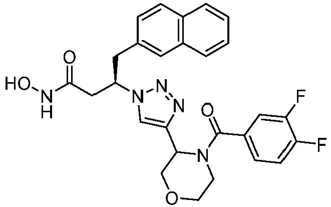

**(3R)-3-[4-[4-(3,4-difluorobenzoyl)morpholin-3-yl]triazol-1-yl]-4-(2-naphthyl)butanehydroxamic acid** (**95**). Compound **95** was obtained according to general procedure C from azide **107** and (3,4-difluorophenyl)(3-ethynylmorpholino)methanone followed by general procedure G as a white solid (43 mg, 55% over 2 steps). Purity: 95%, LC tR= 2.38 min, MS (ESI+): m/z = 522 [M + H]⁺. ¹H NMR (300 MHz, MeOD-d₄) δ (ppm): 7.76-7.72 (m, 2H), 7.69-7.65 (m, 1H), 7.58 (d, J = 11.9 Hz, 1H), 7.41-7.36 (m, 3H), 7.29-7.21 (m, 3H), 7.11 (br s, 1H), 5.37-5.27 (m, 1H), 4.23-3.99 (m, 1H), 3.75-3.23 (m, 5H), 3.13-2.90 (m, 2H), 2.66-2.56 (m, 0.5H), 2.36 (br s, 0.5H). ¹³C NMR (75 MHz, MeOD-d₄) δ (ppm): 170.4 (br s), 168.6, 168.5, 152.6 (dd, J = 250.4, 12.4 Hz), 151.4 (dd, J = 248.8, 13.9 Hz), 145.1, 144.6, 135.6, 135.5, 134.8, 133.9, 133.8, 133.5-133.2 (m), 129.4, 129.3, 128.6 (2C), 128.1, 128.0, 127.21, 127.17, 126.8, 126.4, 125.5, 125.5-125.1 (m), 118.8 (d, J = 18.0 Hz), 118.1 (br d, J = 17.5 Hz), 118.0 (d, J = 18.9 Hz), 69.4, 67.6, 67.5, 61.6, 61.4, 42.8, 42.7, 38.8, 38.7. HRMS m/z calculated for C₂₇H₂₅F₂N₅O₄ [M+H]⁺ 522.1953, found 522.1949.

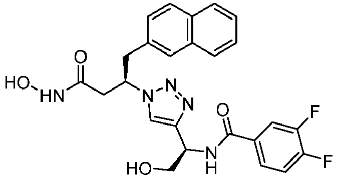

**3,4-difluoro-N-[(1R)-2-hydroxy-1-[1-[(1R)-3-(hydroxyamino)-1-(2-naphthylmethyl)-3-oxo-propyl]triazol-4-yl]ethyl]benzamide** (**96**). Compound **96** was obtained according to general procedure C from azide **107** and (R)-3,4-difluoro-N-(1-hydroxybut-3-yn-2-yl)benzamide followed by general procedure G as a white solid (39 mg, 43% over 2 steps). Purity: 95%, LC t_{R}= 2.25 min, MS (ESI+): m/z = 496 [M + H]⁺. ¹H NMR (300 MHz, DMSO-d₆) δ (ppm): 8.75 (d, J = 8.4 Hz, 1H), 7.98-7.89 (m, 1H), 7.86-7.66 (m, 4H), 7.55 (td, J = 10.4, 8.4 Hz, 1H), 7.46-7.38 (m, 3H), 7.11 (dd, J = 8.4, 1.4 Hz, 1H), 5.28-2.14 (m, 2H), 4.99 (br s, 1H), 3.69 (dd, J = 10.5, 5.5 Hz, 1H), 3.60 (dd, J = 10.5, 8.4 Hz, 1H), 3.30-3.17 (m, 2H), 2.81 (dd, J = 15.0, 8.7 Hz, 1H), 2.69 (dd, J = 15.0, 5.5 Hz, 1H). ¹³C NMR (75 MHz, DMSO-d₆) δ (ppm): 165.5, 163.9, 151.3 (dd, J = 250.2, 12.6 Hz), 149.0 (dd, J = 246.2, 12.9 Hz), 145.8, 134.6, 132.9, 131.8 (2C), 127.8, 127.4 (3C), 127.3, 126.0, 125.6, 125.0 (dd, J = 7.2, 3.0 Hz), 122.4, 117.4 (d, J = 17.5 Hz), 116.9 (d, J = 18.0 Hz), 63.1, 58.9, 49.1, 41.1, 37.0. HRMS m/z calculated for C₂₅H₂₃F₂N₅O₄ [M+H]⁺ 496.1796, found 496.1795.

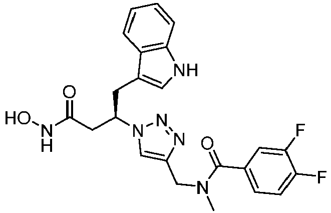

**3,4-difluoro-N-[[1-[(1R)-3-(hydroxyamino)-1-(1H-indol-3-ylmethyl)-3-oxopropyl]triazol-4-yl]methyl]-N-methyl-benzamide** (**97**). Compound **97** was obtained according to general procedure C from azide **131** and 3,4-difluoro-N-methyl-N-(prop-2-yn-1-yl)benzamide followed by general procedure G as a faint pink solid (22 mg, 4% over 2 steps). Purity: 90%, LC t_{R}= 2.23 min, MS (ESI+): m/z = 469 [M + H]⁺. ¹H NMR (300 MHz, CDCl₃) δ (ppm): 10.0, (s, 1H), 7.50 (s, 1H), 7.37 (d, J = 8.00 Hz, 2H), 7.24 (d, J = 8.00 Hz, 2H), 6.96-6.82 (m, 4H), 5.19 (s, 1H) 4.54-4.29 (m, 2H), 3.31-3.28 (m, 2H) 2.70 (s, 3H), 1.36-1.18 (m, 2H). ¹³C NMR (75 MHz, CDCl₃) δ (ppm): 166.4, 151.4, 151.2, 149.1, 148.9, 136.4, 133.9, 133.9, 127.3, 124.4, 123.6, 123.5, 124.3, 118.8, 118.1, 117.4, 116.9, 116.7, 111.3, 109.9, 68.3, 59.2, 37.5, 31.1. HRMS m/z calculated for C₂₃H₂₂F₂N₆O₃ [M+H]⁺ 469.1800, found 469.1800.

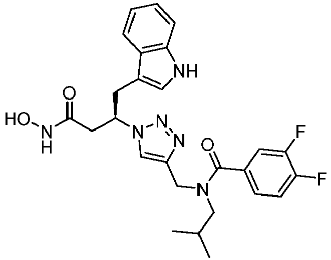

**3,4-difluoro-N-[[1-[(1R)-3-(hydroxyamino)-1-(1H-indol-3-ylmethyl)-3-oxopropyl]triazol-4-yl]methyl]-N-isobutyl-benzamide** (**98**). Compound **98** was obtained according to general procedure C from azide **131** and 3,4-difluoro-N-isobutyl-N-(prop-2-yn-1-yl)benzamide followed by general procedure G as a faint pink solid (55 mg, 18% over 2 steps). Purity: 99%, LC tR= 2.53 min, MS (ESI+): m/z = 511 [M + H]⁺. ¹H NMR (300 MHz, CDCl₃) δ (ppm): 8.73, (s, 1H), 7.33-7.29, (m, 1H), 7.23-7.20 (m 2H), 7.51 (m, 1H), 7.11-6.96 (m, 4H), 5.19 (s, 1H) 4.54-4.29 (m, 2H), 3.31-3.28 (m, 2H) 2.69 (s, 3H), 1.36-1.31 (m, 1H), 1.17-1.03 (m, 1H), 0.85-0.60 (m, 6H). ¹³C NMR (75 MHz, CDCl₃) δ (ppm): 170.3, 167.2, 152.6, 152.4, 157.7, 151.5, 148.2, 142.7, 135.9, 132.7, 127.1, 124.0, 123.5, 122.0, 119.5, 118.0, 117.7, 117.5, 116.6, 116.4, 111.4, 109.9, 59.5, 56.8, 40.5, 37.8, 30.9, 29.7, 26.7, 19.6. HRMS m/z calculated for C₂₆H₂₈F₂N₆O₃ [M+H]⁺ 511.2269, found 511.2260.

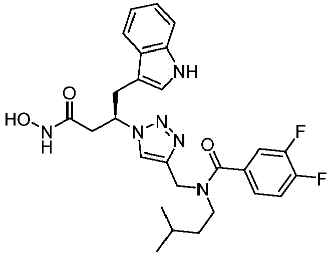

**3,4-difluoro-N-[[1-[(1R)-3-(hydroxyamino)-1-(1H-indol-3-ylmethyl)-3-oxopropyl]triazol-4-yl]methyl]-N-isopentyl-benzamide** (**99**). Compound **99** was obtained according to general procedure C from azide **131** and 3,4-difluoro-N-isopentyl-N-(prop-2-yn-1-yl)benzamide followed by general procedure G as a white solid (57 mg, 53% over 2 steps). Purity: 98%, LC t_{R}= 2.68 min, MS (ESI+): m/z = 525 [M + H]⁺. ¹H NMR (300 MHz, CDCl₃) δ (ppm): 9.03 (s, 1H), 7.31-7.27 (m, 1H), 7.20-7.15 (t, J = 6.96 Hz, 1H), 7.10-6.96 (m, 4H), 6.90 (s, 2H), 6.73 (s, 1H), 5.17 (s, 1H), 4.47-4.35 (m, 2H), 3.22 (s, 2H) 2.89 (s, 2H), 1.43-1.39 (m, 2H), 1.26 (s, 1H), 0.84-0.66 (m, 6H). ¹³C NMR (75 MHz, CDCl₃) δ (ppm): 170.0, 167.5, 151.5, 149.4, 149.2, 148.3, 148.2, 142.7, 135.9, 132.4, 127.2, 124.0, 123.7, 121.8, 119.3, 117.9, 117.7, 117.5, 116.4, 111.4, 109.7, 59.5, 47.8, 40.3, 37.5, 37.0, 31.0, 25.5, 22.14. HRMS m/z calculated for C₂₇H₃₀F₂N₆O₃ [M+H]⁺ 525.2426, found 525.2418.

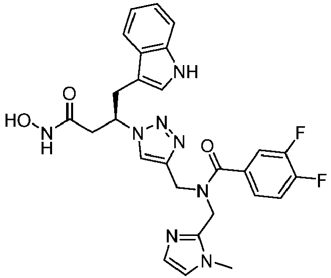

**3,4-difluoro-N-[[1-[(1R)-3-(hydroxyamino)-1-(1H-indol-3-ylmethyl)-3-oxopropyl]triazol-4-yl]methyl]-N-[(1-methylimidazol-2-yl)methyl]benzamide** (**100**). Compound **100** was obtained according to general procedure C from azide **131** and 3,4-difluoro-N-((1-methyl-1H-imidazol-2-yl)methyl)-N-(prop-2-yn-1-yl)benzamide followed by general procedure G as a brown solid (60 mg, 52% over 2 steps). Purity: 93%, LC t_{R}= 2.03 min, MS (ESI+): m/z = 549 [M + H]⁺. ¹H NMR (300 MHz, MeOD) δ (ppm): 7.47 (s, 1H), 7.36-7.25 (m, 5H), 7.04 (t, J = 6.70 Hz, 1H), 6.95-6.92 (m, 3H), 6.87 (s, 1H), 5.23 (s, 1H), 4.93 (s, 1H), 4.48-4.40 (m, 3H), 3.55 (s, 2H) 3.32-3.30 (m, 3H), 2.90-2.89 (m, 2H). HRMS m/z calculated for C₂₇H₂₆F₂N₈O₃ [M+H]⁺ 549.2174, found 549.2175.

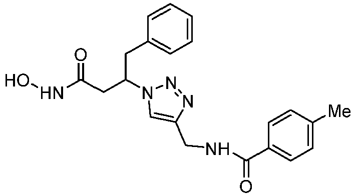

**N-[1-(1-Hydroxycarbamoylmethyl-2-phenyl-ethyl)-1H-[1,2,3]triazol-4-ylmethyl]-4-methyl-benzamide** (**101**). Compound **101** was obtained according to general procedure B from azide **108** and 4-methyl-N-(prop-2-yn-1-yl)benzamide as a white solid (20 mg, 54%), Purity: 97%, LC t_{R} = 2.10 min, MS (ESI+): m/z = 394 [M + H]⁺. ¹H NMR (DMSO-*d₆*) δ: 8.89 (t, J = 5.6 Hz, 1H), 8.37 (s, 1H), 7.78 (s, 1H), 7.75 (d, J = 8.0 Hz, 2H), 7.26 (d, J = 8.0 Hz, 2H), 7.19-7.10 (m, 3H), 6.99-6.96 (m, 2H), 5.14 (m, 1H), 4.42 (d, J = 5.6 Hz, 2H), 3.11 (d, J = 7.6 Hz, 2H), 2.72 (dd, J = 8.9 and 15.1 Hz, 1H), 2.58 (d, J = 5.5 and 15.1 Hz, 1H), 2.34 (s, 3H). ¹³C NMR (DMSO-*d₆*) δ (ppm): 166.4, 165.9, 145.1, 141.6, 137.4, 131.8, 129.4, 129.2 (2C), 128.7 (2C), 127.8 (2C), 127.1 (2C), 123.0, 59.3, 41.3, 37.5, 35.2, 21.4. HRMSm/z calculated for C₂₁H₂₄N₅O₃ [M + H]⁺ 394.1879, found 394.1892.

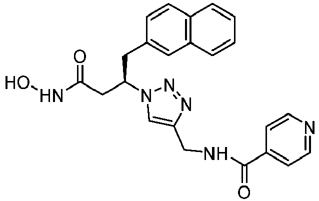

**Methyl (3R)-4-(2-naphthyl)-3-[4-[(pyridine-4-carbonylamino)methyl]triazol-1-yl]butanoate** (**102**). Compound **102** was obtained according to general procedure C from azide **107** and N-(prop-2-yn-1-yl)isonicotinamide followed by general procedure E as a white foam (105 mg, 98%). Purity: 100%, LC t_{R} = 2.02 min, MS (ESI+): m/z = 431 [M + H]⁺. ¹H NMR (MeOD) δ (ppm): 8.64 (s, 1H), 7.71-7.61 (m, 5H), 7.54 (s, 1H), 7.42 (s, 1H), 7.38-7.15 (m, 2H), 7.12 (dd, J = 1.2 Hz, J = 8.4 Hz, 1H), 5.30-5.23 (m, 1H), 4.50 (s, 2H), 3.41-3.32 (m, 2H), 2.99-2.82 (m, 2H). ¹³C NMR (MeOD) δ(ppm): 168.1, 167.0, 150.6 (2C), 144.9, 143.0, 135.0, 134.4, 133.5, 129.0, 128.6 (2C), 128.3 (2C), 127.7, 126.9, 126.6, 124.8, 122.7, 61.2, 42.3, 38.8, 35.8. HRMS m/z calculated for C₂₃H₂₃N₆O₃ [M + H]⁺ 433.1988, found 433.2008.

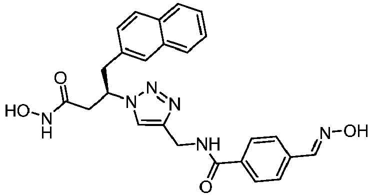

**N-[[1-[(1R)-3-(hydroxyamino)-1-(2-naphthylmethyl)-3-oxo-propyl]triazol-4-yl]methyl]-4-(hydroxycarbamoyl)benzamide** (**103**). Compound **103** was obtained according to general procedure C from azide **107** and (E)-4-((methylimino)methyl)-N-(prop-2-yn-1-yl)benzamide followed by general procedure F as a white foam (20 mg, 67%), Purity: 100%, LC t_{R} = 2.18 min, MS (ESI+): m/z = 473 [M + H]⁺. ¹H NMR (MeOD) δ (ppm): 8.51 (s, 1H), 7.80 (s, 4H), 7.77-7.61 (m, 4H), 7.42 (s, 1H), 7.38-7.14 (m, 2H), 7.11 (dd, J = 1.5 Hz, J = 8.4 Hz, 1H), 5.31-5.26 (m, 1H), 4.50 (s, 2H), 3.41-3.37 (m, 2H), 3.00-2.85 (m, 2H). ¹³C NMR (MeOD) δ (ppm): 168.9, 168.6, 167.1, 145.6, 138.1, 136.4, 135.4, 134.8, 133.8, 129.2, 128.8, 128.7, 128.5 (2C), 128.3 (2C), 128.0 (2C), 127.1, 126.7, 124.9, 61.5, 42.5, 38.9, 36.0. HRMS m/z calculated for C₂₅H₂₅N₆O₄ [M + H]⁺ 473.1985, found 473.1960.

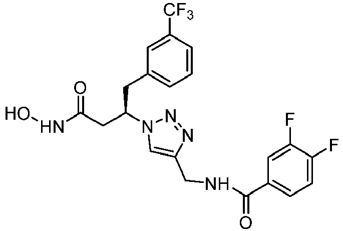

**3,4-difluoro-N-[[1-[(1R)-3-(hydroxyamino)-3-oxo-1-[[3-(trifluoromethyl)phenyl]methyl]propyl]triazol-4-yl]methyl]benzamide** (**104**). Compound **104** was obtained according to general procedure C from azide **126** and 3,4-difluoro-N-(prop-2-yn-1-yl)benzamide followed by general procedure F as a white powder (44 mg, 62% over 2 steps), Purity 99%, LC-MS t_{R} = 2.45 min, MS (ESI+): m/z = 484 [M + H]⁺. ¹H NMR (DMSO-*d₆*), δ (ppm): 2.62 (dd, J = 5.3 Hz, J = 15.2 Hz, 1H), 2.77 (dd, J = 9.1 Hz, J = 15.2 Hz, 1H), 3.24 (d, J = 4.31 Hz, 2H), 4.21 (d, J = 5.78 Hz, 2H), 5.20 (m, 1H), 7.26 (d, J = 7.67 Hz, 1H), 7.31 (s, 1H), 7.39 (t, J = 7.6 Hz, 1H), 7.48 (d, J = 7.8 Hz, 1H), 7.56 (Dd, J = 2.1 Hz, J = 9.6 Hz, 1H), 7.72-7.77 (m, 1H), 7.84 (s, 1H), 7.89 (td, J = 2.1 Hz, J = 7.9 Hz, 1H), 8.79 (s, 1H, NH hydroxamic acid), 9.09 (t, J = 5.78 Hz, 1H), 10.51 (s, 1H). ¹⁹F NMR (DMSO-*d₆*), δ (ppm): -61.6, -135.1, -138.6. ¹³C NMR (DMSO-*d₆*), δ (ppm): 34.7, 36.9, 40.0, 58.5, 116.5, 116.7, 122.7, 123.3, 124.8, 125.4, 129.2, 131.5, 131.6, 133.0, 138.3, 144.2, 163.8, 165.3. HRMS m/z calculated for C₂₁H₁₉F₅N₅O₃ [M + H]⁺ 484.1406, found 484.1408.

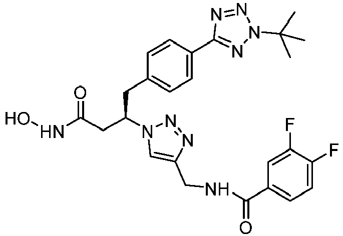

**N-({1-[(2R)-1-[4-(2-tert-butyl-2H-1,2,3,4-tetrazol-5-yl)phenyl]-3-(hydroxycarbamoyl)propan2-yl]-1H-1,2,3-triazol-4-yl}methyl)-3,4-difluorobenzamide** (105). Compound **105** was obtained according to general procedure C from azide **127** and 3,4-difluoro-N-(prop-2-yn-1-yl)benzamide followed by general procedure F as a white solid (61 mg, 47%). Purity 99%, LC t_{R} = 2.48 min, MS (ESI-): m/z = 538 [M-H]⁻. ¹H NMR (DMSO-*d₆*) δ (ppm): 10.52 (s, 1H), 9.09 (t, J = 5.7 Hz, 1H), 8.81 (s, 1H), 7.87 (dd, J = 2.0 Hz, J = 7.8 Hz, 1H ), 7.85 (s, 1H), 7.82 (d, J = 8.2 Hz, 2H), 7.72 (m, 1H), 7.51 (dd, J = 2.0 Hz, J = 8.3 Hz, 1H), 7.12 (d, J = 8.2 Hz, 2H), 5.19 (m, 1H), 4.42 (d, J = 5.7 Hz, 2H), 3.20 (m, 2H), 2.77 (dd, J = 8.7 Hz, J = 15.1 Hz, 1H), 2.66 (dd, J = 5.7 Hz, J = 15.1 Hz, 1H), 1.71 (s, 9H). ¹³C NMR (DMSO-d₆) δ (ppm): 165.4, 163.8, 163.4, 144.2, 139.3, 131.5, 129.7, 126.2, 125.5, 124.7, 122.6, 117.5, 116.5, 63.8, 58.6, 40.6, 37.0, 34.8, 28.7. ¹⁹F NMR (DMSO-d₆) δ (ppm): -135.1 (s), -138.4 (s). HRMS m/z calculated for C₂₅H₂₈F₂N₉O₃ [M+H]⁺: 540.2283, found: 540.2292.

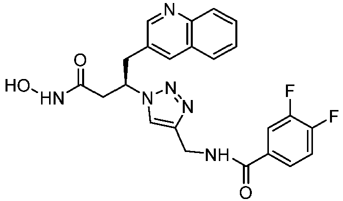

**(R)-3,4-difluoro-N-((1-(4-(hydroxyamino)-4-oxo-1-(quinolin-3-yl)butan-2-yl)-1H-1,2,3-triazol-4-yl)methyl)benzamide** (**143**). Compound **143** was obtained according to general procedure C from azide **140** and 3,4-difluoro-N-(prop-2-yn-1-yl)benzamide followed by general procedure G as a white solid (59 mg, 49%). Purity 96%, LC t_{R} = 2.03 min, MS (ESI+): m/z = 467 [M + H]⁺. ¹H NMR (MeOD) δ (ppm): 8.46 (d, J = 2.0 Hz, 1H), 7.89 (d, J = 2.0 Hz, 1H), 7.86 (d, J = 9.1 Hz, 1H), 7.72 (s, 1H), 7.71 (dd, J = 8.4 and 1.0 Hz, 1H), 7.65 (dd, J = 7.0 and 1.7 Hz, 1H), 7.63-7.59 (m, 1H), 7.59-7.53 (m, 1H), 7.45 (ddd, J = 8.0, 7.0 and 1.0 Hz, 1H), 7.31 (ddd, J = 10.3, 8.4 and 8.2 Hz, 1H), 5.40-5.30 (m, 1H), 4.47 (d, J = 4.3 Hz, 2H), 3.51 (dd, J = 14.5 and 5.5 Hz, 1H), 3.46 (dd, J = 14.1 and 9.2 Hz, 1H), 3.00 (dd, J = 15.0 and 8.8 Hz, 1H), 2.92 (dd, J = 15.0 and 5.9 Hz, 1H). ¹³C NMR (MeOD) δ (ppm): 168.3, 167.3, 153.7 (dd, J = 252.3 and 12.6 Hz), 152.2, 151.2 (dd, J = 247.5 and 13.2 Hz), 147.5, 145.8, 137.9, 132.5 (dd, J = 4.8 and 3.6 Hz), 131.3, 130.8, 129.3, 128.9, 128.7, 128.2, 125.5 (dd, J = 7.2 and 3.6 Hz), 125.0, 118.4 (d, J = 18.0 Hz), 117.9 (d, J = 18.6 Hz), 61.0, 39.3, 38.9, 36.0. HRMS m/z calculated for C₂₃H₂₀F₂N₆O₃ [M+H]⁺ 467.1643, found 467.1642.

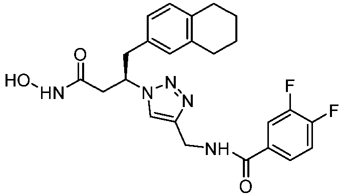

**(R)-3,4-difluoro-N-((1-(4-(hydroxyamino)-4-oxo-1-(5,6,7,8-tetrahydronaphthalen-2-yl)butan-2-yl)-1H-1,2,3-triazol-4-yl)methyl)benzamide** (**144**). Compound **144** was obtained according to general procedure C from azide **141** and 3,4-difluoro-N-(prop-2-yn-1-yl)benzamide followed by general procedure G as a white solid (51 mg, 67%). Purity: 96%, LC t_{R} = 2.48 min, MS (ESI+): m/z = 470 [M+H]⁺. ¹H NMR (MeOD) δ (ppm): 7.76 (ddd, J = 11.2, 7.7 and 2.2 Hz, 1H), 7.71-7.65 (m, 1H), 7.62 (s, 1H), 7.36 (ddd, J = 10.2, 8.4 and 8.2 Hz, 1H), 6.79 (d, J = 7.7 Hz, 1H), 6.64 (br d, J = 8.1 and 1.6 Hz, 1H), 6.62 (br s, 1H), 5.17-5.06 (m, 1H), 4.53 (s, 2H), 3.16-3.05 (m, 2H), 2.87 (dd, J = 14.9 and 9.0 Hz, 1H), 2.77 (dd, J = 14.9 and 5.6 Hz, 1H), 2.62-2.50 (m, 4H), 1.68-1.64 (m, 4H). ¹³C NMR (MeOD) δ (ppm): 168.6, 167.3, 153.8 (dd, J = 252.3 and 12.6 Hz), 151.3 (dd, J = 248.1 and 13.2 Hz), 145.4, 138.2, 136.7, 134.8, 132.7 (dd, J = 4.8 and 3.6 Hz), 130.6, 130.2, 127.2, 125.6 (dd, J = 7.2 and 3.6 Hz), 124.9, 118.5 (d, J = 18.0 Hz), 117.9 (d, J = 19.2 Hz), 61.7, 42.0, 38.8, 36.1, 30.2, 29.9, 24.30, 24.26. HRMS m/z calculated for C₂₄H₂₅F₂N₅O₃ [M+H]⁺ 470.2004, found 470.2000.

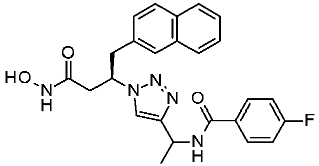

**4-fluoro-N-(1-(1-((R)-4-(hydroxyamino)-1-(naphthalen-2-yl)-4-oxobutan-2-yl)-1H-1,2,3-triazol-4-yl)ethyl)benzamide** (**164**). Compound **164** was obtained according to general procedure C' from azide **109** and N-(but-3-yn-2yl)-4-fluorobenzamide, followed by general procedure G' as a white solid (61 mg, 58% over 2 steps). Purity: 99%, LC t_{R} = 2.32 min, MS (ESI+): m/z = 462 [M + H]⁺. Diastereoisomeric mixture, (R,S)/(R,R) ratio = 4:1. ¹H NMR (MeOD-*d₄*) δ (ppm): 7.81-7.76 (m, 2H), 7.74-7.61 (m, 3H), 7.58 (s, 0.2H), 7.53 (s, 0.8H), 7.41-7.33 (m, 3H), 7.17-7.10 (m, 3H), 5.31-5.21 (m, 2H), 3.45-3.35 (m, 2H), 3.25-3.14 (m, 0.2H), 2.98 (dd, *J* = 14.8, 9.0 Hz, 0.8H), 2.97 (dd, *J* = 14.8, 8.9 Hz, 0.2H), 2.87 (dd, *J* = 14.9, 5.5 Hz, 0.8H), 1.48 (d, *J* = 7.0 Hz, 0.6H), 1.44 (d, *J* = 7.0 Hz, 2.4H). ¹³C NMR (MeOD-*d₄*) δ (ppm): major (R,S): 168.6, 168.2, 166.2 (d, *J* = 250.2 Hz), 150.3, 135.4, 134.8, 133.9, 131.8 (d, *J* = 2.7 Hz), 131.1 (d, *J* = 9.0 Hz, 2C), 129.2, 128.8, 128.5 (2C), 128.0, 127.1, 126.8, 123.9, 116.3 (d, *J* = 22.2 Hz, 2C), 61.5, 43.3, 42.5, 38.8, 20.4. Minor (R,R): 168.6, 168.2, 166.2 (d, *J* = 250.2 Hz), 150.4, 135.4, 134.8, 133.9, 131.8 (d, *J* = 2.7 Hz), 131.1 (d, *J* = 9.0 Hz, 2C), 129.2, 128.8, 128.5 (2C), 128.0, 127.1, 126.8, 123.6, 116.3 (d, *J* = 22.2 Hz, 2C), 61.5, 43.3, 42.5, 38.8, 20.5. HRMS: m/z calculated for C₂₅H₂₅N₅O₃F [M + H]⁺: 462.1941; found: 462.1942.

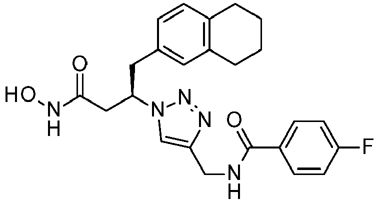

**(R)-4-fluoro-N-((1-(4-(hydroxyamino)-4-oxo-1-(5,6,7,8-tetrahydronaphthalen-2-yl)butan-2-yl)-1H-1,2,3-triazol-4-yl)methyl)benzamide** (**165**). Compound **165** was obtained according to general procedure C' from azide **141** and N-(prop-2-ynyl)-4-fluorobenzamide followed by general procedure G' as a white solid (35 mg, 72% over 2 steps). Purity: 99.5%, LC t_{R} = 2.40 min, MS (ESI+): m/z = 452 [M + H]⁺. ¹H NMR (MeOD-*d₄*) δ (ppm): 7.92-7.85 (m, 2H), 7.62 (s, 1H), 7.23-7.14 (m, 2H), 6.79 (d, *J* = 7.5 Hz, 1H), 6.67-6.61 (m, 2H), 5.17-5.07 (m, 1H), 4.54 (s, 2H), 3.21-3.06 (m, 2H), 2.88 (dd, *J* = 14.9 and 9.0 Hz, 1H), 2.78 (dd, *J* = 14.9 and 5.6 Hz, 1H), 2.64-2.48 (m, 4H), 1.73-1.58 (m, 4H). ¹³C NMR (MeOD-*d₄*) δ (ppm): 168.6, 168.6, 166.3 (d, *J* = 249.3 Hz), 145.6, 138.2, 136.7, 134.7, 131.6 (d, *J* = 3.2 Hz), 131.0 (d, *J* = 9.2 Hz, 2C), 130.6, 130.2, 127.1, 124.8, 116.4 (d, *J* = 22.2 Hz, 2C), 61.6, 42.0, 38.8, 36.0, 30.2, 29.9, 24.3, 24.2. HRMS: m/z calculated for C₂₄H₂₇NO₃F, [M+H]⁺: 452.2098; found: 452.2096.

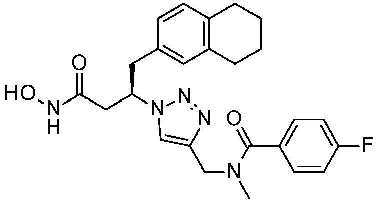

**(R)-4-fluoro-N-((1-(4-(hydroxyamino)-4-oxo-1-(5,6,7,8-tetrahydronaphthalen-2-yl)butan-2-yl)-1H-1,2,3-triazol-4-yl)methyl)-N-methylbenzamide** (**166**). Compound **166** was obtained according to general procedure C' from azide **141** and N-methyl-N-(prop-2-ynyl)-4-fluorobenzamide followed by general procedure G' as an off-white solid (28 mg, 56% over 2 steps). Purity: 99%, LC t_{R} = 2.42 min, MS (ESI+): m/z = 466 [M + H]⁺. Mixture of amide isomer, approximate ratio: 1/0.7. ¹H NMR (MeOD-*d₄*) δ (ppm): 7.66 (br s, 0.6H), 7.47 (br s, 2.4H), 7.21-7.12 (m, 2H), 6.85 (br s, 1H), 6.70 (s, 0.8H), 6.67 (s, 1.2H), 5.15 (br s, 1H), 4.76-4.61 (m, 1H), 4.41 (br s, 1H), 3.19 (dd, *J* = 13.8 and 5.4 Hz, 1H), 3.11 (dd, *J* = 13.8 and 9.8 Hz, 1H), 2.97-2.79 (m, 5H), 2.68-2.54 (m, 4H), 1.70 (br s, 4H). ¹³C NMR (MeOD-*d₄*) δ (ppm): major isomer: 172.6, 168.5, 164.9 (d, *J* = 249.5 Hz), 143.6 (br), 138.2, 136.8, 134.9, 133.2 (m), 130.7 (d, *J* = 9.4 Hz, 2C), 130.6, 130.2, 127.1, 125.5, 116.5 (d, *J* = 22.4 Hz, 2C), 61.7, 47.5, 42.1, 38.8, 37.8, 30.2, 29.9, 24.3, 24.3. Minor isomer: 172.6, 168.5, 164.9 (d, *J* = 249.5 Hz), 143.6 (br), 138.2, 136.8, 134.9, 133.2 (m), 130.7 (d, *J* = 9.4 Hz, 2C), 130.6, 130.2, 127.1, 125.5, 116.5 (d, *J* = 22.4 Hz, 2C), 61.0, 47.5, 43.3, 42.3, 33.5, 30.8, 30.2, 24.3, 24.3. HRMS: m/z calculated for C₂₅H₂₉N₅O₃F [M + H]⁺: 466.2254; found: 466.2248.

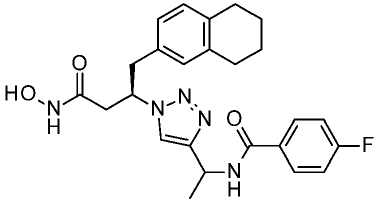

**4-fluoro-N-(1-(1-((R)-4-(hydroxyamino)-4-oxo-1-(5,6,7,8-tetrahydronaphthalen-2-yl)butan-2-yl)-1H-1,2,3-triazol-4-yl)ethyl)benzamide** (**167**). Compound **167** was obtained according to general procedure C' from azide **141** and N-(but-3-yn-2yl)-4-fluorobenzamide, followed by general procedure G' as a white solid (50 mg, 72% over 2 steps). Purity: 95%, LC t_{R} = 2.47 min, MS (ESI+): m/z = 466 [M + H]⁺. R,R isomer as major product, but signal of R,S isomer also visible, approximate ratio: 1:0.4. ¹H NMR (MeOD-*d₄*) δ (ppm): 7.91-7.85 (m, 2H), 7.57 (s, 1H), 7.21-7.13 (m, 2H), 6.84-6.79 (m, 1H), 6.67-6.61 (m, 2H), 5.33 (q, *J* = 6.9 Hz, 1H), 5.14-5.05 (m, 1H), 3.20-3.05 (m, 2H), 2.89 (dd, *J* = 14.9 and 9.1 Hz, 1H), 2.78 (dd, *J* = 14.9 and 5.4 Hz, 1H), 2.67-2.51 (m, 4H), 1.73-1.64 (m, 4H), 1.54 (d, *J* = 7.0 Hz, 3H). ¹³C NMR (MeOD-*d₄*) δ (ppm): 168.6, 168.2, 166.2 (d, *J* = 249.6 Hz), 150.1, 138.2, 136.8, 134.8, 131.9-131.8 (m), 131.1 (d, *J* = 9.0 Hz, 2C), 130.6, 130.2, 127.1, 123.8, 116.3 (d, *J* = 22.3 Hz, 2C), 61.7, 43.3, 42.1, 38.7, 37.1, 30.2, 29.9, 24.3, 24.3, 20.6. HRMS: m/z calculated for C₂₅H₂₉N₅O₃F [M + H]⁺: 466.2254; found: 466.2256.

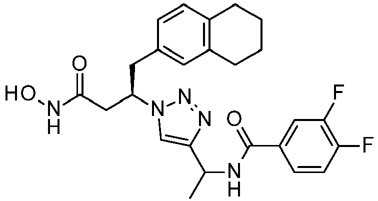

**3,4-difluoro-N-(1-(1-((R)-4-(hydroxyamino)-4-oxo-1-(5,6,7,8-tetrahydronaphthalen-2-yl)butan-2-yl)-1H-1,2,3-triazol-4-yl)ethyl)benzamide** (**168**). Compound **168** was obtained according to general procedure C' from azide **142** and N-(but-3-yn-2yl)-3,4-difluorobenzamide as a white solid (31 mg, 29%). Purity: 99%, LC t_{R} = 2.52 min, MS (ESI+): m/z = 484 [M + H]⁺. Mixture of (R,S) and (R,R) isomers: ¹H NMR (CDCl₃ + 1% TMS) δ (ppm): 7.81-7.73 (m, 1H), 7.72-7.65 (m, 1H), 7.35 (dtd, *J* = 10.4 and 8.2 and 2.8 Hz, 1H), 6.82 (br t, *J* = 7.1 Hz, 1H), 6.68-6.63 (m, 1H), 6.61 (s, 1H), 5.31 (br q, *J* = 7.0 Hz, 1H), 5.16-5.05 (m, 1H), 3.18-3.04 (m, 2H), 2.89 (ddd, J = 14.9 and 9.0 and 2.8 Hz, 1H), 2.78 (dd, *J* = 14.9 and 5.2 Hz, 1H), 2.65-2.54 (m, 4H), 1.72-1.64 (m, 4H), 1.55+1.54 (2d, *J* = 7.0 Hz, 3H). ¹³C NMR (CDCl₃ + 1% TMS) δ (ppm): (R,S) isomer: 168.6, 166.8, 153.7 (dd, *J* = 252.3 and 12.6 Hz), 151.3 (dd, *J* = 248.6 and 13.2 Hz), 150.0, 138.2, 136.8, 134.8, 133.0-132.7 (m), 130.6, 130.2, 127.2, 125.7 (dd, *J* = 7.2 and 3.6 Hz), 123.9, 118.4 (d, *J* = 18.0 Hz), 118.0 (d, *J* = 18.5 Hz), 61.7, 43.4, 42.1, 38.7, 30.2, 29.9, 24.3, 24.3, 20.5. (R,R) isomer: 168.6, 166.8, 153.7 (dd, *J* = 252.3 and 12.6 Hz), 151.3 (dd, *J* = 248.6 and 13.2 Hz), 150.1, 138.2, 136.8, 134.8, 133.0-132.7 (m), 130.6, 130.2, 127.2, 125.7 (dd, *J* = 7.2 and 3.6 Hz), 123.6, 118.4 (d, *J* = 18.0 Hz), 118.0 (d, *J* = 18.5 Hz), 61.7, 43.4, 42.1, 38.7, 30.2, 29.9, 24.3, 24.3, 20.6. HRMS: m/z calculated for C₂₅H₂₈N₅O₃F₂ [M + H]⁺ = 484.2160; found: 484.2160.

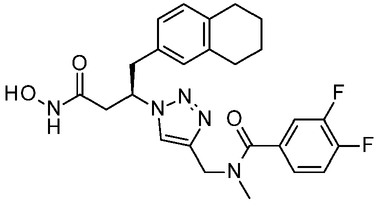

**(R)-3,4-difluoro-N-((1-(4-(hydroxyamino)-4-oxo-1-(5,6,7,8-tetrahydronaphthalen-2-yl)butan-2-yl)-1H-1,2,3-triazol-4-yl)methyl)-N-methylbenzamide** (**169**). Compound **169** was obtained according to general procedure C' from azide **141** and N-methyl-N-(prop-2-ynyl)-3,4-difluorobenzamide followed by general procedure G' as an off-white solid (19 mg, 18% over 2 steps). Purity: 97%, LC t_{R} = 2.50 min, MS (ESI+): m/z = 484 [M + H]⁺. Mixture of amide isomers, approximate ratio: 1.5/1: ¹H NMR (MeOD-*d₄*) δ (ppm): 7.72 (br s, 0.6H), 7.50 (br s, 0.4H), 7.44-7.31 (m, 2H), 7.26 (br s, 1H), 6.93-6.83 (m, 1H), 6.74-6.60 (m, 2H), 5.21-5.09 (m, 1H), 4.74-4.59 (m, 1H), 4.41 (br s, 1H), 3.24-3.07 (m, 2H), 2.96-2.79 (m, 5H), 2.70-2.52 (m, 4H), 1.70 (br s, 4H). ¹³C NMR (MeOD-*d₄*) δ (ppm): major isomer: 168.6, 152.5 (dd, *J* = 249.3 and 12.6 Hz), 151.4 (dd, *J* = 249.3 and 13.2 Hz), 143.5, 143.0, 138.2, 136.8, 134.9, 134.2-134.0 (m), 130.6, 130.2, 127.1, 125.6, 125.3 (br), 118.8 (d, *J* = 17.9 Hz), 117.9 (d, *J* = 18.8 Hz), 61.8, 42.1, 38.8, 37.7, 30.2, 29.9, 24.3, 24.3. Minor isomer: 168.6, 152.5 (dd, *J* = 249.3 and 12.6 Hz), 151.4 (dd, *J* = 249.3 and 13.2 Hz), 143.5, 143.0, 138.2, 136.8, 134.9, 134.2-134.0 (m), 130.6, 130.2, 127.1, 125.6, 125.3 (br), 118.8 (d, *J* = 17.9 Hz), 117.9 (d, *J* = 18.8 Hz), 61.8, 47.4, 43.3, 33.4, 30.2, 29.9, 24.3, 24.3. HRMS: m/z calculated for C₂₅H₂₈N₅O₃F₂ [M + H]⁺: 484.2160; found: 484.2150.

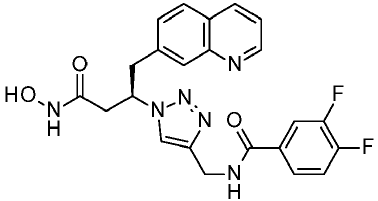

**(R)-3,4-difluoro-N-((1-(4-(hydroxyamino)-4-oxo-1-(quinolin-7-yl)butan-2-yl)-1H-1,2,3-triazol-4-yl)methyl)benzamide (170).** Compound **170** was obtained according to general procedure C' from azide **151** and N-(prop-2-ynyl)-3,4-difluorobenzamide followed by general procedure G' as an off-white solid (24 mg, 59% over 2 steps). Purity: 98%, LC t_{R} = 1.97 min, MS (ESI+): m/z = 467 [M + H]⁺. ¹H NMR (MeOD-*d₄*) δ (ppm): 8.70 (dd, *J* = 4.4 and 1.6 Hz, 1H), 8.19 (dd, *J* = 8.2 and 1.0 Hz, 1H), 7.75 (d, *J* = 8.4 Hz, 1H), 7.68-7.55 (m, 4H), 7.41 (dd, *J* = 8.2 and 4.4 Hz, 1H), 7.37-7.28 (m, 2H), 5.39-5.29 (m, 1H), 4.50 (d, *J* = 15.3 Hz, 1H), 4.44 (d, *J* = 15.3 Hz, 1H), 3.51 (dd, *J* = 13.8 and 5.5 Hz, 1H), 3.45 (dd, *J* = 13.8 and 8.9 Hz, 1H), 3.00 (dd, *J* = 15.0 and 9.0 Hz, 1H), 2.90 (dd, *J* = 15.0 and 5.6 Hz, 1H).¹³C NMR (MeOD-*d₄*) δ (ppm): 168.4, 167.3, 153.7 (dd, *J* = 252.2 and 12.6 Hz), 151.3, 151.3 (dd, *J* = 248.0 and 13.1 Hz), 148.5, 145.6, 140.4, 138.0, 132.6 (dd, *J* = 4.1 and 3.6 Hz), 129.5, 129.3, 129.0, 128.7, 125.5 (dd, *J* = 7.2 and 3.6 Hz), 124.9, 122.3, 118.5 (d, *J* = 18.0 Hz), 117.9 (d, *J* = 18.6 Hz), 61.2, 42.4, 38.9, 36.0. HRMS: m/z calculated for C₂₃H₂₁F₂N₆O₃ [M + H]⁺: 467.1643; found: 467.1645.

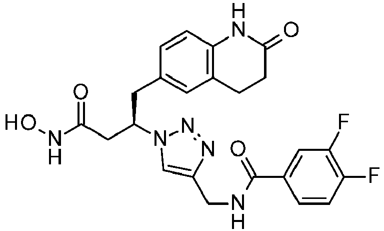

**(R)-3,4-difluoro-N-((1-(4-(hydroxyamino)-4-oxo-1-(2-oxo-1,2,3,4-tetrahydroquinolin-6-yl)butan-2-yl)-1H-1,2,3-triazol-4-yl)methyl)benzamide** (**171**). Compound **171** was obtained according to general procedure C' from azide **153** and N-(prop-2-ynyl)-3,4-difluorobenzamide followed by general procedure G' as an off-white solid (29 mg, 58% over 2 steps). Purity: 97%, LC t_{R} = 1.78 min, MS (ESI+): m/z = 485 [M + H]⁺. ¹H NMR (MeOD-*d₄*) δ (ppm): 7.75 (ddd, *J* = 11.2 and 7.7 and 2.1 Hz, 1H), 7.69-7.62 (m, 1H), 7.64 (s, 1H), 7.36 (ddd, *J* = 10.2 and 8.4 and 8.2 Hz, 1H), 6.82-6.77 (m, 1H), 6.78 (s, 1H), 6.68-6.64 (m, 1H), 5.19-5.09 (m, 1H), 4.52 (s, 2H), 3.19 (dd, *J* = 13.8 and 5.7 Hz, 1H), 3.13 (dd, *J* = 13.8 and 9.2 Hz, 1H), 2.90 (dd, *J* = 15.1 and 8.8 Hz, 1H), 2.80 (dd, *J* = 15.0 and 5.7 Hz, 1H), 2.79-2.68 (m, 2H), 2.43 (t, *J* = 7.5 Hz, 2H). ¹³C NMR (MeOD-*d₄*) δ (ppm): 173.7, 168.5, 167.4, 153.8 (dd, *J* = 252.3 and 12.6 Hz), 151.4 (dd, *J* = 248.1 and 13.2 Hz), 145.5, 137.7, 132.7-132.5 (m), 132.6, 129.5, 129.0, 125.5 (dd, *J* = 7.3 and 3.6 Hz), 125.3, 124.9, 118.6 (d, *J* = 18.0 Hz), 117.9 (d, *J* = 18.6 Hz), 116.6, 61.6, 41.7, 38.7, 36.1, 31.4, 26.0. HRMS: m/z calculated for C₂₃H₂₃N₆O₄F₂ [M + H]⁺: 485.1749; found: 485.1742.

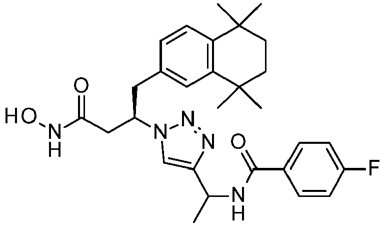

**4-fluoro-N-(1-(1-((R)-4-(hydroxyamino)-4-oxo-1-(5,5,8,8-tetramethyl-5,6,7,8-tetrahydronaphthalen-2-yl)butan-2-yl)-1H-1,2,3-triazol-4-yl)ethyl)benzamide** (**172**). Compound **172** was obtained according to general procedure C' from azide **154** and N-(but-3-yn-2yl)-4-fluorobenzamide, followed by general procedure G' as a white solid (75 mg, 59% over 2 steps). Purity: 99%, LC t_{R} = 2.82 min, MS (ESI+): m/z = 522 [M + H]⁺. ¹H NMR (MeOD-*d₄*) δ (ppm): 7.90-7.85 (m, 2H), 7.60 (s, 1H), 7.19-7.11 (m, 3H), 6.83 (br s, 1H), 6.79 (dd, *J* = 7.9 and 1.4 Hz, 1H), 5.33 (q, *J* = 6.9 Hz, 1H), 5.16-5.06 (m, 1H), 3.26-3.10 (m, 2H), 2.89 (dd, *J* = 14.9 and 9.0 Hz, 1H), 2.79 (dd, *J* = 14.9 and 5.5 Hz, 1H), 1.61 (br s, 4H), 1.56+1.52 (2d, *J* = 6.9 Hz, 3H), 1.18+1.09 (2s, 12H).¹³C NMR (MeOD-*d₄*) δ (ppm): (R,R) isomer: 168.6 , 168.2, 166.2 (d, *J* = 249.1 Hz), 150.0, 146.0, 144.5, 134.8, 131.9-131.8 (m), 131.1 (d, *J* = 9.0 Hz, 2C), 128.2, 127.8, 127.3, 123.9, 116.3 (d, *J* = 22.2 Hz, 2C), 61.7, 43.3, 42.0, 38.9, 36.1 (2C), 35.0, 34.8, 32.2 (4C), 20.6. (R,S) isomer: 168.6, 168.2, 166.2 (d, *J* = 249.1 Hz), 150.1, 146.0, 144.5, 135.0, 131.9-131.8 (m), 131.1 (d, *J* = 9.0 Hz, 2C), 128.2, 127.8, 127.3, 123.7, 116.3 (d, *J* = 22.2 Hz, 2C), 60.8, 43.3, 42.3, 38.9, 36.1 (2C), 35.0, 34.8, 32.3 (4C), 20.7. HRMS: m/z calculated for C₂₉H₃₇O₃N₅F [M + H]⁺: 522.2880; found: 522.2888.

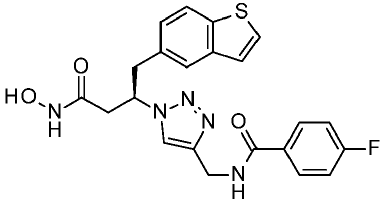

**(R)-N-((1-(1-(benzo[b]thiophen-5-yl)-4-(hydroxyamino)-4-oxobutan-2-yl)-1H-1,2,3-triazol-4-yl)methyl)-4-fluorobenzamide** (**173**). Compound **173** was obtained according to general procedure C' from azide **155** and N-(prop-2-ynyl)-4-fluorobenzamide followed by general procedure G' as a white solid (47 mg, 70% over 2 steps). Purity: 99.5%, LC t_{R} = 2.20 min, MS (ESI+): m/z = 454 [M + H]⁺. ¹H NMR (MeOD-*d₄*) δ (ppm): 7.85-7.78 (m, 2H), 7.66 (d, *J* = 8.2 Hz, 1H), 7.61 (s, 1H), 7.46-7.44 (m, 2H), 7.21-7.13 (m, 3H), 6.96 (dd, *J* = 8.2 and 1.1 Hz, 1H), 5.29-5.19 (m, 1H), 4.54 (dd, *J* = 15.5 and 4.1 Hz, 1H), 4.47 (dd, *J* = 15.5 and 4.1 Hz, 1H), 3.43-3.28 (m, 2H), 2.94 (dd, *J* = 14.9 and 8.9 Hz, 1H), 2.84 (dd, *J* = 14.9 and 5.6 Hz, 1H). ¹³C NMR (MeOD-*d₄*) δ (ppm): 168.7, 168.5, 166.2 (d, *J* = 250.4 Hz), 145.7, 141.4, 139.8, 133.9, 131.6 (d, *J* = 3.3 Hz), 131.0 (d, *J* = 9.0 Hz, 2C), 128.0, 126.3, 124.9, 124.8, 124.6, 123.4, 116.4 (d, *J* = 22.2 Hz, 2C), 61.8, 42.2, 38.8, 36.0. HRMS: m/z calculated for C₂₂H₂₂N₅O₃SF [M + H]⁺: 454.1349; found 454.1348.

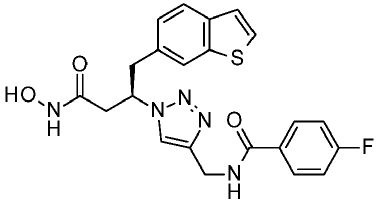

**(R)-N-((1-(1-(benzo[b]thiophen-6-yl)-4-(hydroxyamino)-4-oxobutan-2-yl)-1H-1,2,3-triazol-4-yl)methyl)-4-fluorobenzamide (174).** Compound **174** was obtained according to general procedure C' from azide **156** and N-(prop-2-ynyl)-4-fluorobenzamide followed by general procedure G' as a white solid (43 mg, 73% over 2 steps). Purity: 99.5%, LC t_{R} = 2.20 min, MS (ESI+): m/z = 454 [M + H]⁺. ¹H NMR, MeOD, δ (ppm): 7.84-7.78 (m, 2H), 7.66-759 (m, 2H), 7.51 (br s, 1H), 7.43 (d, *J* = 5.4 Hz, 1H), 7.20 (dd, *J* = 5.4, 1.0 Hz, 1H), 7.19-7.12 (m, 2H), 6.98 (dd, *J* = 8.2, 1.3 Hz, 1H), 5.29-5.18 (m, 1H), 4.52 (d, *J* = 15.8 Hz, 1H), 4.47 (d, *J* = 15.8 Hz, 1H), 3.38 (dd, *J* = 13.8, 5.6 Hz, 1H), 3.32 (dd, *J* = 13.8, 9.0 Hz, 1H), 2.93 (dd, *J* = 14.9, 9.0 Hz, 1H), 2.83 (dd, *J* = 14.9, 5.5 Hz, 1H). ¹³C NMR, MeOD, δ (ppm): 168.8, 168.5, 166.2 (d, *J* = 250.6 Hz), 145.7, 141.4, 140.0, 134.1, 131.6 (d, *J* = 2.8 Hz), 131.0 (d, *J* = 9.0 Hz, 2C), 127.4, 126.4, 124.9, 124.6, 124.5, 123.6, 116.3 (d, *J* = 22.2 Hz, 2C), 61.7, 42.3, 38.8, 36.0. HRMS: m/z calculated for C₂₂H₂₁N₅O₃SF, [M + H]⁺: 454.1349; found: 454.1351.

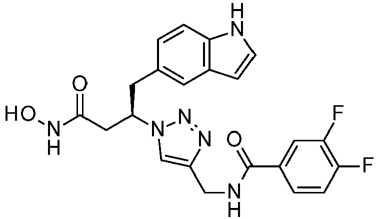

**(R)-3,4-difluoro-N-((1-(4-(hydroxyamino)-1-(1H-indol-5-yl)-4-oxobutan-2-yl)-1H-1,2,3-triazol-4-yl)methyl)benzamide (175).** Compound **175** was obtained according to general procedure C' from azide **157** and N-(prop-2-ynyl)-3,4-difluorobenzamide followed by general procedure G' as an off-white solid (37 mg, 54% over 2 steps). Purity: 97%, LC t_{R} = 2.07 min, MS (ESI+): m/z = 455 [M + H]⁺. ¹H NMR (MeOD-*d₄*) δ (ppm): 7.68 (ddd, *J* = 11.3, 7.7, 2.1 Hz, 1H), 7.62-7.56 (m, 1H), 7.55 (s, 1H), 7.34 (ddd, *J* = 10.2, 8.5, 8.0 Hz, 1H), 7.19 (br d, *J* = 8.3 Hz, 1H), 7.15 (d, *J* = 0.7 Hz, 1H), 7.12 (d, *J* = 3.1 Hz, 1H), 6.76 (dd, *J* = 8.3, 1.6 Hz, 1H), 6.25 (dd, *J* = 3.1, 0.7 Hz, 1H), 5.23-5.14 (m, 1H), 4.53 (dd, *J* = 16.1, 4.3 Hz, 1H), 4.46 (dd, *J* = 16.1, 4.3 Hz, 1H), 3.30 (dd, *J* = 13.5, 5.8 Hz, 1H), 3.24 (dd, *J* = 13.5, 8.6 Hz, 1H), 2.91 (dd, *J* = 14.9, 9.1 Hz, 1H), 2.81 (dd, *J* = 14.9, 5.5 Hz, 1H). ¹³C NMR (MeOD-*d₄*) δ (ppm): 168.8, 167.5, 153.7 (dd, *J* = 151.2 and 12.8 Hz), 151.3 (dd, *J* = 247.9 and 13.1 Hz), 145.2, 136.7, 132.7 (dd, *J* = 4.8 and 3.2 Hz), 129.6, 128.0, 126.0, 125.5 (dd, *J* = 7.2 and 4.2 Hz), 124.9, 123.2, 121.5, 118.5 (d, *J* = 17.9 Hz), 117.9 (d, *J* = 18.4 Hz), 112.2, 102.1, 62.3, 42.8, 38.7, 36.1. HRMS: m/z calculated for C₂₂H₂N₆O₃F₂, [M + H]⁺: 455.1643; found: 455.1641.

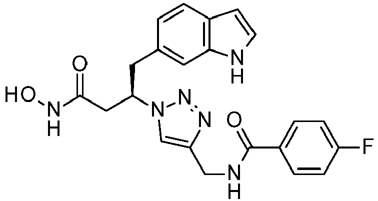

**(R)-4-fluoro-N-((1-(4-(hydroxyamino)-1-(1H-indol-6-yl)-4-oxobutan-2-yl)-1H-1,2,3-triazol-4-yl)methyl)benzamide (176).** Compound **176** was obtained according to general procedure C' from azide **158** and N-(prop-2-ynyl)-4-fluorobenzamide followed by general procedure G' as a white solid (41 mg, 73% over 2 steps). Purity: 99.5%, LC t_{R} = 2.08 min, MS (ESI+): m/z = 437 [M + H]⁺. ¹H NMR (MeOD-*d₄*) δ (ppm): 7.83-7.77 (m, 2H), 7.54 (s, 1H), 7.33 (d, *J* = 8.0 Hz, 1H), 7.17-7.13 (m, 2H), 7.11 (d, *J* = 3.1 Hz, 1H), 7.00 (s, 1H), 6.65 (dd, *J* = 8.0 and 1.0 Hz, 1H), 6.30 (d, *J* = 3.1 Hz, 1H), 5.25-5.14 (m, 1H), 4.53 (d, *J* = 15.6 Hz, 1H), 4.47 (d, *J* = 15.6 Hz, 1H), 3.33-3.22 (m, 2H), 2.91 (dd, *J* = 14.9 and 8.9 Hz, 1H), 2.80 (dd, *J* = 14.9 and 5.4 Hz, 1H). ¹³C NMR (MeOD-*d₄*) δ (ppm): 168.9, 168.7, 166.2 (d, *J* = 250.3 Hz), 145.4, 137.8, 131.7 (d, *J* = 3.2 Hz), 131.0 (d, *J* = 9.6 Hz, 2C), 130.6, 128.4, 125.7, 124.9, 121.2 (2C), 116.4 (d, *J* = 22.2 Hz, 2C), 112.5, 102.1, 62.2, 42.8, 38.8, 36.0. HRMS: m/z calculated for C₂₂H₂₂N₆O₃F [M + H]⁺: 437.1735; found: 437.1737.

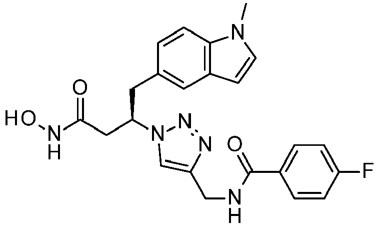

**(R)-4-fluoro-N-((1-(4-(hydroxyamino)-1-(1-methyl-1H-indol-5-yl)-4-oxobutan-2-yl)-1H-1,2,3-triazol-4-yl)methyl)benzamide (177).** Compound **177** was obtained according to general procedure C' from azide **159** and N-(prop-2-ynyl)-4-fluorobenzamide followed by general procedure G' as a light yellow wax (6 mg, 14% over 2 steps). Purity: 97%, LC t_{R} = 2.23 min, MS (ESI+): m/z = 451 [M + H]⁺. ¹H NMR (MeOD-*d₄*) δ (ppm): 7.83-7.79 (m, 2H), 7.53 (s, 1H), 7.20-7.12 (m, 4H), 7.03 (d, *J* = 3.0 Hz, 1H), 6.78 (dd, *J* = 8.3 and 1.5 Hz, 1H), 6.23 (dd, *J* = 3.0 and 0.4 Hz, 1H), 5.23-5.14 (m, 1H), 4.50 (s, 2H), 3.66 (s, 3H), 3.34-3.20 (m, 2H), 2.90 (dd, *J* = 14.9 and 9.0 Hz, 1H), 2.80(dd, *J* = 14.9 and 5.5 Hz, 1H). ¹³C NMR (MeOD-*d₄*) δ (ppm): 168.8 (2C), 166.2 (d, *J* = 250.8 Hz), 145.5, 137.3, 131.7 (d, *J* = 3.6 Hz), 131.0 (d, *J* = 9.0 Hz, 2C), 130.4, 130.2, 128.1, 124.8, 123.3, 121.9, 116.3 (d, *J* = 22.2 Hz, 2C), 110.2, 101.4, 62.3, 42.7, 38.7, 36.0, 32.8. HRMS: m/z calculated for C₂₃H₂₄N₆O₃F [M + H]⁺: 451.1894; found: 451.1893.

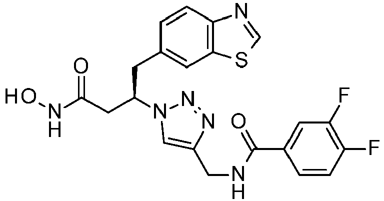

**(R)-N-((1-(1-(benzo[d]thiazol-6-yl)-4-(hydroxyamino)-4-oxobutan-2-yl)-1H-1,2,3-triazol-4-yl)methyl)-3,4-difluorobenzamide (178).** Compound **178** was obtained according to general procedure C' from azide **160** and N-(prop-2-ynyl)-3,4-difluorobenzamide followed by general procedure G' as a colourless solid (15 mg, 55% over 2 steps). Purity: 98%, LC t_{R} = 1.95 min, MS (ESI+): m/z = 473 [M + H]⁺. ¹H NMR (MeOD-*d₄*) δ (ppm): 9.11 (s, 1H), 7.85 (d, *J* = 8.4 Hz, 1H), 7.68 (ddd, *J* = 11.2 and 7.7 and 2.1 Hz, 1H), 7.68 (br s, 1H), 7.63 (s, 1H), 7.63-7.57 (m, 1H), 7.35 (ddd, *J* = 10.3 and 8.4 and 8.1 Hz, 1H), 7.20 (dd, *J* = 8.4 and 1.5 Hz, 1H), 5.34-5.23 (m, 1H), 4.48 (s, 2H), 3.44 (dd, *J* = 13.7 and 5.5 Hz, 1H), 3.38 (dd, *J* = 13.7 and 9.2 Hz, 1H), 2.95 (dd, *J* = 14.9 and 8.8 Hz, 1H), 2.86 (dd, *J* = 14.9 and 5.6 Hz, 1H). ¹³C NMR (MeOD-*d₄*) δ (ppm): 168.4, 167.4, 156.9, 153.8 (dd, *J* = 252.3 and 12.6 Hz), 151.3 (dd, *J* = 248.0 and 13.2 Hz), 145.6, 136.1, 135.2, 132.6 (dd, *J* = 4.8 and 3.6 Hz), 128.7, 125.5 (dd, *J* = 7.2 and 3.6 Hz), 124.9, 123.8, 123.5, 118.5 (d, *J* = 18.0 Hz), 117.9 (d, *J* = 18.6 Hz), 61.5, 42.1, 38.8, 36.0. HRMS: m/z calculated for C₂₁H₁₉N₆O₃SF₂ [M + H]⁺: 473.1207; found: 473.1209.

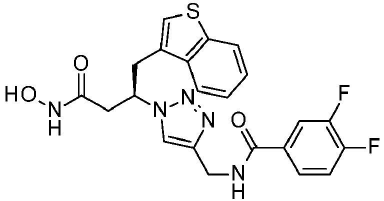

**(R)-N-((1-(1-(benzo[b]thiophen-3-yl)-4-(hydroxyamino)-4-oxobutan-2-yl)-1H-1,2,3-triazol-4-yl)methyl)-3,4-difluorobenzamide** (**179**). Compound **179** was obtained according to general procedure C' from azide **161** and N-(prop-2-ynyl)-3,4-difluorobenzamide followed by general procedure G' as an off-white solid (25 mg, 45% over 2 steps). Purity: 98%, LC t_{R} = 2.30 min, MS (ESI+): m/z = 472 [M + H]⁺. ¹H NMR (MeOD-*d₄*) δ (ppm): 7.82-7.72 (m, 2H), 7.70 (dd, *J* = 7.6 and 2.2 Hz, 1H), 7.66-7.61 (m, 1H), 7.55 (s, 1H), 7.40-7.24 (m, 3H), 7.00 (s, 1H), 5.37-5.27 (m, 1H), 4.50 (d, *J* = 15.5 Hz, 1H), 4.45 (d, *J* = 15.5 Hz, 1H), 3.54 (dd, *J* = 14.5 and 5.7 Hz, 1H), 3.47 (dd, *J* = 14.5 and 9.0 Hz, 1H), 2.98 (dd, *J* = 14.9 and, 8.2 Hz, 1H), 2.91 (dd, *J* = 14.9 and 6.4 Hz, 1H). ¹³C NMR (MeOD-*d₄*) δ (ppm): 168.5, 167.4, 153.8 (dd, *J* = 252.2 and 12.6 Hz), 151.3 (dd, *J* = 248.2 and 13.2 Hz), 145.6, 141.6, 139.6, 132.8-132.5 (m), 132.2, 125.6 (dd, *J* = 7.6 and 3.7 Hz), 125.5, 125.5, 125.2, 124.8, 123.7, 122.4, 118.5 (d, *J* = 18.3 Hz), 118.0 (d, *J* = 19.2 Hz), 60.0, 38.8, 36.0, 35.0. HRMS: m/z calculated for C₂₂H₂₀N₅O₃SF₂, [M+H]⁺: 472.1255; found: 472.1254.

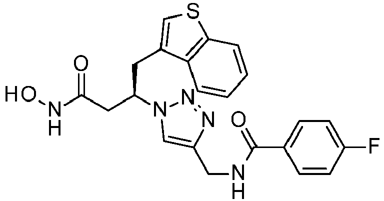

**(R)-N-((1-(1-(benzo[b]thiophen-3-yl)-4-(hydroxyamino)-4-oxobutan-2-yl)-1H-1,2,3-triazol-4-yl)methyl)-4-fluorobenzamide (180).** Compound **180** was obtained according to general procedure C' from azide **161** and N-(prop-2-ynyl)-4-fluorobenzamide followed by general procedure G' as an off-white solid (21 mg, 41% over 2 steps). Purity: 99.5%, LC t_{R} = 2.22 min, MS (ESI+): m/z = 454 [M + H]⁺. ¹H NMR (MeOD-*d₄*) δ (ppm): 7.88-7.80 (m, 2H), 7.76-7.72 (m, 2H), 7.55 (s, 1H), 7.34 (ddd, *J* = 7.9 and 7.1 and 1.2 Hz, 1H), 7.27 (ddd, *J* = 7.9 and 7.1 and 1.2 Hz, 1H), 7.22-7.14 (m, 2H), 7.01 (s, 1H), 5.37-5.27 (m, 1H), 4.51 (d, *J* = 15.5 Hz, 1H), 4.46 (d, *J* = 15.5 Hz, 1H), 3.54 (dd, *J* = 14.6 and 5.7 Hz, 1H), 3.47 (dd, *J* = 14.6 and 9.3 Hz, 1H), 2.97 (dd, *J* = 14.9 and 8.1 Hz, 1H), 2.91 (dd, *J* = 14.9 and 6.4 Hz, 1H). ¹³C NMR (MeOD-*d₄*) δ (ppm): 168.8, 168.6, 166.3 (d, *J* = 250.3 Hz), 145.8, 141.6, 139.7, 132.2, 131.6 (d, *J* = 3.4 Hz), 131.0 (d, *J* = 9.0 Hz, 2C), 125.5, 125.5, 124.7, 123.8, 122.4, 116.3 (d, *J* = 22.1 Hz, 2C), 60.0, 38.8, 36.0, 35.0. HRMS: m/z calculated for C₂₂H₂₁N₅O₃FS [M + H]⁺: 454.1349; found: 454.1346.

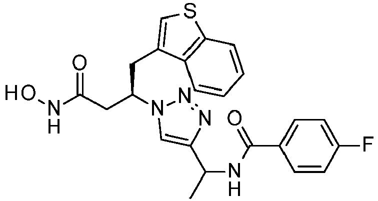

**N-(1-(1-((R)-1-(benzo[b]thiophen-3-yl)-4-(hydroxyamino)-4-oxobutan-2-yl)-1H-1,2,3-triazol-4-yl)ethyl)-4-fluorobenzamide** (**181**). Compound **181** was obtained according to general procedure C' from azide **161** and N-(but-3-yn-2yl)-4-fluorobenzamide, followed by general procedure G' as a white solid (23 mg, 48% over 2 steps). Purity: 95%, LC t_{R} = 2.28 min, MS (ESI+): m/z = 468 [M + H]⁺. Mixture of diastereoisomers, estimated (R,R)/(R,S) ratio: 2.3/1 ¹H NMR (MeOD-*d₄*) δ (ppm): 7.87-7.75 (m, 3.2H), 7.71 (br d, *J* = 7.5 Hz, 0.8H), 7.52 (s, 0.3H), 7.49 (s, 0.7H), 7.38-7.26 (m, 2H), 7.21-7.12 (m, 2H), 7.03 (s, 0.7H), 7.02 (s, 0.3H), 5.37-5.22 (m, 1H), 5.26 (q, *J* = 7.1 Hz, 1H), 3.60-3.43 (m, 2H), 3.04-2.89 (m, 2H), 1.49 (d, *J* = 7.1 Hz, 0.9H), 1.46 (d, *J* = 7.1 Hz, 2.1H). ¹³C NMR (MeOD-*d₄*) δ (ppm): Major isomer, (R,R): 168.6, 168.3, 166.2 (d, *J* = 250.0 Hz), 150.3, 141.6, 139.7, 132.3, 131.9 (d, *J* = 3.2 Hz), 131.1 (d, *J* = 9.0 Hz, 2C), 125.5, 125.5, 125.3, 123.8, 123.7, 122.4, 116.3 (d, *J* = 22.1 Hz, 2C), 60.1, 43.3, 38.7, 35.1, 20.4. Minor isomer, (R,S): 168.6, 168.3, 166.2 (d, *J* = 250.0 Hz), 150.5, 141.6, 139.7, 132.2, 131.9 (d, *J* = 3.2 Hz), 131.1 (d, *J* = 9.0 Hz, 2C), 125.6, 125.5, 125.3, 123.8, 123.5, 122.4, 116.3 (d, *J* = 22.1 Hz, 2C), 60.0, 43.3, 38.7, 35.1, 20.6. HRMS: m/z calculated for C₂₃H₂₃NO₃FS [M + H]⁺: 468.1506; found: 468.1499.

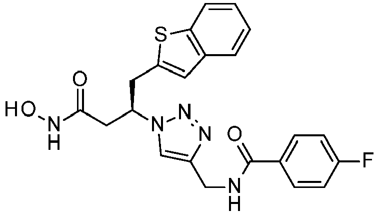

**(S)-N-((1-(1-(benzo[b]thiophen-2-yl)-4-(hydroxyamino)-4-oxobutan-2-yl)-1H-1,2,3-triazol-4-yl)methyl)-4-fluorobenzamide** (**182**). Compound **182** was obtained according to general procedure C' from azide **162** and N-(prop-2-ynyl)-4-fluorobenzamide followed by general procedure G' as a purple-brown solid (9 mg, 34% over 2 steps). Purity: 97%, LC t_{R} = 2.23 min, MS (ESI+): m/z = 454 [M + H]⁺. ¹H NMR (MeOD-*d₄*) δ (ppm): 7.83-7.77 (m, 2H), 7.56 (s, 1H), 7.67-7.61 (m, 1H), 7.57-7.51 (m, 1H), 7.24-7.11 (m, 4H), 6.89 (s, 1H), 5.33-5.23 (m, 1H), 4.54 (s, 2H), 3.61 (dd, *J* = 14.7 and 8.6 Hz, 1H), 3.55 (dd, *J* = 14.6 and 5.6 Hz, 1H), 2.94 (dd, *J* = 15.1 and 8.9 Hz, 1H), 2.87 (dd, *J* = 15.1 and 5.8 Hz, 1H). ¹³C NMR (MeOD-*d₄*) δ (ppm): 167.4, 166.9, 164.8 (d, *J* = 250.0 Hz), 144.5, 139.8, 139.6, 139.1, 130.2 (d, *J* = 3.6 Hz), 129.6 (d, *J* = 9.0 Hz, 2C), 123.9, 123.7, 123.6, 123.1, 122.7, 121.5, 114.9 (d, *J* = 22.2 Hz, 2C), 59.7, 37.4, 35.5, 34.6. HRMS: m/z calculated for C₂₂H₂₁N₅O₃FS [M + H]⁺: 454.1349; found: 454.1345.

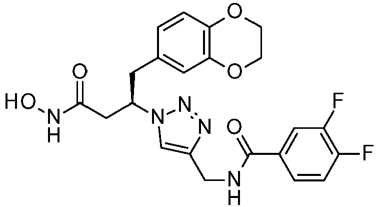

**(R)-N-((1-(1-(2,3-dihydrobenzo[b][1,4]dioxin-6-yl)-4-(hydroxyamino)-4-oxobutan-2-yl)-1H-1,2,3-triazol-4-yl)methyl)-3,4-difluorobenzamide (183).** Compound **183** was obtained according to general procedure C' from azide **163** and N-(prop-2-ynyl)-3,4-difluorobenzamide as a beige solid (90mg, 60%). Purity: 99%, LC t_{R} = 2.08 min, MS (ESI+): m/z = 474 [M + H]⁺. ¹H NMR (MeOD-*d₄*) δ (ppm): 7.76 (ddd, *J* = 11.2 and 7.7 and 2.1 Hz, 1H), 7.70-7.64 (m, 1H), 7.62 (s, 1H), 7.36 (ddd, *J* = 10.2 and 8.4 and 8.2 Hz, 1H), 6.56 (d, *J* = 8.2 Hz, 1H), 6.48 (d, *J* = 1.8 Hz, 1H), 6.38 (dd, *J* = 8.2 and 1.8 Hz, 1H), 5.15-5.05 (m, 1H), 4.59-4.46 (m, 2H), 4.10 (s, 4H), 3.12 (dd, *J* = 13.9 and 5.7 Hz, 1H), 3.06 (dd, *J* = 13.9 and 9.1 Hz, 1H), 2.87 (dd, *J* = 14.9 and 9.1 Hz, 1H), 2.77 (dd, *J* = 14.9 and 5.5 Hz, 1H). ¹³C NMR (MeOD-*d₄*) δ (ppm): 168.6, 167.5, 153.8 (dd, *J* = 252.9 and 13.2 Hz), 151.3 (dd, *J* = 248.1 and 12.6 Hz), 145.4, 144.9, 144.0, 132.8-132.7 (m), 130.8, 125.6 (dd, *J* = 7.2 and 3.6 Hz), 124.9, 122.8, 118.6, 118.5 (d, *J* = 19.0 Hz), 118.1, 118.0 (d, *J* = 20.0 Hz), 65.5 (2C), 61.6, 41.7, 38.7, 36.1. HRMS: m/z calculated for C₂₂H₂₂N₅O₅F₂, [M + H]⁺: 474.1589; found: 474.1592.

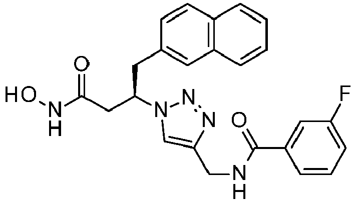

**(R)-3-fluoro-N-((1-(4-(hydroxyamino)-1-(naphthalen-2-yl)-4-oxobutan-2-yl)-1H-1,2,3-triazol-4-yl)methyl)benzamide (184).** Compound **184** was obtained according to general procedure C' from azide **109** and 3-fluoro-N-prop-2-ynyl-benzamide followed by general procedure G' as a white solid (34 mg, 27% over 2 steps). Purity: 99%, LC tr = 2.37 min, MS (ESI+): m/z = 448 [M + H]⁺. ¹H NMR (DMSO-*d₆*) δ (ppm): 10.5 (br s, 1H), 9.07 (t, *J* = 5.8 Hz, 1H), 8.8 (s, 1H), 7.9 (s, 1H), 7.79-7.61 (m, 5H), 7.56-7.49 (m, 1H), 7.36 (br s, 1H), 7.44-7.35 (m, 3H), 7.13 (dd, *J* = 8.5 and 1.5 Hz, 1H), 5.27 (quint, *J* = 7.0 Hz, 1H), 4.43 (d, *J* = 5.8 Hz, 2H), 3.31-3.28 (m, 2H), 2.78 (dd, *J* = 15.0 and 9.0 Hz, 1H), 2.66 (dd, *J* = 15.0 and 5.5 Hz, 1H). ¹³C NMR (DMSO-*d₆*) δ (ppm): 166.0, 165.1, 162.5 (d, *J* = 250.0 Hz), 144.7, 137.0 (d, *J* = 6.0 Hz), 135.0, 133.3, 132.3, 130.9 (d, *J* = 8.0 Hz), 128.2, 127.8, 127.7, 126.4, 126.0, 118.6 (d, *J* = 22.0 Hz), 114.6 (d, *J* = 22.0 Hz), 59.2, 41.3, 37.6, 35.3. HRMS m/z calculated for C₂₄H₂₃N₅O₃F [M + H]⁺ 448.1785, found 448.1785.

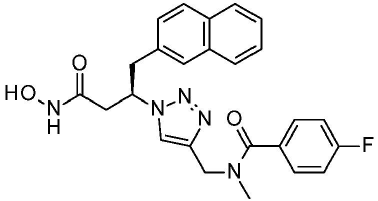

**(R)-4-fluoro-N-((1-(4-(hydroxyamino)-1-(naphthalen-2-yl)-4-oxobutan-2-yl)-1H-1,2,3-triazol-4-yl)methyl)-N-methylbenzamide (185).** Compound **185** was obtained according to general procedure C' from azide **109** and 4-fluoro-N-methyl-N-prop-2-ynyl-benzamide followed by general procedure G' as a white solid (58 mg, 45% over 2 steps). Purity: 99%, LC tr = 2.38 min, MS (ESI+): m/z = 462 [M + H]⁺. ¹H NMR (MeOD-*d₄*) δ(ppm): 7.73-7.61 (m, 3H), 7.56 (br s, 1H), 7.41-7.30 (m, 4H), 5.21 (dd, *J* = 8.4 and 1.5 Hz, 2H), 7.13-6.96 (m, 2H), 5.32 (br s, 1H), 4.68 (d, *J* = 15.4 Hz, 0.6H), 4.47 (d, *J* = 15.4 Hz, 0.7H), 4.32 (br s, 0.7H), 3.47 (dd, *J* = 14.0 and 5.0 Hz, 1H), 3.38-3.35 (m, 1H), 3.05 (dd, *J* = 15.0 and 8.0 Hz, 1H), 2.94 (dd, *J* = 15.0 and 5.0 Hz, 1H), 2.73 (br s, 1H) 2.55 (br s, 2H). ¹³C NMR (MeOD-*d₄*) δ(ppm): 171.1, 167.1, 163.7 (d, *J* = 248.9 Hz), 142.2, 142.0, 134.1, 133.4, 132.4, 131.6, 129.2, 127.9, 127.4, 127.2, 127.1, 126.6, 125.8, 125.6, 124.4, 115.1 (d, *J* = 22.2 Hz, 2C), 60.2, 41.8, 41.2, 37.5, 36.0. HRMS m/z calculated for C₂₅H₂₅N₅O₃F [M + H]⁺ 462.1941, found 462.1944.

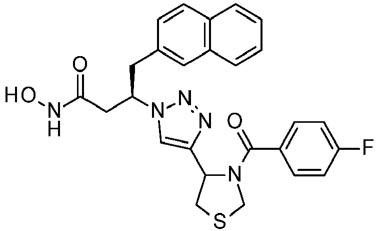

**(3R)-3-(4-(3-(4-fluorobenzoyl)thiazolidin-4-yl)-1H-1,2,3-triazol-1-yl)-N-hydroxy-4-(naphthalen-2-yl)butanamide (186).** Compound **186** was obtained according to general procedure C' from azide **109** and (4-ethynylthiazolidin-3-yl)-(4-fluorophenyl)methanone followed by general procedure G' as a white solid (82 mg, 59% over 2 steps). Purity: 95%, LC tr = 2.57 min, MS (ESI+): m/z = 506 [M + H]⁺. ¹H NMR (DMSO-*d₆*) δ (ppm): 10.56 (s, 1H), 8.85 (s, 1H), 8.05 (m, 1H), 7.76 (m, 3H), 7.45 (m, 6H), 7.15 (m, 3H), 5.26 (m, 1H), 4.28 (m, 1H), 3.53 (m, 5H), 3.09 (m, 1H), 2.82 (m, 2H). ¹³C NMR (DMSO-*d₆*) δ (ppm): 172.6(min), 171.1(maj), 168.1, 166.0, 163.5 (d, *J* = 250.0 Hz), 143.3, 146.2 (d, *J* = 16.0 Hz), 135.0, 134.9, 133.3, 132.7, 132.3, 130.2, 128.2, 127.9, 127.8, 127.7, 126.5, 126.1, 115.9, 115.6, 59.6, 55.4, 41.6, 37.6, 35.8. HRMS m/z calculated for C₂₆H₂₅N₅O₃FS [M + H]⁺ 506.1662, found 506.1654.

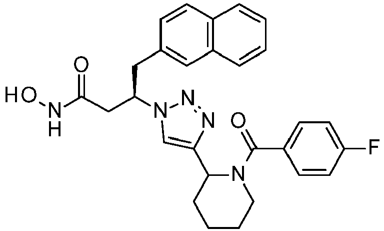

**(3R)-3-(4-(1-(4-fluorobenzoyl)piperidin-2-yl)-1H-1,2,3-triazol-1-yl)-N-hydroxy-4-(naphthalen-2-yl)butanamide (187).** Compound **187** was obtained according to general procedure C' from azide **109** and (2-ethynyl-1-piperidyl)-(4-fluorophenyl)methanone followed by general procedure G' as a white solid (105 mg, 73% over 2 steps). Purity: 95%, LC tr = 2.58 min, MS (ESI+): m/z = 502 [M + H]⁺. ¹H NMR (DMSO-*d₆*) δ (ppm): 10.58 (s, 1H), 8.85 (s, 1H), 7.85-7.65 (m, 4H), 7.50-7.30 (m, 5H), 7.28-7.09 (m, 3H), 5.35-5.21 (m, 1H), 4.80-3.80 (m, 1H), 3.45-3.35 (m, 2H), 3.30-3.14 (m, 1H), 2.99-2.78 (m, 2H) 2.32-1.05 (m 7H). ¹³C NMR (DMSO-*d₆*) δ (ppm): 169.1, 169.0, 166.0, 162.9 (d, *J* = 249.5 Hz) 144.9, 135.0, 133.3, 132.3, 129.4, 128.2, 128.1, 127.8, 127.7, 127.6, 126.4, 126.0, 123.7, 115.8 (d, *J* = 21.5 Hz), 59.5, 45.6, 41.7, 37.7, 28.1, 25.5, 19.6, 19.3. HRMS m/z calculated for C₂₈H₂₈N₅O₃F [M + H]⁺ 502.2254, found 502.2246.

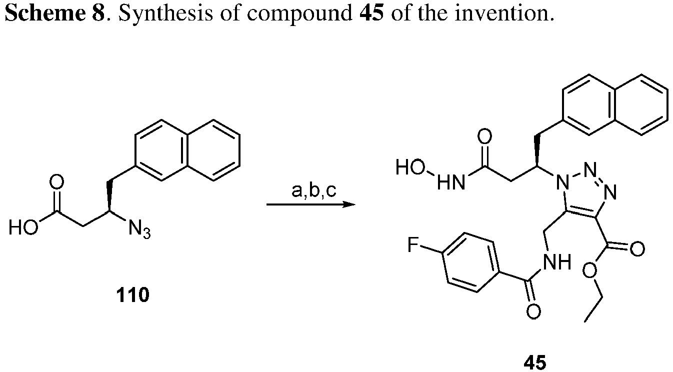

Reagent and conditions: (a) ethyl 4-(N-(tert-butoxycarbonyl)-4-fluorobenzamido)but-2-ynoate, dioxane, 130°C MW, 6 h; (b) TFA, CH₂Cl₂, rt, 2 h; (c) (i) EDCI, HOBt, NMM, DMF, rt, 15 min, (ii) NH₂OTBDMS, rt, 16 h.

**Ethyl 5-[[(4-fluorobenzoyl)amino]methyl]-3-[(1R)-3-(hydroxyamino)-1-(2-naphthylmethyl)-3-oxo-propyl]triazole-4-carboxylate (45).** Azide **110** (226 mg, 0.89 mmol, 1 eq.) and ethyl 4-(N-(tert-butoxycarbonyl)-4-fluorobenzamido)but-2-ynoate (373 mg, 1.07 mmol, 1.2 eq.) were added in dioxane (2.5 mL) in a microwave vessel and the resulting mixture was heated 6 h at 130°C (200 W). After cooling, the solvent was evaporated and the residue was treated 2 h at room temperature with a solution of TFA in dichloromethane (10%, 20mL). TFA and dichloromethane were evaporated and the crude was purified by flash chromatography on silica gel (CH₂Cl₂/MeOH 100:0 to 95:5 (v/v)) to afford a mixture of two regioisomers (215 mg). Then, to a solution of this mixture in DMF (5 mL) were added HOBt (300 mg, 1.95 mmol, 2.3 eq.), EDCI (238 mg, 1.24 mmol, 1.5 eq.) and NMM (0.514 mL, 4.7 mmol, 5.6 eq.). After 15 min of stirring, NH₂-OTBDMS (173 mg, 1.17 mmol, 1.4 eq.) was finally introduced and the reaction mixture was stirred overnight. The solvent was evaporated and the crude was purified by flash chromatography on silica gel (CH₂Cl₂/MeOH 100:0 to 95:5 (v/v)) to afford compound **45** as a white solid (33 mg, 7%). Purity: 95%, LC t_{R} = 2.95 min, MS (ESI+) m/z = 520 [M+H]⁺. ¹H NMR (DMSO) δ (ppm): 1.27 (t, J = 7.0 Hz, 3H), 2.73 (dd, J = 4.5 and 15.6 Hz, 1H), 2.93 (dd, J = 10.2 and 15.0 Hz, 1H), 4.28 (q, J = 7.0 Hz, 2H), 4.35-4.40 (m, 1H), 4.95 (dd, J = 6.4 and 14.7, 1H), 5.40-5.42 (m, 1H), 7.20-7.27 (m, 3H), 7.42-7.45 (m, 2H), 7.53 (br s, 1H), 7.64-7.69 (m, 2H), 7.76-7.79 (m, 1H), 7.86-7.91 (m, 2H), 8.77 (br s, 1H), 8.81(br s, 1H), 10.62 (br s, 1H). ¹³C NMR (DMSO) δ (ppm): 14.5, 29.5, 31.4, 41.2, 57.1, 61.0, 115.6 (d, J_{C-F} = 21 Hz), 126.2, 126.6, 127.6, 127.8, 127.9, 128.2, 128.4, 130.5 (d, J_{C-F} = 9 Hz), 132.3, 133.3, 134.5, 136.6, 139.0, 161.2, 164.4 (d, J_{C-F} = 247 Hz), 165.4, 165.9. HRMS m/z calculated for C₂₇H₂₇FN₅O₅ [M+H]⁺ 520.1996, found 520.1977.

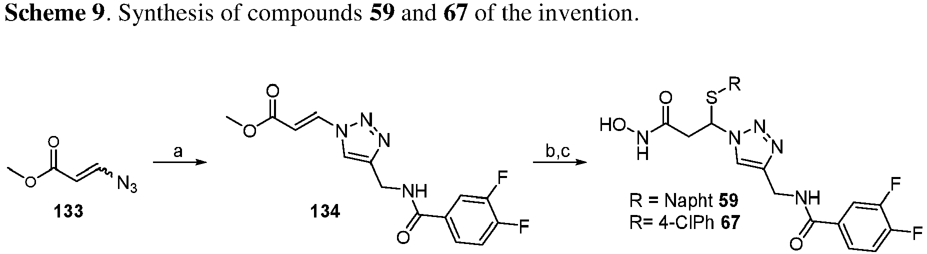

Reagent and conditions: (a) alkyne, CuSO₄.5H₂O, sodium ascorbate, CH₃CN/H₂O/dioxane, rt, 16 h; (b) thiol, DIPEA, MeOH, 0°C, 30 min; (c) NH₂OH, KCN, H₂O, MeOH, rt, 16 h.

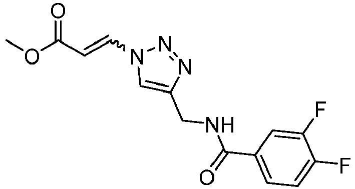

**Methyl 3-(4-((3,4-difluorobenzamido)methyl)-1H-1,2,3-triazol-1-yl)acrylate (134).** Compound **134** was obtained according to general procedure C from azide **133** as a yellow solid. LC t_{R}: 2.39 min, MS (ESI+) m/z = 323 [M+H]⁺.

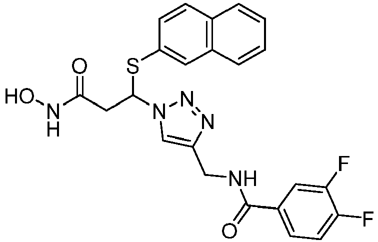

**3,4-difluoro-N-((1-(3-(hydroxyamino)-1-(naphthalen-2-ylthio)-3-oxopropyl)-1H-1,2,3-triazol-4-yl)methyl)benzamide (59).** Compound **159** was obtained according to general procedure R followed by general procedure F as a white solid (15 mg, 25%). Purity: 99%, LC t_{R}: 2.87 min, MS (ESI+) m/z = 484 [M+H]⁺. HRMS m/z calculated for C₂₃H₁₉F₂N₅O₃S [M+H]⁺ 484.1255, found 484.1255.

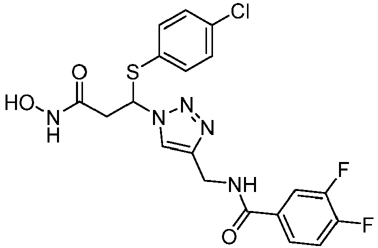

**N-[[1-[1-(4-chlorophenyl)sulfanyl-3-(hydroxyamino)-3-oxo-propyl]triazol-4-yl]methyl]-3,4-difluoro-benzamide (67).** Compound **67** was obtained according to general procedure R followed by general procedure F as a white foam (19 mg, 44%), Purity: 100%, LC t_{R} = 2.48 min, MS (ESI+): m/z = 468 [M + H]⁺. ¹H NMR (MeOD) δ (ppm): 3.05 (dd, J = 6.7 Hz, J = 15.1 Hz, 1H), 3.15 (dd, J = 8.2 Hz, J = 15.1 Hz, 1H), 4.55 (s, 2H), 6.25 (dd, J = 6.7 Hz, J = 6.8 Hz, 1H), 7.23 (s, 4H), 7.37 (td, J = 8.2 Hz, J = 10.3 Hz, 1H), 7.70 (dddd, J = 2.0 Hz, J = 4.2 Hz,J = 5.6 Hz, J = 8.0 Hz, 1H), 7.78 (ddd, J = 2.1 Hz, J = 7.6 Hz, J = 11.3 Hz, 1H), 7.85 (s, 1H). ¹³C NMR (MeOD) δ (ppm): 36.2, 37.7, 65.3, 117.9 (d, J = 18.8 Hz), 118.6 (d, J = 18.2 Hz), 124.0, 125.6 (dd, J = 3.7 Hz, J = 7.3 Hz), 130.2, 130.5 (2C), 132.6, 136.9, 137.2 (2C), 146.4, 151.4 (dd, J = 13.0 Hz, J = 247.8 Hz), 153.8 (dd, J = 13.0 Hz, J = 252.5 Hz), 166.8, 167.5. ¹⁹F NMR (MeOD) δ (ppm): - 136.1 (d, J = 20.5), -139.8 (d, J = 20.5). HRMS m/z calculated for C₁₉H₁₇ClF₂SN₅O₃ [M + H]⁺ 468.10709, found 468.0758.

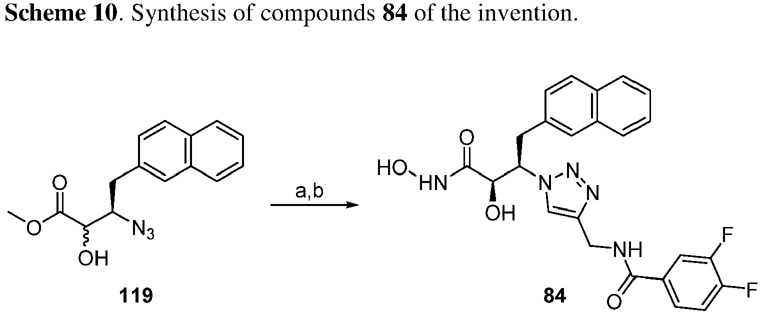

Reagent and conditions: (a) alkyne, CuSO₄.5H₂O, sodium ascorbate, H₂O/dioxane, rt, 16 h; (b) NH₂OH, KCN, H₂O, MeOH, rt, 16 h.

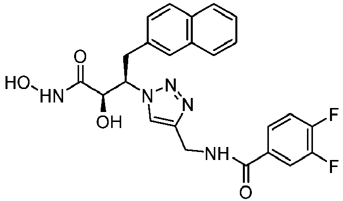

**3,4-difluoro-N-[[1-[(1R,2S)-2-hydroxy-3-(hydroxyamino)-1-(2-naphthylmethyl)-3-oxo-propyl]triazol-4-yl]methyl]benzamide (84).** Compound **84** was obtained according to general procedure C from azide **119** and 3,4-difluoro-N-(prop-2-yn-1-yl)benzamide followed by general procedure G as a white foam (8 mg, 10% over 2 steps). Purity: 100%, LC t_{R} = 2.40 min, MS (ESI+): m/z = 482 [M + H]. ¹H NMR (MeOD) δ (ppm): 3.47 (d, J = 7.8 Hz, 1H), 4.50 (br s, 1H), 4.52 (q, J = 15.0 Hz, 2H), 5.36 (ddd, J = 3.6 Hz, J = 7.7 Hz, J = 8.1 Hz, 1H), 7.26 (m, 4H), 7.51 (br s, 1H), 7.60-7.76 (m, 5H), 7.98 (s, 1H). ¹³C NMR (DMSO-d₆) δ (ppm): 34.8, 37.2, 64.2, 71.3, 116.7 (d, J = 18.4 Hz), 117.5 (d, J = 17.2 Hz), 123.1, 124.8 (dd, J = 3.3 Hz, J = 7.4 Hz), 125.5, 125.9, 127.1, 127.2, 127.3, 127.4, 127.7, 131.6, 131.8, 132.8, 134.6, 143.9, 149.1 (dd, J = 12.9 Hz, J = 246.5 Hz), 153.3 (dd, J = 10.3 Hz, J = 250.8 Hz), 163.8, 166.9. ¹⁹F NMR (MeOD) δ (ppm): -139.9 (d, J = 20.3 Hz), - 136.3 (d, J = 20.3 Hz). HRMS m/z calculated for C₂₄H₂₂F₂N₅O₄ [M + H]⁺ 482.1640, found 482.1622.

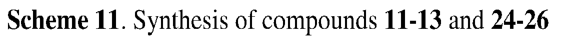

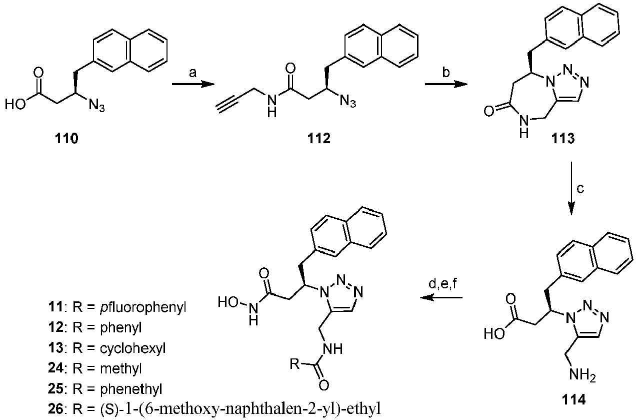

Reagent and conditions: (a) propargylamine, DMTMM, EtOAc, rt, 3 h, 60%; (b) DMF, reflux, 24 h, 84%; (c) HCl 6M, H₂O, MW, 85°C, 1 h, quantitative yield; (d) RCOCl, dioxane, sat. K2CO3, 0°C to rt, 3-5 h; (e) O-tritylhydroxylamine, EDCI, HOBt, N-methylmorpholine, DMF, rt, overnight; (f) TFA, TIS2Cl2, rt, 2 h, 3 steps 3-24%.

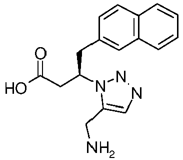

**(R)-3-(5-Aminomethyl-[1,2,3]triazol-1-yl)-4-naphthalen-2-yl-butyric acid (114).** (R)-3-Azido-4-naphthalen-2-yl-butyric acid **(110)** (1.85 g, 7.25 mmol) and propargylamine (0.56 mL, 8.70 mmol) were dissolved in EtOAc (55 mL), and the resulting solution was stirred at room temperature for 10 min. DMTMM (2.41 g, 8.70 mmol) was added and the reaction was stirred at room temperature for 3 h. The mixture was washed twice with 1M HCl (aq), twice with saturated NaHCO₃ (aq) and twice with saturated NaCl (aq), dried over MgSO₄, filtered and concentrated under reduced pressure. The residue was purified by flash chromatography on silica gel using CH₂Cl₂ as eluent. (R)-3-Azido-4-naphthalen-2-yl-N-prop-2-ynyl-butyramide **112** was obtained as a yellowish oil (1.270 g, 60%). Purity: 90%, LC t_{R}= 2.85 min (method A), MS (ESI+): m/z = 293 [M + H]⁺. ¹H NMR (CDCl₃) δ (ppm): 7.85-7.80 (m, 3H), 7.69 (s, 1H), 7.52-7.45 (m, 2H), 7.37 (dd, J = 1.6 and 8.4 Hz, 1H), 5.80 (sl, 1H), 4.29-4.23 (m, 1H), 4.15-3.99 (m, 2H), 3.06 (d, J = 6.8 Hz, 1H), 2.45 (dd, J = 4.5 and 14.9 Hz, 1H), 2.31 (dd, J = 8.6 and 14.9 Hz, 1H), 2.25 (t, J = 2.5 Hz, 2H). ¹³C NMR (CDCl₃) δ (ppm): 169.2, 134.2, 133.5, 132.5, 128.4, 128.2, 127.7, 127.6, 127.4, 126.3, 125.8, 79.1, 71.9, 60.3, 40.7, 29.3. The azido compound **112** (1.25 g, 4.28 mmol) was dissolved in DMF (110 mL) and was heated to reflux and left stirring overnight to allow cyclisation. After cooling down the mixture was diluted with EtOAc and was washed with H₂O (3 times). The organic phase was dried with MgSO₄ and evaporated under reduced pressure to give (R)-8-Naphthalen-2-ylmethyl-4,5,7,8-tetrahydro-1,2,5,8a-tetraaza-azulen-6-one **113** (1.05 g, 84%) as a beige solid. Purity: 95%, LC t_{R} = 2.24 min (method A), MS (ESI+): m/z = 293 [M + H]⁺. ¹H NMR (CDCl₃) δ (ppm): 7.84-7.78 (m, 3H), 7.66 (s, 1H), 7.52-7.45 (m, 3H), 7.27 (dd, J = 1.6 and 8.4 Hz, 2H), 6.65 (sl, 1H), 5.21-5.18 (m, 1H), 4.38 (dd, J = 5.3 and 17.0 Hz, 1H), 4.27 (dd, J = 6.0 and 17.0 Hz, 1H), 3.71 (dd, J = 3.3 and 13.6 Hz, 1H), 3.55 (dd, J = 8.8 and 13.6 Hz, 1H), 2.98-2.96 (m, 2H). ¹³C NMR (CDCl₃) δ (ppm): 172.3, 133.4, 132.8, 132.7, 132.6, 131.2, 128.9, 128.5, 127.7, 127.6, 127.6, 126.3, 126.0, 58.0, 42.2, 35.5. A 6 M solution of HCl (aq) (10.5 mL) was added to **113** (0.250 g, 0.86 mmol) and splitted in four microwaves tubes. The mixture was heated under microwave conditions at 85°C for 1 h (Discover - CEM, Method standard: power max 200 W, ramp time 20 min, hold time 60 min, T 85°C, internal pressure max 20 bar). The solution was evaporated under reduced pressure to give compound **114** (322 mg, quant.) as an orange solid. Purity: 97%, LC t_{R} = 1.90 min (method A), MS (ESI+): m/z = 311 [M + H]⁺. ¹H NMR (DMSO-d₆) δ (ppm): 8.49 (sl, 2H), 7.87-7.76 (m, 3H), 7.66 (s, 1H), 7.58 (s, 1H), 7.48-7.45 (m, 2H), 7.28 (dd, J = 1.6 and 8.4 Hz, 2H), 5.26-5.13 (m, 1H), 3.95 (dd, J = 6.3 and 15.8 Hz, 1H), 4.27 (dd, J = 5.2 and 15.8 Hz, 1H), 3.44 (dd, J = 6.3 and 14.0 Hz, 1H), 3.32 (dd, J = 8.6 and 14.0 Hz, 1H), 3.19 (dd, J = 9.3 and 17.3 Hz, 1H), 3.06 (dd, J = 4.5 and 17.3 Hz, 1H). ¹³C NMR (DMSO-d₆) δ (ppm): 172.3, 134.8, 133.3, 133.2, 132.4, 132.1, 128.4, 128.2, 128.0, 127.8, 126.7, 126.2, 56.6, 41.4, 31.7.

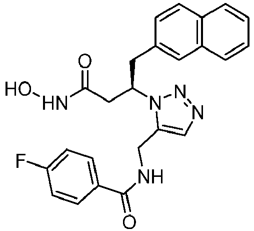

**(R)-4-Fluoro-N-[3-(1-hydroxycarbamoylmethyl-2-naphthalen-2-yl-ethyl)-3H-[1,2,3]triazol-4-ylmethyl]-benzamide (11).** Compound **11** was obtained according to general procedure I from azide **114** and 4-fluoro-benzoyl chloride followed by general procedure D as a white solid (0.012 g, 3 steps 3%). Purity: 97%, LC t_{R} = 2.42 min (method A), MS (ESI+): m/z = 448 [M + H]⁺. ¹H NMR (CD₃CN) δ (ppm): 9.21 (s, 1H), 7.86-7.68 (m, 6H), 7.47-7.40 (m, 4H), 7.13 (t, J = 8.9 Hz, 3H), 5.33-5.19 (m, 1H), 4.43 (dd, J = 6.8 and 15.0 Hz, 1H), 3.86 (dd, J = 3.7 and 15.0 Hz, 1H), 3.46-3.43 (m, 2H), 3.13 (dd, J = 10.5 and 15.5 Hz, 1H), 2.92 (dd, J = 4.1 and 15.5 Hz, 1H). ¹³C NMR (CD₃CN) δ (ppm): 167.0, 165.5, 164.6 (d, J = 247 Hz), 146.2, 135.0, 134.7, 133.3, 132.5, 130.2, 129.9, 129.8, 128.0, 127.7, 127.5, 127.4, 127.1, 126.2, 125.8, 115.2 (d, J_{C-F} = 21.8 Hz, 2C), 56.9, 41.4, 37.9, 31.3. HRMS m/z calculated for C₂₄H₂₃N₅O₃F [M + H]⁺ 448.1785, found 448.1788.

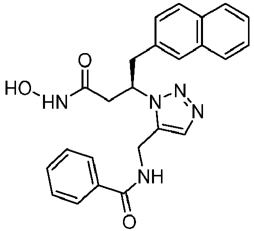

**(R)-N-[3-(1-Hydroxycarbamoylmethyl-2-naphthalen-2-yl-ethyl)-3H-[1,2,3]triazol-4-ylmethyl]-benzamide (12).** Compound **12** was obtained according to general procedure I from azide **114** and benzoyl chloride followed by general procedure D as a white solid (0.106 g, 3 steps 24%). Purity: 96%, LC t_{R} = 2.39 min (method A), MS (ESI⁺): m/z = 430 [M + H]⁺. ¹H NMR (DMSO-d₆) δ (ppm): 10.58 (s, 1H), 8.80 (m, 2H), 7.84-7.68 (m, 5H), 7.52-7.40 (m, 7H), 7.23 (dd, J = 1.6 and 8.4 Hz, 1H), 5.38-5.28 (m, 1H), 4.48 (dd, J = 6.4 and 15.5 Hz, 1H), 4.15 (dd, J = 4.3 and 15.5 Hz, 1H), 3.42-3.28 (m, 2H), 2.94 (dd, J = 9.9 and 15.0 Hz, 1H), 2.65 (dd, J = 4.5 and 15.0 Hz, 1H). ¹³C NMR (DMSO-d₆) δ (ppm): 166.5, 166.3, 135.6, 135.0, 134.1, 133.3, 132.9, 132.3, 131.9, 128.7, 128.3, 128.1, 127.9, 127.8, 126.6, 126.1, 56.4, 41.6, 37.9, 31.9. HRMS m/z calculated for C₂₄H₂₄N₅O₃ [M + H]⁺ 430.1879, found 430.1880.

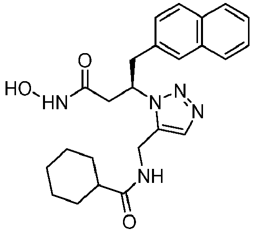

**(R)-Cyclohexanecarboxylic acid [3-(1-hydroxycarbamoylmethyl-2-naphthalen-2-yl-ethyl)-3H-[1,2,3]triazol-4-ylmethyl]-amide (13).** Compound **13** was obtained according to general procedure I from azide **114** and cyclohexanecarbonyl chloride followed by general procedure D as a white solid (0.041 g, 3 steps 8%). Purity: 98%, LC t_{R} = 2.47 min (method A), MS (ESI+): m/z = 436 [M + H]⁺. ¹H NMR (CD₃CN) δ (ppm): 9.29 (s, 0.7H), 7.85-7.75 (m, 3H), 7.49-7.46 (m, 3H), 7.31 (s, 1H), 7.17 (d, J = 8.3 Hz, 1H), 7.31 (s, 1H), 6.69 (sl, 1H), 5.23-5.08 (m, 1H), 3.97 (sl, 2H), 3.69-3.47 (m, 1H), 3.43 (d, J = 7.3 Hz, 2H), 3.15-2.88 (m, 3H), 1.64 (d, J = 12.2 Hz, 5H), 1.29-1.18 (m, 5H). ¹³C NMR (CD₃CN) δ (ppm): 176.3, 166.9, 146.0, 136.0, 134.7, 133.3, 132.3, 131.9, 128.1, 127.8, 127.6, 127.5, 127.2, 126.3, 125.9, 57.1, 44.4, 41.2, 38.0, 31.1, 29.1, 25.5 (2C), 25.3. HRMS m/z calculated for C₂₄H₃₀N₅O₃ [M + H]⁺ 436.2349, found 430.2353.

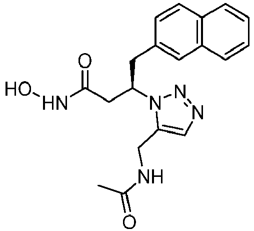

**(R)-3-[5-(Acetylamino-methyl)-[1,2,3]triazol-1-yl]-N-hydroxy-4-naphthalen-2-yl-butyramide (24).** Compound **24** was obtained according to general procedure I from azide **114** and acetyl chloride followed by general procedure D as a white solid (0.034 g, 3 steps 11%). Purity: 100%, LC t_{R} = 2.00 min (method A), MS (ESI⁺): m/z = 368 [M + H]⁺. ¹H NMR (CD₃CN) δ (ppm): 9.47 (s, 1H), 7.84-7.71 (m, 3H), 7.47-7.45 (m, 3H), 7.31 (s, 1H), 7.15 (d, J = 8.4 Hz, 1H), 9.47 (s, 1H), 5.22-5.11 (m, 1H), 4.06 (dd, J = 6.3 and 15.6 Hz, 1H), 3.85 (dd, J = 4.8 and 15.6 Hz, 1H), 3.41 (d, J = 7.8 Hz, 2H), 3.06 (dd, J = 10.2 and 15.0 Hz, 1H), 2.88 (dd, J = 4.2 and 15.0 Hz, 1H), 1.70 (s, 2.8H). ¹³C NMR (CD₃CN) δ (ppm): 170.2, 167.0, 135.6, 134.7, 133.3, 132.3, 132.1, 128.1, 127.8, 127.5, 127.5, 127.2, 126.2, 125.8, 57.0, 41.4, 38.0, 31.0, 21.8. HRMS m/z calculated for C₁₉H₂₂N₅O₃ [M + H]⁺ 368.1723, found 368.1725.

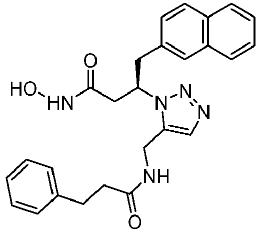

**(R)-N-hydroxy-4-naphthalen-2-yl-3-{5-[(3-phenyl-propionylamino)-methyl]-[1,2,3]triazol-1-yl}-butyramide (25).** Compound **25** was obtained according to general procedure I from azide **114** and 3-phenyl-propionyl chloride followed by general procedure D as a white solid (0.030 g, 3 steps 11%). Purity: 100%, LC t_{R} = 2.45 min (method A), MS (ESI+): m/z = 458 [M + H]⁺. ¹H NMR (CD₃CN) δ (ppm): 9.53 (s, 1H), 7.82-7.72 (m, 3H), 7.46-7.43 (m, 3H), 7.29-7.10 (m, 7H), 6.94 (m, 1H), 5.13-5.04 (m, 1H), 3.91 (dd, J = 5.4 and 15.6 Hz, 1H), 4.15 (dd, J = 4.2 and 15.6 Hz, 1H), 3.38 (d, J = 7.5 Hz, 2H), 3.09-2.96 (m, 2H), 2.80 (t, J = 7.5 Hz, 2H), 2.27 (t, J = 7.5 Hz, 2H). ¹³C NMR (CD₃CN) δ (ppm): 172.2, 167.0, 146.0, 141.2, 135.5, 134.6, 133.3, 132.3, 132.0, 128.3, 128.1, 127.8, 127.5 (2C), 127.5, 127.2, 126.3, 126.0, 125.9, 117.3, 57.1, 41.4, 37.9, 37.0, 31.1, 30.9. HRMS m/z calculated for C₂₆H₂₈N₅O₃ [M + H]⁺ 458.2192, found 458.2209.

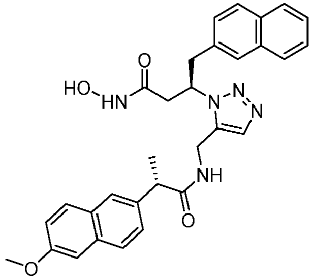

**(R,S)-N-hydroxy-3-(5-{[2-(6-methoxy-naphthalen-2-yl)-propionylamino]-methyl}-[1,2,3]triazol-1-yl)-4-naphthalen-2-yl-butyramide (26)** Compound **26** was obtained according to general procedure I from azide **114** and 2-(6-methoxy-naphthalen-2-yl)-propionyl chloride followed by general procedure D as a white solid (0.026 g, 3 steps 16%). Purity: 100%, LC t_{R} = 2.70 min (method A), MS (ESI⁺): m/z = 538 [M + H]⁺. ¹H NMR (CD₃CN) δ (ppm): 9.34 (s, 1H), 7.82-7.79 (m, 1H), 7.71-7.63 (m, 5H), 7.47-7.42 (m, 3H), 7.34 (d, J = 8.4 Hz, 1H), 7.18 (s, 2H), 7.09 (dd, J = 1.8 and 8.7 Hz, 2H), 7.01 (m, 1H), 5.19-5.08 (m, 1H), 3.93 (d, J = 4.8 Hz, 1H), 3.86 (s, 4H), 3.52 (q, J = 6.9 Hz, 1H), 3.34 (d, J = 6.3 Hz, 2H), 4.15 (dd, J = 2.1 and 12.3 Hz, 1H), 2.94 (dd, J = 3.3 and 14.7 Hz, 1H), 1.39 (d, J = 6.9, 3H). ¹³C NMR (CD₃CN) δ (ppm): 174.2, 166.9, 157.6, 136.9, 135.5, 134.6, 133.7, 133.3, 132.3, 131.9, 129.1, 128.8, 128.0, 127.8, 127.5, 127.5, 127.2, 126.9, 126.3, 126.2, 125.8, 118.7, 117.3, 105.7, 56.9, 55.0, 45.9, 41.3, 37.7, 31.3, 17.6. HRMS m/z calculated for C₃₁H₃₂N₅O₄ [M + H]⁺ 538.2454, found 538.2449.

### 4. Synthesis of pyrazole

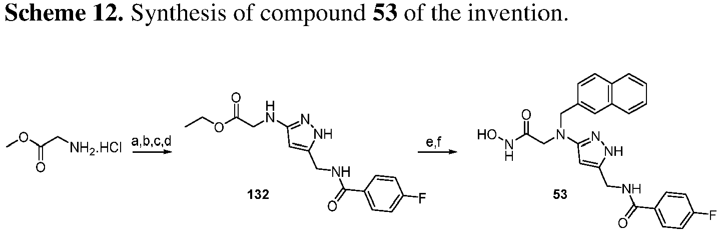

Reagent and conditions: (a) 4-fluorobenzoyl chloride, Et₃N, CH₂Cl₂, rt, 3 h; (b) (i) CH₃CN, nBuLi, THF, -78°C to rt, 3.5 h; (c) NH₂NH₂.H₂O, EtOH, reflux, overnight; (d) ethyl glyoxylate, NaBH₃CN, glacial acetic acid, MeOH, 45°C, 3 h; (e) naphthaldehyde, NaBH₃CN, acetic acid, MeOH, reflux, 4.5 h; (f) (i) NH₂OH.HCl, KOH, Na₂SO₄, MeOH, 42°C, overnight, (ii) KCN, DIEA, 55°C, 45 h.

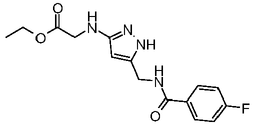

**Ethyl 2-[[5-[[(4-fluorobenzoyl)amino]methyl]-1H-pyrazol-3-yl]amino]acetate (132-TVE).** To a solution of methyl-2-aminoacetate hydrochloride (6 g, 47.9 mmol, 1.1 equiv) and triethylamine (12.8 mL, 91.4 mmol, 2.1 equiv) in dichloromethane (32 mL) was added dropwise 4-fluorobenzoyl chloride (5.15 mL, 43.5 mmol, 1 equiv). The resulting mixture was stirred at room temperature for 3h. The organic layer was then washed with HCl (1N), water, brine and was dried over MgSO₄. Evaporation of the solvent afforded the desired product which was used without further purification. White/yellow solid (9 g, 98%). Purity: 100%, LC t_{R} = 2.47 min, MS (ESI-): m/z = 210 [M-H]⁻. ¹H NMR (CDCl₃) δ (ppm) 3.77 (s, 3H), 4.19 (d, J = 5.4 Hz, 2H), 7.04-7.09 (m, 3H), 7.80-7.84 (m, 2H). ¹³C NMR (CDCl₃) δ (ppm) 41.7, 52.4, 115.6 (J_{C-F} = 21.9 Hz), 129.5 (J_{C-F} = 8.9 Hz), 129.7 (J_{C-F} = 2.9 Hz), 164.9 (J_{C-F} = 251.0 Hz), 166.6, 170.6. To a stirred solution of MeCN (2.30 mL, 44.8 mmol, 2.1 equiv) in anhydrous THF (40 mL) under inert atmosphere was added n-BuLi (28.6 mL, 45.8 mmol, 1.6 M in hexane, 2.2 equiv) at -78°C. After the solution was stirred for 30 min at the same temperature, previously obtained methyl 2-[(4-fluorobenzoyl)amino]acetate (4.5 g, 21.3 mmol, 1 equiv) in solution in 20 mL of anhydrous THF was added. The reaction mixture was allowed to warm at room temperature for 3 hours. HCl 1N was added in order to quench the reaction and until the pH of the aqueous layer was equal to 1. The phases were separated. The aqueous layer was extracted three times with EtOAc. The combined organic layers were washed with brine, dried over MgSO₄ and the solvent was removed by evaporation. A solution of the residue in ethanol (30 mL) was mixed with hydrazine monohydrate (3.2 mL, 63.9 mmol, 3 equiv) and was refluxed overnight. Ethanol was evaporated and the residue was triturated in water and methanol. The precipitate was then filtered and the desired product was used for the next step without further purification. Brown solid (5 g, 44%). Purity: 95%, MS (ESI+): m/z = 235 [M+H]⁺. ¹H NMR (DMSO) δ (ppm) 4.28 (d, J = 5.7 Hz, 2H), 4.62 (br s, 2H), 5.26 (s, 1H), 7.24-7.30 (m, 2H), 7.93-7.98 (m, 2H), 8.91 (br s, 1H), 11.25 (br s, 1H). To a solution of N-[(3-amino-1H-pyrazol-5-yl)methyl]-4-fluoro-benzamide (2.2 g, 9.39 mmol) in anhydrous methanol (100 mL) were added ethyl glyoxylate (2.23 mL, 11.3 mmol), NaBH₃CN (708 mg, 11.3 mmol) and finally glacial acetic acid (0.52 mL, 9.39 mmol). The resulting mixture was heated at 45°C for 3h and allowed to cool at room temperature. Ice and water were then added and methanol was evaporated. EtOAc and water were then added, the aqueous layer was extracted three times with EtOAc. The combined organic layer was washed with brine, dried over MgSO₄ and the solvent was evaporated. The crude was purified by flash chromatography on silica gel (CH₂Cl₂/Methanol: 100/0 to 95/5) to give the desired compound as a white solid (790 mg, 26%). Purity: 90%, MS (ESI+): m/z = 321 [M+H]⁺. ¹H NMR (DMSO) δ (ppm) 4.28 (d, J = 5.7 Hz, 2H), 4.62 (br s, 2H), 5.26 (s, 1H), 7.24-7.30 (m, 2H), 7.93-7.98 (m, 2H), 8.91 (br s, 1H), 11.25 (br s, 1H).

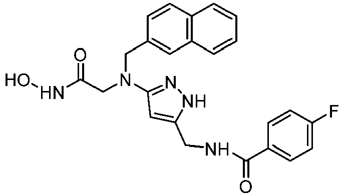

**4-fluoro-N-((3-((2-(hydroxyamino)-2-oxoethyl)(naphthalen-2-ylmethyl)amino)-1H-pyrazol-5-yl)methyl)benzamide (53).** To a solution of ethyl 2-[[5-[[(4-fluorobenzoyl)amino]methyl]-1H-pyrazol-3-yl]amino]acetate **132** (1 equiv) in anhydrous methanol were added naphthaldehyde, acetic acid and finally NaBH₃CN (1.3 equiv). The resulting mixture was refluxed 1 h. NaBH₃CN (1.3 equiv) was then added and the mixture was refluxed 1h30. NaBH₃CN (1.3 equiv) was added and the reaction was refluxed 2h. Water was added to the reaction mixture and methanol was evaporated. Water and EtOAc were then added and the layers were separated. Aqueous layer were extracted (2x) and the combined organic layers were washed with brine and dried over MgSO₄. Evaporation of the solvent afforded the crude which was purified by flash chromatography on silica gel (CH₂Cl₂/MeOH: 99/1 to 95/5) to afford the desired compound (100 mg, 12%). To a solution of hydroxylamine hydrochloride (180 mg, 2.59 mmol, 28 equiv) in dry MeOH (1.2 mL) was added KOH (121 mg, 2.2 mmol, 23 equiv). The mixture was sonicated 5 min at room temperature. Na₂SO₄ was added in high excess. Filtration of the solids and dropwise addition to a solution of methyl (3R)-3-[4-[[(4-fluorobenzoyl)amino]methyl]triazol-1-yl]-4-(2-naphthyl)butanoate (42 mg, 0.094 mmol, 1 equiv) in dry MeOH (1 mL). The mixture was stirred at 42°C overnight. KCN (12 mg, 0.188 mmol, 2 equiv) and DIEA (36.5 mg, 0.282 mmol, 3 equiv) were added, and the mixture was stirred to 55°C for 45 h. The mixture was cooled down to rt, partitioned between EtOAC and water acidified to pH 4 (using aq. saturated NH4Cl and diluted aq. HCl). Extraction with EtOAc, drying with Na₂SO₄ and evaporation afforded a yellow oily residue as crude product, which was purified by preparative HPLC to give the desired compound as a white solid (17.5 mg, 42%). LC t_{R}: 2.45 min, MS (ESI+) m/z = 448 [M+H]+. ¹H NMR (MeOD-d₄) δ (ppm): 8.42 (s, 1H), 7.88-7.77 (m, 5H), 7.70 (s, 1H), 7.45-7.42 (m, 3H), 7.14 (t, J = 17.4 and 8.7 Hz, 2H), 5.70 (s, 1H), 4.69 (s, 2H), 4.49 (s, 2H), 3.71 (s, 2H). ¹³C NMR (MeOD-d₄) δ (ppm): 169.6, 168.8, 136.6, 134.6, 134.0, 131.4, 130.8, 130.7, 129.2, 128.6, 128.5, 127.3, 127.0, 126.8, 126.6, 116.4, 116.1, 90.5, 55.9, 54.6, 53.8, 51.9, 36.4, 30.6. ¹⁹F NMR (MeOD) δ (ppm): -109.6. HRMS m/z calculated for C₂₄H₂₃N₅O₃F [M + H]⁺ 448.1795, found 448.1785.

### 5. Synthesis of oxadiazoles

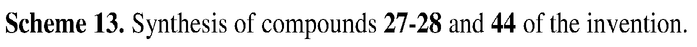

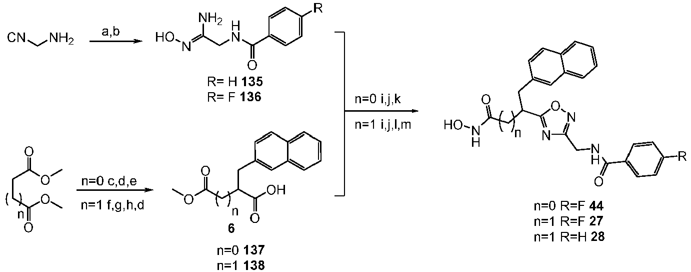

Reagent and conditions: (a) RCOCl, pyridine, rt, overnight; (b) hydroxylamine.HCl, triethylamine, MeOH, rt or reflux, overnight; (c) 2-naphthaldehyde, proline, DMSO, rt, 24 h; (d) Pd/C, ammonium formate, EtOH, rt, overnight; (e) NaOH, MeOH, rt, overnight; (f) 2-naphthaldehyde, t-BuOK, t-BuOH, reflux, 3 h; (g) NaOH 2M, EtOH, reflux, 16 h; (h) Amberlyst-15H⁺, MeOH, reflux, overnight; (i) (*i*) CDI, CH₂Cl₂, rt, 10 min, (*ii*) 3-4, rt, overnight, (*iii*) DMF, reflux, 3 h; (j) (*i*) NaOH 2M, EtOH/THF, rt, 16 h, (*ii*) HCl 1N; (k) O-(tert-butyldimethylsilyl)hydroxylamine, HOBt, EDCI, NMM, rt, overnight; (l) O-tritylhydroxylamine, EDCI, HOBt, N-methylmorpholine, DMF, rt, overnight; (m) TFA, TIS, CH₂Cl₂, rt, 2 h.

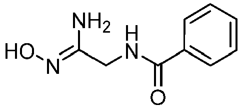

**N-(N-hydroxycarbamimidoylmethyl)-benzamide (135).** Compound **134** was obtained according to general procedure S as a white solid (574 mg, 80%). Purity: 93%, LC t_{R} = 1.37 min (method A), MS (ESI+): m/z = 194 [M + H]⁺. ¹H NMR (DMSO-d₆) δ (ppm): 9.04 (br s, 1H), 8.70 (br s, 1H), 7.86 (d, J = 7.2 Hz, 2H), 7.53-7.46 (m, 3H), 5.38 (br s, 2H), 3.86 (d, J = 5.4 Hz, 2H). ¹³C NMR (DMSO-d₆) δ (ppm): 167.2, 151.0, 134.5, 131.8, 128.8, 127.7,46.1.

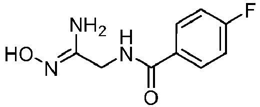

**4-fluoro-N-(N-hydroxycarbamimidoylmethyl)-benzamide (136).** Compound **136** was obtained according to general procedure S as a white solid (2.28 g, 95%). Purity: 100%, LC t_{R} = 1.37 min (method A), MS (ESI+): m/z = 212 [M + H]⁺. ¹H NMR (DMSO-d₆) δ (ppm): 9.05 (br s, 1H), 8.78 (br t, J = 6.0 Hz, 1H), 7.97-7.92 (m, 2H), 7.29 (t, J = 9.0 Hz, 2H), 5.39 (br s, 2H), 3.85 (d, J = 6.0 Hz, 2H). ¹³C NMR (DMSO-d₆) δ (ppm): 166.1, 162.7, 150.9, 131.0, 130.4 (d, J_{C-F} = 9 Hz), 115.6 (d, J_{C-F} = 22 Hz), 45.9.

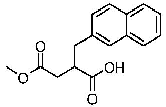

**2-naphthalen-2-ylmethyl-succinic acid 4-methyl ester (137).** To a refluxing suspension of *t*-BuOK (2.59 g, 23.1 mmol, 1.2 eq) in *t*-BuOH (17 mL) was carefully added a solution of dimethyl succinate (3.3 mL, 25.0 mmol, 1.3 eq) and 2-naphthaldehyde (3.00 g, 19.2 mmol, 1 eq) in t-BuOH (17 mL). The reaction mixture was stirred at reflux temperature for 3 h, after which the solvent was removed under vacuum. The residue was dissolved in 1M HCl (17 mL) and this solution was extracted with EtOAc (3x50 mL). The organic layers were dried (MgSO₄) and concentrated. The resulting monoacid was dissolved and EtOH (24 mL) and aqueous NaOH (2 M, 48 mL) were added. The resultant mixture was stirred under reflux for 16 h, followed by evaporation of the EtOH under reduced pressure. Extra H₂O (60 mL) and NaOH (2 M, 10 mL) was added and the mixture was washed with EtOAc (3x60 mL). Next, the aqueous layer was acidified (pH≈1) with 1M HCl, extracted with EtOAc (2x120 mL) and the organic layers were dried (MgSO₄) and concentrated. The resulting diacid was dissolved in MeOH (7.4 mL). Amberlyst-15H⁺ (1.3 g) was added and the reaction mixture was heated under reflux for 16 h. The mixture was filtered over Celite and concentrated under vacuum, resulting in crude ester. The product was purified by flash chromatography (CH₂Cl₂/MeOH 100:0 to 98:2 (v/v)) to give compound 2-naphthalen-2-ylmethylene-succinic acid 4-methyl ester as a white amorphous solid (2.067 g, 40%). Purity: 75%, LC t_{R} = 3.09 min (method A), MS (ESI+): m/z = 271 [M + H]⁺. ¹H NMR (CDCl₃) δ (ppm): 8.21 (br s, 1H), 7.89-7.86 (m, 4H), 7.58-7.47 (m, 3H), 3.80 (s, 3H), 3.67 (s, 2H). ¹³C NMR (CDCl₃) δ (ppm): 172.8, 171.6, 144.4, 133.4, 133.1, 132.1, 129.3, 128.5, 127.7, 127.2, 126.7, 126.2, 125.3, 52.3, 33.3. Under an atmosphere of argon, Pd/C (0.35 g) and ammonium formate (1.64 g) was added to a solution of the previous acid (0.780 g, 2.89 mmol) in ethanol (29 mL). The mixture was stirred at room temperature for 16 h, then it was filtered over Celite. Solvent was removed under vacuum. The residue was solubilized in water and acidified (pH≈1) with 1M HCl, extracted with CH₂Cl₂ (2×120 mL) and the collected organic layers were dried (MgSO₄) and concentrated. The residue was purified by flash chromatography (CH₂Cl₂/MeOH 100:0 to 98:2 (v/v)) to give compound **111** as a yellow oil (0.663 g, 84%). Purity: 84%, LC t_{R} = 2.65 min (method A), MS (ESI-): m/z = 271 [M - H]⁻. ¹H NMR (MeOD) δ (ppm): 9.74 (br s, 1H, OH), 7.89-7.84 (m, 3H), 7.70 (s, 1H), 7.54-7.48 (m, 2H), 7.39 (d, J = 8.4 Hz, 1H), 3.66 (s, 3H), 3.37-3.32 (m, 2H), 3.00 (dd, J = 10.5 and 15.3 Hz, 1H), 2.75 (dd, J = 8.7 and 17.1 Hz, 1H), 2.51 (dd, J = 4.5 and 17.1 Hz, 1H). ¹³C NMR (MeOD) δ (ppm): 180.1, 172.2, 135.7, 133.5, 132.4, 128.3, 127.7, 127.6, 127.6, 127.2, 126.2, 125.7, 42.9, 37.5, 34.6.

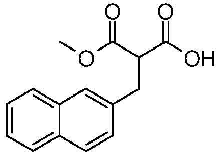

**2-Naphthalen-2-ylmethyl-malonic acid monomethyl ester (138).** To a solution of naphthaldehyde (3.5 g, 22.4 mmol) in DMSO (10 mL) was added proline (253 mg, 3.2 mmol) and the mixture was stirred 15 min at room temperature. Dimethylmalonate (5.1 mL, 44.8 mmol) was then added and the reaction media was stirred for 24 hours. EtOAc was added and the organic layer was washed with water, brine and was dried over MgSO₄. Evaporation of the solvent afforded the crude which was purified by flash chromatography on silica gel (cyclohexane/EtOAc: 100:0 to 90:10). Viscous yellow oil (1.6 g, 26%). Purity: 100%, LC t_{R} = 3.40 min, MS (ESI+): m/z = 271 [M+H]⁺. ¹H NMR (CDCl₃) δ (ppm): 3.87 (s, 6H), 7.45-7.56 (m, 3H), 7.80-7.85 (m, 3H), 7.93 (s, 2H). ¹³C NMR (CDCl₃) δ (ppm): 52.7, 125.0, 125.5, 126.8, 127.7, 127.8, 128.7, 128.8, 130.3, 131.1, 133.1, 134.1, 143.1, 164.6, 167.3. To a solution of 2-Naphthalen-2-ylmethylene-malonic acid dimethyl ester (1.52 g, 5.64 mmol) in degazed EtOH (100 mL) were added ammonium formate (3.2 g, 50.8 mmol) and palladium (10%) on charcoal (600 mg, 0.56 mmol). The reaction was stirred for 4 hours and the media was then filtered over celite. The residue was dissolved in EtOAc, washed with water and brine and finally dried over MgSO₄. Evaporation of the solvent afforded the desired product which was used without further purification. Yellow oil (1.53 g, Quant.). Purity: 100%, LC t_{R} = 3.37 min, MS (ESI+): m/z = 273 [M+H]⁺. ¹H NMR (CDCl₃) δ (ppm): 3.42 (d, J = 7.8 Hz, 2H), 3.70 (s, 6H), 3.82 (t, J = 7.8 Hz, 1H), 7.34 (dd, J = 1.5 Hz and 8.4 Hz, 1H), 7.42-7.49 (m, 2H), 7.67 (s, 1H), 7.77-7.82 (m, 3H). ¹³C NMR (CDCl₃) δ (ppm): 35.0, 52.6, 53.6, 125.7, 126.1, 127.0, 127.4, 127.7, 128.3, 132.4, 133.5, 135.3, 169.3. NaOH (259 mg, 6.46 mmol) was added to a solution of 2-Naphthalen-2-ylmethyl-malonic acid dimethyl ester (1.53 g, 5.62 mmol) in MeOH (10 mL). The reaction was stirred overnight and the solvent was removed. The residue was dissolved in aqueous NaHCO₃ and washed twice with CH₂Cl₂. The aqueous layer was acidified with fuming HCl and then extracted with EtOAc. The combined organic layer was dried over MgSO₄ and the solvent was evaporated to afford the desired product as a white solid (1.45 g, Quant.). Purity: 100%, LC t_{R} = 2.77 min. ¹H NMR (CDCl₃) δ (ppm): 3.41 (dd, J = 2.7 and 7.8 Hz, 2H), 3.70 (s, 3H), 3.83 (t, J = 7.8 Hz, 1H), 7.33 (dd, J = 1.8 and 8.4 Hz, 1H), 7.42-7.50 (m, 2H), 7.67 (s, 1H), 7.76-7.82 (m, 3H), 8.70 (br s, 1H)

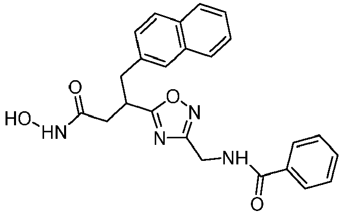

**N-[5-(1-hydroxycarbamoylmethyl-2-naphthalen-2-yl-ethyl)-[1,2,4]oxadiazol-3-ylmethyl]-benzamide (28).** Compound **28** was obtained according to general procedure T from **135** and **138** followed by general procedure P followed by general procedure D as a colorless oil (65 mg, 41%). Purity: 93%, LC t_{R} = 2.87 min (method A), MS (ESI+): m/z = 431 [M + H]⁺. ¹H NMR (acetone-d₆) δ (ppm): 8.29 (br s, 1H,), 7.95-7.75 (m, 5H), 7.61 (s, 1H), 7.55-7.42 (m, 5H), 7.29-7.27 (m, 1H), 4.66 (s, 2H), 3.96-3.93 (m, 1H), 3.24 (d, J = 6.6 Hz, 2H), 2.66-2.62 (m, 2H). ¹³C NMR (acetone-d₆) δ (ppm): 181.5, 168.2, 167.0, 166.6, 135.5, 134.3, 133.6, 132.4, 131.4, 128.4 (2C), 128.0, 127.6 (2C), 127.5, 127.3(3C), 126.0, 125.6, 38.8, 36.4, 35.1, 34.6. HRMS m/z calculated for C₂₄H₂₃N₄O₄ [M + H]⁺ 431.1719, found 431.1727.

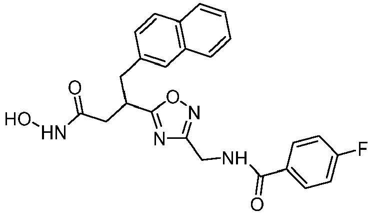

**4-fluoro-N-[5-(1-hydroxycarbamoylmethyl-2-naphthalen-2-yl-ethyl)-[1,2,4]oxadiazol-3-ylmethyl]-benzamide (27).** Compound **27** was obtained according to general procedure T from **136** and **138** followed by general procedure P followed by general procedure D as a colorless oil (44 mg, 28%). Purity: 100%, LC t_{R} = 2.90 min (method A), MS (ESI+): m/z = 449 [M + H]⁺. ¹H NMR (acetone-d₆) δ (ppm): 8.35 (br t, 1H, NH), 8.02-7.98 (m, 2H), 7.82-7.74 (m, 3H), 7.60 (s, 1H), 7.46-7.40 (m, 2H), 7.29-7.20 (m, 3H), 4.65 (d, J = 6.0 Hz, 2H), 4.00-3.90 (m, 1H), 3.24 (d, J = 7.2 Hz, 2H), 2.65 (t, J = 8.7 Hz, 2H). ¹³C NMR (acetone-d₆) δ (ppm): 181.5, 168.1, 167.1, 165.7, 164.6 (d, J_{C-F} = 248 Hz), 163.7, 135.5, 133.5, 132.4, 130.7, 130.0, 129.9, 128.0, 127.6 (2C), 127.5, 127.3, 126.0, 125.6, 115.2 (d, J_{C-F} = 22 Hz), 38.8, 36.4, 35.1, 34.6. HRMS m/z calculated for C₂₄H₂₂N₄O₄F [M + H]⁺ 449.1625, found 449.1668.

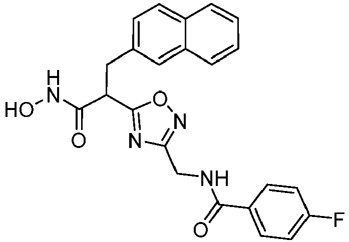

**4-Fluoro-N-[5-(1-hydroxycarbamoyl-2-naphthalen-2-yl-ethyl)-[1,2,4]oxadiazol-3-ylmethyl]-benzamide (44).** To a solution of the carboxylic acid oxadiazole (1 equiv), obtained according to general procedure T from **136** and **138** followed by general procedure P, in DMF were added HOBt (5 equiv), EDCI (4 equiv), and N-methylmorpholine (12 equiv) and the reaction media was stirred 15 min at room temperature. O-(*tert*-butyldimethylsilyl)hydroxylamine (3 equiv) was then added and the resulting mixture was stirred overnight. DMF was evaporated and the residue was dissolved in EtOAc, and washed with water and brine. The organic layer was then dried over MgSO₄ and the solvent was evaporated. Purification by flash chromatography to afford a yellow solid (20 mg, 2%). Purity: 96%, LC t_{R} = 2.85 min, MS (ESI+): m/z = 435 [M+H]+. ¹H NMR (DMSO-*d₆*) δ (ppm): 3.46 (d, J = 7.5 Hz, 2H), 4.24 (t, J = 7.8 Hz, 1H), 4.57 (d, J = 6.0 Hz, 2H), 7.31-7.37 (m, 2H), 7.39-7.42 (m, 1H), 7.45-7.49 (m, 2H), 7.72 (s, 1H), 7.77-7.88 (m, 3H), 7.94-7.99 (m, 2H), 9.13 (s, 1H), 9.22 (t, J = 5.7 Hz, 1H), 10.94 (s, 1H). ¹³C NMR (DMSO-*d₆*) δ (ppm) 35.2, 35.5, 44.1, 115.9 (J_{C-F} = 22 Hz), 120.0, 126.1, 126.6, 127.8, 127.9, 128.3, 130.4 (J_{C-F} = 9 Hz), 130.6, 132.4, 133.4, 135.6, 163.7, 164.5 (J_{C-F} = 247 Hz), 165.7, 168.8, 177.6. HRMS m/z calculated for C₂₃H₂₀N₄O₄F [M + H]⁺ 435.1469, found 435.1438.

### BIOLOGICAL EVALUATIONS

### Materials and methods

### 1. Cell culture

### (a) OPM2 cell culture

Human multiple myeloma cells OPM2 (ACC 50, DSMZ) are maintained at 0.3-0.7 10⁶ cells/mL in RPMI1640 medium supplemented with GlutaMAX^{™}, 10% fetal bovine serum and 1% penicillin-streptomycin.

### (b) HeLa cell culture

Human HeLa cells (uterine cervical carcinoma) are maintained in DMEM medium supplemented with GlutaMAX^{™}, 10% fetal bovine serum and 1% penicillin-streptomycin.

### (c) MCF7 cell culture

Human epithelial MCF7 cells (mammary gland adenocarcinoma) are maintained in EMEM medium supplemented with GlutaMAX^{™}, 10% fetal bovine serum, 0.01 mg insulin and 1% penicillin-streptomycin.

### (d) RPMI 8226 and MOPC315.BM cell culture

RPMI 8226 and MOPC315.BM cells were cultured in RPMI 1640 medium supplemented with L-glutamine, non-essential amino acids, 2-mercaptoethanol and 10% fetal calf serum.

### (e) TLM1 cell culture

Canine melanoma TLM-1 cells are maintained in DMEM medium supplemented with GlutaMAX^{™}, 10% fetal bovine serum, 1X Hepes and 1% penicillin-streptomycin.

### 2. High Content Screening to identify compounds that sensitize cells to RE stressors and boost apoptosis.

We developed a cell-based, high content assay allowing the rapid detection and quantification of apoptotic cells. Caspase-3/7 activity was detected in real-time using the NucView^{™} 488 Caspase-3 substrate (Biotium), where the caspase-3/7 substrate peptide sequence DEVD is attached to a nucleic acid dye. The NucView^{™} 488 reagent crosses the plasma membrane to enter the cytoplasm, where the dye is unable to bind to DNA and remains non-fluorescent. Once the substrate has been cleaved by activated caspase-3/7 in apoptotic cells, the high-affinity DNA dye is released and migrates to the cell nucleus to stain the nucleus with a bright green fluorescence. All cell nuclei were also labeled with Hoechst 33342 (Ozyme).

For OPM2, briefly, nanoliters of compound and cytotoxic agent in DMSO stock solutions were first transferred and combined in 384-microtiter well plate using an Echo555 liquid handler (Labcyte). The exact volume of each solution to be transferred was calculated to achieve subsequently the target concentration in the 70 µL volume incubate. Final DMSO content was maintained at 0.2%. Then, 70 µL of cells treated with 1 µM NucView^{™} 488 and 20 ng/mL Hoechst 33342 were distributed to the plates to obtain a final density of 12,000 cells per well. The microplates were incubated at 37°C and 5% CO₂ in an automated LiCONiC STR240 UB cell incubator, and images were acquired at various times at 405 nm and 488 nm with the high-resolution automated confocal microscope INCell 6000 (GE). Four fields per well were read with the 20x objective in a confocal mode. The BioCel workstation was managed by VWorks automation control software (Agilent).

For HeLa and MCF7 cells, briefly, cells were seeded in 384-microtiter well plate (1500 HeLa cells or 5000 MCF7 cells in 40 µL full medium per well) and let to attach overnight. Then the compound of the invention and the RE stressor were transferred to the cell culture plate using an Echo555 liquid handler (Labcyte) and 30 µL medium containing NucView^{™} and Hoechst 33342 were added. Final NucView^{™} 488 and Hoechst 33342 concentrations were 1 µM and 20 ng/mL, respectively. DMSO content was maintained at 0.2%. The microplates were incubated at 37°C and 5% CO₂ in an automated LiCONiC STR240 UB cell incubator, and images were acquired at various times at 405 nm and 488 nm with the high-resolution automated confocal microscope INCell 6000 (GE). Four fields per well were read with the 20x objective in a confocal mode. The BioCel workstation was managed by VWorks automation control software (Agilent).

For all cells, Columbus software (Perkin) was used for image quantification and analysis. First, Hoechst 33342 staining was used for an automated detection of nuclei, in which the mean fluorescence intensity for NucView^{™} 488 was quantified. Inspection of control cells at initial time was used to set a threshold level of NucView^{™} 488 fluorescence to separate non apoptotic cells from apoptotic cells. Finally, for each well, the result was expressed as percentage of apoptotic cells in the total cell population. CC₅₀ values were calculated from concentration-response curves by a nonlinear regression analysis at four parameters using Excel Fit or GraphPad Prim softwares.

Activities were calculated as either:
- Ratio of % of apoptoptic cells. % of apoptotic cells of cytotoxic agent was calculated in absence and in presence of a compound of the invention.

Activity = (% of apoptotic cells with cytotoxic agent in combination with compound of the invention) / (% of apoptotic cells with cytotoxic agent alone).
- Ratio of CC₅₀: The CC₅₀ of the cytotoxic agent was calculated in absence and in presence of a compound of the invention.

Activity = (CC₅₀ of agent alone) / (CC₅₀ of agent with compound).
- Ratio of AUC. Activity = (Area of the dose-concentration curve of cytotoxic agent combined with one concentration of compound) / (Area of the dose-concentration curve of cytotoxic agent alone). (Lehar J. et al, 2008 (molecular systems biology 4:215)).

### 3. Effect of the combination of epoxomicin and compound (3) of the invention on murine and human myelomas.

Cells were seeded in 24-well culture plates at 150,000 cells/well/mL. Compounds (EPX and 3) were dissolved and added to culture medium at a final concentration of 1%. Cells were treated with protease inhibitors for 24 and 48 hours before analysis for apoptosis and cell death by flow cytometry. The cells were centrifuged at 300 × g for 5 minutes and washed with cold FACS buffer (PBS 1X with 2 mM EDTA and 0.5% BSA. Then the cells were resuspended in Annexin V binding buffer (BioLegend) at a concentration of 10⁶ cells/mL, and 100 µL of each sample was transferred to a 5 mL FACS tube. 5 µL of Annexin V-FITC Ab (BioLegend) and 5 µL of propidium iodide (BioLegend) were added to each sample and incubated 15 min at RT. Finally, 400 µL of Annexin V binding buffer was added to each tube and the cells were analyzed using BD FACSCanto or BD Fortessa machines within an hour. Annexin+ but PI- cells were identified as apoptotic and Annexin+ PI+ as dead cells.

### 4. Effect of the combination of X-ray radiation and compounds of the invention on canine melanoma TLM1 cell line

TLM-1 cells were seeded with the BioTek MicroFlo^{™} dispenser in µClear^{®} black 384-well plates (Greiner) at the density of 20,000 cells/mL in 50 µL. Compounds for pretreatment were added 2h after the seeding, using an Echo 550 (Labcyte). The next day, cells were washed five times with 40 µL media without phenol red with a plate washer (Hydrospeed, Tecan). Cells were irradiated with 4, 6, 8, 12, 16, 20 and 24 Gray (Gy). Around 1.5h after irradiation, 20 µL of media containing compounds at the desired concentration, Hoechst 33342 (40 ng/mL, Thermofisher) for nuclei staining and propidium iodide (1µg/mL, Thermofisher) for labeling dead cells were added to the cell plates with a Bravo liquid handler (Agilent). This step was repeated every 72h after radiation. Plates were incubated at 37°C and 5% CO₂. Images were acquired at various times with DAPI filter set (ex.405/em.455nm) and Texas Red filter set (ex.561/em.605nm) with the high-resolution automated confocal microscope INCell 6000 (GE) in 20X magnification in non-confocal mode. 4 images per well were collected. Images were analyzed with Columbus software (Perkin Elmer). In this system we used predefined building blocks to measure different parameters on nuclei detected. We applied a threshold based on propidium iodide intensity in nuclei of controls to select and count dead cells. By dividing the number of dead cells by the number of nuclei detected we obtained the percentage of dead cells. AUC were determined in one experiment with triplicate incubates on percentage of cells labelled with propidium iodide and were corrected by the percentage of cells labelled with propidium iodide at 0 Gy. Ratio of AUC corrected was determined as followed: Activity = (Area of the dose-concentration curve of cytotoxic agent combined with one concentration of compound corrected by the effect of the compound alone at 0Gy) / (Area of the dose-concentration curve of radiation alone corrected by the effect of the vehicle at 0Gy).

### 5. Effect of the combination of UV-C radiation and compounds of the invention on canine melanoma TLM1 cell line

TLM-1 cells were seeded in µClear^{®} black 384-well plates (Greiner) at the density of 60,000 cells/mL in 50 µL. Compounds were added 24h after the seeding, using an acoustic nanodispensor Echo 550 (Labcyte). The next day, cells were washed five times with 40 µL phosphate buffer saline (PBS) with a plate washer (Hydrospeed, Tecan). 10 µL of PBS was left per well. Plate was submitted to UV-C (Stratalinker^{®} UV Crosslinker, Artisan Technologies Group) with different doses (J/m²). After UV-C radiation, compounds were added using an acoustic nanodispensor Echo 550 (Labcyte). We complete to 50 µL of media per well. Plates were incubated at 37°C and 5% CO_{2.} Some hours before image acquisition, propidium iodide (1 µg/mL, Thermofisher) for labeling dead cells and Hoechst 33342 (80 ng/mL, Thermofisher) for nuclei staining were added using an acoustic nanodispensor Echo 550 (Labcyte). Images were acquired at various times with DAPI filter set (ex.405/em.455nm) and Texas Red filter set (ex.561/em.605nm) with the high-resolution automated confocal microscope INCell 6000 (GE) in 20X magnification in non-confocal mode. 4 images per well were collected. Images were analyzed with Columbus software (Perkin Elmer). In this system we used predefined building blocks to measure different parameters on nuclei detected. We applied a threshold based on propidium iodide intensity in nuclei of controls to select and count dead cells. By dividing the number of dead cells by the number of nuclei detected we obtained the percentage of dead cells. Activity refers to the ratio of the percentage of cells labelling with propidium iodide with pre-post treatment with the compound on the percentage of cells labelling with propidium iodide with pre-post treatment with vehicle, 48h after the UV-C radiations.

### Results

### Effect of compounds of the invention on cytotoxicity of proteasome inhibitors in human multiple myeloma cell lines

Compounds of the invention increase proteasome inhibitor cytotoxicity in human multiple myeloma cell lines: for example, Table 2 shows activities of compounds of the invention in combination with carfilzomib (CFZ) on OPM2 cells. Results show that activities of compounds of the invention in combination with CFZ on OMP2 cells are higher compared to the activity of CFZ alone.

**Table 2. Activities of compounds of the invention in combination with CFZ on OPM2**

| **Compound** | **Activity^{a}** | **Compound** | **Activity^{a}** | **Compound** | **Activity^{a}** | **Compound** | **Activity^{a}** |
|---|---|---|---|---|---|---|---|
| **1** | 150 | **28** | 394 | **55** | 132 | **82** | 195 |
| **2** | 234 | **29** | 387 | **56** | 210 | **83** | 187 |
| **3** | 239 | **30** | 294 | **57** | 283 | **84** | 252 |
| **4** | 213 | **31** | 251 | **58** | 245 | **85** | 172 |
| **5** | 142 | **32** | 314 | **59** | 301 | **86** | 262 |
| **6** | 170 | **33** | 163 | **60** | 233 | **87** | 209 |
| **7** | 158 | **34** | 152 | **61** | 300 | **88** | 230 |
| **8** | 141 | **35** | 179 | **62** | 327 | **89** | 1089 |
| **9** | 161 | **36** | 153 | **63** | 168 | **90** | 147 |
| **10** | 131 | **37** | 189 | **64** | 209 | **91** | 135 |
| **11** | 247 | **38** | 177 | **65** | 248 | **92** | 137 |
| **12** | 211 | **39** | 163 | **66** | 158 | **93** | 370 |
| **13** | 232 | **40** | 152 | **67** | 292 | **94** | 432 |
| **14** | 199 | **41** | 130 | **68** | 172 | **95** | 331 |
| **15** | 218 | **42** | 197 | **69** | 150 | **96** | 299 |
| **16** | 201 | **43** | 255 | **70** | 178 | **97** | 208 |
| **17** | 214 | **44** | 133 | **71** | 176 | **98** | 431 |
| **18** | 157 | **45** | 137 | **72** | 158 | **99** | 691 |
| **19** | 306 | **46** | 221 | **73** | 145 | **100** | 203 |
| **20** | 421 | **47** | 244 | **74** | 291 | **143** | 143 |
| **21** | 272 | **48** | 311 | **75** | 210 | **144** | 522 |
| **22** | 208 | **49** | 152 | **76** | 176 | **146** | 318 |
| **23** | 260 | **50** | 157 | **77** | 229 | **170** | 613 |
| **24** | 167 | **51** | 166 | **78** | 233 | **178** | 290 |
| **25** | 298 | **52** | 248 | **79** | 199 | | |
| **26** | 385 | **53** | 135 | **80** | 280 | | |
| **27** | 497 | **54** | 282 | **81** | 362 | | |

| | | | | | | | |
|---|---|---|---|---|---|---|---|
| ^{a} %OPM2 apoptotic cells of (CFZ+compound of the invention) vs. CFZ alone | | | | | | | |

### Effect of compounds of the invention on cytotoxicity of protein glycosylation inhibitors in human multiple myeloma cell lines

Compounds of the invention increase cytotoxicity of tunicamycin (TUN) in human multiple myeloma cell line (OPM2) (Table 3). Results show that activities of compounds of the invention in combination with TUN on OMP2 cells are higher compared to the actity of TUN alone.

**Table 3. Activities of compounds of the invention in combination with TUN on OPM2.**

| **Compound** | **Activity^{a}** | **Compound** | **Activity^{a}** | **Compound** | **Activity^{a}** |
|---|---|---|---|---|---|
| **1** | 162 | **30** | 138 | **72** | 191 |
| **2** | 217 | 32 | 170 | **74** | 739 |
| **3** | 849 | **42** | 211 | **75** | 405 |
| **4** | 231 | **43** | 631 | **76** | 176 |
| **5** | 220 | **44** | 283 | **77** | 189 |
| **6** | 310 | **45** | 179 | **78** | 332 |
| **9** | 668 | **47** | 130 | **80** | 736 |
| **10** | 134 | **48** | 212 | **81** | 831 |
| **11** | 774 | **54** | 595 | **83** | 152 |
| **12** | 222 | **55** | 137 | **85** | 218 |
| **13** | 202 | **57** | 324 | **86** | 437 |
| **14** | 130 | **58** | 388 | **87** | 326 |
| **17** | 132 | **59** | 157 | **89** | 546 |
| **19** | 207 | **61** | 178 | **93** | 150 |
| **20** | 644 | **62** | 929 | **94** | 2567 |
| **23** | 221 | **63** | 185 | **101** | 171 |
| **25** | 237 | **64** | 220 | **102** | 133 |
| **26** | 439 | **67** | 163 | **103** | 132 |
| **27** | 745 | **68** | 167 | **104** | 152 |
| **28** | 483 | **69** | 136 | **105** | 131 |
| **29** | 647 | **70** | 142 | | |

| | | | | | |
|---|---|---|---|---|---|
| ^{a} %OPM2 apoptotic cells of (TUN+compound of the invention) vs. TUN alone | | | | | |

Compounds of the invention increase cytotoxicity of epoxomycin (EPX) in multiple myeloma cell lines (RPMI and MOPC315.BM) (Figure 1).

Figure 1 shows that the cytotoxicity of epoxomicin on multiple myeloma cell lines RPMI 8226 (Fig. 1A) and MOPC 315.BM (Fig. 1B) is increased when used in combination with compound **3** of the invention at 7.5 µM, 15 µM and 30 µM.

### Effect of compounds of the invention on cytotoxicity of proteasome inhibitors in cervical cancer cell lines

Compounds of the invention boost proteasome inhibitor cytotoxicity in cells other than multiple myeloma cell lines: for example, Table 4 shows activities of compounds of the invention in combination with CFZ on cervical cancer cell lines (Hela) (Table 4). Results show that activities of compounds of the invention in combination with CFZ on HeLa cells are higher compared to the activity of CFZ alone.

**Table 4. Activities of compounds of the invention in combination with CFZ on HeLa.**

| **Compound** | **Activity^{a}** | **Compound** | **Activity^{a}** |
|---|---|---|---|
| **3** | 383 | **58** | 402 |
| **12** | 379 | **62** | 455 |
| **20** | 1100 | **75** | 883 |
| **27** | 573 | **80** | 757 |
| **32** | 922 | **81** | 913 |
| **43** | 498 | **86** | 548 |
| **54** | 221 | | |

| | | | |
|---|---|---|---|
| ^{a} %Hela apoptotic cells of (CFZ+compound of the invention) vs. CFZ alone | | | |

### Effect of compounds of the invention on the potency of proteasome inhibitors in human multiple myeloma cell lines and in cervical cancer cell lines

Compounds of the invention increase the potency of proteasome inhibitors such as carfilzomib: lower doses of carfilzomib can be used to reach the same effect when used in combination with compounds of the invention. As shown in Table 5 & Table 6, the ratios (CC₅₀ of proteasome inhibitor alone) / (CC₅₀ of proteasome inhibitor in combination with compound of the invention) is higher than 1.

**Table 5. Examples of CFZ potency increase with compounds of the invention on OPM2.**

| **Compound** | **Activity^{a}** | **Compound** | **Activity^{a}** | **Compound** | **Activity^{a}** | **Compound** | **Activity^{a}** |
|---|---|---|---|---|---|---|---|
| **3** | 1.9 | **58** | 1.4 | **95** | 2.1 | **172** | 3.9 |
| **6** | 1.3 | **62** | 1.6 | **96** | 1.4 | **173** | 2.1 |
| **12** | 1.2 | **44** | 1.2 | **81** | 2.3 | **174** | 2.0 |
| **12** | 1.2 | **74** | 1.6 | **98** | 2.0 | **174** | 1.5 |
| **13** | 1.2 | **75** | 1.7 | **99** | 2.5 | **176** | 1.3 |
| **20** | 2.1 | **58** | 1.4 | **88** | 1.7 | **177** | 1.7 |
| **23** | 1.1 | **78** | 2.0 | **143** | 1.1 | **178** | 1.3 |
| **25** | 1.4 | **80** | 2.2 | **144**^{b} | 1.8 | **179** | 1.6 |
| **26** | 1.8 | **81** | 2.3 | **146** | 1.4 | **180** | 1.8 |
| **27** | 2.4 | **86** | 1.7 | **164** | 2.0 | **181** | 1.9 |
| **28** | 2.0 | **87** | 1.7 | **165** | 1.9 | **182** | 2.7 |
| **29** | 1.6 | **88** | 1.7 | **166**^{b} | 2.1 | **183** | 1.2 |
| **32** | 1.9 | **89** | 7.6 | **167** | 2.5 | **184** | 2.4 |
| **43** | 1.7 | **90** | 1.4 | **168** | 2.8 | **185** | 1.8 |
| **44** | 1.2 | **91** | 1.4 | **169** | 3.3 | **186** | 2.9 |
| **54** | 1.8 | **92** | 1.7 | **170** | 1.5 | **187** | 3.4 |
| **57** | 1.7 | **93** | 2.3 | **171** | 1.2 | | |

| | | | | | | | |
|---|---|---|---|---|---|---|---|
| ^{a} ratio (CC₅₀ of CFZ alone) / (CC₅₀ of CFZ in combination with compound of the invention (7.5 µM)). ^{b} calculated with compound at a concentration of 3.7 µM. | | | | | | | |

**Table 6. Examples of CFZ potency increase with compounds of the invention on various human cancer cell lines.**

| **Cells** | **OPM2** | **Hela** | **MCF7** | **Cells** | **OPM2** | **Hela** | **MCF7** |
|---|---|---|---|---|---|---|---|
| **Compound** | **Activity^{a}** | **Activity^{a}** | **Activity^{b}** | **Compound** | **Activity^{a}** | **Activity^{a}** | **Activity^{b}** |
| **3** | 1.9 | 2.8 | 2.1 | **58** | 1.4 | 2.7 | 2.0 |
| **12** | 1.2 | 2.4 | 1.6 | **62** | 1.6 | 3.0 | 2.6 |
| **20** | 2.1 | 6.3 | 3.4 | **75** | 1.7 | 4.3 | 2.1 |
| **27** | 2.4 | 3.0 | 2.8 | **80** | 2.2 | 3.6 | 2.4 |
| **32** | 1.9 | 4.5 | 2.7 | **81** | 2.3 | 3.5 | 2.5 |
| **43** | 1.7 | 2.6 | 2.6 | **86** | 1.7 | 2.6 | 1.7 |
| **54** | 1.8 | 2.3 | 2.2 | **89** | 7.6 | nd | nd |

| | | | | | | | |
|---|---|---|---|---|---|---|---|
| ^{a} ratio (CC₅₀ of CFZ alone) / (CC₅₀ of CFZ in combination with compound of the invention (7.5 µM)); ^{b} ratio (CC₅₀ of CFZ alone) / (CC₅₀ of CFZ in combination with compound of the invention (15 µM)). | | | | | | | |

### Effect of compounds of the invention on the potency of HDAC inhibitors and proteasome inhibitors in human multiple myeloma cell lines and in cervical cancer cell lines

Compounds of the invention increase the potency of other cytotoxic agents, in particular HDAC inhibitors such as Panobinostat (PAN) and proteasome inhibitors such as Nelfinavir (NEL): lower doses of cytotoxic agents can be used to reach the same effect when used in combination with compound **3** of the invention (Table 7 & Table 8).

**Table 7. Panobinostat potency increase by compound (3), on OPM2.**

| **Agent** | **Activity^{a}** |
|---|---|
| PAN | 2.1 |

| | |
|---|---|
| ^{a} ratio of AUC of cytotoxity of PAN with and without 15 µM (**3**). | |

**Table 8. Nelfinavir potency increase by compound (3), on Hela.**

| **Agent** | **Activity^{a}** |
|---|---|
| NEL | 1.5 |

| | |
|---|---|
| ^{a} ratio of AUC of cytotoxity of NEL with and without 15 µM **(3).** | |

### Effect of the combination of X-ray radiation and compounds of the invention on canine melanoma TLM1 cell line

Compounds **3, 80, 93, 144, 168, 169, 186, 187** of the invention increase the potency of X-ray radiations: lower doses of X-ray radiations can be used to reach the same effect when used in combination with compounds **3, 80, 93, 144, 168, 169, 186,** or **187** of the invention (Table 9).

**Table 9. Examples of X-ray radiation potency increase with compounds 3, 80, 93, 144, 168, 169, 186, 187 of the invention on TLM1.**

| **Compound** | **Activity^{a}** | **Compound** | **Activity^{a}** | **Compound** | **Activity^{a}** | **Compound** | **Activity^{a}** |
|---|---|---|---|---|---|---|---|
| **3** | 1.8 | **93** | 1.2 | **168** | 2.4 | **186** | 2.0 |
| **80** | 2.7 | **144** | 2.4 | **169** | 2.5 | **187** | 2.4 |

| | | | | | | | |
|---|---|---|---|---|---|---|---|
| ^{a} ratio of (corrected AUC of X-ray radiation in combination with compound of the invention (30 µM)/ corrected AUC of X-ray radiation alone) | | | | | | | |

### Effect of the combination of UV-C radiation and compounds of the invention on canine melanoma TLM1 cell line

Compounds **3** and **80** of the invention increase the potency of UV-C radiations: lower doses of UV-C radiations can be used to reach the same effect when used in combination with compounds **3** or **80** of the invention (Table 10).

**Table 10. Examples of UV-C radiation potency increase with compounds 3 and 80 of the invention on TLM1.**

| **Compound** | **Activity^{a}** |
|---|---|
| **3** | 1.3 |
| **80** | 2.3 |

| | |
|---|---|
| ^{a} TLM-1 cells were treated with 15 µM of compound of the invention 24h before the UV-C radiation and just after UV-C radiation. Activity refers to the ratio of the percentage of cells labelling with propidium iodide with pre-post treatment with the compound at 15 µM on the percentage of cells labelling with propidium iodide with pre-post treatment with vehicle, 48h after the UV-C radiations at 400 J/m². Results are the mean of ratio obtained in three independent experiments. | |

## Claims

1. A compound of Formula I: 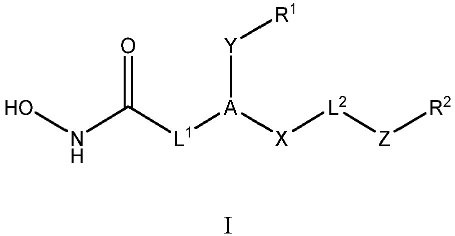
and pharmaceutically acceptable salts and solvates thereof,
wherein
**L¹** is inexistent or is selected from -CH₂- and -CH(OH)-;
**A** is CH or N;
**Y** is -CH₂- or -S-;
**R¹** is selected from aryl, heteroaryl, arylalkyl and arylalkenyl wherein said aryl, heteroaryl, arylalkyl or arylalkenyl is optionally substituted by one or more substituents independently selected from C1-C4-alkyl, halogen, C1-C4-haloalkyl, hydroxy-C1-C4-alkyl, acetylamino, acetylamino-C1-C4-alkyl and C1-C4-alkylaminocarboxyl;
**R²** is selected from aryl, heteroaryl, arylalkyl, cycloalkyl, heterocycloalkyl, C1-C6-alkyl, C1-C4-alkyloxycarbonyl, C1-C4-alkoxy, arylamino, wherein said aryl, arylalkyl, cycloalkyl or heterocycloalkyl is optionally substituted by one or more substituents independently selected from C1-C4-alkoxy, halogen, C1-C4-haloalkyl, C1-C4-alkyl, C1-C2-alkoxy-C1-C2-alkoxy, C1-C4-alkyloxycarbonyl, acetylamino, di(C1-C4-alkyl)amino-C1-C4-alkyl and hydroxycarbamoyl;
**X** is selected from 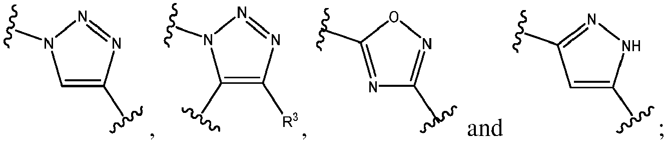
**R³** is selected from H and C(O)OR⁴;
**R⁴** is C1-C4-alkyl;
**L²** is linear or branched C1-C6-alkyl, optionally substituted by a group **R⁵**;
**R⁵** is selected from linear or branched C1-C4-alkyl, CH₂-OH, CHOH-CH₃, CH₂-C(O)NH₂, CH₂-CH₂-C(O)NH₂, CH₂-COOH, CH₂-CH₂-COOH, (CH₂)₄-NH₂, (CH₂)₄-NH-C(NH₂⁺)-NH₂, CH₂-Imidazolyl, CH₂-Indolyl, CH₂-SH, CH₂-CH₂-S-CH₃, CH₂-Ph, CH₂-Ph-OH, CH₂-OR⁶, CH₂-COOR⁶, CH₂-CH₂-COOR⁶, CH₂-SR⁶ and CH₂-Ph-OR⁶;
**R⁶** is selected from Me, Bn, Ph and Ac;
**Z** is selected from -NR⁷(CO)-, -NHSO₂-, -CH₂-CH₂-, -NH-, -NH-CH(C1-C6-alkyl)-, -O-CH₂-, -C(O)NH- and -O-; and
**R⁷** is selected from H, linear or branched C1-C6-alkyl and (1-methylimidazol-2-yl)-C1-C2-alkyl, or
**R⁷** and **L²** form together with the nitrogen atom they are attached to a 5- or 6-membered heterocyclyl group;
with the proviso that the compound of Formula I is none of the following:
4-fluoro-N-[1-(R-2-hydroxycarbamoyl-1-naphthalen-2-ylmethyl-ethyl)-1H-[1,2,3]triazol-4-ylmethyl]-benzamide,
N-[1-((R)-2-hydroxycarbamoyl-1-naphthalen-2-ylmethyl-ethyl)-1H-[1,2,3]triazol-4-ylmethyl]-benzamide,
N-[1-((R)-2-hydroxycarbamoyl-1-naphthalen-2-ylmethyl-ethyl)-1H-[1,2,3]triazol-4-ylmethyl] -4-methyl-benzamide,
N-[1-((R)-2-hydroxycarbamoyl-1-naphthalen-2-ylmethyl-ethyl)-1H-[1,2,3]triazol-4-ylmethyl]-4-methoxy-benzamide,
cyclohexanecarboxylic acid [1-(R-2-hydroxycarbamoyl-1-naphthalen-2-ylmethyl-ethyl)-1H-[1,2,3]triazol-4-ylmethyl]-amide,
(R)-N-hydroxy-4-naphthalen-2-yl-3-[4-(phenylacetylamino-methyl)-[1,2,3]triazol-1-yl]-butyramide,
(R)-N-hydroxy-4-naphthalen-2-yl-3-{4-[(2-p-tolyl-acetylamino)-methyl]-[1,2,3]triazol-1-yl}-butyramide,
(R)-N-hydroxy-4-naphthalen-2-yl-3-{4-[(3-phenyl-propionylamino)-methyl]-[1,2,3]triazol-1-yl}-butyramide,
(R)-3-{4-[(4-fluoro-benzenesulfonylamino)-methyl]-[1,2,3]triazol-1-yl}-N-hydroxy-4-naphthalen-2-yl-butyramide,
N-[1-(1-hydroxycarbamoylmethyl-2-phenyl-ethyl)-1H-[1,2,3]triazol-4-ylmethyl]-4-methyl-benzamide,
N-[1-(1-hydroxycarbamoylmethyl-2-phenyl-ethyl)-1H-[1,2,3]triazol-4-ylmethyl]-4-fluoro-benzamide,
N-[5-(1-hydroxycarbamoylmethyl-2-naphthalen-2-yl-ethyl)-[1,2,4]oxadiazol-3-ylmethyl]-benzamide,
4-fluoro-N-[5-(1-hydroxycarbamoylmethyl-2-naphthalen-2-yl-ethyl)-[1,2,4]oxadiazol-3-ylmethyl]-benzamide,
(R)-4-fluoro-N-[1-(1-hydroxycarbamoyl-2-naphthalen-2-yl-ethyl)-1H-[1,2,3]triazol-4-ylmethyl]-benzamide,
(S)-4-fluoro-N-[1-(2-hydroxycarbamoyl-1-naphthalen-2-ylmethyl-ethyl)-1H-[1,2,3]triazol-4-ylmethyl]-benzamide,
(R)-4-fluoro-N-[3-(1-hydroxycarbamoylmethyl-2-naphthalen-2-yl-ethyl)-3H-[1,2,3]triazol-4-ylmethyl]-benzamide,
(R)-cyclohexanecarboxylic acid [3-(1-hydroxycarbamoylmethyl-2-naphthalen-2-yl-ethyl)-3H-[1,2,3]triazol-4-ylmethyl]-amide, and
(R)-N-hydroxy-4-naphthalen-2-yl-3-{5-[(3-phenyl-propionylamino)-methyl]-[1,2,3]triazol-1-yl}-butyramide.

2. The compound according to claim 1, wherein **L¹** is -CH₂-.

3. The compound according to any of claims 1 and 2, wherein **A** is CH.

4. The compound according to any of claims 1 to 3, wherein **Y** is -CH₂-.

5. The compound according to any of claims 1 to 4, wherein **L²** is selected from -CH₂-, - CH₂-CH₂-, -C(CH₃)₂- and -CH(R⁵)-.

6. The compound according to any of claims 1 to 5, wherein **R⁵** is selected from CH₃, CH₂-OH, CH₂-Ph-OH and CH₂-Ph-OR⁶.

7. The compound according to any of claims 1 to 6, having Formula II: 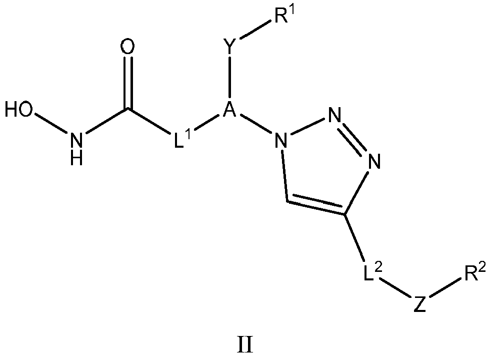 and pharmaceutically acceptable salts and solvates thereof,
wherein
**L¹**, **L²**, **A**, **Y**, **Z**, **R¹**, **R²**, **R⁵**, **R⁶** and **R⁷** are as defined in claim 1.

8. The compound according to any of claims 1 to 6, having Formula III: 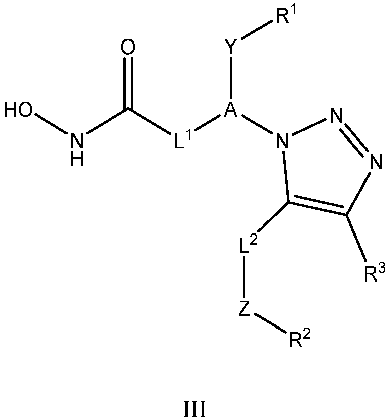 and pharmaceutically acceptable salts and solvates thereof,
wherein
**L¹**, **L²**, **A, Y, Z, R¹**, **R²**, **R³**, **R⁴**, **R⁵**, **R⁶** and **R⁷** are as defined in claim 1.

9. The compound according to any of claims 1 to 6, having Formula IV: 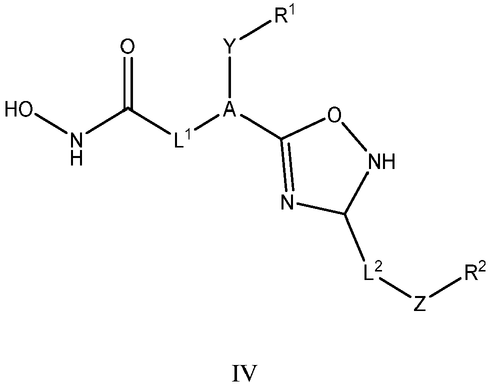 and pharmaceutically acceptable salts and solvates thereof,
wherein
**L¹**, **L²**, **A**, **Y**, **Z**, **R¹**, **R²**, **R⁵**, **R⁶** and **R⁷** are as defined in claim 1.

10. The compound according to any of claims 1 to 6, having Formula V: 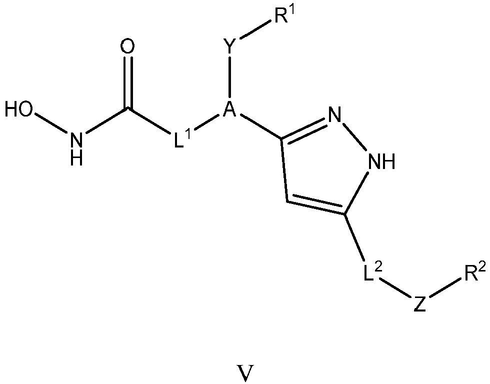 and pharmaceutically acceptable salts and solvates thereof,
wherein
**L¹**, **L²**, **A**, **Y**, **Z**, **R¹**, **R²**, **R⁵**, **R⁶** and **R⁷** are as defined in claim 1.

11. The compound according to any of claims 1 to 10, selected from the group consisting of:
N-[1-(R-2-Hydroxycarbamoyl-1-naphthalen-2-ylmethyl-ethyl)-1H-[1,2,3]triazol-4-ylmethyl]-4-trifluoromethyl-benzamide;
(R)-N-Hydroxy-4-naphthalen-2-yl-3-{4-[(toluene-4-sulfonylamino)-methyl]-[1,2,3]triazol-1-yl}-butyramide;
(R)-3-[4-(Benzenesulfonylamino-methyl)-[1,2,3]triazol-1-yl]-N-hydroxy-4-naphthalen-2-yl-butyramide;
(R)-3-{4-[(4-Fluoro-benzenesulfonylamino)-methyl]-[1,2,3]triazol-1-yl}-N-hydroxy-4-naphthalen-2-yl-butyramide;
(R)-N-[3-(1-Hydroxycarbamoylmethyl-2-naphthalen-2-yl-ethyl)-3H-[1,2,3]triazol-4-ylmethyl]-benzamide;
(R)-N-[1-(2-hydroxycarbamoyl-1-naphthalen-2-ylmethyl-ethyl)-1H-[1,2,3]triazol-4-ylmethyl] -N-methyl-benzamide;
(R)-4,4-difluoro-cyclohexanecarboxylic acid [1-(2-hydroxycarbamoyl-1-naphthalen-2-ylmethyl-ethyl)-1H-[1,2,3]triazol-4-ylmethyl]-amide;
(R)-3- {4-[(2-ethyl-butyrylamino)-methyl]-[1,2,3]triazol-1-yl}-N-hydroxy-4-naphthalen-2-yl-butyramide;
(R)-3-[4-(acetylamino-methyl)-[1,2,3]triazol-1-yl]-N-hydroxy-4-naphthalen-2-yl-butyramide;
(R)-3-{4-[(4-fluoro-phenylamino)-methyl]-[1,2,3]triazol-1-yl}-N-hydroxy-4-naphthalen-2-yl-butyramide;
*tert*-butyl ((1-((R)-4-(hydroxyamino)-1-(naphthalen-2-yl)-4-oxobutan-2-yl)-1H-1,2,3-triazol-4-yl)methyl)-L-leucinate;
(R)-N-hydroxy-4-naphthalen-2-yl-3-{4-[(3-phenyl-ureido)-methyl]-[1,2,3]triazol-1-yl}-butyramide;
(R)-[1-(2-hydroxycarbamoyl-1-naphthalen-2-ylmethyl-ethyl)-1H-[1,2,3]triazol-4-ylmethyl]-carbamic acid tert-butyl ester;
(R)-3-(4-benzyloxymethyl-[1,2,3]triazol-1-yl)-N-hydroxy-4-naphthalen-2-yl-butyramide;
(R)-3-[5-(acetylamino-methyl)-[1,2,3]triazol-1-yl]-N-hydroxy-4-naphthalen-2-yl-butyramide;
(R)-N-hydroxy-4-naphthalen-2-yl-3-{5-[(3-phenyl-propionylamino)-methyl]-[1,2,3]triazol-1-yl}-butyramide;
(R,S)-N-hydroxy-3-(5-{[2-(6-methoxy-naphthalen-2-yl)-propionylamino]-methyl}-[1,2,3]triazol-1-yl)-4-naphthalen-2-yl-butyramide;
(R)-4-fluoro-N-{2-[1-(1-hydroxycarbamoyl-2-naphthalen-2-yl-ethyl)-1H-[1,2,3]triazol-4-yl]-ethyl}-benzamide;
(R)-N-{1-[1-(4-bromo-benzyl)-2-hydroxycarbamoyl-ethyl]-1H-[1,2,3]triazol-4-ylmethyl } -4-fluoro-benzamide;
(R)-N-[1-(1-biphenyl-4-ylmethyl-2-hydroxycarbamoyl-ethyl)-1H-[1,2,3]triazol-4-ylmethyl]-4-fluoro-benzamide;
(R)-4-fluoro-N-{1-[2-hydroxycarbamoyl-1-(4'-hydroxymethyl-biphenyl-4-ylmethyl)-ethyl]-1H-[1,2,3]triazol-4-ylmethyl}-benzamide;
(R)-4'-(2-{4-[(4-fluoro-benzoylamino)-methyl]-[1,2,3]triazol-1-yl}-3-hydroxycarbamoyl-propyl)-biphenyl-4-carboxylic acid methylamide;
(R)-N-{1-[1-(4'-acetylamino-biphenyl-4-ylmethyl)-2-hydroxycarbamoyl-ethyl]-1H-[1,2,3]triazol-4-ylmethyl}-4-fluoro-benzamide;
(R)-N-(1-{1-[4'-(acetylamino-methyl)-biphenyl-4-ylmethyl]-2-hydroxycarbamoyl-ethyl}-1H-[1,2,3]triazol-4-ylmethyl)-4-fluoro-benzamide;
(R)-4-fluoro-N-{1-[2-hydroxycarbamoyl-1-(3'-hydroxymethyl-biphenyl-4-ylmethyl)-ethyl]-1H-[1,2,3]triazol-4-ylmethyl}-benzamide;
(R)-4'-(2-{4-[(4-fluoro-benzoylamino)-methyl]-[1,2,3]triazol-1-yl}-3-hydroxycarbamoyl-propyl)-biphenyl-3-carboxylic acid methylamide;
(R)-N-{1-[1-(3'-acetylamino-biphenyl-4-ylmethyl)-2-hydroxycarbamoyl-ethyl]-1H-[1,2,3]triazol-4-ylmethyl}-4-fluoro-benzamide;
(R)-formic acid 4'-(2-{4-[(4-fluoro-benzoylamino)-methyl]-[1,2,3]triazol-1-yl}-3-hydroxycarbamoyl-propyl)-biphenyl-3-ylmethyl ester;
(R)-4-fluoro-N-{1-[2-hydroxycarbamoyl-1-(4-pyrimidin-5-yl-benzyl)-ethyl]-1H-[1,2,3]triazol-4-ylmethyl}-benzamide;
4-{[1-(2-hydroxycarbamoyl-1-naphthalen-2-ylmethyl-ethyl)-1H-[1,2,3]triazol-4-ylmethyl]-carbamoyl}-piperidine-1-carboxylic acid tert-butyl ester;
4-fluoro-N-[5-(1-hydroxycarbamoyl-2-naphthalen-2-yl-ethyl)-[1,2,4]oxadiazol-3-ylmethyl]-benzamide;
Ethyl 5-[[(4-fluorobenzoyl)amino]methyl]-3-[(1R)-3-(hydroxyamino)-1-(2-naphthylmethyl)-3-oxo-propyl]triazole-4-carboxylate;
2,4-Difluoro-N-[1-(2-hydroxycarbamoyl-1-naphthalen-2-ylmethyl-ethyl)-1H-[1,2,3]triazol-4-ylmethyl]-benzamide;
(R)-N-{1-[1-(3-bromo-benzyl)-2-hydroxycarbamoyl-ethyl]-1H-[1,2,3]triazol-4-ylmethyl } -4-fluoro-benzamide;
(R)-N-[1-(1-biphenyl-3-ylmethyl-2-hydroxycarbamoyl-ethyl)-1H-[1,2,3]triazol-4-ylmethyl]-4-fluoro-benzamide;
(R)-N-{1-[1-(4'-acetylamino-biphenyl-3 -ylmethyl)-2-hydroxycarbamoyl-ethyl] -1H-[1,2,3]triazol-4-ylmethyl]-4-fluoro-benzamide;
(R)-4-fluoro-N-{1-[2-hydroxycarbamoyl-1-(4'-hydroxymethyl-biphenyl-3-ylmethyl)-ethyl]-1H-[1,2,3]triazol-4-ylmethyl}-benzamide;
(R)-N-{1-[1-(3'-acetylamino-biphenyl-3-ylmethyl)-2-hydroxycarbamoyl-ethyl]-1H-[1,2,3]triazol-4-ylmethyl}-4-fluoro-benzamide;
(R)-4-fluoro-N-{1-[2-hydroxycarbamoyl-1-(3'-hydroxymethyl-biphenyl-3-ylmethyl)-ethyl]-1H-[1,2,3]triazol-4-ylmethyl}-benzamide;
4-fluoro-N-((3-((2-(hydroxyamino)-2-oxoethyl)(naphthalen-2-ylmethyl)amino)-1H-pyrazol-5-yl)methyl)benzamide;
3,4-difluoro-N-[[1-[(1R)-3-(hydroxyamino)-1-(2-naphthylmethyl)-3-oxopropyl]triazol-4-yl]methyl]benzamide;
4-[(dimethylamino)methyl]-N-[[1-[(1R)-3-(hydroxyamino)-1-(2-naphthylmethyl)-3-oxo-propyl]triazol-4-yl]methyl]benzamide;
4-fluoro-N-[2-[1-[(1R)-3-(hydroxyamino)-1-(2-naphthylmethyl)-3-oxopropyl]triazol-4-yl]ethyl]benzamide);
3,4-difluoro-N-[2-[1-[(1R)-3-(hydroxyamino)-1-(2-naphthylmethyl)-3-oxopropyl]triazol-4-yl]ethyl]benzamide;
3-{4-[(4-Fluoro-phenylcarbamoyl)-methyl]-[1,2,3]triazol-1-yl}-N-hydroxy-4-naphthalen-2-yl-butyramide;
3,4-difluoro-N-((1-(3-(hydroxyamino)-1-(naphthalen-2-ylthio)-3-oxopropyl)-1H-1,2,3-triazol-4-yl)methyl)benzamide;
N-[[1-[(1R)-3-(hydroxyamino)-1-(2-naphthylmethyl)-3-oxo-propyl]triazol-4-yl]methyl]-3,4-dimethoxy-benzamide;
N-[[1-[(1R)-3-(hydroxyamino)-1-(2-naphthylmethyl)-3-oxo-propyl]triazol-4-yl]methyl]-1,3-benzodioxole-5-carboxamide;
3-chloro-4-fluoro-N-[[1-[(1R)-3-(hydroxyamino)-1-(2-naphthylmethyl)-3-oxopropyl]triazol-4-yl]methyl]benzamide;
2,3,4-Trifluoro-N-[1-(1-hydroxycarbamoylmethyl-2-naphthalen-2-yl-ethyl)-1H-[1,2,3]triazol-4-ylmethyl] -benzamide;
methyl(3R)-3-[4-[[(3,4-difluorobenzoyl)amino]methyl]triazol-1-yl]-4-(1-naphthyl)butanoate;
N-[[1-[(1R)-1-[(4-chlorophenyl)methyl]-3-(hydroxyamino)-3-oxo-propyl]triazol-4-yl]methyl]-3,4-difluoro-benzamide;
N-[[1-[(1R)-3-(hydroxyamino)-1-(2-naphthylmethyl)-3-oxo-propyl]triazol-4-yl]methyl]pyrimidine-4-carboxamide;
N-[[1-[1-(4-chlorophenyl)sulfanyl-3-(hydroxyamino)-3-oxo-propyl]triazol-4-yl]methyl]-3,4-difluoro-benzamide;
N-[[1-[(1R)-3-(hydroxyamino)-1-(2-naphthylmethyl)-3-oxo-propyl]triazol-4-yl]methyl]-4-(2-methoxyethoxy)benzamide;
N-[[1-[(1R)-3-(hydroxyamino)-1-(2-naphthylmethyl)-3-oxo-propyl]triazol-4-yl]methyl]-1-methyl-imidazole-4-carboxamide;
3,4-difluoro-N-[[1-[(1R)-3-(hydroxyamino)-3-oxo-1-(2-phenylethyl)propyl]triazol-4-yl]methyl]benzamide;
tert-butyl 3-[[1-[(1R)-3-(hydroxyamino)-1-(2-naphthylmethyl)-3-oxo-propyl]triazol-4-yl]methylcarbamoyl]morpholine-4-carboxylate;
2-chloro-4-fluoro-N-[[1-[(1R)-3-(hydroxyamino)-1-(2-naphthylmethyl)-3-oxopropyl]triazol-4-yl]methyl]benzamide;
4-hydroxy-N-[[1-[(1R)-3-(hydroxyamino)-1-(2-naphthylmethyl)-3-oxopropyl]triazol-4-yl]methyl]benzamide;
3-{4-[(3,4-Difluoro-phenylcarbamoyl)-methyl]-[1,2,3]triazol-1-yl}-N-hydroxy-4-naphthalen-2-yl-butyramide;
3,4-difluoro-N-[[1-[(E,1R)-1-[2-(hydroxyamino)-2-oxo-ethyl]-4-phenyl-but-3-enyl] triazol-4-yl] methyl] benzamide;
3,4-difluoro-N-[[1-[(1R)-3-(hydroxyamino)-3-oxo-1-[[4-(trifluoromethyl)phenyl]methyl]propyl]triazol-4-yl]methyl]benzamide;
3,4-Difluoro-N-{1-[1-(1-hydroxycarbamoylmethyl-2-naphthalen-2-yl-ethyl)-1H-[1,2,3]triazol-4-yl]-1-methyl-ethyl}-benzamide;
3,4-difluoro-N-[1-[1-[3-(hydroxyamino)-1-(2-naphthylmethyl)-3-oxo-propyl]triazol-4-yl]ethyl]benzamide;
3-[4-(2-Acetylamino-phenoxymethyl)-[1,2,3]triazol-1-yl]-N-hydroxy-4-naphthalen-2-yl-butyramide;
4-fluoro-N-[[1-[(1R)-3-(hydroxyamino)-1-(2-naphthylmethyl)-3-oxo-propyl]triazol-4-yl]methyl]-3-methyl-benzamide;
N-(3,4-difluorophenyl)-3-[1-[3-(hydroxyamino)-1-(2-naphthylmethyl)-3-oxo-propyl]triazol-4-yl]propanamide;
N-[[1-[(1S)-1-(3,4-dihydro-1H-isoquinolin-2-ylmethyl)-3-(hydroxyamino)-3-oxo-propyl]triazol-4-yl]methyl]-3,4-difluoro-benzamide;
3,4-difluoro-N-[[1-[(1R)-3-(hydroxyamino)-1-(1H-indol-3-ylmethyl)-3-oxo-propyl]triazol-4-yl]methyl]benzamide;
3,4-difluoro-N-[[1-[(1R,2S)-2-hydroxy-3-(hydroxyamino)-1-(2-naphthylmethyl)-3-oxo-propyl]triazol-4-yl]methyl]benzamide;
3,4-Difluoro-N-{1-[1-(1(R)-hydroxycarbamoylmethyl-2-naphthalen-2-yl-ethyl)-1H-[1,2,3]triazol-4-yl]-(S)-ethyl}-benzamide;
3,4-Difluoro-N-{1-[1-(1(R)-hydroxycarbamoylmethyl-2-naphthalen-2-yl-ethyl)-1H-[1,2,3]triazol-4-yl]-(R)-ethyl}-benzamide;
Methyl (3R)-3-[4-[[(3,4-difluorobenzoyl)-methyl-amino]methyl]triazol-1-yl]-4-(2-naphthyl)butanoate;
(3R)-3-[4-[(2S)-1-(3,4-difluorobenzoyl)pyrrolidin-2-yl]triazol-1-yl]-4-(1H-indol-3-yl)butanehydroxamic acid;
N-[2-(4-benzyloxyphenyl)-1-[1-[(1R)-3-(hydroxyamino)-1-(1H-indol-3-ylmethyl)-3-oxo-propyl]triazol-4-yl]ethyl]-3,4-difluoro-benzamide;
(3R)-3-[4-[4-(3,4-difluorobenzoyl)morpholin-3-yl]triazol-1-yl]-4-(1H-indol-3-yl)butanehydroxamic acid;
3,4-difluoro-N-[1-[1-[(1R)-3-(hydroxyamino)-1-(1H-indol-3-ylmethyl)-3-oxo-propyl]triazol-4-yl]ethyl]benzamide;
3,4-difluoro-N-[(1R)-2-hydroxy-1-[1-[(1R)-3-(hydroxyamino)-1-(1H-indol-3-ylmethyl)-3-oxo-propyl]triazol-4-yl]ethyl]benzamide;
(3R)-3-[4-[(2S)-1-(3,4-difluorobenzoyl)pyrrolidin-2-yl]triazol-1-yl]-4-(2-naphthyl)butanehydroxamic acid;
N-[2-(4-benzyloxyphenyl)-1-[1-[(1R)-3-(hydroxyamino)-1-(2-naphthylmethyl)-3-oxo-propyl]triazol-4-yl]ethyl]-3,4-difluoro-benzamide;
(3R)-3-[4-[4-(3,4-difluorobenzoyl)morpholin-3-yl]triazol-1-yl]-4-(2-naphthyl)butanehydroxamic acid;
3,4-difluoro-N-[(1R)-2-hydroxy-1-[1-[(1R)-3-(hydroxyamino)-1-(2-naphthylmethyl)-3-oxo-propyl]triazol-4-yl]ethyl]benzamide;
3,4-difluoro-N-[[1-[(1R)-3-(hydroxyamino)-1-(1H-indol-3-ylmethyl)-3-oxo-propyl]triazol-4-yl]methyl]-N-methyl-benzamide;
3,4-difluoro-N-[[1-[(1R)-3-(hydroxyamino)-1-(1H-indol-3-ylmethyl)-3-oxo-propyl]triazol-4-yl]methyl]-N-isobutyl-benzamide;
3,4-difluoro-N-[[1-[(1R)-3-(hydroxyamino)-1-(1H-indol-3-ylmethyl)-3-oxo-propyl]triazol-4-yl]methyl]-N-isopentyl-benzamide;
3,4-difluoro-N-[[1-[(1R)-3-(hydroxyamino)-1-(1H-indol-3-ylmethyl)-3-oxo-propyl]triazol-4-yl]methyl]-N-[(1-methylimidazol-2-yl)methyl]benzamide ;
methyl (3R)-4-(2-naphthyl)-3-[4-[(pyridine-4-carbonylamino)methyl]triazol-1-yl]butanoate;
N-[[1-[(1R)-3-(hydroxyamino)-1-(2-naphthylmethyl)-3-oxo-propyl]triazol-4-yl]methyl]-4-(hydroxycarbamoyl)benzamide;
3,4-difluoro-N-[[1-[(1R)-3-(hydroxyamino)-3-oxo-1-[[3-(trifluoromethyl)phenyl]methyl]propyl]triazol-4-yl]methyl]benzamide;
N-({1-[(2R)-1-[4-(2-tert-butyl-2H-1,2,3,4-tetrazol-5-yl)phenyl]-3-(hydroxycarbamoyl)propan2-yl]-1H-1,2,3-triazol-4-yl}methyl)-3,4-difluorobenzamide;
(R)-3,4-difluoro-N-((1-(4-(hydroxyamino)-4-oxo-1-(quinolin-3-yl)butan-2-yl)-1H-1,2,3-triazol-4-yl)methyl)benzamide;
(R)-3,4-difluoro-N-((1-(4-(hydroxyamino)-4-oxo-1-(5,6,7,8-tetrahydronaphthalen-2-yl)butan-2-yl)-1H-1,2,3-triazol-4-yl)methyl)benzamide;
3,4-Difluoro-N -[1-[1-[(1R)-3-(hydroxyamino)-1-(1H-indol-3-ylmethyl)-3-oxo-propyl]triazol-4-yl]-2-(4-hydroxyphenyl)ethyl]benzamide;
4-fluoro-N-(1-(1-((R)-4-(hydroxyamino)-1-(naphthalen-2-yl)-4-oxobutan-2-yl)-1H-1,2,3-triazol-4-yl)ethyl)benzamide;
(R)-4-fluoro-N-((1-(4-(hydroxyamino)-4-oxo-1-(5,6,7,8-tetrahydronaphthalen-2-yl)butan-2-yl)-1H-1,2,3-triazol-4-yl)methyl)benzamide;
(R)-4-fluoro-N-((1-(4-(hydroxyamino)-4-oxo-1-(5,6,7,8-tetrahydronaphthalen-2-yl)butan-2-yl)-1H-1,2,3-triazol-4-yl)methyl)-N-methylbenzamide;
4-fluoro-N-(1-(1-((R)-4-(hydroxyamino)-4-oxo-1-(5,6,7,8-tetrahydronaphthalen-2-yl)butan-2-yl)-1H-1,2,3-triazol-4-yl)ethyl)benzamide;
3,4-difluoro-N-(1-(1-((R)-4-(hydroxyamino)-4-oxo-1-(5,6,7,8-tetrahydronaphthalen-2-yl)butan-2-yl)-1H-1,2,3-triazol-4-yl)ethyl)benzamide;
(R)-3,4-difluoro-N-((1-(4-(hydroxyamino)-4-oxo-1-(5,6,7,8-tetrahydronaphthalen-2-yl)butan-2-yl)-1H-1,2,3-triazol-4-yl)methyl)-N-methylbenzamide;
(R)-3,4-difluoro-N-((1-(4-(hydroxyamino)-4-oxo-1-(quinolin-7-yl)butan-2-yl)-1H-1,2,3-triazol-4-yl)methyl)benzamide;
(R)-3,4-difluoro-N-((1-(4-(hydroxyamino)-4-oxo-1-(2-oxo-1,2,3,4-tetrahydroquinolin-6-yl)butan-2-yl)-1H-1,2,3-triazol-4-yl)methyl)benzamide;
4-fluoro-N-(1-(1-((R)-4-(hydroxyamino)-4-oxo-1-(5,5,8,8-tetramethyl-5,6,7,8-tetrahydronaphthalen-2-yl)butan-2-yl)-1H-1,2,3-triazol-4-yl)ethyl)benzamide;
(R)-N-((1-(1-(benzo[b]thiophen-5-yl)-4-(hydroxyamino)-4-oxobutan-2-yl)-1H-1,2,3-triazol-4-yl)methyl)-4-fluorobenzamide;
(R)-N-((1-(1-(benzo[b]thiophen-6-yl)-4-(hydroxyamino)-4-oxobutan-2-yl)-1H-1,2,3-triazol-4-yl)methyl)-4-fluorobenzamide;
(R)-3,4-difluoro-N-((1-(4-(hydroxyamino)-1-(1H-indol-5-yl)-4-oxobutan-2-yl)-1H-1,2,3-triazol-4-yl)methyl)benzamide;
(R)-4-fluoro-N-((1-(4-(hydroxyamino)-1-(1H-indol-6-yl)-4-oxobutan-2-yl)-1H-1,2,3-triazol-4-yl)methyl)benzamide;
(R)-4-fluoro-N-((1-(4-(hydroxyamino)-1-(1-methyl-1H-indol-5-yl)-4-oxobutan-2-yl)-1H-1,2,3-triazol-4-yl)methyl)benzamide;
(R)-N-((1-(1-(benzo[d]thiazol-6-yl)-4-(hydroxyamino)-4-oxobutan-2-yl)-1H-1,2,3-triazol-4-yl)methyl)-3,4-difluorobenzamide;
(R)-N-((1-(1-(benzo[b]thiophen-3-yl)-4-(hydroxyamino)-4-oxobutan-2-yl)-1H-1,2,3-triazol-4-yl)methyl)-3,4-difluorobenzamide;
(R)-N-((1-(1-(benzo[b]thiophen-3-yl)-4-(hydroxyamino)-4-oxobutan-2-yl)-1H-1,2,3-triazol-4-yl)methyl)-4-fluorobenzamide;
N-(1-(1-((R)-1-(benzo[b]thiophen-3-yl)-4-(hydroxyamino)-4-oxobutan-2-yl)-1H-1,2,3-triazol-4-yl)ethyl)-4-fluorobenzamide;
(S)-N-((1-(1-(benzo[b]thiophen-2-yl)-4-(hydroxyamino)-4-oxobutan-2-yl)-1H-1,2,3-triazol-4-yl)methyl)-4-fluorobenzamide;
(R)-N-((1-(1-(2,3-dihydrobenzo[b][1,4]dioxin-6-yl)-4-(hydroxyamino)-4-oxobutan-2-yl)-1H-1,2,3-triazol-4-yl)methyl)-3,4-difluorobenzamide;
(R)-3-fluoro-N-((1-(4-(hydroxyamino)-1-(naphthalen-2-yl)-4-oxobutan-2-yl)-1H-1,2,3-triazol-4-yl)methyl)benzamide;
(R)-4-fluoro-N-((1-(4-(hydroxyamino)-1-(naphthalen-2-yl)-4-oxobutan-2-yl)-1H-1,2,3-triazol-4-yl)methyl)-N-methylbenzamide;
(3R)-3-(4-(3-(4-fluorobenzoyl)thiazolidin-4-yl)-1H-1,2,3-triazol-1-yl)-N-hydroxy-4-(naphthalen-2-yl)butanamide; and
(3R)-3-(4-(1-(4-fluorobenzoyl)piperidin-2-yl)-1H-1,2,3-triazol-1-yl)-N-hydroxy-4-(naphthalen-2-yl)butanamide.

12. A pharmaceutical composition comprising a compound according to any of claims 1 to 11 or a pharmaceutically acceptable salt or solvate thereof and at least one pharmaceutically acceptable carrier, diluent, excipient and/or adjuvant.

13. A compound according to any of Claims 1 to 11 or a pharmaceutically acceptable salt or solvate thereof for use as a medicament.

14. A compound of Formula I: 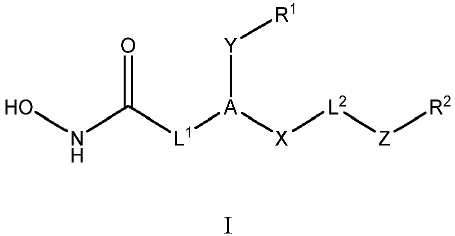 or a pharmaceutically acceptable salt or solvate thereof for use in treating and/or preventing cancer,
wherein
L¹ is inexistent or is selected from -CH₂- and -CH(OH)-;
A is CH or N;
Y is -CH₂- or -S-;
**R¹** is selected from aryl, heteroaryl, arylalkyl and arylalkenyl wherein said aryl, heteroaryl, arylalkyl or arylalkenyl is optionally substituted by one or more substituents independently selected from C1-C4-alkyl, halogen, C1-C4-haloalkyl, hydroxy-C1-C4-alkyl, acetylamino, acetylamino-C1-C4-alkyl and C1-C4-alkylaminocarboxyl;
**R²** is selected from aryl, heteroaryl, arylalkyl, cycloalkyl, heterocycloalkyl, C1-C6-alkyl, C1-C4-alkyloxycarbonyl, C1-C4-alkoxy, arylamino, wherein said aryl, arylalkyl, cycloalkyl or heterocycloalkyl is optionally substituted by one or more substituents independently selected from C1-C4-alkoxy, halogen, C1-C4-haloalkyl, C1-C4-alkyl, C1-C2-alkoxy-C1-C2-alkoxy, C1-C4-alkyloxycarbonyl, acetylamino, di(C1-C4-alkyl)amino-C1-C4-alkyl and hydroxycarbamoyl;
X is selected from 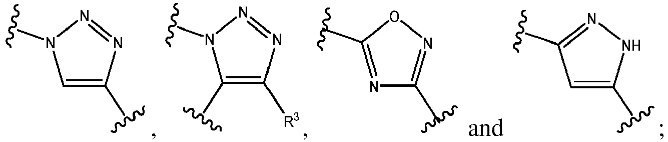
**R³** is selected from H and C(O)OR⁴;
**R⁴** is C1-C4-alkyl;
**L²** is linear or branched C1-C6-alkyl, optionally substituted by a group **R⁵**;
**R⁵** is selected from linear or branched C1-C4-alkyl, CH₂-OH, CHOH-CH₃, CH₂-C(O)NH₂, CH₂-CH₂-C(O)NH₂, CH₂-COOH, CH₂-CH₂-COOH, (CH₂)₄-NH₂, (CH₂)₄-NH-C(NH₂⁺)-NH₂, CH₂-Imidazolyl, CH₂-Indolyl, CH₂-SH, CH₂-CH₂-S-CH₃, CH₂-Ph, CH₂-Ph-OH, CH₂-OR⁶, CH₂-COOR⁶, CH₂-CH₂-COOR⁶, CH₂-SR⁶ and CH₂-Ph-OR⁶;
**R⁶** is selected from Me, Bn, Ph and Ac;
**Z** is selected from -NR⁷(CO)-, -NHSO₂-, -CH₂-CH₂-, -NH-, -NH-CH(C1-C6-alkyl)-, -O-CH₂-, -C(O)NH- and -O-; and
**R⁷** is selected from H, linear or branched C1-C6-alkyl and (1-methylimidazol-2-yl)-C1-C2-alkyl, or
**R⁷** and **L²** form together with the nitrogen atom they are attached to a 5- or 6-membered heterocyclyl group.

15. The compound for use according to claim 14, wherein the compound is a sensitizer for chemotherapy of malignant tumors.

16. The compound for use according to claim 15, wherein the chemotherapy of malignant tumors involves a proteasome inhibitor, a HDAC inhibitor or a protein glycosylation inhibitor.

17. The compound for use according to any of claims 14 to 16, wherein the cancer is selected from the group consisting of multiple myeloma, cervical cancer and breast cancer.

18. The compound for use according to any of claims 14 to 17, wherein the compound is selected from the group consisting of:
N-[1-((R)-2-Hydroxycarbamoyl-1-naphthalen-2-ylmethyl-ethyl)-1H-[1,2,3]triazol-4-ylmethyl]-benzamide;
N-[1-((R)-2-Hydroxycarbamoyl-1-naphthalen-2-ylmethyl-ethyl)-1H-[1,2,3]triazol-4-ylmethyl]-4-methoxy-benzamide;
4-Fluoro-N-[1-(R-2-hydroxycarbamoyl-1-naphthalen-2-ylmethyl-ethyl)-1H-[1,2,3]triazol-4-ylmethyl]-benzamide;
N-[1-(R-2-Hydroxycarbamoyl-1-naphthalen-2-ylmethyl-ethyl)-1H-[1,2,3]triazol-4-ylmethyl]-4-trifluoromethyl-benzamide;
(R)-N-Hydroxy-4-naphthalen-2-yl-3-{4-[(toluene-4-sulfonylamino)-methyl]-[1,2,3]triazol-1-yl}-butyramide;
(R)-3-{4-[(4-Fluoro-benzenesulfonylamino)-methyl]-[1,2,3]triazol-1-yl}-N-hydroxy-4-naphthalen-2-yl-butyramide;
(R)-3-[4-(Benzenesulfonylamino-methyl)-[1,2,3]triazol-1-yl]-N-hydroxy-4-naphthalen-2-yl-butyramide;
(R)-N-Hydroxy-4-naphthalen-2-yl-3-{4-[(2-p-tolyl-acetylamino)-methyl]-[1,2,3]triazol-1-yl}-butyramide;
(R)-3 - {4- [(4-Fluoro-benzenesulfonylamino)-methyl] - [1,2,3]triazol-1-yl} -N-hydroxy-4-naphthalen-2-yl-butyramide;
(R)-N-Hydroxy-4-naphthalen-2-yl-3-{4-[(3-phenyl-propionylamino)-methyl]-[1,2,3]triazol-1-yl}-butyramide;
(R)-4-Fluoro-N-[3-(1-hydroxycarbamoylmethyl-2-naphthalen-2-yl-ethyl)-3H-[1,2,3]triazol-4-ylmethyl] -benzamide;
(R)-N-[3-(1-Hydroxycarbamoylmethyl-2-naphthalen-2-yl-ethyl)-3H-[1,2,3]triazol-4-ylmethyl]-benzamide;
(R)-Cyclohexanecarboxylic acid [3-(1-hydroxycarbamoylmethyl-2-naphthalen-2-yl-ethyl)-3H-[1,2,3]triazol-4-ylmethyl]-amide;
(R)-N-[1-(2-hydroxycarbamoyl-1-naphthalen-2-ylmethyl-ethyl)-1H-[1,2,3]triazol-4-ylmethyl] -N-methyl-benzamide;
(R)-4,4-difluoro-cyclohexanecarboxylic acid [1-(2-hydroxycarbamoyl-1-naphthalen-2-ylmethyl-ethyl)-1H-[1,2,3]triazol-4-ylmethyl]-amide;
(R)-N-hydroxy-4-naphthalen-2-yl-3-[4-(phenylacetylamino-methyl)-[1,2,3]triazol-1-yl]-butyramide;
(R)-3- {4- [(2-ethyl-butyrylamino)-methyl] - [1,2,3]triazol-1-yl} -N-hydroxy-4-naphthalen-2-yl-butyramide;
(R)-3-[4-(acetylamino-methyl)-[1,2,3]triazol-1-yl]-N-hydroxy-4-naphthalen-2-yl-butyramide;
(R)-3 - {4- [(4-fluoro-phenylamino)-methyl] -[1,2,3] triazol-1-yl} -N-hydroxy-4-naphthalen-2-yl-butyramide;
*tert-butyl* ((1-((R)-4-(hydroxyamino)-1-(naphthalen-2-yl)-4-oxobutan-2-yl)-1H-1,2,3-triazol-4-yl)methyl)-L-leucinate;
(R)-N-hydroxy-4-naphthalen-2-yl-3-{4-[(3-phenyl-ureido)-methyl]-[1,2,3]triazol-1-yl}-butyramide;
(R)-[1-(2-hydroxycarbamoyl-1-naphthalen-2-ylmethyl-ethyl)-1H-[1,2,3]triazol-4-ylmethyl]-carbamic acid tert-butyl ester;
(R)-3-(4-benzyloxymethyl-[1,2,3]triazol-1-yl)-N-hydroxy-4-naphthalen-2-yl-butyramide;
(R)-3-[5-(Acetylamino-methyl)-[1,2,3]triazol-1-yl]-N-hydroxy-4-naphthalen-2-yl-butyramide;
(R)-N-hydroxy-4-naphthalen-2-yl-3-{5-[(3-phenyl-propionylamino)-methyl]-[1,2,3]triazol-1-yl}-butyramide;
(R,S)-N-hydroxy-3-(5-{[2-(6-methoxy-naphthalen-2-yl)-propionylamino]-methyl}-[1,2,3]triazol-1-yl)-4-naphthalen-2-yl-butyramide;
4-fluoro-N-[5-(1-hydroxycarbamoylmethyl-2-naphthalen-2-yl-ethyl)-[1,2,4]oxadiazol-3-ylmethyl]-benzamide;
N-[5-(1-hydroxycarbamoylmethyl-2-naphthalen-2-yl-ethyl)-[1,2,4]oxadiazol-3-ylmethyl]-benzamide;
(R)-4-Fluoro-N-{2-[1-(1-hydroxycarbamoyl-2-naphthalen-2-yl-ethyl)-1H-[1,2,3]triazol-4-yl]-ethyl}-benzamide;
(S)-4-fluoro-N-[1-(2-hydroxycarbamoyl-1-naphthalen-2-ylmethyl-ethyl)-1H-[1,2,3]triazol-4-ylmethyl] -benzamide;
(R)-N-{1-[1-(4-bromo-benzyl)-2-hydroxycarbamoyl-ethyl]-1H-[1,2,3]triazol-4-ylmethyl } -4-fluoro-benzamide;
(R)-N-[1-(1-biphenyl-4-ylmethyl-2-hydroxycarbamoyl-ethyl)-1H-[1,2,3]triazol-4-ylmethyl]-4-fluoro-benzamide;
(R)-4-fluoro-N-{1-[2-hydroxycarbamoyl-1-(4'-hydroxymethyl-biphenyl-4-ylmethyl)-ethyl] -1H-[1,2,3] triazol-4-ylmethyl}-benzamide;
(R)-4'-(2- {4- [(4-fluoro-benzoylamino)-methyl] - [1,2,3]triazol-1-yl}-3-hydroxycarbamoyl-propyl)-biphenyl-4-carboxylic acid methylamide;
(R)-N-{1-[1-(4'-acetylamino-biphenyl-4-ylmethyl)-2-hydroxycarbamoyl-ethyl]-1H-[1,2,3]triazol-4-ylmethyl}-4-fluoro-benzamide;
(R)-N-(1-{1-[4'-(acetylamino-methyl)-biphenyl-4-ylmethyl]-2-hydroxycarbamoyl-ethyl}-1H-[1,2,3]triazol-4-ylmethyl)-4-fluoro-benzamide;
(R)-4-fluoro-N-{1-[2-hydroxycarbamoyl-1-(3'-hydroxymethyl-biphenyl-4-ylmethyl)-ethyl]-1H-[1,2,3]triazol-4-ylmethyl}-benzamide;
(R)-4'-(2- {4- [(4-fluoro-benzoylamino)-methyl]-[1,2,3]triazol-1-yl}-3-hydroxycarbamoyl-propyl)-biphenyl-3-carboxylic acid methylamide;
(R)-N-{1-[1-(3'-acetylamino-biphenyl-4-ylmethyl)-2-hydroxycarbamoyl-ethyl]-1H-[1,2,3]triazol-4-ylmethyl}-4-fluoro-benzamide;
(R)-Formic acid 4'-(2-{4-[(4-fluoro-benzoylamino)-methyl]-[1,2,3]triazol-1-yl}-3-hydroxycarbamoyl-propyl)-biphenyl-3-ylmethyl ester;
(R)-4-fluoro-N-{ 1-[2-hydroxycarbamoyl-1-(4-pyrimidin-5-yl-benzyl)-ethyl]-1H-[1,2,3]triazol-4-ylmethyl}-benzamide;
4-{[1-(2-Hydroxycarbamoyl-1-naphthalen-2-ylmethyl-ethyl)-1H-[1,2,3]triazol-4-ylmethyl]-carbamoyl}-piperidine-1-carboxylic acid tert-butyl ester;
(R)-4-Fluoro-N-[1-(1-hydroxycarbamoyl-2-naphthalen-2-yl-ethyl)-1H-[1,2,3]triazol-4-ylmethyl]-benzamide;
4-Fluoro-N-[5-(1-hydroxycarbamoyl-2-naphthalen-2-yl-ethyl)-[1,2,4]oxadiazol-3-ylmethyl]-benzamide;
Ethyl 5-[[(4-fluorobenzoyl)amino]methyl]-3-[(1R)-3-(hydroxyamino)-1-(2-naphthylmethyl)-3-oxo-propyl]triazole-4-carboxylate;
2,4-Difluoro-N-[1-(2-hydroxycarbamoyl-1-naphthalen-2-ylmethyl-ethyl)-1H-[1,2,3]triazol-4-ylmethyl] -benzamide;
(R)-N-{1-[1-(3-bromo-benzyl)-2-hydroxycarbamoyl-ethyl]-1H-[1,2,3]triazol-4-ylmethyl } -4-fluoro-benzamide;
(R)-N-[1-(1-biphenyl-3-ylmethyl-2-hydroxycarbamoyl-ethyl)-1H-[1,2,3]triazol-4-ylmethyl]-4-fluoro-benzamide;
(R)-N-{1-[1-(4'-acetylamino-biphenyl-3 -ylmethyl)-2-hydroxycarbamoyl-ethyl] -1H-[1,2,3]triazol-4-ylmethyl}-4-fluoro-benzamide;
(R)-4-fluoro-N-{1-[2-hydroxycarbamoyl-1-(4'-hydroxymethyl-biphenyl-3-ylmethyl)-ethyl]-1H-[1,2,3]triazol-4-ylmethyl}-benzamide;
(R)-N-{1-[1-(3'-acetylamino-biphenyl-3-ylmethyl)-2-hydroxycarbamoyl-ethyl]-1H-[1,2,3]triazol-4-ylmethyl}-4-fluoro-benzamide;
(R)-4-fluoro-N-{1-[2-hydroxycarbamoyl-1-(3'-hydroxymethyl-biphenyl-3-ylmethyl)-ethyl]-1H-[1,2,3]triazol-4-ylmethyl}-benzamide;
4-fluoro-N-((3-((2-(hydroxyamino)-2-oxoethyl)(naphthalen-2-ylmethyl)amino)-1H-pyrazol-5-yl)methyl)benzamide;
3,4-difluoro-N-[[1-[(1R)-3-(hydroxyamino)-1-(2-naphthylmethyl)-3-oxo-propyl]triazol-4-yl]methyl]benzamide;
4-[(dimethylamino)methyl]-N-[[1-[(1R)-3-(hydroxyamino)-1-(2-naphthylmethyl)-3-oxo-propyl]triazol-4-yl]methyl]benzamide;
4-fluoro-N-[2-[1-[(1R)-3-(hydroxyamino)-1-(2-naphthylmethyl)-3-oxo-propyl]triazol-4-yl]ethyl]benzamide);
3,4-difluoro-N-[2-[1-[(1R)-3-(hydroxyamino)-1-(2-naphthylmethyl)-3-oxo-propyl]triazol-4-yl]ethyl]benzamide;
3-{4-[(4-Fluoro-phenylcarbamoyl)-methyl]-[1,2,3]triazol-1-yl}-N-hydroxy-4-naphthalen-2-yl-butyramide;
3,4-difluoro-N-((1-(3-(hydroxyamino)-1-(naphthalen-2-ylthio)-3-oxopropyl)-1H-1,2,3-triazol-4-yl)methyl)benzamide;
N-[[1-[(1R)-3-(hydroxyamino)-1-(2-naphthylmethyl)-3-oxo-propyl]triazol-4-yl]methyl]-3,4-dimethoxy-benzamide;
N-[[1-[(1R)-3-(hydroxyamino)-1-(2-naphthylmethyl)-3-oxo-propyl]triazol-4-yl]methyl]-1,3-benzodioxole-5-carboxamide;
3-chloro-4-fluoro-N-[[1-[(1R)-3-(hydroxyamino)-1-(2-naphthylmethyl)-3-oxo-propyl]triazol-4-yl]methyl]benzamide;
2,3,4-Trifluoro-N-[1-(1-hydroxycarbamoylmethyl-2-naphthalen-2-yl-ethyl)-1H-[1,2,3]triazol-4-ylmethyl] -benzamide;
methyl(3R)-3-[4-[[(3,4-difluorobenzoyl)amino]methyl]triazol-1-yl]-4-(1-naphthyl)butanoate;
N-[[1-[(1R)-1-[(4-chlorophenyl)methyl]-3-(hydroxyamino)-3-oxo-propyl]triazol-4-yl]methyl]-3,4-difluoro-benzamide;
N-[[1-[(1R)-3-(hydroxyamino)-1-(2-naphthylmethyl)-3-oxo-propyl]triazol-4-yl]methyl]pyrimidine-4-carboxamide;
N-[[1-[1-(4-chlorophenyl)sulfanyl-3-(hydroxyamino)-3-oxo-propyl]triazol-4-yl]methyl]-3,4-difluoro-benzamide;
N-[[1-[(1R)-3-(hydroxyamino)-1-(2-naphthylmethyl)-3-oxo-propyl]triazol-4-yl]methyl]-4-(2-methoxyethoxy)benzamide;
N-[[1-[(1R)-3-(hydroxyamino)-1-(2-naphthylmethyl)-3-oxo-propyl]triazol-4-yl]methyl]-1-methyl-imidazole-4-carboxamide;
3,4-difluoro-N-[[1-[(1R)-3-(hydroxyamino)-3-oxo-1-(2-phenylethyl)propyl]triazol-4-yl]methyl]benzamide;
tert-butyl 3-[[1-[(1R)-3-(hydroxyamino)-1-(2-naphthylmethyl)-3 -oxo-propyl] triazol-4-yl]methylcarbamoyl]morpholine-4-carboxylate;
2-chloro-4-fluoro-N-[[1-[(1R)-3-(hydroxyamino)-1-(2-naphthylmethyl)-3-oxo-propyl]triazol-4-yl]methyl]benzamide;
4-hydroxy-N-[[1-[(1R)-3-(hydroxyamino)-1-(2-naphthylmethyl)-3-oxo-propyl]triazol-4-yl]methyl]benzamide;
3-{4-[(3,4-Difluoro-phenylcarbamoyl)-methyl]-[1,2,3]triazol-1-yl}-N-hydroxy-4-naphthalen-2-yl-butyramide;
3,4-difluoro-N-[[1-[(E,1R)-1-[2-(hydroxyamino)-2-oxo-ethyl]-4-phenyl-but-3-enyl] triazol-4-yl] methyl] benzamide;
3,4-difluoro-N-[[1-[(1R)-3-(hydroxyamino)-3-oxo-1-[[4-(trifluoromethyl)phenyl]methyl]propyl]triazol-4-yl]methyl]benzamide;
3,4-Difluoro-N-{1-[1-(1-hydroxycarbamoylmethyl-2-naphthalen-2-yl-ethyl)-1H-[1,2,3]triazol-4-yl]-1-methyl-ethyl}-benzamide;
3,4-difluoro-N-[1-[1-[3-(hydroxyamino)-1-(2-naphthylmethyl)-3-oxo-propyl]triazol-4-yl]ethyl]benzamide;
3-[4-(2-Acetylamino-phenoxymethyl)-[1,2,3]triazol-1-yl]-N-hydroxy-4-naphthalen-2-yl-butyramide;
4-fluoro-N-[[1-[(1R)-3-(hydroxyamino)-1-(2-naphthylmethyl)-3-oxo-propyl]triazol-4-yl]methyl]-3-methyl-benzamide;
N-(3,4-difluorophenyl)-3-[1-[3-(hydroxyamino)-1-(2-naphthylmethyl)-3-oxo-propyl]triazol-4-yl]propanamide;
N-[[1-[(1S)-1-(3,4-dihydro-1H-isoquinolin-2-ylmethyl)-3-(hydroxyamino)-3-oxo-propyl] triazol-4-yl] methyl] -3 ,4-difluoro-benzamide;
3,4-difluoro-N-[[1-[(1R)-3-(hydroxyamino)-1-(1H-indol-3-ylmethyl)-3-oxo-propyl]triazol-4-yl]methyl]benzamide;
3,4-difluoro-N-[[1-[(1R,2S)-2-hydroxy-3-(hydroxyamino)-1-(2-naphthylmethyl)-3-oxo-propyl]triazol-4-yl]methyl]benzamide;
3,4-Difluoro-N-{1-[1-(1(R)-hydroxycarbamoylmethyl-2-naphthalen-2-yl-ethyl)-1H-[1,2,3]triazol-4-yl]-(S)-ethyl}-benzamide;
3,4-Difluoro-N-{1-[1-(1(R)-hydroxycarbamoylmethyl-2-naphthalen-2-yl-ethyl)-1H-[1,2,3]triazol-4-yl]-(R)-ethyl}-benzamide;
Methyl (3R)-3-[4-[[(3,4-difluorobenzoyl)-methyl-amino]methyl]triazol-1-yl]-4-(2-naphthyl)butanoate;
(3R)-3-[4-[(2S)-1-(3,4-difluorobenzoyl)pyrrolidin-2-yl]triazol-1-yl]-4-(1H-indol-3-yl)butanehydroxamic acid;
N-[2-(4-benzyloxyphenyl)-1-[1-[(1R)-3-(hydroxyamino)-1-(1H-indol-3-ylmethyl)-3-oxo-propyl]triazol-4-yl]ethyl]-3,4-difluoro-benzamide;
(3R)-3-[4-[4-(3,4-difluorobenzoyl)morpholin-3-yl]triazol-1-yl]-4-(1H-indol-3-yl)butanehydroxamic acid;
3,4-difluoro-N-[1-[1-[(1R)-3-(hydroxyamino)-1-(1H-indol-3-ylmethyl)-3-oxo-propyl]triazol-4-yl]ethyl]benzamide;
3,4-difluoro-N-[(1R)-2-hydroxy-1-[1-[(1R)-3-(hydroxyamino)-1-(1H-indol-3-ylmethyl)-3-oxo-propyl]triazol-4-yl]ethyl]benzamide;
(3R)-3-[4-[(2S)-1-(3,4-difluorobenzoyl)pyrrolidin-2-yl]triazol-1-yl]-4-(2-naphthyl)butanehydroxamic acid;
N-[2-(4-benzyloxyphenyl)-1-[1-[(1R)-3-(hydroxyamino)-1-(2-naphthylmethyl)-3-oxo-propyl]triazol-4-yl]ethyl]-3,4-difluoro-benzamide;
(3R)-3-[4-[4-(3,4-difluorobenzoyl)morpholin-3-yl]triazol-1-yl]-4-(2-naphthyl)butanehydroxamic acid;
3,4-difluoro-N-[(1R)-2-hydroxy-1-[1-[(1R)-3-(hydroxyamino)-1-(2-naphthylmethyl)-3-oxo-propyl]triazol-4-yl]ethyl]benzamide;
3,4-difluoro-N-[[1-[(1R)-3-(hydroxyamino)-1-(1H-indol-3-ylmethyl)-3-oxo-propyl]triazol-4-yl]methyl]-N-methyl-benzamide;
3,4-difluoro-N-[[1-[(1R)-3-(hydroxyamino)-1-(1H-indol-3-ylmethyl)-3-oxo-propyl]triazol-4-yl]methyl]-N-isobutyl-benzamide;
3,4-difluoro-N-[[1-[(1R)-3-(hydroxyamino)-1-(1H-indol-3-ylmethyl)-3-oxo-propyl]triazol-4-yl]methyl]-N-isopentyl-benzamide;
3,4-difluoro-N-[[1-[(1R)-3-(hydroxyamino)-1-(1H-indol-3-ylmethyl)-3-oxo-propyl]triazol-4-yl]methyl]-N-[(1-methylimidazol-2-yl)methyl]benzamide;
N-[1-(1-Hydroxycarbamoylmethyl-2-phenyl-ethyl)-1H-[1,2,3]triazol-4-ylmethyl]-4-methyl-benzamide;
methyl (3R)-4-(2-naphthyl)-3-[4-[(pyridine-4-carbonylamino)methyl]triazol-1-yl]butanoate;
N-[[1-[(1R)-3-(hydroxyamino)-1-(2-naphthylmethyl)-3-oxo-propyl]triazol-4-yl]methyl]-4-(hydroxycarbamoyl)benzamide;
3,4-difluoro-N-[[1-[(1R)-3-(hydroxyamino)-3-oxo-1-[[3-(trifluoromethyl)phenyl]methyl]propyl]triazol-4-yl]methyl]benzamide;
N-({1-[(2R)-1-[4-(2-tert-butyl-2H-1,2,3,4-tetrazol-5-yl)phenyl]-3-(hydroxycarbamoyl)propan2-yl]-1H-1,2,3-triazol-4-yl}methyl)-3,4-difluorobenzamide;
(R)-3,4-difluoro-N-((1-(4-(hydroxyamino)-4-oxo-1-(quinolin-3-yl)butan-2-yl)-1H-1,2,3-triazol-4-yl)methyl)benzamide;
(R)-3,4-difluoro-N-((1-(4-(hydroxyamino)-4-oxo-1-(5,6,7,8-tetrahydronaphthalen-2-yl)butan-2-yl)-1H-1,2,3-triazol-4-yl)methyl)benzamide;
3,4-Difluoro-N-[1-[1-[(1R)-3-(hydroxyamino)-1-(1H-indol-3-ylmethyl)-3-oxo-propyl]triazol-4-yl]-2-(4-hydroxyphenyl)ethyl]benzamide;
4-fluoro-N-(1-(1-((R)-4-(hydroxyamino)-1-(naphthalen-2-yl)-4-oxobutan-2-yl)-1H-1,2,3-triazol-4-yl)ethyl)benzamide;
(R)-4-fluoro-N-((1-(4-(hydroxyamino)-4-oxo-1-(5,6,7,8-tetrahydronaphthalen-2-yl)butan-2-yl)-1H-1,2,3-triazol-4-yl)methyl)benzamide;
(R)-4-fluoro-N-((1-(4-(hydroxyamino)-4-oxo-1-(5,6,7,8-tetrahydronaphthalen-2-yl)butan-2-yl)-1H-1,2,3-triazol-4-yl)methyl)-N-methylbenzamide;
4-fluoro-N-(1-(1-((R)-4-(hydroxyamino)-4-oxo-1-(5,6,7,8-tetrahydronaphthalen-2-yl)butan-2-yl)-1H-1,2,3-triazol-4-yl)ethyl)benzamide;
3,4-difluoro-N-(1-(1-((R)-4-(hydroxyamino)-4-oxo-1-(5,6,7,8-tetrahydronaphthalen-2-yl)butan-2-yl)-1H-1,2,3-triazol-4-yl)ethyl)benzamide;
(R)-3,4-difluoro-N-((1-(4-(hydroxyamino)-4-oxo-1-(5,6,7,8-tetrahydronaphthalen-2-yl)butan-2-yl)-1H-1,2,3-triazol-4-yl)methyl)-N-methylbenzamide;
(R)-3,4-difluoro-N-((1-(4-(hydroxyamino)-4-oxo-1-(quinolin-7-yl)butan-2-yl)-1H-1,2,3-triazol-4-yl)methyl)benzamide;
(R)-3,4-difluoro-N-((1-(4-(hydroxyamino)-4-oxo-1-(2-oxo-1,2,3,4-tetrahydroquinolin-6-yl)butan-2-yl)-1H-1,2,3-triazol-4-yl)methyl)benzamide;
4-fluoro-N-(1-(1-((R)-4-(hydroxyamino)-4-oxo-1-(5,5,8,8-tetramethyl-5,6,7,8-tetrahydronaphthalen-2-yl)butan-2-yl)-1H-1,2,3-triazol-4-yl)ethyl)benzamide;
(R)-N-((1-(1-(benzo[b]thiophen-5-yl)-4-(hydroxyamino)-4-oxobutan-2-yl)-1H-1,2,3-triazol-4-yl)methyl)-4-fluorobenzamide;
(R)-N-((1-(1-(benzo[b]thiophen-6-yl)-4-(hydroxyamino)-4-oxobutan-2-yl)-1H-1,2,3-triazol-4-yl)methyl)-4-fluorobenzamide;
(R)-3,4-difluoro-N-((1-(4-(hydroxyamino)-1-(1H-indol-5-yl)-4-oxobutan-2-yl)-1H-1,2,3-triazol-4-yl)methyl)benzamide;
(R)-4-fluoro-N-((1-(4-(hydroxyamino)-1-(1H-indol-6-yl)-4-oxobutan-2-yl)-1H-1,2,3-triazol-4-yl)methyl)benzamide;
(R)-4-fluoro-N-((1-(4-(hydroxyamino)-1-(1-methyl-1H-indol-5-yl)-4-oxobutan-2-yl)-1H-1,2,3-triazol-4-yl)methyl)benzamide;
(R)-N-((1-(1-(benzo[d]thiazol-6-yl)-4-(hydroxyamino)-4-oxobutan-2-yl)-1H-1,2,3-triazol-4-yl)methyl)-3,4-difluorobenzamide;
(R)-N-((1-(1-(benzo[b]thiophen-3-yl)-4-(hydroxyamino)-4-oxobutan-2-yl)-1H-1,2,3-triazol-4-yl)methyl)-3,4-difluorobenzamide;
(R)-N-((1-(1-(benzo[b]thiophen-3-yl)-4-(hydroxyamino)-4-oxobutan-2-yl)-1H-1,2,3-triazol-4-yl)methyl)-4-fluorobenzamide;
N-(1-(1-((R)-1-(benzo[b]thiophen-3-yl)-4-(hydroxyamino)-4-oxobutan-2-yl)-1H-1,2,3-triazol-4-yl)ethyl)-4-fluorobenzamide;
(S)-N-((1-(1-(benzo[b]thiophen-2-yl)-4-(hydroxyamino)-4-oxobutan-2-yl)-1H-1,2,3-triazol-4-yl)methyl)-4-fluorobenzamide;
(R)-N-((1-(1-(2,3-dihydrobenzo[b][1,4]dioxin-6-yl)-4-(hydroxyamino)-4-oxobutan-2-yl)-1H-1,2,3-triazol-4-yl)methyl)-3,4-difluorobenzamide;
(R)-3-fluoro-N-((1-(4-(hydroxyamino)-1-(naphthalen-2-yl)-4-oxobutan-2-yl)-1H-1,2,3-triazol-4-yl)methyl)benzamide;
(R)-4-fluoro-N-((1-(4-(hydroxyamino)-1-(naphthalen-2-yl)-4-oxobutan-2-yl)-1H-1,2,3-triazol-4-yl)methyl)-N-methylbenzamide;
(3R)-3-(4-(3-(4-fluorobenzoyl)thiazolidin-4-yl)-1H-1,2,3-triazol-1-yl)-N-hydroxy-4-(naphthalen-2-yl)butanamide; and
(3R)-3-(4-(1-(4-fluorobenzoyl)piperidin-2-yl)-1H-1,2,3-triazol-1-yl)-N-hydroxy-4-(naphthalen-2-yl)butanamide.

19. The compound for use according to any of claims 14 to 18, wherein the compound is used in combination with radiation therapy.

## Patentansprüche

1. Verbindung der Formel I: 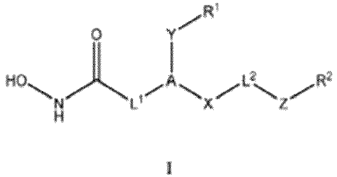 und pharmazeutisch annehmbare Salze und Solvate davon, wobei
**L¹** nicht vorhanden ist oder ausgewählt ist aus -CH₂- und -CH(OH)-,
**A** CH oder N ist,
Y -CH₂- oder -S- ist,
R¹ ausgewählt ist aus Aryl, Heteroaryl, Arylalkyl und Arylalkenyl, wobei das Aryl, Heteroaryl, Arylalkyl oder Arylalkenyl gegebenenfalls durch einen oder mehrere Substituenten substituiert ist, die unabhängig ausgewählt sind aus Cl-C4-Alkyl, Halogen, Cl-C4-Haloalkyl, Hydroxy-Cl-C4-Alkyl, Acetylamino, Acetylamino-Cl-C4-Alkyl und Cl-C4-Alkylaminocarboxyl,
**R²** ausgewählt ist aus Aryl, Heteroaryl, Arylalkyl, Cycloalkyl, Heterocycloalkyl, Cl-C6-Alkyl, Cl-C4-Alkyloxycarbonyl, Cl-C4-Alkoxy, Arylamino, wobei das Aryl, Arylalkyl, Cycloalkyl oder Heterocycloalkyl gegebenenfalls durch einen oder mehrere Substituenten substituiert ist, die unabhängig ausgewählt sind aus Cl-C4-Alkoxy, Halogen, Cl-C4-Haloalkyl, Cl-C4-Alkyl, Cl-C2-Alkoxy-Cl-C2-Alkoxy, Cl-C4-Alkyloxycarbonyl, Acetylamino, Di(Cl-C4-Alkyl)amino-C1-C4-alkyl und Hydroxycarbamoyl,
**X** ausgewählt ist aus 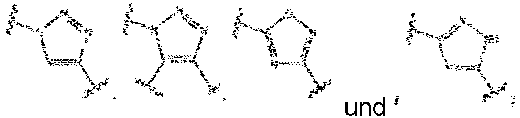
**R³** ausgewählt ist aus H und C(O)OR⁴,
**R⁴** Cl-C4-Alkyl ist,
**L²** lineares oder verzweigtes Cl-C6-Alkyl ist, das gegebenenfalls durch eine Gruppe **R⁵** substituiert ist,
**R⁵** ausgewählt ist aus linearem oder verzweigtem Cl-C4-Alkyl, CH₂-OH, CHOH-CH₃, CH₂-C(O)NH₂, CH₂-CH₂-C(O)NH₂, CH₂-COOH, CH₂-CH₂-COOH, (CH₂)₄-NH₂, (CH₂)₄- NH-C(NH₂⁺)-NH₂, CH₂-Imidazolyl, CH₂-Indolyl, CH₂-SH, CH₂-CH₂-S-CH₃, CH₂-Ph, CH₂-Ph-OH, CH₂-OR⁶, CH₂-COOR⁶, CH₂-CH₂-COOR⁶, CH₂-SR⁶ und CH₂-Ph-OR⁶,
**R⁶** ausgewählt ist aus Me, Bn, Ph und Ac,
**Z** ausgewählt ist aus -NR⁷(CO)-, -NHSO₂-, -CH₂-CH₂-, -NH-, -NH-CH(Cl-C6-alkyl)-, -O-CH₂-, -C(O)NH- und -O- und
**R** ausgewählt ist aus H, linearem oder verzweigtem Cl-C6-Alkyl und (I-Methylimidazol-2-yl)-Cl-C2-Alkyl oder
**R⁷** und **L²** zusammen mit dem Stickstoffatom, an das sie gebunden sind, eine 5- oder 6-gliedrige Heterocyclylgruppe bilden,
mit der Maßgabe, dass die Verbindung der Formel I keine der folgenden ist:
4-Fluor-N-[I-(R-2-hydroxycarbamoyl-1-naphthalin-2-ylmethyl-ethyl)-1H-[1,2,3]triazol-4-ylmethyl]-benzamid,
N-[I-((R)-2-Hydroxycarbamoyl-1-naphthalin-2-ylmethyl-ethyl)-1H-[1 ,2,3]triazol-4-ylmethyl]-benzamid,
N-[1-((R)-2-Hydroxycarbamoyl-1-naphthalin-2-ylmethyl-ethyl)-1H-[1,2,3]triazol-4-ylmethyl]-4-methyl-benzamid,
N-[1-((R)-2-Hydroxycarbamoyl-1-naphthalin-2-ylmethyl-ethyl)-1H-[1 ,2,3]triazol-4-ylmethyl]-4-methoxy-benzamid,
Cyclohexancarbonsäure [1-(R-2-hydroxycarbamoyl-1-naphthalin-2-ylmethyl-ethyl)-1H-[1,2,3]triazol-4-ylmethyl]-amid,
(R)-N-Hydroxy-4-naphthalin-2-yl-3-[4-(phenylacetylamino-methyl)-[1,2,3]triazol-1-yl]-butyramid,
(R)-N-Hydroxy-4-naphthalin-2-yl-3-{4-[(2-p-tolyl-acetylamino)-methyl]-[1,2,3]triazol-1-yl}-butyramid,
(R)-N-Hydroxy-4-naphthalin-2-yl-3-{4-[(3-phenyl-propionylamino)-methyl]-[1,2,3]triazol-1-yl}-butyramid,
(R)-3-{4-[(4-Fluor-benzolsulfonylamino)-methyl]-[1,2,3]triazol-1-yl}-N-hydroxy-4-naphthalin-2-yl-butyramid,
N-[1-(1-Hydroxycarbamoylmethyl-2-phenyl-ethyl)-1H-[1,2,3]triazol-4-ylmethyl]-4-methyl-benzamid,
N-[1-(1-Hydroxycarbamoylmethyl-2-phenyl-ethyl)-1H-[1,2,3]triazol-4-ylmethyl]-4-fluor-benzamid,
N-[5-(1-Hydroxycarbamoylmethyl-2-naphthalin-2-yl-ethyl)-[1,2,4]oxadiazol-3-ylmethyl]-benzamid,
4-Fluor-N-[5-(1-hydroxycarbamoylmethyl-2-naphthalin-2-yl-ethyl)-[1,2,4]oxadiazol-3-ylmethyl]-benzamid,
(R)-4-Fluor-N-[1-(1-hydroxycarbamoyl-2-naphthalin-2-yl-ethyl)-1H-[1 ,2,3]triazol-4-ylmethyl]-benzamid,
(S)-4-Fluor-N-[1-(2-hydroxycarbamoyl-1-naphthalin-2-ylmethyl-ethyl)-1H-[1,2,3]triazol-4-ylmethyl]-benzamid,
(R)-4-Fluor-N-[3-(1-hydroxycarbamoylmethyl-2-naphthalin-2-yl-ethyl)-3H-[1,2,3]triazol-4-ylmethyl]-benzamid,
(R)-Cyclohexancarbonsäure[3-(1-hydroxycarbamoylmethyl-2-naphthalin-2-yl-ethyl)-3H-[1,2,3]triazol-4-ylmethyl]-amid und
(R)-N-Hydroxy-4-naphthalin-2-yl-3-{5-[(3-phenyl-propionylamino)-methyl]-[1,2,3]triazol-1-yl}-butyramid.

2. Verbindung nach Anspruch 1, wobei L¹ -CH₂- ist.

3. Verbindung nach einem der Ansprüche 1 und 2, wobei A CH ist.

4. Verbindung nach einem der Ansprüche 1 bis 3, wobei Y -CH₂- ist.

5. Verbindung nach einem der Ansprüche 1 bis 4, wobei L² ausgewählt ist aus - CH₂-, -CH₂-CH₂-, -C(CH₃)₂- und -CH(R⁵)-.

6. Verbindung nach einem der Ansprüche 1 bis 5, wobei R⁵ ausgewählt ist aus CH₃, CH₂-OH, CH₂-Ph-OH und CH₂-Ph-OR⁶.

7. Verbindung nach einem der Ansprüche 1 bis 6 mit der Formel II: 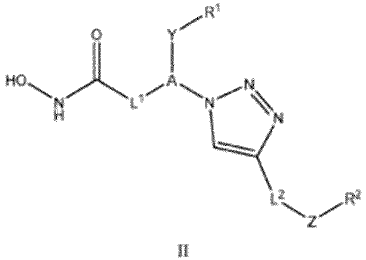 und pharmazeutisch annehmbare Salze und Solvate davon,
wobei
**L¹, L², A, Y, Z, R¹, R², R⁵, R⁶** und **R⁷** wie in Anspruch 1 definiert sind.

8. Verbindung nach einem der Ansprüche 1 bis 6 mit der Formel III: 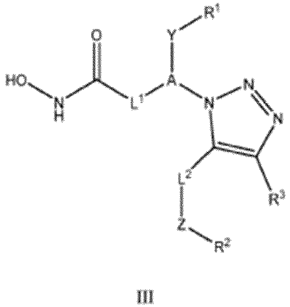 und pharmazeutisch annehmbare Salze und Solvate davon,
wobei
**L¹, L², A, Y, Z, R¹, R², R³, R⁴, R⁵, R⁶** und **R⁷**wie in Anspruch 1 definiert sind.

9. Verbindung nach einem der Ansprüche 1 bis 6 mit der Formel IV: 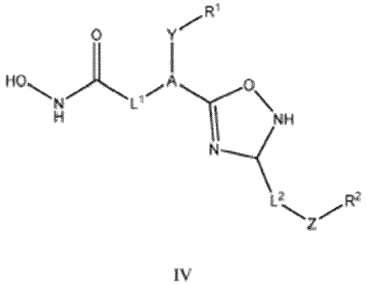 und pharmazeutisch annehmbare Salze und Solvate davon,
wobei
**L¹, L², A, Y, Z, R¹, R², R⁵, R⁶** und **R⁷** wie in Anspruch 1 definiert sind.

10. Verbindung nach einem der Ansprüche 1 bis 6 mit der Formel V: 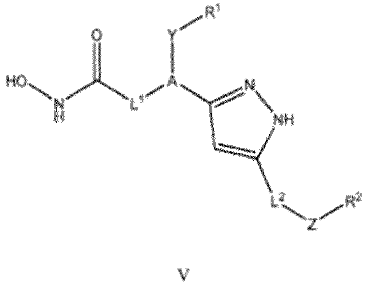 und pharmazeutisch annehmbare Salze und Solvate davon,
wobei
**L¹**, **L², A, Y, Z, R¹, R², R⁵, R⁶** und **R⁷** wie in Anspruch 1 definiert sind.

11. Verbindung nach einem der Ansprüche 1 bis 10, ausgewählt aus der Gruppe bestehend aus:
N-[1-(R-2-Hydroxycarbamoyl-1-naphthalin-2-ylmethyl-ethyl)-1H-[1,2,3]triazol-4-ylmethyl]-4-trifluormethyl-benzamid,
(R)-N-Hydroxy-4-naphthalin-2-yl-3-{4-[(toluol-4-sulfonylamino)-methyl]-[1,2,3]triazol-1-yl}-butyramid,
(R)-3-[4-(Benzolsulfonylamino-methyl)-[1,2,3]triazol-1-yl]-N-hydroxy-4-naphthalin-2-yl-butyramid,
(R)-3-{4-[(4-Fluor-benzolsulfonylamino)-methyl]-[1,2,3]-triazol-1-yl}-N-hydroxy-4-naphthalin-2-yl-butyramid,
(R)-N-[3-(1-Hydroxycarbamoylmethyl-2-naphthalin-2-yl-ethyl)-3H-[1,2,3]triazol-4-ylmethyl]-benzamid,
(R)-N-[1-(2-Hydroxycarbamoyl-1-naphthalin-2-ylmethyl-ethyl)-1H-[1 ,2,3]triazol-4-ylmethyl]-N-methyl-benzamid,
(R)-4,4-Difluorcyclohexancarbonsäure[1-(2-hydroxycarbamoyl-1-naphthalin-2-ylmethyl-ethyl)-1H-[1,2,3]triazol-4-ylmethyl]-amid,
(R)-3-{4-[(2-Ethyl-butyrylamino)-methyl]-[1,2,3]triazol-1-yl}-N-hydroxy-4-naphthalin-2-yl-butyramid,
(R)-3-[4-(Acetylamino-methyl)-[1,2,3]triazol-1-yl]-N-hydroxy-4-naphthalin-2-yl-butyramid,
(R)-3-{4-[(4-Fluor-phenylamino)-methyl]-[1,2,3]triazol-1-yl}-N-hydroxy-4-naphthalin-2-yl-butyramid,
tert-Butyl ((1-((R)-4-(hydroxyamino)-1-(naphthalin-2-yl)-4-oxobutan-2-yl)-1H-1,2,3-triazol-4-yl)methyl)-L-leucinat,
(R)-N-Hydroxy-4-naphthalin-2-yl-3-{4-[(3-phenyl-ureido)-methyl]-[1,2,3]triazol-1-yl]-butyramid,
(R)-[1-(2-Hydroxycarbamoyl-1 -naphthalin-2-ylmethyl-ethyl)-1H-[1,2,3]triazol-4-ylmethyl]-carbaminsäure-tert-butylester,
(R)-3-(4-Benzyloxymethyl-[1,2,3]triazol-1-yl)-N-hydroxy-4-naphthalin-2-yl-butyramid,
(R)-3-[5-(Acetylamino-methyl)-[1,2,3]triazol-1-yl]-N-hydroxy-4-naphthalin-2-yl-butyramid,
(R)-N-Hydroxy-4-naphthalin-2-yl-3-{5-[(3-phenyl-propionylamino)-methyl]-[1,2,3]triazol-1-yl}-butyramid,
(R,S)-N-Hydroxy-3-(5-{[2-(6-methoxy-naphthalin-2-yl)-propionylamino]-methyl}-[1,2,3]triazol-1-yl)-4-naphthalin-2-yl-butyramid,
(R)-4-Fluor-N-{2-[1-(1-hydroxycarbamoyl-2-naphthalin-2-yl-ethyl)-1H-[1,2,3]triazol-4-yl]-ethyl}-benzamid,
(R)-N-{1-[1-(4-Brom-benzyl)-2-hydroxycarbamoyl-ethyl]-1H-[1 ,2,3]triazol-4-ylmethyl}-4-fluor-benzamid,
(R)-N-[1-(1-Biphenyl-4-ylmethyl-2-hydroxycarbamoyl-ethyl)-1H-[1 ,2,3]triazol-4-ylmethyl]-4-fluor-benzamid,
(R)-4-Fluor-N-{1-[2-hydroxycarbamoyl-1-(4'-hydroxymethyl-biphenyl-4-ylmethyl)-ethyl]-1H-[1,2,3]triazol-4-ylmethyl}-benzamid,
(R)-4'-(2-{4-[(4-Fluor-benzoylamino)-methyl]-[1,2,3]thazol-1-yl}-3-hydroxycarbamoyl-propyl)-biphenyl-4-carbonsäuremethylamid,
(R)-N-{1-[1-(4'-Acetylamino-biphenyl-4-ylmethyl)-2-hydroxycarbamoyl-ethyl]-1H-[1,2,3]triazol-4-ylmethyl}-4-fluor-benzamid,
(R)-N-(1-{1-[4'-(Acetylamino-methyl)-biphenyl-4-ylmethyl]-2-hydroxycarbamoyl-ethyl]-1H-[1,2,3]triazol-4-ylmethyl)-4-fluor-benzamid,
(R)-4-Fluor-N-{1-[2-hydroxycarbamoyl-1-(3'-hydroxymethyl-biphenyl-4-ylmethyl)-ethyl]-1H-[1,2,3]triazol-4-ylmethyl}-benzamid,
(R)-4'-(2-{4-[(4-Fluor-benzoylamino)-methyl]-[1,2,3]triazol-1-yl}-3-hydroxycarbamoyl-propyl)-biphenyl-3-carbonsäuremethylamid,
(R)-N-{1-[1-(3'-Acetylamino-biphenyl-4-ylmethyl)-2-hydroxycarbamoyl-ethyl]-1H-[1,2,3]triazol-4-ylmethyl}-4-fluor-benzamid,
(R)-Ameisensäure 4'-(2-{4-[(4-fluor-benzoylamino)-methyl]-[1,2,3]triazol-1-yl}-3-hydroxycarbamoyl-propyl)-biphenyl-3-ylmethylester,
(R)-4-Fluor-N-{1-[2-hydroxycarbamoyl-1-(4-pyrimidin-5-yl-benzyl)-ethyl]-1H-[1,2,3]triazol-4-ylmethyl} -benzamid,
4-{[1-(2-Hydroxycarbamoyl-1 -naphthalin-2-ylmethyl-ethyl)-1H-[1 ,2,3]triazol-4-ylmethyl]-carbamoyl]-piperidin-1-carbonsäure-tert-butylester,
4-Fluor-N-[5-(1-hydroxycarbamoyl-2-naphthalin-2-yl-ethyl)-[1,2,4]oxadiazol-3-ylmethyl]-benzamid,
Ethyl-5-[[(4-fluorbenzoyl)amino]methyl]-3-[(1R)-3-(hydroxyamino)-1-(2-naphthylmethyl)-3-oxo-propyl]triazol-4-carboxylat,
2,4-Difluor-N-[1-(2-hydroxycarbamoyl-1-naphthalin-2-ylmethyl-ethyl)-1H-[1,2,3]triazol-4-ylmethyl]-benzamid,
(R)-N-{1-[1-(3-Brom-benzyl)-2-hydroxycarbamoyl-ethyl]-1H-[1 ,2,3]triazol-4-ylmethyl}-4-fluor-benzamid,
(R)-N-[1-(1-Biphenyl-3-ylmethyl-2-hydroxycarbamoyl-ethyl)-1H-[1 ,2,3]triazol-4-ylmethyl]-4-fluor-benzamid,
(R)-N-{1-[1-(4'-Acetylamino-biphenyl-3-ylmethyl)-2-hydroxycarbamoyl-ethyl]-1H-[1,2,3]triazol-4-ylmethyl}-4-fluor-benzamid,
(R)-4-Fluor-N-{1-[2-hydroxycarbamoyl-1-(4'-hydroxymethyl-biphenyl-3-ylmethyl)-ethyl]-1H-[1,2,3]triazol-4-ylmethyl}-benzamid,
(R)-N-{1-[1-(3'-Acetylamino-biphenyl-3-ylmethyl)-2-hydroxycarbamoyl-ethyl]-1H-[1,2,3]triazol-4-ylmethyl}-4-fluor-benzamid,
(R)-4-Fluor-N-{1-[2-hydroxycarbamoyl-1-(3'-hydroxymethyl-biphenyl-3-ylmethyl)-ethyl]-1H-[1,2,3]triazol-4-ylmethyl}-benzamid,
4-Fluor-N-((3-((2-(hydroxyamino)-2-oxoethyl)(naphthalin-2-ylmethyl)amino)-1H-pyrazol-5-yl)methyl)benzamid,
3,4-Difluor-N-[[1-[(1R)-3-(hydroxyamino)-1-(2-naphthylmethyl)-3-oxo-propyl]triazol-4-yl]methyl]benzamid,
4-[(Dimethylamin-[(1R)-3-(hydroxyamino)-1-(2-naphthylmethyl)-3-oxo-propyl]triazol-4-yl]methyl]benzamid,
4-Fluor-N-[2-[1-[(1R)-3-(hydroxyamino)-1-(2-naphthylmethyl)-3-oxo-propyl]triazol-4-yl]ethyl]benzamid),
3,4-Difluor-N-[2-[1-[(1R)-3-(hydroxyamino)-1-(2-naphthylmethyl)-3-oxo-propyl]triazol-4-yl]ethyl]benzamid,
3-{4-[(4-Fluor-phenylcarbamoyl)-methyl]-[1,2,3]triazol-1-yl]-N-hydroxy-4-naphthalin-2-yl-butyramid,
3,4-Difluor-N-((1-(3-(hydroxyamino)-1-(naphthalin-2-ylthio)-3-oxopropyl)-1H-1,2,3triazol-4-yl)methyl)benzamid,
N-[[1-[(1R)-3-(Hydroxyamino)-1-(2-naphthylmethyl)-3-oxo-propyl]triazol-4-yl]methyl]-3,4-dimethoxy-benzamid,
N-[[1-[(1R)-3-(Hydroxyamino)-1-(2-naphthylmethyl)-3-oxo-propyl]triazol-4-yl]methyl]-1,3-benzodioxol-5-carboxamid,
3-Chlor-4-fluor-N-[[1-[(1R)-3-(hydroxyamino)-1-(2-naphthylmethyl)-3-oxo-propyl]triazol-4-yl]methyl]benzamid,
2,3,4-Trifluor-N-[1-(1-hydroxycarbamoylmethyl-2-naphthalin-2-yl-ethyl)-1H-[1,2,3]triazol-4-ylmethyl]-benzamid,
Methyl(3R)-3-[4-[[(3,4-difluorbenzoyl)amino]methyl]triazol-1-yl]-4-(1-naphthyl)butanoat,
N-[[1-[(1R)-1-[(4-Chlorphenyl)methyl]-3-(hydroxyamino)-3-oxo-propyl]triazol-4-yl]methyl]-3,4-difluor-benzamid,
N-[[1-[(1R)-3-(Hydroxyamino)-1-(2-naphthylmethyl)-3-oxo-propyl]triazol-4-yl]methyl]pyrimidin-4-carboxamid,
N-[[1-[1-(4-Chlorphenyl)sulfanyl-3-(hydroxyamino)-3-oxo-propyl]triazol-4-yl]methyl]-3,4-difluor-benzamid,
N-[[1-[(1R)-3-(Hydroxyamino)-1-(2-naphthylmethyl)-3-oxo-propyl]triazol-4-yl]methyl]-4-(2-methoxyethoxy)benzamid,
N-[[1-[(1R)-3-(Hydroxyamino)-1-(2-naphthylmethyl)-3-oxo-propyl]triazol-4-yl]methyl]-1-methyl-imidazol-4-carboxamid,
3,4-Difluo-[(1R)-3-(hydroxyamino)-3-oxo-1-(2-phenylethyl)propyl]triazol-4-yl]methyl]benzamid,
tert-Butyl 3-[[1-[(1R)-3-(hydroxyamino)-1-(2-naphthylmethyl)-3-oxo-propyl]triazol-2-yl]methylcarbamoyl]morpholin-4-carboxylat,
2-Chlor-4-fluor-N-[[1-[(1R)-3-(hydroxyamino)-1-(2-naphthylmethyl)-3-oxo-propyl]triazol-4-yl]methyl]benzamid,
4-Hydroxy-N-[[1-[(1R)-3-(hydroxyamino)-1-(2-naphthylmethyl)-3-oxo-propyl]triazol-4-yl]methyl]benzamid,
3-{4-[(3,4-Difluor-phenylcarbamoyl)-methyl]-[1,2,3]-triazol-1-yl}-N-hydroxy-4-naphthalin-2-yl-butyramid,
3,4-Difluor-N-[[1-[(E,1R)-1 -[2-(hydroxyamino)-2-oxo-ethyl]-4-phenyl-but-3-enyl]triazol-4-yl]methyl]benzamid,
3,4-Difluor-N-[[1-[(1R)-3-(hydroxyamino)-3-oxo-1-[[4-(trifluormethyl)phenyl]methyl]propyl]triazol-4-yl]methyl]benzamid,
3,4-Difluor-N-{1-[1-(1 -hydroxycarbamoylmethyl-2-naphthalin-2-yl-ethyl)-1H-[1,2,3]Triazol-4-yl]-1-methyl-ethyl}-benzamid,
3,4-Difluor-N-[1-[1-[3-(hydroxyamino)-1-(2-naphthylmethyl)-3-oxo-propyl]triazol-4-yl]ethyl]benzamid,
3-[4-(2-Acetylamino-phenoxymethyl)-[1,2,3]triazol-1-yl]-N-hydroxy-4-naphthalin-2-yl-butyramid,
4-Fluo-[(1R)-3-(hydroxyamino)-1-(2-naphthylmethyl)-3-oxo-propyl]triazol-4-yl]methyl]-3-methyl-benzamid,
N-(3,4-Difluorphenyl)-3-[1-[3-(hydroxyamino)-1-(2-naphthylmethyl)-3-oxo-propyl]triazol-4-yl]propanamid,
N-[[1 -[(1S)-1-(3,4-Dihydro-1H-isochinolin-2-ylmethyl)-3-(hydroxyamino)-3-oxo-propyl]triazol-4-yl]methyl]- 3,4-difluor-benzamid,
3,4-Difluor-N-[[1-[(1R)-3-(hydroxyamino)-1-(1H-indol-3-ylmethyl)-3-oxo-propyl]triazol-4-yl]methyl]benzamid,
3,4-Difluor-N-[[1-[(1R,2S)-2-hydroxy-3-(hydroxyamino)-1-(2-naphthylmethyl)-3-oxo-propyl]triazol-4-yl]methyl]benzamid,
3,4-Difluor-N-{1-[1-(1(R)-hydroxycarbamoylmethyl-2-naphthalin-2-yl-ethyl)-1H-[1,2,3]triazol-4-yl]-(S)-ethyl}-benzamid,
3,4-3Difluor-N-{1-[1-(1(R)-hydroxycarbamoylmethyl-2-naphthalin-2-yl-ethyl)-1H-[1,2,3]triazol-4-yl]-(R)-ethyl}-benzamid,
Methyl-(3R)-3-[4-[[(3,4-difluorbenzoyl)-methyl-amino]methyl]triazol-1-yl]-4-(2-naphthyl)butanoat,
(3R)-3-[4-[(2S)-1-(3,4-Difluorbenzoyl)pyrrolidin-2-yl]triazol-1-yl]-4-(1H-indol-3-yl)butanhydroxamsäure,
N-[2-(4-Benzyloxyphenyl)-1-[1-[(1R)-3-(Hydroxyamino)-1-(1H-indol-3-ylmethyl)-3-oxo-propyl]triazol- 4-yl]ethyl]-3,4-difluor-benzamid,
(3R)-3-[4-[4-(3,4-Difluorbenzoyl)morpholin-3-yl]triazol-1-yl]-4-(1H-indol-3-yl)butanhydroxamsäure,
3,4-Difluor-N-[1-[1-[(1R)-3-(hydroxyamino)-1-(1H-indol-3-ylmethyl)-3-oxo-propyl]triazol-4-yl]ethyl]benzamid,
3,4-Difluor-N-[(1R)-2-hydroxy-1-[1-[(1R)-3-(hydroxyamino)-1-(1H-indol-3-ylmethyl)-3-oxo-propyl]triazol-4-yl]ethyl]benzam id,
(3R)-3-[4-[(2S)-1-(3,4-Difluorbenzoyl)pyrrolidin-2-yl]triazol-1-yl]-4-(2-naphthyl)butanhydroxamsäure,
N-[2-(4-Benzyloxyphenyl)-1-[1-[(1R)-3-(hydroxyamino)-1 -(2-naphthylmethyl)-3-oxo-propyl]triazol-4-yl]ethyl]-3,4-difluor-benzamid,
(3R)-3-[4-[4-(3,4-Difluorbenzoyl)morpholin-3-yl]triazol-1-yl]-4-(2-naphthyl)butanhydroxamsäure,
3,4-Difluor-N-[(1R)-2-hydroxy-1-[1-[(1R)-3-(hydroxyamino)-1-(2-naphthylmethyl)-3-oxo-propyl]triazol-4-yl]ethyl]benzam id,
3,4-Difluor-N-[[1-[(IR)-3-(hydroxyamino)-1-(1H-indol-3-ylmethyl)-3-oxo-propyl]triazol-4-yl]methyl]-N- methyl-benzamid,
3,4-Difluor-N-[[1-[(IR)-3-(hydroxyamino)-1-(1H-indol-3-ylmethyl)-3-oxo-propyl]triazol-4-yl]methyl]-N- isobutyl-benzamid,
3,4-Difluor-N-[[1-[(IR)-3-(hydroxyamino)-1-(1H-indol-3-ylmethyl)-3-oxo-propyl]triazol-4-yl]methyl]- N-isopentyl-benzamid,
3,4-Difluor-N-[[1-[(IR)-3-(hydroxyamino)-1-(1H-indol-3-ylmethyl)-3-oxo-propyl]triazol-4-yl]methyl]-N- [(1-methylimidazol-2-yl)methyl]benzamid,
Methyl-(3R)-4-(2-naphthyl)-3-[4-[(pyridin-4-carbonylamino)methyl]triazol-1-yl]butanoat,
N-[[1-[(1R)-3-(Hydroxyamino)-1-(2-naphthylmethyl)-3-oxo-propyl]triazol-4-yl]methyl]-4-(hydroxycarbamoyl)benzamid,
3,4-Difluor-N-[[1-[(1R)-3-(hydroxyamino)-3-oxo-1-[[3-(trifluormethyl)phenyl]methyl]propyl]triazol-4-yl]methyl]benzamid,
N-({1-[(2R)-1-[4-(2-tert-butyl-2H-1,2,3,4-tetrazol-5-yl)phenyl]-3-(hydroxycarbamoyl)propan2-yl]-1H-1,2,3-triazol-4-yl]methyl)-3,4-difluorbenzamid,
(R)-3,4-Difluor-N-((1-(4-(hydroxyamino)-4-oxo-1-(chinolin-3-yl)butan-2-yl)-1H-1,2,3-triazol -4-yl)methyl)benzamid,
(R)-3,4-Difluor-N-((I-(4-(hydroxyamino)-4-oxo-1-(5,6,7,8-tetrahydronaphthalin-2-yl)butan-2-yl)-1H-1,2,3-triazol-4-yl)methyl)benzamid,
3,4-Difluor-N-[1-[1-[(IR)-3-(hydroxyamino)-1-(1H-indol-3-ylmethyl)-3-oxo-propyl]triazol-4-yl]-2- (4-hydroxyphenyl)ethyl]benzamid,
4-Fluor-N-(1-(1-((R)-4-(hydroxyamino)-1-(naphthalin-2-yl)-4-oxobutan-2-yl)-1H-1,2,3- triazol-4-yl)ethyl)benzamid,
(R)-4-Fluor-N-((1-(4-(hydroxyamino)-4-oxo-1-(5,6,7,8-tetrahydronaphthalin-2-yl)butan-2-yl)-1H -1,2,3-triazol-4-yl)methyl)benzamid,
(R)-4-Fluor-N-((1-(4-(hydroxyamino)-4-oxo-1-(5,6,7,8-tetrahydronaphthalin-2-yl)butan-2-yl)-1H -1,2,3-triazol-4-yl)methyl)-N-methylbenzamid,
4-Fluor-N-(1-(1-((R)-4-(hydroxyamino)-4-oxo-1-(5,6,7,8-tetrahydronaphthalin-2-yl)butan-2-yl)-1H-1,2,3-triazol-4-yl)ethyl)benzamid,
3,4-Difluor-N-(1-(1-((R)-4-(hydroxyamino)-4-oxo-1-(5,6,7,8-tetrahydronaphthalin-2-yl)butan-2- yl)-1H-1,2,3-triazol-4-yl)ethyl)benzamid,
(R)-3,4-Difluor-N-((1-(4-(hydroxyamino)-4-oxo-1-(5,6,7,8-tetrahydronaphthalin-2-yl)butan-2-yl)-1H-1,2,3-triazol-4-yl)methyl)-N-methylbenzamid,
(R)-3,4-Difluor-N-((1-(4-(hydroxyamino)-4-oxo-1-(chinolin-7-yl)butan-2-yl)-1H-1,2,3 -triazol-4-yl)methyl)benzamid,
(R)-3,4-Difluor-N-((1-(4-(hydroxyamino)-4-oxo-1-(2-oxo-1,2,3,4-tetrahydrochinolin-6-yl)butan- 2-yl)-1H-1 ,2,3-triazol-4-yl)methyl)benzamid,
4-Fluor-N-(1-(1-((R)-4-(hydroxyamino)-4-oxo-1-(5,5,8,8-tetramethyl-5,6,7,8-tetrahydronaphthalin- 2-yl)butan-2-yl)-1H-1,2,3-triazol-4-yl)ethyl)benzamid,
(R)-N-((1-(1-(Benzo[b]thiophen-5-yl)-4-(hydroxyamino)-4-oxobutan-2-yl)-1H-1,2,3-triazol- 4-yl)methyl)-4-fluorbenzamid,
(R)-N-((1-(1-(Benzo[b]thiophen-6-yl)-4-(hydroxyamino)-4-oxobutan-2-yl)-1H-1,2,3-triazol- 4-yl)methyl)-4-fluorbenzamid,
(R)-3,4-Difluor-N-((1 -(4-(hydroxyamino)-1-(1H-indol-5-yl)-4-oxobutan-2-yl)-1H-1,2,3-triazol-4-yl)methyl)benzamid,
(R)-4-Fluor-N-((1-(4-(hydroxyamino)-1-(1H-indol-6-yl)-4-oxobutan-2-yl)-1H-1,2,3-triazol-4-yl)methyl)benzamid,
(R)-4-Fluor-N-((1-(4-(hydroxyamino)-1-(1-methyl-1H-indol-5-yl)-4-oxobutan-2-yl)-1H-1,2,3-triazol-4-yl)methyl)benzamid,
(R)-N-((1-(1-(Benzo[d]thiazol-6-yl)-4-(hydroxyamino)-4-oxobutan-2-yl)-1H-1,2,3-triazol- 4-yl)methyl)-3,4-difluorbenzamid,
(R)-N-((1-(1-(Benzo[b]thiophen-3-yl)-4-(hydroxyamino)-4-oxobutan-2-yl)-1H-1,2,3-triazol- 4-yl)methyl)-3,4-difluorbenzamid,
(R)-N-((1-(1-(Benzo[b]thiophen-3-yl)-4-(hydroxyamino)-4-oxobutan-2-yl)-1H-1,2,3-triazol- 4-yl)methyl)-4-fluorbenzamid,
N-(1-(1-((R)-1-(Benzo[b]thiophen-3-yl)-4-(hydroxyamino)-4-oxobutan-2-yl)-1H-1,2,3- triazol-4-yl)ethyl)-4-fluorbenzamid,
(S)-N-((1-(1-(Benzo[b]thiophen-2-yl)-4-(hydroxyamino)-4-oxobutan-2-yl)-1H-1,2,3-triazol- 4-yl)methyl)-4-fluorbenzamid,
(R)-N-((1-(1-(2,3-Dihydrobenzo[b][1,4]dioxin-6-yl)-4-(hydroxyamino)-4-oxobutan-2-yl)-1H -1,2,3-triazol-4-yl)methyl)-3,4-difluorbenzamid,
(R)-3-Fluor-N-((1-(4-(hydroxyamino)-1-(naphthalin-2-yl)-4-oxobutan-2-yl)-1H-1,2,3-triazol- 4-yl)methyl)benzamid,
(R)-4-Fluor-N-((1-(4-(hydroxyamino)-1-(naphthalin-2-yl)-4-oxobutan-2-yl)-1H-1,2,3-triazol- 4-yl)methyl)benzamid,
(3R)-3-(4-(3-(4-Fluorbenzoyl)thiazolidin-4-yl)-1H-1,2,3-triazol-1-yl)-N-hydroxy-4-(naphthalin-2- yl)butanamid und
(3R)-3-(4-(1-(4-Fluorbenzoyl)piperidin-2-yl)-1H-1,2,3-triazol-1-yl)-N-hydroxy-4-(naphthalin-2 -yl)butanamid.

12. Pharmazeutische Zusammensetzung, umfassend eine Verbindung nach einem der Ansprüche 1 bis 11 oder ein pharmazeutisch annehmbares Salz oder Solvat davon und mindestens einen pharmazeutisch annehmbaren Träger, Verdünner, Hilfsstoff und/oder ein pharmazeutisch annehmbares Adjuvans.

13. Verbindung nach einem der Ansprüche 1 bis 11 oder ein pharmazeutisch annehmbares Salz oder Solvat davon zur Verwendung als ein Arzneimittel.

14. Verbindung der Formel I: 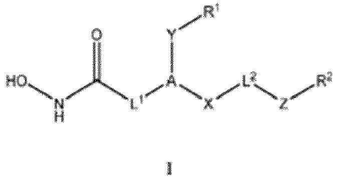 oder ein pharmazeutisch annehmbares Salz oder Solvat davon zur Verwendung bei der Behandlung und/oder Vorbeugung von Krebs,
wobei
**L¹** nicht vorhanden ist oder ausgewählt ist aus -CH₂- und -CH(OH)-,
**A** CH oder N ist,
**Y** -CH₂- oder -S- ist,
**R¹** ausgewählt ist aus Aryl, Heteroaryl, Arylalkyl und Arylalkenyl, wobei das Aryl, Heteroaryl, Arylalkyl oder Arylalkenyl gegebenenfalls durch einen oder mehrere Substituenten substituiert ist, die unabhängig ausgewählt sind aus Cl-C4-Alkyl, Halogen, Cl-C4-Haloalkyl, Hydroxy-Cl-C4-Alkyl, Acetylamino, Acetylamino-Cl-C4-Alkyl und Cl-C4-Alkylaminocarboxyl,
**R²** ausgewählt ist aus Aryl, Heteroaryl, Arylalkyl, Cycloalkyl, Heterocycloalkyl, Cl-C6-Alkyl, Cl-C4-Alkyloxycarbonyl, Cl-C4-Alkoxy, Arylamino, wobei das Aryl, Arylalkyl, Cycloalkyl oder Heterocycloalkyl gegebenenfalls durch einen oder mehrere Substituenten substituiert ist, die unabhängig ausgewählt sind aus Cl-C4-Alkoxy, Halogen, Cl-C4-Haloalkyl, Cl-C4-Alkyl, Cl-C2-Alkoxy-Cl-C2-Alkoxy, Cl-C4-Alkyloxycarbonyl, Acetylamino, Di(Cl-C4-Alkyl)amino-C1-C4-alkyl und Hydroxycarbamoyl,
X ausgewählt ist aus 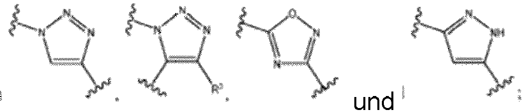
**R³** ausgewählt ist aus H und C(O)OR⁴
**R⁴** CI-C4-Alkyl ist,
**L²** lineares oder verzweigtes CI-C6-Alkyl ist, das gegebenenfalls durch eine Gruppe **R⁵** substituiert ist,
**R⁵** ausgewählt ist aus linearem oder verzweigtem Cl-C4-Alkyl, CH₂-OH, CHOH-CH₃, CH₂-C(O)NH₂, CH₂-CH₂-C(O)NH₂, CH₂-COOH, CH₂-CH₂-COOH, (CH₂)₄-NH₂, (CH₂)₄- NH-C(NH₂⁺)-NH₂, CH₂-Imidazolyl, CH₂-Indolyl, CH₂-SH, CH₂-CH₂-S-CH₃, CH₂-Ph, CH₂-Ph-OH, CH₂-OR⁶, CH₂-COOR⁶, CH₂-CH₂-COOR⁶, CH₂-SR⁶ und CH₂-Ph-OR⁶,
**R⁶** ausgewählt ist aus Me, Bn, Ph und Ac,
**Z** ausgewählt ist aus -NR⁷(CO)-, -NHSO₂-, -CH₂-CH₂-, -NH-, -NH-CH(CI-C6-alkyl)-, -O-CH₂-, -C(O)NH- und -O- und
**R⁷** ausgewählt ist aus H, linearem oder verzweigtem C1-C6-Alkyl und (1-Methylimidazol-2-yl)-Cl-C2-Alkyl oder
**R⁷** und **L²** zusammen mit dem Stickstoffatom, an das sie gebunden sind, eine 5- oder 6-gliedrige Heterocyclylgruppe bilden.

15. Verbindung zur Verwendung nach Anspruch 14, wobei die Verbindung ein Sensibilisator für die Chemotherapie von bösartigen Tumoren ist.

16. Verbindung zur Verwendung nach Anspruch 15, wobei die Chemotherapie von bösartigen Tumoren einen Proteasom-Inhibitor, einen HDAC-Inhibitor oder einen Inhibitor der Proteinglykosylierung umfasst.

17. Verbindung zur Verwendung nach einem der Ansprüche 14 bis 16, wobei der Krebs ausgewählt ist aus der Gruppe bestehend aus Multiplem Myelom, Gebärmutterhalskrebs und Brustkrebs.

18. Verbindung zur Verwendung nach einem der Ansprüche 14 bis 17, wobei die Verbindung ausgewählt ist aus der Gruppe bestehend aus:
N-[1 -((R)-2-Hydroxycarbamoyl-1-naphthalin-2-ylmethyl-ethyl)-1H-[1,2,3]triazol-4-ylmethyl]-benzamid,
N-[1 -((R)-2-Hydroxycarbamoyl-1-naphthalin-2-ylmethyl-ethyl)-1H-[1,2,3]triazol-4-ylmethyl]-4-methoxy-benzamid,
4-Fluor-N-[1-(R-2-hydroxycarbamoyl-1-naphthalin-2-ylmethyl-ethyl)-1H-[1,2,3]triazol-4-ylmethyl]-benzamid,
N-[1-(R-2-Hydroxycarbamoyl-1-naphthalin-2-ylmethyl-ethyl)-1H-[1,2,3]triazol-4-ylmethyl]-4-trifluormethyl-benzamid,
(R)-N-Hydroxy-4-naphthalin-2-yl-3-{4-[(toluol-4-sulfonylamino)-methyl]-[1,2,3]triazol-1-yl}-butyramid,
(R)-3-{4-[(4-Fluor-benzolsulfonylamino)-methyl]-[1,2,3]-triazol-1-yl}-N-hydroxy-4-naphthalin-2-yl-butyramid,
(R)-3-[4-(Benzolsulfonylamino-methyl)-[1,2,3]triazol-1-yl]-N-hydroxy-4-naphthalin-2-yl-butyramid,
(R)-N-Hydroxy-4-naphthalin-2-yl-3-{4-[(2-p-tolyl-acetylamino)-methyl]-[1,2,3]triazol-1-yl}-butyramid,
(R)-3-{4-[(4-Fluor-benzolsulfonylamino)-methyl]-[1,2,3]-triazol-1-yl}-N-hydroxy-4-naphthalin-2-yl-butyramid,
(R)-N-Hydroxy-4-naphthalin-2-yl-3-{4-[(3-phenyl-propionylamino)-methyl]-[1,2,3]triazol-1-yl}-butyramid,
(R)-4-Fluor-N-[3-(1-hydroxycarbamoylmethyl-2-naphthalin-2-yl-ethyl)-3H-[1,2,3]triazol-4-ylmethyl]-benzamid,
(R)-N-[3-(1-Hydroxycarbamoylmethyl-2-naphthalin-2-yl-ethyl)-3H-[1,2,3]triazol-4-ylmethyl]-benzamid,
(R)-Cyclohexancarbonsäure[3-(1-hydroxycarbamoylmethyl-2-naphthalin-2-yl-ethyl)-3H-[1,2,3]triazol-4-ylmethyl]-amid,
(R)-N-[1 -(2-Hydroxycarbamoyl-1-naphthalin-2-ylmethyl-ethyl)-1H-[1 ,2,3]triazol-4-ylmethyl]-N-methyl-benzamid,
(R)-4,4-Difluor-cyclohexancarbonsäure [1-(2-hydroxycarbamoyl-1-naphthalin-2-ylmethyl-ethyl)-1H-[1,2,3]triazol-4-ylmethyl]-amid,
(R)-N-Hydroxy-4-naphthalin-2-yl-3-[4-(phenylacetylamino-methyl)-[1,2,3]triazol-1-yl]-butyramid,
(R)-3-{4-[(2-Ethyl-butyrylamino)-methyl]-[1,2,3]triazol-1-yl}-N-hydroxy-4-naphthalin-2-yl-butyramid,
(R)-3-[4-(Acetylamino-methyl)-[1,2,3]triazol-1-yl]-N-hydroxy-4-naphthalin-2-yl-butyramid,
(R)-3-{4-[(4-Fluor-phenylamino)-methyl]-[1,2,3]triazol-1-yl}-N-hydroxy-4-naphthalin-2-yl-butyramid,
tert-Butyl ((1-((R)-4-(hydroxyamino)-1-(naphthalin-2-yl)-4-oxobutan-2-yl)-1H-1,2,3-triazol-4-yl )Methyl)-L-leucinat,
(R)-N-Hydroxy-4-naphthalin-2-yl-3-{4-[(3-phenyl-ureido)-methyl]-[1,2,3]triazol-1-yl]-butyramid,
(R)-[1-(2-Hydroxycarbamoyl-1-naphthalin-2-ylmethyl-ethyl)-1H-[1,2,3]triazol-4-ylmethyl]-carbaminsäure-tert-butylester,
(R)-3-(4-Benzyloxymethyl-[1,2,3]triazol-1-yl)-N-hydroxy-4-naphthalin-2-yl-butyramid,
(R)-3-[5-(Acetylamino-methyl)-[1,2,3]triazol-1-yl]-N-hydroxy-4-naphthalin-2-yl-butyramid,
(R)-N-Hydroxy-4-naphthalin-2-yl-3-{5-[(3-phenyl-propionylamino)-methyl]-[1,2,3]triazol-1-yl}-butyramid,
(R,S)-N-Hydroxy-3-(5-{[2-(6-methoxy-naphthalin-2-yl)-propionylamino]-methyl}-[1,2,3]triazol-1-yl)-4-naphthalin-2-yl-butyramid,
4-Fluor-N-[5-(1-hydroxycarbamoylmethyl-2-naphthalin-2-yl-ethyl)-[1,2,4]oxadiazol-3-ylmethyl]-benzamid,
N-[5-(1-Hydroxycarbamoylmethyl-2-naphthalin-2-yl-ethyl)-[1,2,4]oxadiazol-3-ylmethyl]-benzamid,
(R)-4-Fluor-N-{2-[1-(1-hydroxycarbamoyl-2-naphthalin-2-yl-ethyl)-1H-[1,2,3]triazol-4-yl]-ethyl}-benzamid,
(S)-4-Fluor-N-[1-(2-hydroxycarbamoyl-1-naphthalin-2-ylmethyl-ethyl)-1H-[1,2,3]triazol-4-ylmethyl]-benzamid,
(R)-N-{1-[1-(4-Brom-benzyl)-2-hydroxycarbamoyl-ethyl]-1H-[1,2,3]triazol-4-ylmethyl}-4-fluor-benzamid,
(R)-N-[1-(1-Biphenyl-4-ylmethyl-2-hydroxycarbamoyl-ethyl)-1H-[1,2,3]triazol-4-ylmethyl]-4-fluor-benzamid,
(R)-4-Fluor-N-{1-[2-hydroxycarbamoyl-1-(4'-hydroxymethyl-biphenyl-4-ylmethyl)-ethyl]-1H-[1,2,3]triazol-4-ylmethyl}-benzamid,
(R)-4'-(2-{4-[(4-Fluor-benzoylamino)-methyl]-[1,2,3]triazol-1-yl}-3-hydroxycarbamoyl-propyl)-biphenyl-4-carbonsäuremethylamid,
(R)-N-{1-[1-(4'-Acetylamino-biphenyl-4-ylmethyl)-2-hydroxycarbamoyl-ethyl]-1H-[1,2,3]triazol-4-ylmethyl}-4-fluor-benzamid,
(R)-N-(1-{1-[4'-(Acetylamino-methyl)-biphenyl-4-ylmethyl]-2-hydroxycarbamoyl-ethyl]-1H-[1,2,3]triazol-4-ylmethyl)-4-fluor-benzamid,
(R)-4-Fluor-N-{1-[2-hydroxycarbamoyl-1-(3'-hydroxymethyl-biphenyl-4-ylmethyl)-ethyl]-1H-[1,2,3]triazol-4-ylmethyl}-benzamid,
(R)-4'-(2-{4-[(4-Fluor-benzoylamino)-methyl]-[1,2,3]triazol-1-yl}-3-hydroxycarbamoyl-propyl)-biphenyl-3-carbonsäuremethylamid,
(R)-N-{1-[1-(3'-Acetylamino-biphenyl-4-ylmethyl)-2-hydroxycarbamoyl-ethyl]-1H-[1,2,3]triazol-4-ylmethyl}-4-fluor-benzamid,
(R)-Ameisensäure 4'-(2-{4-[(4-fluor-benzoylamino)-methyl]-[1,2,3]triazol-1-yl}-3-hydroxycarbamoyl-propyl)-biphenyl- 3-ylmethylester,
(R)-4-Fluor-N-{1-[2-hydroxycarbamoyl-1-(4-pyrimidin-5-yl-benzyl)-ethyl]-1H-[1,2,3]triazol-4-ylmethyl} -benzamid,
4-{[1-(2-Hydroxycarbamoyl-1-naphthalin-2-ylmethyl-ethyl)-1H-[1 ,2,3]triazol-4-ylmethyl]-carbamoyl]-piperidin-1-carbonsäure-tert-butylester,
(R)-4-Fluor-N-[1-(1-hydroxycarbamoyl-2-naphthalin-2-yl-ethyl)-1H-[1,2,3]triazol-4-ylmethyl]-benzamid,
4-Fluor-N-[5-(1-hydroxycarbamoyl-2-naphthalin-2-yl-ethyl)-[1,2,4]oxadiazol-3-ylmethyl]-benzamid,
Ethyl-5-[[(4-fluorbenzoyl)amino]methyl]-3-[(1R)-3-(hydroxyamino)-1-(2-naphthylmethyl)-3-oxo-propyl]triazol-4-carboxylat,
2,4-Difluor-N-[1-(2-hydroxycarbamoyl-1-naphthalin-2-ylmethyl-ethyl)-1H-[1,2,3]triazol-4-ylmethyl]-benzamid,
(R)-N-{1-[1-(3-Brom-benzyl)-2-hydroxycarbamoyl-ethyl]-1H-[1,2,3]triazol-4-ylmethyl}-4-fluor-benzamid,
(R)-N-[1-(1-Biphenyl-3-ylmethyl-2-hydroxycarbamoyl-ethyl)-1H-[1,2,3]triazol-4-ylmethyl]-4-fluor-benzamid,
(R)-N-{1-[1-(4'-Acetylamino-biphenyl-3-ylmethyl)-2-hydroxycarbamoyl-ethyl]-1H-[1,2,3]triazol-4-ylmethyl}-4-fluor-benzamid,
(R)-4-Fluor-N-{1-[2-hydroxycarbamoyl-1-(4'-hydroxymethyl-biphenyl-3-ylmethyl)-ethyl]-1H-[1,2,3]triazol-4-ylmethyl}-benzamid,
(R)-N-{1-[1-(3'-Acetylamino-biphenyl-3-ylmethyl)-2-hydroxycarbamoyl-ethyl]-1H-[1,2,3]triazol-4-ylmethyl}-4-fluor-benzamid,
(R)-4-Fluor-N-{1-[2-hydroxycarbamoyl-1-(3'-hydroxymethyl-biphenyl-3-ylmethyl)-ethyl]-1H-[1,2,3]triazol-4-ylmethyl}-benzamid,
4-Fluor-N-((3-((2-(hydroxyamino)-2-oxoethyl)(naphthalin-2-ylmethyl)amino)-1H-pyrazol-5-yl)methyl)benzamid,
3.4- 3,4-Difluor-N-[[1-[(1R)-3-(hydroxyamino)-1-(2-naphthylmethyl)-3-oxo-propyl]triazol-4-yl]methyl]benzamid,
4-[(Dimethylamino)methyl]-N-[[1-[(1R)-3-(hydroxyamino)-1-(2-naphthylmethyl)-3-oxo-propyl]triazol-4-yl]methyl]benzamid,
4-Fluor-N-[2-[1-[(1R)-3-(hydroxyamino)-1-(2-naphthylmethyl)-3-oxo-propyl]triazol-4-yl]ethyl]benzamid),
3,4-Difluor-N-[2-[1-[(1R)-3-(hydroxyamino)-1-(2-naphthylmethyl)-3-oxo-propyl]triazol-4-yl]ethyl]benzamid,
3-{4-[(4-Fluor-phenylcarbamoyl)-methyl]-[1,2,3]triazol-1-yl]-N-hydroxy-4-naphthalin-2-yl-butyramid,
3,4-Difluor-N-((1-(3-(hydroxyamino)-1-(naphthalin-2-ylthio)-3-oxopropyl)-1H-1,2,3-triazol-4-yl)methyl)benzamid,
N-[[1-[(1R)-3-(Hydroxyamino)-1-(2-naphthylmethyl)-3-oxo-propyl]triazol-4-yl]methyl]-3,4-dimethoxy-benzamid,
N-[[1-[(1R)-3-(Hydroxyamino)-1-(2-naphthylmethyl)-3-oxo-propyl]triazol-4-yl]methyl]-1,3-benzodioxol-5-carboxamid,
3-Chlor-4-fluor-N-[[1-[(1R)-3-(hydroxyamino)-1-(2-naphthylmethyl)-3-oxo-propyl]triazol-4-yl]methyl]benzamid,
2,3,4-Trifluor-N-[1-(1-hydroxycarbamoylmethyl-2-naphthalin-2-yl-ethyl)-1H-[1,2,3]triazol-4-ylmethyl]-benzamid,
Methyl(3R)-3-[4-[[(3,4-difluorbenzoyl)amino]methyl]triazol-1-yl]-4-(1-naphthyl)butanoat,
N-[[1-[(1R)-1-[(4-Chlorphenyl)methyl]-3-(hydroxyamino)-3-oxo-propyl]triazol-4-yl]methyl]-3,4-difluor-benzamid,
N-[[1-[(1R)-3-(Hydroxyamino)-1-(2-naphthylmethyl)-3-oxo-propyl]triazol-4-yl]methyl]pyrimidin-4-carboxamid,
N-[[1-[1-(4-Chlorphenyl)sulfanyl-3-(hydroxyamino)-3-oxo-propyl]triazol-4-yl]methyl]-3,4-difluor-benzamid,
N-[[1-[(1R)-3-(Hydroxyamino)-1-(2-naphthylmethyl)-3-oxo-propyl]triazol-4-yl]methyl]-4-(2-methoxyethoxy)benzamid,
N-[[1-[(1R)-3-(Hydroxyamino)-1-(2-naphthylmethyl)-3-oxo-propyl]triazol-4-yl]methyl]-1-methyl-imidazol-4-carboxamid,
3,4-Difluor-N-[[1-[(1R)-3-(hydroxyamino)-3-oxo-1-(2-phenylethyl)propyl]triazol-4-yl]methyl]benzamid,
tert-Butyl 3-[[1-[(1R)-3-(hydroxyamino)-1-(2-naphthylmethyl)-3-oxo-propyl]triazol-2-yl]methylcarbamoyl]morpholin-4-carboxylat,
2-Chlor-4-fluor-N-[[1-[(1R)-3-(hydroxyamino)-1-(2-naphthylmethyl)-3-oxo-propyl]triazol-4-yl]methyl]benzamid,
4-Hydroxy-N-[[1-[(1R)-3-(hydroxyamino)-1-(2-naphthylmethyl)-3-oxo-propyl]triazol-4-yl]methyl]benzamid,
3-{4-[(3,4-Difluor-phenylcarbamoyl)-methyl]-[1,2,3]-triazol-1-yl}-N-hydroxy-4-naphthalin-2-yl-butyramid,
3,4-Difluor-N-[[1-[(E,1R)-1-[2-(hydroxyamino)-2-oxo-ethyl]-4-phenyl-but-3-enyl]triazol-4-yl]methyl]benzamid,
3,4-Difluor-N-[[1-[(1R)-3-(hydroxyamino)-3-oxo-1-[[4-(trifluormethyl)phenyl]methyl]propyl]triazol-4-yl]methyl]benzamid,
3,4-Difluor-N-{1-[1-(1-hydroxycarbamoylmethyl-2-naphthalin-2-yl-ethyl)-1H-[1,2,3]triazol-4-yl]-1-methyl-ethyl}-benzamid,
3,4-Difluor-N-[1-[1-[3-(hydroxyamino)-1-(2-naphthylmethyl)-3-oxo-propyl]triazol-4-yl]ethyl]benzamid,
3-[4-(2-Acetylamino-phenoxymethyl)-[1,2,3]triazol-1-yl]-N-hydroxy-4-naphthalin-2-yl-butyramid,
4-Fluor-N-[[1-[(1R)-3-(hydroxyamino)-1-(2-naphthylmethyl)-3-oxo-propyl]triazol-4-yl]methyl]-3-methyl-benzamid,
N-(3,4-Difluorphenyl)-3-[1-[3-(hydroxyamino)-1-(2-naphthylmethyl)-3-oxo-propyl]triazol-4-yl]propanamid,
N-[[1-[(1S)-1-(3,4-Dihydro-1H-isochinolin-2-ylmethyl)-3-(hydroxyamino)-3-oxo-propyl]triazol-4-yl]methyl]- 3,4-difluor-benzamid,
3,4-Difluor-N-[[1-[(1R)-3-(hydroxyamino)-1-(1H-indol-3-ylmethyl)-3-oxo-propyl]triazol-4-yl]methyl]benzamid,
3,4-Difluor-N-[[1-[(1R,2S)-2-hydroxy-3-(hydroxyamino)-1-(2-naphthylmethyl)-3-oxo-propyl]triazol-4-yl]methyl]benzamid,
3,4-Difluor-N-{1-[1-(1(R)-hydroxycarbamoylmethyl-2-naphthalin-2-yl-ethyl)-1H-[1,2,3]triazol-4-yl]-(S)-ethyl}-benzamid,
3,4-Difluor-N-{1-[1-(1(R)-hydroxycarbamoylmethyl-2-naphthalin-2-yl-ethyl)-1H-[1,2,3]triazol-4-yl]-(R)-ethyl}-benzamid,
Methyl-(3R)-3-[4-[[(3,4-difluorbenzoyl)-methyl-amino]methyl]triazol-1-yl]-4-(2-naphthyl)butanoat,
(3R)-3-[4-[(2S)-1-(3,4-Difluorbenzoyl)pyrrolidin-2-yl]triazol-1-yl]-4-(1H-indol-3-yl)butanhydroxamsäure,
N-[2-(4-Benzyloxyphenyl)-1-[1-[(1R)-3-(Hydroxyamino)-1-(1H-indol-3-ylmethyl)-3-oxo-propyl]triazol- 4-yl]ethyl]-3,4-difluor-benzamid,
(3R)-3-[4-[4-(3,4-Difluorbenzoyl)morpholin-3-yl]triazol-1-yl]-4-(1H-indol-3-yl)butanhydroxamsäure,
3,4-Difluor-N-[1-[1-[(1R)-3-(hydroxyamino)-1-(1H-indol-3-ylmethyl)-3-oxo-propyl]triazol-4-yl]ethyl]benzamid,
3,4-Difluor-N-[(1R)-2-hydroxy-1-[1-[(1R)-3-(hydroxyamino)-1-(1H-indol-3-ylmethyl)-3-oxo- propyl]triazol-4-yl]ethyl]benzamid,
(3R)-3-[4-[(2S)-1-(3,4-Difluorbenzoyl)pyrrolidin-2-yl]triazol-1-yl]-4-(2-naphthyl)butanhydroxamsäure,
N-[2-(4-Benzyloxyphenyl)-1-[1-[(1R)-3-(hydroxyamino)-1-(2-naphthylmethyl)-3-oxo-propyl]triazol-4-yl]ethyl]-3,4-difluor-benzamid,
(3R)-3-[4-[4-(3,4-Difluorbenzoyl)morpholin-3-yl]triazol-1-yl]-4-(2-naphthyl)butanhydroxamsäure,
3,4-Difluor-N-[(1R)-2-hydroxy-1-[1-[(1R)-3-(hydroxyamino)-1-(2-naphthylmethyl)-3-oxo-propyl]triazol-4-yl]ethyl]benzam id,
3,4-Difluor-N-[[1-[(1R)-3-(hydroxyamino)-1-(1H-indol-3-ylmethyl)-3-oxo-propyl]triazol-4-yl]methyl]-N- methyl-benzamid,
3,4-Difluor-N-[[1-[(1R)-3-(hydroxyamino)-1-(1H-indol-3-ylmethyl)-3-oxo-propyl]triazol-4-yl]methyl]-N- isobutyl-benzamid,
3,4-Difluor-N-[[1-[(1R)-3-(hydroxyamino)-1-(1H-indol-3-ylmethyl)-3-oxo-propyl]triazol-4-yl]methyl]- N-isopentyl-benzamid,
3,4-Difluor-N-[[1-[(IR)-3-(hydroxyamino)-1-(1H-indol-3-ylmethyl)-3-oxo-propyl]triazol-4-yl]methyl]-N- [(1-methylimidazol-2-yl)methyl]benzamid,
N-[1-(1-Hydroxycarbamoylmethyl-2-phenyl-ethyl)-1H-[1,2,3]triazol-4-ylmethyl]-4-methyl-benzamid,
Methyl-(3R)-4-(2-naphthyl)-3-[4-[(pyridin-4-carbonylamino)methyl]triazol-1-yl]butanoat,
N-[[1-[(1R)-3-(Hydroxyamino)-1-(2-naphthylmethyl)-3-oxo-propyl]triazol-4-yl]methyl]-4-(hydroxycarbamoyl)benzamid,
3,4-Difluor-N-[[1-[(1R)-3-(hydroxyamino)-3-oxo-1-[[3-(trifluormethyl)phenyl]methyl]propyl]triazol-4-yl]methyl]benzamid,
N-({1-[(2R)-1-[4-(2-tert-butyl-2H-1,2,3,4-tetrazol-5-yl)phenyl]-3-(hydroxycarbamoyl)propan2-yl]-1H-1,2,3-triazol-4-yl]methyl)-3,4-difluorbenzamid,
(R)-3,4-Difluor-N-((1-(4-(hydroxyamino)-4-oxo-1-(chinolin-3-yl)butan-2-yl)-1H-1,2,3-triazol-4-yl)methyl)benzamid,
(R)-3,4-Difluor-N-((I-(4-(hydroxyamino)-4-oxo-1-(5,6,7,8-tetrahydronaphthalin-2-yl)butan-2-yl) -1H-1,2,3-triazol-4-yl)methyl)benzamid,
3,4-Difluor-N-[1-[1 -[(IR)-3-(hydroxyamino)-1-(1H-indol-3-ylmethyl)-3-oxo-propyl]triazol-4-yl]-2- (4-hydroxyphenyl)ethyl]benzamid,
4-Fluor-N-(1-(1-((R)-4-(hydroxyamino)-1-(naphthalin-2-yl)-4-oxobutan-2-yl)-1H-1,2,3- triazol-4-yl)ethyl)benzamid,
(R)-4-Fluor-N-((1-(4-(hydroxyamino)-4-oxo-1-(5,6,7,8-tetrahydronaphthalin-2-yl)butan-2-yl)-1H -1,2,3-triazol-4-yl)methyl)benzamid,
(R)-4-Fluor-N-((1-(4-(hydroxyamino)-4-oxo-1-(5,6,7,8-tetrahydronaphthalin-2-yl)butan-2-yl)-1H -1,2,3-triazol-4-yl)methyl)-N-methylbenzamid,
4-Fluor-N-(1-(1-((R)-4-(hydroxyamino)-4-oxo-1-(5,6,7,8-tetrahydronaphthalin-2-yl)butan-2-yl) -1H-1,2,3-triazol-4-yl)ethyl)benzamid,
3,4-Difluor-N-(1-(1-((R)-4-(hydroxyamino)-4-oxo-1 -(5,6,7,8-tetrahydronaphthalin-2-yl)butan-2- yl)-1H-1,2,3-triazol-4-yl)ethyl)benzamid,
(R)-3,4-Difluor-N-((1-(4-(hydroxyamino)-4-oxo-1-(5,6,7,8-tetrahydronaphthalin-2-yl)butan-2-yl) -1H-1,2,3-triazol-4-yl)methyl)-N-methylbenzamid,
(R)-3,4-Difluor-N-((1-(4-(hydroxyamino)-4-oxo-1-(chinolin-7-yl)butan-2-yl)-1H-1,2,3 -triazol-4-yl)methyl)benzamid,
(R)-3,4-Difluor-N-((1-(4-(hydroxyamino)-4-oxo-1-(2-oxo-1,2,3,4-tetrahydrochinolin-6-yl)butan- 2-yl)-1H-1,2,3-triazol-4-yl)methyl)benzamid,
4-Fluor-N-(I-(I-((R)-4-(hydroxyamino)-4-oxo-I-(5,5,8,8-tetramethyl-5,6,7,8-tetrahydronaphthalin- 2-yl)butan-2-yl)-1H-1,2,3-triazol-4-yl)ethyl)benzamid,
(R)-N-((1-(1-(Benzo[b]thiophen-5-yl)-4-(hydroxyamino)-4-oxobutan-2-yl)-1H-1,2,3-triazol- 4-yl)methyl)-4-fluorbenzamid,
(R)-N-((1-(1-(Benzo[b]thiophen-6-yl)-4-(hydroxyamino)-4-oxobutan-2-yl)-1H-1,2,3-triazol- 4-yl)methyl)-4-fluorbenzamid,
(R)-3,4-Difluor-N-((1-(4-(hydroxyamino)-1-(1H-indol-5-yl)-4-oxobutan-2-yl)-1H-1,2,3-triazol-4-yl)methyl)benzamid,
(R)-4-Fluor-N-((1 -(4-(hydroxyamino)-1-(1H-indol-6-yl)-4-oxobutan-2-yl)-1H-1,2,3-triazol-4-yl)methyl)benzamid,
(R)-4-Fluor-N-((1-(4-(hydroxyamino)-1-(1-methyl-1H-indol-5-yl)-4-oxobutan-2-yl)-1H-1,2,3-triazol-4-yl)methyl)benzamid,
(R)-N-((1-(1-(Benzo[d]thiazol-6-yl)-4-(hydroxyamino)-4-oxobutan-2-yl)-1H-1,2,3-triazol- 4-yl)methyl)-3,4-difluorbenzamid,
(R)-N-((1-(1-(Benzo[b]thiophen-3-yl)-4-(hydroxyamino)-4-oxobutan-2-yl)-1H-1,2,3-triazol- 4-yl)methyl)-3,4-difluorbenzamid,
(R)-N-((1-(1-(Benzo[b]thiophen-3-yl)-4-(hydroxyamino)-4-oxobutan-2-yl)-1H-1,2,3-triazol- 4-yl)methyl)-4-fluorbenzamid,
N-(1-(1-((R)-1-(Benzo[b]thiophen-3-yl)-4-(hydroxyamino)-4-oxobutan-2-yl)-1H-1,2,3- triazol-4-yl)ethyl)-4-fluorbenzamid,
(S)-N-((1-(1-(Benzo[b]thiophen-2-yl)-4-(hydroxyamino)-4-oxobutan-2-yl)-1H-1,2,3-triazol- 4-yl)methyl)-4-fluorbenzamid,
(R)-N-((1-(1-(2,3-Dihydrobenzo[b][1,4]dioxin-6-yl)-4-(hydroxyamino)-4-oxobutan-2-yl)-1H -1,2,3-triazol-4-yl)methyl)-3,4-difluorbenzamid,
(R)-3-Fluor-N-((1-(4-(hydroxyamino)-1-(naphthalin-2-yl)-4-oxobutan-2-yl)-1H-1,2,3-triazol- 4-yl)methyl)benzamid,
(R)-4-Fluor-N-((1-(4-(hydroxyamino)-1-(naphthalin-2-yl)-4-oxobutan-2-yl)-1H-1,2,3-triazol- 4-yl)methyl)-N-methylbenzamid,
(3R)-3-(4-(3-(4-Fluorbenzoyl)thiazolidin-4-yl)-1H-1,2,3-triazol-1-yl)-N-hydroxy-4-(naphthalin-2- yl)butanamid und
(3R)-3-(4-(1-(4-Fluorbenzoyl)piperidin-2-yl)-1H-1,2,3-triazol-1-yl)-N-hydroxy-4-(naphthalin-2-yl)butanamid.

19. Verbindung zur Verwendung nach einem der Ansprüche 14 bis 18, wobei die Verbindung in Kombination mit Strahlentherapie eingesetzt wird.

## Revendications

1. Composé de formule I : 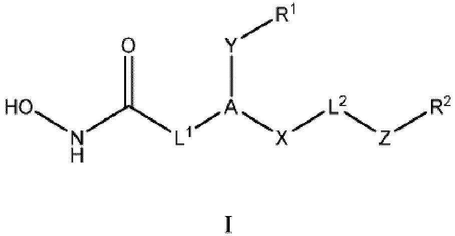
et les sels et solvates pharmaceutiquement acceptables de celui-ci,
dans lequel
L¹ est inexistant ou est sélectionné parmi -CH₂- et -CH(OH)- ;
A est CH ou N ;
Y est -CH₂- ou -S- ;
R¹ est sélectionné parmi un aryle, un hétéroaryle, un arylalkyle et un arylalcényle dans lequel ledit aryle, hétéroaryle, arylalkyle ou arylalcényle est facultativement substitué par un ou plusieurs substituants indépendamment sélectionnés parmi un alkyle en C1-C4, un halogène, un halogénoalkyle en C1-C4, un hydroxy-(alkyle en C1-C4), un acétylamino, un acétylamino-(alkyle en C1-C4) et un (alkyl en C1-C4)-aminocarboxyle ;
R² est sélectionné parmi un aryle, un hétéroaryle, un arylalkyle, un cycloalkyle, un hétérocycloalkyle, un alkyle en C1-C6, un (alkyl en C1-C4)-oxycarbonyle, un alcoxy en C1-C4, un arylamino, dans lequel ledit aryle, arylalkyle, cycloalkyle ou hétérocycloalkyle est facultativement substitué par un ou plusieurs substituants indépendamment sélectionnés parmi un alcoxy en C1-C4, un halogène, un halogénoalkyle en C1-C4, un alkyle en C1-C4, un (alcoxy en C1-C2)-(alcoxy en C1-C2), un (alkyl en C1-C4)-oxycarbonyle, un acétylamino, un di(alkyl en C1-C4)amino-(alkyle en C1-C4) et un hydroxycarbamoyle ;
X est sélectionné parmi 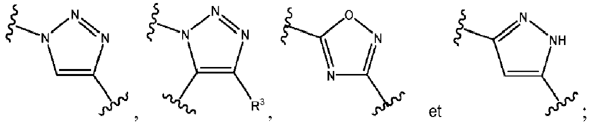
R³ est sélectionné parmi H et C(O)OR⁴ ;
R⁴ est un alkyle en C1-C4 ;
L² est un alkyle en C1-C6 linéaire ou ramifié, facultativement substitué par un groupe R⁵ ;
R⁵ est sélectionné parmi un alkyle en C1-C4 linéaire ou ramifié, CH₂-OH, CHOH-CH₃, CH₂-C(O)NH₂, CH₂-CH₂-C(O)NH₂, CH₂-COOH, CH₂-CH₂-COOH, (CH₂)₄-NH₂, (CH₂)₄-NH-C(NH₂⁺)-NH₂, un CH₂-imidazolyle, un CH₂-indolyle, CH₂-SH, CH₂-CH₂-S-CH₃, CH₂-Ph, CH₂-Ph-OH, CH₂-OR⁶, CH₂-COOR⁶, CH₂-CH₂-COOR⁶, CH₂-SR⁶ et CH₂-Ph-OR⁶ ;
R⁶ est sélectionné parmi Me, Bn, Ph et Ac ;
Z est sélectionné parmi -NR⁷(CO)-, -NHSO₂-, -CH₂-CH₂-, -NH-, un -NH-CH(alkyle en C1-C6)-, -O-CH₂-, -C(O)NH- et -O- ; et
R⁷ est sélectionné parmi H, un alkyle en C1-C6 linéaire ou ramifié et un (1-méthylimidazol-2-yl)-(alkyle en C1-C2), ou
R⁷ et L² forment conjointement à l'atome d'azote auquel ils sont liés un groupe hétérocyclyle à 5 ou 6 chaînons ;
à condition que le composé de formule I ne soit aucun des suivants :
4-fluoro-N-[1-(R-2-hydroxycarbamoyl-1-naphtalén-2-ylméthyl-éthyl)-1H-[1,2,3]triazol-4-ylméthyl]-benzamide,
N-[1-((R)-2-hydroxycarbamoyl-1-naphtalén-2-ylméthyl-éthyl)-1H-[1,2,3]triazol-4-ylméthyl]-benzamide,
N-[1-((R)-2-hydroxycarbamoyl-1-naphtalén-2-ylméthyl-éthyl)-1H-[1,2,3]triazol-4-ylméthyl]-4-méthyl-benzamide,
N-[1-((R)-2-hydroxycarbamoyl-1-naphtalén-2-ylméthyl-éthyl)-1H-[1,2,3]triazol-4-ylméthyl]-4-méthoxy-benzamide,
[1-(R-2-hydroxycarbamoyl-1-naphtalén-2-ylméthyl-éthyl)-1H-[1,2,3]triazol-4-ylméthyl]-amide de l'acide cyclohexanecarboxylique,
(R)-N-hydroxy-4-naphtalén-2-yl-3-[4-(phénylacétylamino-méthyl)[1,2,3]triazol-1-yl]-butyramide,
(R)-N-hydroxy-4-naphtalén-2-yl-3-{4-[(2-p-tolyl-acétylamino)-méthyl]-[1,2,3]triazol-1-yl}-butyramide,
(R)-N-hydroxy-4-naphtalén-2-yl-3-{4-[(3-phényl-propionylamino)-méthyl]-[1,2,3]triazol-1-yl}-butyramide,
(R)-3-{4-[(4-fluoro-benzènesulfonylamino)-méthyl]-[1,2,3]triazol-1-yl}-N-hydroxy-4-naphtalén-2-yl-butyramide,
N-[1-(1-hydroxycarbamoylméthyl-2-phényl-éthyl)-1H-[1,2,3]triazol-4-ylméthyl]-4-méthyl-benzamide,
N-[1-(1-hydroxycarbamoylméthyl-2-phényl-éthyl)-1H-[1,2,3]triazol-4-ylméthyl]-4-fluoro-benzamide,
N-[5-(1-hydroxycarbamoylméthyl-2-naphtalén-2-yl-éthyl)-[1,2,4]oxadiazol-3-ylméthyl]-benzamide,
4-fluoro-N-[5-(1-hydroxycarbamoylméthyl-2-naphtalén-2-yl-éthyl)-[1,2,4]oxadiazol-3 -ylméthyl]-benzamide,
(R)-4-fluoro-N-[1-(1-hydroxycarbamoyl-2-naphtalén-2-yl-éthyl)-1H-[1,2,3]triazol-4-ylméthyl]-benzamide,
(S)-4-fluoro-N-[1-(2-hydroxycarbamoyl-1-naphtalén-2-ylméthyl-éthyl)-1H-[1,2,3]triazol-4-ylméthyl]-benzamide,
(R)-4-fluoro-N-[3-(1-hydroxycarbamoylméthyl-2-naphtalén-2-yl-éthyl)-3H-[1,2,3]triazol-4-ylméthyl]-benzamide,
[3-(1-hydroxycarbamoylméthyl-2-naphtalén-2-yl-éthyl)-3H-[1,2,3]triazol-4-ylméthyl]-amide de l'acide (R)-cyclohexanecarboxylique, et
(R)-N-hydroxy-4-naphtalén-2-yl-3-{5-[(3-phényl-propionylamino)-méthyl]-[1,2,3]triazol-1-yl}-butyramide.

2. Composé selon la revendication 1, dans lequel L¹ est -CH₂-.

3. Composé selon l'une quelconque des revendications 1 et 2, dans lequel A est CH.

4. Composé selon l'une quelconque des revendications 1 à 3, dans lequel Y est -CH₂-.

5. Composé selon l'une quelconque des revendications 1 à 4, dans lequel L² est sélectionné parmi -CH₂-, -CH₂-CH₂-, -C(CH₃)₂- et -CH(R⁵)-.

6. Composé selon l'une quelconque des revendications 1 à 5, dans lequel R⁵ est sélectionné parmi CH₃, CH₂-OH, CH₂-Ph-OH et CH₂-Ph-OR⁶.

7. Composé selon l'une quelconque des revendications 1 à 6, de formule II : 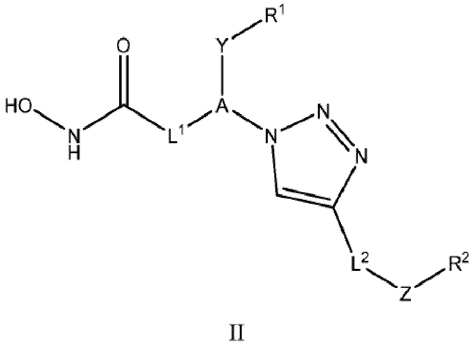 et les sels et solvates pharmaceutiquement acceptables de celui-ci,
dans lequel
L¹, L², A, Y, Z, R¹, R², R⁵, R⁶ et R⁷ sont tels que définis dans la revendication 1.

8. Composé selon l'une quelconque des revendications 1 à 6, de formule III : 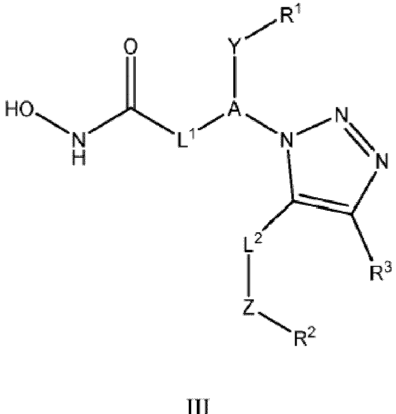 et les sels et solvates pharmaceutiquement acceptables de celui-ci,
dans lequel
L¹, L², A, Y, Z, R¹, R², R³, R⁴, R⁵, R⁶ et R⁷ sont tels que définis dans la revendication 1.

9. Composé selon l'une quelconque des revendications 1 à 6, de formule IV : 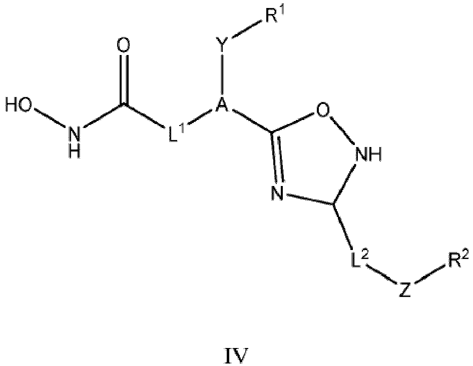 et les sels et solvates pharmaceutiquement acceptables de celui-ci,
dans lequel
L¹, L², A, Y, Z, R¹, R², R⁵, R⁶ et R⁷ sont tels que définis dans la revendication 1.

10. Composé selon l'une quelconque des revendications 1 à 6, de formule V : 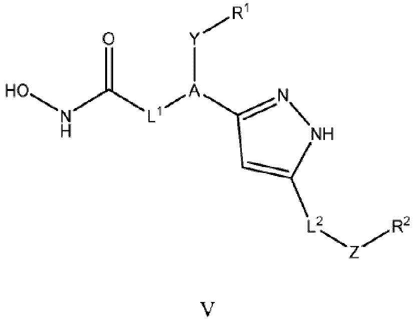 et les sels et solvates pharmaceutiquement acceptables de celui-ci,
dans lequel
L¹, L², A, Y, Z, R¹, R², R⁵, R⁶ et R⁷ sont tels que définis dans la revendication 1.

11. Composé selon l'une quelconque des revendications 1 à 10, sélectionné dans le groupe constitué par :
N-[1-(R-2-hydroxycarbamoyl-1-naphtalén-2-ylméthyl-éthyl)-1H-[1,2,3]triazol-4-ylméthyl]-4-trifluorométhyl-benzamide ;
(R)-N-hydroxy-4-naphtalén-2-yl-3-{4-[(toluène-4-sulfonylamino)-méthyl]-[1,2,3]triazol-1-yl}-butyramide ;
(R)-3-[4-(benzènesulfonylamino-méthyl)-[1,2,3]triazol-1-yl]-N-hydroxy-4-naphtalén-2-yl-butyramide ;
(R)-3-{4-[(4-fluoro-benzènesulfonylamino)-méthyl]-[1,2,3]triazol-1-yl}-N-hydroxy-4-naphtalén-2-yl-butyramide ;
(R)-N-[3-(1-hydroxycarbamoylméthyl-2-naphtalén-2-yl-éthyl)-3H-[1,2,3]triazol-4-ylméthyl]-benzamide ;
(R)-N-[1-(2-hydroxycarbamoyl-1-naphtalén-2-ylméthyl-éthyl)-1H-[1,2,3]triazol-4-ylméthyl]-N-méthyl-benzamide ;
[1-(2-hydroxycarbamoyl-1-naphtalén-2-ylméthyl-éthyl)-1H-[1,2,3]triazol-4-ylméthyl]-amide de l'acide (R)-4,4-difluoro-cyclohexanecarboxylique ;
(R)-3-{4-[(2-éthyl-butyrylamino)-méthyl]-[1,2,3]triazol-1-yl}-N-hydroxy-4-naphtalén-2-yl-butyramide ;
(R)-3-[4-(acétylamino-méthyl)-[1,2,3]triazol-1-yl]-N-hydroxy-4-naphtalén-2-yl-butyramide ;
(R)-3-[4-[(4-fluoro-phénylamino)-méthyl]-[1,2,3]triazol-1-yl]-N-hydroxy-4-naphtalén-2-yl-butyramide ;
((1-((R)-4-(hydroxyamino)-1-(naphtalén-2-yl)-4-oxobutan-2-yl)-1H-1,2,3-triazol-4-yl)méthyl)-L-leucinate de *tert*-butyle ;
(R)-N-hydroxy-4-naphtalén-2-yl-3-{4-[(3-phényl-uréido)-méthyl]-[1,2,3]triazol-1-yl}-butyramide ;
ester tert-butylique de l'acide (R)-[1-(2-hydroxycarbamoyl-1-naphtalén-2-ylméthyl-éthyl)-1H-[1,2,3]triazol-4-ylméthyl]-carbamique ;
(R)-3-(4-benzyloxyméthyl-[1,2,3]triazol-1-yl)-N-hydroxy-4-naphtalén-2-yl-butyramide ;
(R)-3-[5-(acétylamino-méthyl)-[1,2,3]triazol-1-yl]-N-hydroxy-4-naphtalén-2-yl-butyramide ;
(R)-N-hydroxy-4-naphtalén-2-yl-3-{5-[(3-phényl-propionylamino)-méthyl]-[1,2,3]triazol-1-yl}-butyramide ;
(R,S)-N-hydroxy-3-(5-{[2-(6-méthoxy-naphtalén-2-yl)-propionylamino]-méthyl}-[1,2,3]triazol-1-yl)-4-naphtalén-2-yl-butyramide ;
(R)-4-fluoro-N-{2-[1-(1-hydroxycarbamoyl-2-naphtalén-2-yl-éthyl)-1H-[1,2,3]triazol-4-yl]-éthyl}-benzamide ;
(R)-N-{1-[1-(4-bromo-benzyl)-2-hydroxycarbamoyl-éthyl]-1H-[1,2,3]triazol-4-ylméthyl}-4-fluoro-benzamide ;
(R)-N-[1-(1-biphényl-4-ylméthyl-2-hydroxycarbamoyl-éthyl)-1H-[1,2,3]triazol-4-ylméthyl]-4-fluoro-benzamide ;
(R)-4-fluoro-N-{1-[2-hydroxycarbamoyl-1-(4'-hydroxyméthyl-biphényl-4-ylméthyl)-éthyl]-1H-[1,2,3]triazol-4-ylméthyl}-benzamide ;
méthylamide de l'acide (R)-4'-(2-{4-[(4-fluoro-benzoylamino)-méthyl]-[1,2,3]triazol-1-yl}-3-hydroxycarbamoyl-propyl)-biphényl-4-carboxylique ;
(R)-N-{1-[1-(4'-acétylamino-biphényl-4-ylméthyl)-2-hydroxycarbamoyl-éthyl]-1H-[1,2,3]triazol-4-ylméthyl}-4-fluoro-benzamide;
(R)-N-(1-{1-[4'-(acétylamino-méthyl)-biphényl-4-ylméthyl]-2-hydroxycarbamoyl-éthyl}-1H-[1,2,3]triazol-4-ylméthyl)-4-fluoro-benzamide ;
(R)-4-fluoro-N-{1-[2-hydroxycarbamoyl-1-(3'-hydroxyméthyl-biphényl-4-ylméthyl)-éthyl]-1H-[1,2,3]triazol-4-ylméthyl}-benzamide ;
méthylamide de l'acide (R)-4'-(2-{4-[(4-fluoro-benzoylamino)-méthyl]-[1,2,3]triazol-1-yl}-3-hydroxycarbamoyl-propyl)-biphényl-3-carboxylique ;
(R)-N-{1-[1-(3'-acétylamino-biphényl-4-ylméthyl)-2-hydroxycarbamoyl-éthyl]-1H-[1,2,3]triazol-4-ylméthyl}-4-fluoro-benzamide;
4'-(2-{4-[(4-fluoro-benzoylamino)-méthyl]-[1,2,3]triazol-1-yl}-3-hydroxycarbamoyl-propyl)-biphényl-3-ylméthyl ester de l'acide (R)-formique ;
(R)-4-fluoro-N-{1-[2-hydroxycarbamoyl-1-(4-pyrimidin-5-yl-benzyl)-éthyl]-1H-[1,2,3]triazol-4-ylméthyl}-benzamide ;
ester tert-butylique de l'acide 4-{[1-(2-hydroxycarbamoyl-1-naphtalén-2-ylméthyl-éthyl)-1H-[1,2,3]triazol-4-ylméthyl]-carbamoyl}-pipéridine-1-carboxylique ;
4-fluoro-N-[5-(1-hydroxycarbamoyl-2-naphtalén-2-yl-éthyl)-[1,2,4]oxadiazol-3-ylméthyl]-benzamide ;
5-[[(4-fluorobenzoyl)amino]méthyl]-3-[(1R)-3-(hydroxyamino)-1-(2-naphtylméthyl)-3-oxo-propyl]triazole-4-carboxylate d'éthyle ;
2,4-difluoro-N-[1-(2-hydroxycarbamoyl-1-naphtalén-2-ylméthyl-éthyl)-1H-[1,2,3]triazol-4-ylméthyl]-benzamide ;
(R)-N-{1-[1-(3-bromo-benzyl)-2-hydroxycarbamoyl-éthyl]-1H-[1,2,3]triazol-4-ylméthyl}-4-fluoro-benzamide ;
(R)-N-[1-(1-biphényl-3-ylméthyl-2-hydroxycarbamoyl-éthyl)-1H-[1,2,3]triazol-4-ylméthyl]-4-fluoro-benzamide ;
(R)-N-{1-[1-(4'-acétylamino-biphényl-3-ylméthyl)-2-hydroxycarbamoyl-éthyl]-1H-[1,2,3]triazol-4-ylméthyl}-4-fluoro-benzamide ;
(R)-4-fluoro-N-{1-[2-hydroxycarbamoyl-1-(4'-hydroxyméthyl-biphényl-3-ylméthyl)-éthyl]-1H-[1,2,3]triazol-4-ylméthyl}-benzamide ;
(R)-N-{1-[1-(3'-acétylamino-biphényl-3-ylméthyl)-2-hydroxycarbamoyl-éthyl]-1H-[1,2,3]triazol-4-ylméthyl}-4-fluoro-benzamide ;
(R)-4-fluoro-N-{1-[2-hydroxycarbamoyl-1-(3'-hydroxyméthyl-biphényl-3-ylméthyl)-éthyl]-1H-[1,2,3]triazol-4-ylméthyl}-benzamide ;
4-fluoro-N-((3-((2-(hydroxyamino)-2-oxoéthyl)(naphtalén-2-ylméthyl)amino)-1H-pyrazol-5-yl)méthyl)benzamide ;
3,4-difluoro-N-[[1-[(1R)-3-(hydroxyamino)-1-(2-naphtylméthyl)-3-oxo-propyl]triazol-4-yl]méthyl]benzamide ;
4-[(diméthylamino)méthyl]-N-[[1-[(1R)-3-(hydroxyamino)-1-(2-naphtylméthyl)-3-oxo-propyl]triazol-4-yl]méthyl]benzamide ;
4-fluoro-N-[2-[1-[(1R)-3-(hydroxyamino)-1-(2-naphtylméthyl)-3-oxo-propyl]triazol-4-yl]éthyl]benzamide) ;
3,4-difluoro-N-[2-[1-[(1R)-3-(hydroxyamino)-1-(2-naphtylméthyl)-3-oxo-propyl]triazol-4-yl]éthyl]benzamide ;
3-{4-[(4-fluoro-phénylcarbamoyl)-méthyl]-[1,2,3]triazol-1-yl}-N-hydroxy-4-naphtalén-2-yl-butyramide ;
3,4-difluoro-N-((1-(3-(hydroxyamino)-1-(naphtalén-2-ylthio)-3-oxopropyl)-1H-1,2,3-triazol-4-yl)méthyl)benzamide;
N-[[1-[(1R)-3-(hydroxyamino)-1-(2-naphtylméthyl)-oxo-propyl]triazol-4-yl]méthyl]-3,4-diméthoxy-benzamide ;
N-[[1-[(1R)-3-(hydroxyamino)-1-(2-naphtylméthyl)-oxo-propyl]triazol-4-yl]méthyl]-1,3-benzodioxole-5-carboxamide ;
3 -chloro-4-fluoro-N-[[1-[(1R)-3-(hydroxyamino)-1-(2-naphtylméthyl)-3-oxo-propyl]triazol-4-yl]méthyl]benzamide ;
2,3,4-trifluoro-N-[1-(1-hydroxycarbamoylméthyl-2-naphtalén-2-yl-éthyl)-1H-[1,2,3]triazol-4-ylméthyl]-benzamide ;
méthyl(3R)-3-[4-[[(3,4-difluorobenzoyl)amino]méthyl]triazol-1-yl]-4-(1-naphtyl)butanoate ;
N-[[1-[(1R)-1-[(4-chlorophényl)méthyl]-3-(hydroxyamino)-3-oxo-propyl]triazol-4-yl]méthyl]-3,4-difluoro-benzamide ;
N-[[1-[(1R)-3-(hydroxyamino)-1-(2-naphtylméthyl)-oxo-propyl]triazol-4-yl]méthyl]pyrimidine-4-carboxamide ;
N-[[1-[1-(4-chlorophényl)sulfanyl-3-(hydroxyamino)-3 -oxo-propyl]triazol-4-yl]méthyl]-3,4-difluoro-benzamide ;
N-[[1-[(1R)-3-(hydroxyamino)-1-(2-naphtylméthyl)-oxo-propyl]triazol-4-yl]méthyl]-4-(2-méthoxyéthoxy)benzamide ;
N-[[1-[(1R)-3-(hydroxyamino)-1-(2-naphtylméthyl)-oxo-propyl]triazol-4-yl]méthyl]-1-méthyl-imidazole-4-carboxamide ;
3,4-difluoro-N-[[1-[(1R)-3-(hydroxyamino)-3-oxo-1-(2-phényléthyl)propyl]triazol-4-yl]méthyl]benzamide ;
3-[[1-[(1R)-3-(hydroxyamino)-1-(2-naphtylméthyl)-oxo-propyl]triazol-4-yl]méthyl carbamoyl]morpholine-4-carboxylate de tert-butyle ;
2-chloro-4-fluoro-N-[[1-[(1R)-3-(hydroxyamino)-1-(2-naphtylméthyl)-3-oxo-propyl]triazol-4-yl]méthyl]benzamide ;
4-hydroxy-N-[[1-[(1R)-3-(hydroxyamino)-1-(2-naphtylméthyl)-oxo-propyl]triazol-4-yl]méthyl]benzamide ;
3-{4-[(3,4-difluoro-phénylcarbamoyl)-méthyl]-[1,2,3]triazol-1-yl}-N-hydroxy-4-naphtalén-2-yl-butyramide ;
3,4-difluoro-N-[[1-[(E,1R)-1-[2-(hydroxyamino)-2-oxo-éthyl]-4-phényl-but-3-enyl]triazol-4-yl]méthyl]benzamide ;
3,4-difluoro-N-[[1-[(1R)-3-(hydroxyamino)-3-oxo-1-[[4-(trifluorométhyl)phényl]méthyl]propyl]triazol-4-yl]méthyl]benzamide ;
3,4-difluoro-N-{1-[1-(1-hydroxycarbamoylméthyl-2-naphtalén-2-yl-éthyl)-1H-[1,2,3]triazol-4-yl]-1-méthyl-éthyl}-benzamide ;
3,4-difluoro-N-[1-[1-[3-(hydroxyamino)-1-(2-naphtylméthyl)-3-oxo-propyl]triazol-4-yl]éthyl]benzamide ;
3-[4-(2-acétylamino-phénoxyméthyl)-[1,2,3]triazol-1-yl]-N-hydroxy-4-naphtalén-2-yl-butyramide ;
4-fluoro-N-[[1-[(1R)-3-(hydroxyamino)-1-(2-naphtylméthyl)-3-oxo-propyl]triazol-4-yl]méthyl]-3-méthyl-benzamide ;
N-(3,4-difluorophényl)-3-[1-[3-(hydroxyamino)-1-(2-naphtylméthyl)-3-oxo-propyl]triazol-4-yl]propanamide ;
N-[[1-[(1S)-1-(3,4-dihydro-1H-isoquinoléin-2-ylméthyl)-3-(hydroxyamino)-3-oxo-propyl]triazol-4-yl]méthyl]-3,4-difluoro-benzamide ;
3,4-difluoro-N-[[1-[(1R)-3-(hydroxyamino)-1-(1H-indol-3-ylméthyl)-3-oxo-propyl]triazol-4-yl]méthyl]benzamide ;
3,4-difluoro-N-[[1-[(1R,2S)-2-hydroxy-3-(hydroxyamino)-1-(2-naphtylméthyl)-3-oxo-propyl]triazol-4-yl]méthyl]benzamide ;
3,4-difluoro-N-{1-[1-(1(R)-hydroxycarbamoylméthyl-2-naphtalén-2-yl-éthyl)-1H-[1,2,3]triazol-4-yl]-(S)-éthyl}-benzamide ;
3,4-difluoro-N-{1-[1-(1(R)-hydroxycarbamoylméthyl-2-naphtalén-2-yl-éthyl)-1H-[1,2,3]triazol-4-yl]-(R)-éthyl}-benzamide ;
(3R)-3-[4-[[(3,4-difluorobenzoyl)-méthyl-amino]méthyl]triazol-1-yl]-4-(2-naphtyl)butanoate de méthyle ;
acide (3R)-3-[4-[(2S)-1-(3,4-difluorobenzoyl)pyrrolidin-2-yl]triazol-1-yl]-4-(1H-indol-3-yl)butanehydroxamique ;
N-[2-(4-benzyloxyphényl)-1-[1-[(1R)-3-(hydroxyamino)-1-(1H-indol-3-ylméthyl)-3-oxo-propyl]triazol-4-yl]éthyl]-3,4-difluoro-benzamide ;
acide (3R)-3-[4-[4-(3,4-difluorobenzoyl)morpholin-3-yl]triazol-1-yl]-4-(1H-indol-3-yl)butanehydroxamic ;
3,4-difluoro-N-[1-[1-[(1R)-3-(hydroxyamino)-1-(1H-indol-3-ylméthyl)-3-oxo-propyl]triazol-4-yl]éthyl]benzamide ;
3,4-difluoro-N-[(1R)-2-hydroxy-1-[1-[(1R)-3-(hydroxyamino)-1-(1H-indol-3-ylméthyl)-3-oxo-propyl]triazol-4-yl]éthyl]benzamide ;
acide (3R)-3-[4-[(2S)-1-(3,4-difluorobenzoyl)pyrrolidin-2-yl]triazol-1-yl]-4-(2-naphtyl)butanehydroxamique ;
N-[2-(4-benzyloxyphényl)-1-[1-[(1R)-3-(hydroxyamino)-1-(2-naphtylméthyl)-3-oxo-propyl]triazol-4-yl]éthyl]-3,4-difluoro-benzamide ;
acide (3R)-3-[4-[4-(3,4-difluorobenzoyl)morpholin-3-yl]triazol-1-yl]-4-(2-naphtyl)butanehydroxamique ;
3,4-difluoro-N-[(1R)-2-hydroxy-1-[1-[(1R)-3-(hydroxyamino)-1-(2-naphtylméthyl)-3-oxo-propyl]triazol-4-yl]éthyl]benzamide ;
3,4-difluoro-N-[[1-[(1R)-3-(hydroxyamino)-1-(1H-indol-3-ylméthyl)-3-oxo-propyl]triazol-4-yl]méthyl]-N-méthyl-benzamide ;
3,4-difluoro-N-[[1-[(1R)-3-(hydroxyamino)-1-(1H-indol-3-ylméthyl)-3-oxo-propyl]triazol-4-yl]méthyl]-N-isobutyl-benzamide ;
3,4-difluoro-N-[[1-[(1R)-3-(hydroxyamino)-1-(1H-indol-3-ylméthyl)-3-oxo-propyl]triazol-4-yl]méthyl]-N-isopentyl-benzamide ;
3,4-difluoro-N-[[1-[(1R)-3-(hydroxyamino)-1-(1H-indol-3-ylméthyl)-3-oxo-propyl]triazol-4-yl]méthyl]-N-[(1-méthylimidazol-2-yl)méthyl]benzamide ;
(3R)-4-(2-naphtyl)-3-[4-[(pyridine-4-carbonylamino)méthyl]triazol-1-yl]butanoate de méthyle ;
N-[[1-[(1R)-3-(hydroxyamino)-1-(2-naphtylméthyl)-3-oxo-propyl]triazol-4-yl]méthyl]-4-(hydroxycarbamoyl)benzamide ;
3,4-difluoro-N-[[1-[(1R)-3-(hydroxyamino)-3-oxo-1-[[3-(trifluorométhyl)phényl]méthyl]propyl]triazol-4-yl]méthyl]benzamide ;
N-({1-[(2R)-1-[4-(2-tert-butyl-2H-1,2,3,4-tétrazol-5-yl)phényl]-3-(hydroxycarbamoyl)propan2-yl]-1H-1,2,3-triazol-4-yl}méthyl)-3,4-difluorobenzamide ;
(R)-3,4-difluoro-N-((1-(4-(hydroxyamino)-4-oxo-1-(quinoléin-3-yl)butan-2-yl)-1H-1,2,3-triazol-4-yl)méthyl)benzamide ;
(R)-3,4-difluoro-N-((1-(4-(hydroxyamino)-4-oxo-1-(5,6,7,8-tétrahydronaphtalén-2-yl)butan-2-yl)-1H-1,2,3-triazol-4-yl)méthyl)benzamide ;
3,4-difluoro-N-[1-[1-[(1R)-3-(hydroxyamino)-1-(1H-indol-3-ylméthyl)-3-oxo-propyl]triazol-4-yl]-2-(4-hydroxyphényl)éthyl]benzamide ;
4-fluoro-N-(1-(1-((R)-4-(hydroxyamino)-1-(naphtalén-2-yl)-4-oxobutan-2-yl)-1H-1,2,3-triazol-4-yl)éthyl)benzamide ;
(R)-4-fluoro-N-((1-(4-(hydroxyamino)-4-oxo-1-(5,6,7,8-tétrahydronaphtalén-2-yl)butan-2-yl)-1H-1,2,3-triazol-4-yl)méthyl)benzamide ;
(R)-4-fluoro-N-((1-(4-(hydroxyamino)-4-oxo-1-(5,6,7,8-tétrahydronaphtalén-2-yl)butan-2-yl)-1H-1,2,3-triazol-4-yl)méthyl)-N-méthylbenzamide ;
4-fluoro-N-(1-(1-((R)-4-(hydroxyamino)-4-oxo-1-(5,6,7,8-tétrahydronaphtalén-2-yl)butan-2-yl)-1H-1,2,3-triazol-4-yl)éthyl)benzamide ;
3,4-difluoro-N-(1-(1-((R)-4-(hydroxyamino)-4-oxo-1-(5,6,7,8-tétrahydronaphtalén-2-yl)butan-2-yl)-1H-1,2,3-triazol-4-yl)éthyl)benzamide ;
(R)-3,4-difluoro-N-((1-(4-(hydroxyamino)-4-oxo-1-(5,6,7,8-tétrahydronaphtalén-2-yl)butan-2-yl)-1H-1,2,3-triazol-4-yl)méthyl)-N-méthylbenzamide ;
(R)-3,4-difluoro-N-((1-(4-(hydroxyamino)-4-oxo-1-(quinoléin-7-yl)butan-2-yl)-1H-1,2,3-triazol-4-yl)méthyl)benzamide ;
(R)-3,4-difluoro-N-((1-(4-(hydroxyamino)-4-oxo-1-(2-oxo-1,2,3,4-tétrahydroquinoléin-6-yl)butan-2-yl)-1H-1,2,3-triazol-4-yl)méthyl)benzamide ;
4-fluoro-N-(1-(1-((R)-4-(hydroxyamino)-4-oxo-1-(5,5,8,8-tétraméthyl-5,6,7,8-tétrahydronaphtalén-2-yl)butan-2-yl)-1H-1,2,3-triazol-4-yl)éthyl)benzamide ;
(R)-N-((1-(1-(benzo[b]thiophén-5-yl)-4-(hydroxyamino)-4-oxobutan-2-yl)-1H-1,2,3-triazol-4-yl)méthyl)-4-fluorobenzamide ;
(R)-N-((1-(1-(benzo[b]thiophén-6-yl)-4-(hydroxyamino)-4-oxobutan-2-yl)-1H-1,2,3-triazol-4-yl)méthyl)-4-fluorobenzamide ;
(R)-3,4-difluoro-N-((1-(4-(hydroxyamino)-1-(1H-indol-5-yl)-4-oxobutan-2-yl)-1H-1,2,3-triazol-4-yl)méthyl)benzamide ;
(R)-4-fluoro-N-((1-(4-(hydroxyamino)-1-(1H-indol-6-yl)-4-oxobutan-2-yl)-1H-1,2,3-triazol-4-yl)méthyl)benzamide ;
(R)-4-fluoro-N-((1-(4-(hydroxyamino)-1-(1-méthyl-1H-indol-5-yl)-4-oxobutan-2-yl)-1H-1,2,3-triazol-4-yl)méthyl)benzamide ;
(R)-N-((1-(1-(benzo[d]thiazol-6-yl)-4-(hydroxyamino)-4-oxobutan-2-yl)-1H-1,2,3-triazol-4-yl)méthyl)-3,4-difluorobenzamide ;
(R)-N-((1-(1-(benzo[b]thiophén-3-yl)-4-(hydroxyamino)-4-oxobutan-2-yl)-1H-1,2,3-triazol-4-yl)méthyl)-3,4-difluorobenzamide ;
(R)-N-((1-(1-(benzo[b]thiophén-3-yl)-4-(hydroxyamino)-4-oxobutan-2-yl)-1H-1,2,3-triazol-4-yl)méthyl)-4-fluorobenzamide ;
N-(1-(1-((R)-1-(benzo[b]thiophén-3-yl)-4-(hydroxyamino)-4-oxobutan-2-yl)-1H-1,2,3-triazol-4-yl)éthyl)-4-fluorobenzamide ;
(S)-N-((1-(1-(benzo[b]thiophén-2-yl)-4-(hydroxyamino)-4-oxobutan-2-yl)-1H-1,2,3-triazol-4-yl)méthyl)-4-fluorobenzamide ;
(R)-N-((1-(1-(2,3-dihydrobenzo[b][1,4]dioxin-6-yl)-4-(hydroxyamino)-4-oxobutan-2-yl)-1H-1,2,3-triazol-4-yl)méthyl)-3,4-difluorobenzamide ;
(R)-3-fluoro-N-((1-(4-(hydroxyamino)-1-(naphtalén-2-yl)-4-oxobutan-2-yl)-1H-1,2,3-triazol-4-yl)méthyl)benzamide ;
(R)-4-fluoro-N-((1-(4-(hydroxyamino)-1-(naphtalén-2-yl)-4-oxobutan-2-yl)-1H-1,2,3-triazol-4-yl)méthyl)-N-méthylbenzamide ;
(3R)-3-(4-(3-(4-fluorobenzoyl)thiazolidin-4-yl)-1H-1,2,3-triazol-1-yl)-N-hydroxy-4-(naphtalén-2-yl)butanamide ; et
(3R)-3-(4-(1-(4-fluorobenzoyl)pipéridin-2-yl)-1H-1,2,3-triazol-1-yl)-N-hydroxy-4-(naphtalén-2-yl)butanamide.

12. Composition pharmaceutique comprenant un composé selon l'une quelconque des revendications 1 à 11, ou un sel ou solvate pharmaceutiquement acceptable de celui-ci, et au moins un vecteur, diluant, excipient et/ou adjuvant pharmaceutiquement acceptables.

13. Composé selon l'une quelconque des revendications 1 à 11 ou sel ou solvate pharmaceutiquement acceptables de celui-ci pour leur utilisation en tant que médicament.

14. Composé de formule I : 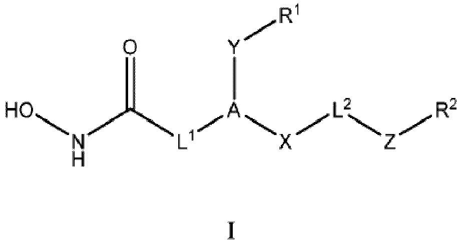 ou un sel ou solvate pharmaceutiquement acceptable de celui-ci pour son utilisation dans le traitement et/ou la prévention du cancer,
dans lequel
L¹ est inexistant ou est sélectionné parmi -CH₂- et -CH(OH)- ;
A est CH ou N ;
Y est -CH₂- ou -S- ;
R¹ est sélectionné parmi un aryle, un hétéroaryle, un arylalkyle et un arylalcényle dans lequel ledit aryle, hétéroaryle, arylalkyle ou arylalcényle est facultativement substitué par un ou plusieurs substituants indépendamment sélectionnés parmi un alkyle en C1-C4, un halogène, un halogénoalkyle en C1-C4, un hydroxy-(alkyle en C1-C4), un acétylamino, un acétylamino-(alkyle en C1-C4) et un (alkyl en C1-C4)-aminocarboxyle ;
R² est sélectionné parmi un aryle, un hétéroaryle, un arylalkyle, un cycloalkyle, un hétérocycloalkyle, un alkyle en C1-C6, un (alkyl en C1-C4)-oxycarbonyle, un alcoxy en C1-C4, un arylamino, dans lequel ledit aryle, arylalkyle, cycloalkyle ou
hétérocycloalkyle est facultativement substitué par un ou plusieurs substituants indépendamment sélectionnés parmi un alcoxy en C1-C4, un halogène, un halogénoalkyle en C1-C4, un alkyle en C1-C4, un (alcoxy en C1-C2)-(alcoxy en C1-C2), un (alkyl en C1-C4)-oxycarbonyle, un acétylamino, un di(alkyl en C1-C4)amino-(alkyle en C1-C4) et un hydroxycarbamoyle ;
X est sélectionné parmi 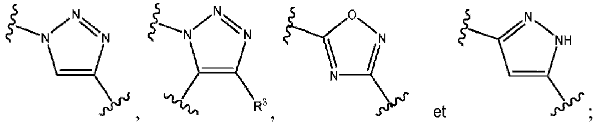
R³ est sélectionné parmi H et C(O)OR⁴ ;
R⁴ est un alkyle en C1-C4 ;
L² est un alkyle en C1-C6 linéaire ou ramifié, facultativement substitué par un groupe R⁵ ;
R⁵ est sélectionné parmi un alkyle en C1-C4 linéaire ou ramifié, CH₂-OH, CHOH-CH₃, CH₂-C(O)NH₂, CH₂-CH₂-C(O)NH₂, CH₂-COOH, CH₂-CH₂-COOH, (CH₂)₄-NH₂, (CH₂)₄-NH-C(NH₂⁺)-NH₂, un CH₂-imidazolyle, un CH₂-indolyle, CH₂-SH, CH₂-CH₂-S-CH₃, CH₂-Ph, CH₂-Ph-OH, CH₂-OR⁶, CH₂-COOR⁶, CH₂-CH₂-COOR⁶,
CH₂-SR⁶ et CH₂-Ph-OR⁶ ;
R⁶ est sélectionné parmi Me, Bn, Ph et Ac ;
Z est sélectionné parmi -NR⁷(CO)-, -NHSO₂-, -CH₂-CH₂-, -NH-, un -NH-CH(alkyle en C1-C6)-, -O-CH₂-, -C(O)NH- et -O- ; et
R⁷ est sélectionné parmi H, un alkyle en C1-C6 linéaire ou ramifié et un (1-méthylimidazol-2-yl)-(alkyle en C1-C2), ou
R⁷ et L² forment conjointement à l'atome d'azote auquel ils sont liés un groupe hétérocyclyle à 5 ou 6 chaînons.

15. Composé pour son utilisation selon la revendication 14, dans lequel le composé est un sensibilisateur pour la chimiothérapie de tumeurs malignes.

16. Composé pour son utilisation selon la revendication 15, dans lequel la chimiothérapie de tumeurs malignes implique un inhibiteur du protéasome, un inhibiteur de HDAC ou un inhibiteur de la glycosylation des protéines.

17. Composé pour son utilisation selon l'une quelconque des revendications 14 à 16, dans lequel le cancer est sélectionné dans le groupe constitué par un myélome multiple, un cancer du col de l'utérus et un cancer du sein.

18. Composé pour son utilisation selon l'une quelconque des revendications 14 à 17, dans lequel le composé est sélectionné dans le groupe constitué par :
N-[1-((R)-2-hydroxycarbamoyl-1-naphtalén-2-ylméthyl-éthyl)-1H-[1,2,3]triazol-4-ylméthyl]-benzamide ;
N-[1-((R)-2-hydroxycarbamoyl-1-naphtalén-2-ylméthyl-éthyl)-1H-[1,2,3]triazol-4-ylméthyl]-4-méthoxy-benzamide ;
4-fluoro-N-[1-(R-2-hydroxycarbamoyl-1-naphtalén-2-ylméthyl-éthyl)-1H-[1,2,3]triazol-4-ylméthyl]-benzamide ;
N-[1-(R-2-hydroxycarbamoyl-1-naphtalén-2-ylméthyl-éthyl)-1H-[1,2,3]triazol-4-ylméthyl]-4-trifluorométhyl-benzamide ;
(R)-N-hydroxy-4-naphtalén-2-yl-3-{4-[(toluène-4-sulfonylamino)-méthyl]-[1,2,3]triazol-1-yl}-butyramide ;
(R)-3-{4-[(4-fluoro-benzènesulfonylamino)-méthyl]-[1,2,3]triazol-1-yl}-N-hydroxy-4-naphtalén-2-yl-butyramide ;
(R)-3-[4-(benzènesulfonylamino-méthyl)-[1,2,3]triazol-1-yl]-N-hydroxy-4-naphtalén-2-yl-butyramide ;
(R)-N-hydroxy-4-naphtalén-2-yl-3-{4-[(2-p-tolyl-acétylamino)-méthyl]-[1,2,3]triazol-1-yl}-butyramide ;
(R)-3-{4-[(4-fluoro-benzènesulfonylamino)-méthyl]-[1,2,3]triazol-1-yl}-N-hydroxy-4-naphtalén-2-yl-butyramide ;
(R)-N-hydroxy-4-naphtalén-2-yl-3-{4-[(3-phényl-propionylamino)-méthyl]-[1,2,3]triazol-1-yl}-butyramide ;
(R)-4-fluoro-N-[3-(1-hydroxycarbamoylméthyl-2-naphtalén-2-yl-éthyl)-3H-[1,2,3]triazol-4-ylméthyl]-benzamide ;
(R)-N-[3-(1-hydroxycarbamoylméthyl-2-naphtalén-2-yl-éthyl)-3H-[1,2,3]triazol-4-ylméthyl]-benzamide ;
[3-(1-hydroxycarbamoylméthyl-2-naphtalén-2-yl-éthyl)-3H-[1,2,3]triazol-4-ylméthyl]-amide de l'acide (R)-cyclohexanecarboxylique ;
(R)-N-[1-(2-hydroxycarbamoyl-1-naphtalén-2-ylméthyl-éthyl)-1H-[1,2,3]triazol-4-ylméthyl]-N-méthyl-benzamide ;
[1-(2-hydroxycarbamoyl-1-naphtalén-2-ylméthyl-éthyl)-1H-[1,2,3]triazol-4-ylméthyl]-amide de l'acide (R)-4,4-difluoro-cyclohexanecarboxylique ;
(R)-N-hydroxy-4-naphtalén-2-yl-3-[4-(phénylacétylamino-méthyl)-[1,2,3]triazol-1-yl]-butyramide ;
(R)-3-{4-[(2-éthyl-butyrylamino)-méthyl]-[1,2,3]triazol-1-yl}-N-hydroxy-4-naphtalén-2-yl-butyramide ;
(R)-3-[4-(acétylamino-méthyl)-[1,2,3]triazol-1-yl]-N-hydroxy-4-naphtalén-2-yl-butyramide ;
(R)-3-{4-[(4-fluoro-phénylamino)-méthyl]-[1,2,3]triazol-1-yl}-N-hydroxy-4-naphtalén-2-yl-butyramide ;
((1-((R)-4-(hydroxyamino)-1-(naphtalén-2-yl)-4-oxobutan-2-yl)-1H-1,2,3-triazol-4-yl)méthyl)-L-leucinate de tert-butyle ;
(R)-N-hydroxy-4-naphtalén-2-yl-3-{4-[(3-phényl-uréido)-méthyl]-[1,2,3]triazol-1-yl}-butyramide ;
ester tert-butylique de l'acide (R)-[1-(2-hydroxycarbamoyl-1-naphtalén-2-ylméthyl-éthyl)-1H-[1,2,3]triazol-4-ylméthyl]-carbamique ;
(R)-3-(4-benzyloxyméthyl-[1,2,3]triazol-1-yl)-N-hydroxy-4-naphtalén-2-yl-butyramide ;
(R)-3-[5-(acétylamino-méthyl)-[1,2,3]triazol-1-yl]-N-hydroxy-4-naphtalén-2-yl-butyramide ;
(R)-N-hydroxy-4-naphtalén-2-yl-3-{5-[(3-phényl-propionylamino)-méthyl]-[1,2,3]triazol-1-yl}-butyramide ;
(R,S)-N-hydroxy-3-(5-{[2-(6-méthoxy-naphtalén-2-yl)-propionylamino]-méthyl}-[1,2,3]triazol-1-yl)-4-naphtalén-2-yl-butyramide ;
4-fluoro-N-[5-(1-hydroxycarbamoylméthyl-2-naphtalén-2-yl-éthyl)-[1,2,4]oxadiazol-3-ylméthyl]-benzamide ;
N-[5-(1-hydroxycarbamoylméthyl-2-naphtalén-2-yl-éthyl)-[1,2,4]oxadiazol-3-ylméthyl]-benzamide ;
(R)-4-fluoro-N-{2-[1-(1-hydroxycarbamoyl-2-naphtalén-2-yl-éthyl)-1H-[1,2,3]triazol-4-yl]-éthyl}-benzamide ;
(S)-4-fluor0-N-[1-(2-hydroxycarbamoyl-1-naphtalén-2-ylméthyl-éthyl)-1H-[1,2,3]triazol-4-ylméthyl]-benzamide ;
(R)-N-{1-[1-(4-bromo-benzyl)-2-hydroxycarbamoyl-éthyl]-1H-[1,2,3]triazol-4-ylméthyl}-4-fluoro-benzamide ;
(R)-N-[1-(1-biphényl-4-ylméthyl-2-hydroxycarbamoyl-éthyl)-1H-[1,2,3]triazol-4-ylméthyl]-4-fluoro-benzamide ;
(R)-4-fluoro-N-{1-[2-hydroxycarbamoyl-1-(4'-hydroxy méthyl-biphényl-4-ylméthyl)-éthyl]-1H-[1,2,3]triazol-4-ylméthyl}-benzamide ;
méthylamide de l'acide (R)-4'-(2-{4-[(4-fluoro-benzoylamino)-méthyl]-[1,2,3]triazol-1-yl}-3-hydroxycarbamoyl-propyl)-biphényl-4-carboxylique ;
(R)-N-{1-[1-(4'-acétylamino-biphényl-4-ylméthyl)-2-hydroxycarbamoyl-éthyl]-1H-[1,2,3]triazol-4-ylméthyl}-4-fluoro-benzamide ;
(R)-N-(1-{1-[4'-(acétylamino-méthyl)-biphényl-4-ylméthyl]-2-hydroxycarbamoyl-éthyl}-1H-[1,2,3]triazol-4-ylméthyl)-4-fluoro-benzamide ;
(R)-4-fluoro-N-{1-[2-hydroxycarbamoyl-1-(3'-hydroxy méthyl-biphényl-4-ylméthyl)-éthyl]-1H-[1,2,3]triazol-4-ylméthyl}-benzamide ;
méthylamide de l'acide (R)-4'-(2-{4-[(4-fluoro-benzoylamino)-méthyl]-[1,2,3]triazol-1-yl}-3-hydroxycarbamoyl-propyl)-biphényl-3-carboxylique ;
(R)-N-{1-[1-(3'-acétylamino-biphényl-4-ylméthyl)-2-hydroxycarbamoyl-éthyl]-1H-[1,2,3]triazol-4-ylméthyl}-4-fluoro-benzamide ;
4'-(2-{4-[(4-fluoro-benzoylamino)-méthyl]-[1,2,3]triazol-1-yl}-3-hydroxycarbamoyl-propyl)-biphényl-3-ylméthyl ester de l'acide (R)-formique ;
(R)-4-fluoro-N-{1-[2-hydroxycarbamoyl-1-(4-pyrimidin-5-yl-benzyl)-éthyl]-1H-[1,2,3]triazol-4-ylméthyl}-benzamide ;
ester tert-butylique de l'acide 4-{[1-(2-hydroxycarbamoyl-1-naphtalén-2-ylméthyl-éthyl)-1H-[1,2,3]triazol-4-ylméthyl]-carbamoyl}-pipéridine-1-carboxylique ;
(R)-4-fluoro-N-[1-(1-hydroxycarbamoyl-2-naphtalén-2-yl-éthyl)-1H-[1,2,3]triazol-4-ylméthyl]-benzamide ;
4-fluoro-N-[5-(1-hydroxycarbamoyl-2-naphtalén-2-yl-éthyl)-[1,2,4]oxadiazol-3-ylméthyl]-benzamide ;
5-[[(4-fluorobenzoyl)amino]méthyl]-3-[(1R)-3-(hydroxyamino)-1-(2-naphtylméthyl)-3-oxo-propyl]triazole-4-carboxylate d'éthyle ;
2,4-difluoro-N-[1-(2-hydroxycarbamoyl-1-naphtalén-2-ylméthyl-éthyl)-1H-[1,2,3]triazol-4-ylméthyl]-benzamide ;
(R)-N-{1-[1-(3-bromo-benzyl)-2-hydroxycarbamoyl-éthyl]-1H-[1,2,3]triazol-4-ylméthyl}-4-fluoro-benzamide ;
(R)-N-[1-(1-biphényl-3-ylméthyl-2-hydroxycarbamoyl-éthyl)-1H-[1,2,3]triazol-4-ylméthyl]-4-fluoro-benzamide ;
(R)-N-{1-[1-(4'-acétylamino-biphényl-3-ylméthyl)-2-hydroxycarbamoyl-éthyl]-1H-[1,2,3]triazol-4-ylméthyl}-4-fluoro-benzamide ;
(R)-4-fluoro-N-{1-[2-hydroxycarbamoyl-1-(4'-hydroxy méthyl-biphényl-3-ylméthyl)-éthyl]-1H-[1,2,3]triazol-4-ylméthyl}-benzamide ;
(R)-N-{1-[1-(3'-acétylamino-biphényl-3-ylméthyl)-2-hydroxycarbamoyl-éthyl]-1H-[1,2,3]triazol-4-ylméthyl}-4-fluoro-benzamide ;
(R)-4-fluoro-N-{1-[2-hydroxycarbamoyl-1-(3-hydroxy méthyl-biphényl-3-ylméthyl)-éthyl]-1H-[1,2,3]triazol-4-ylméthyl}-benzamide ;
4-fluoro-N-((3-((2-(hydroxyamino)-2-oxoéthyl)(naphtalén-2-ylméthyl)amino)-1H-pyrazol-5-yl)méthyl)benzamide ;
3,4-difluoro-N-[[1-[(1R)-3-(hydroxyamino)-1-(2-naphtylméthyl)-3-oxo-propyl]triazol-4-yl]méthyl]benzamide ;
4-[(diméthylamino)méthyl]-N-[[1-[(1R)-3-(hydroxyamino)-1-(2-naphtylméthyl)-3-oxo-propyl]triazol-4-yl]méthyl]benzamide ;
4-fluoro-N-[2-[1-[(1R)-3-(hydroxyamino)-1-(2-naphtylméthyl)-3-oxo-propyl]triazol-4-yl]éthyl]benzamide) ;
3,4-difluoro-N-[2-[1-[(1R)-3-(hydroxyamino)-1-(2-naphtylméthyl)-3-oxo-propyl]triazol-4-yl]éthyl]benzamide ;
3-{4-[(4-fluoro-phénylcarbamoyl)-méthyl]-[1,2,3]triazol-1-yl}-N-hydroxy-4-naphtalén-2-yl-butyramide ;
3,4-difluoro-N-((1-(3-(hydroxyamino)-1-(naphtalén-2-ylthio)-3-oxopropyl)-1H-1,2,3-triazol-4-yl)méthyl)benzamide;
N-[[1-[(1R)-3-(hydroxyamino)-1-(2-naphtylméthyl)-oxo-propyl]triazol-4-yl]méthyl]-3,4-diméthoxy-benzamide ;
N-[[1-[(1R)-3-(hydroxyamino)-1-(2-naphtylméthyl)-oxo-propyl]triazol-4-yl]méthyl]-1,3-benzodioxole-5-carboxamide ;
3-chloro-4-fluoro-N-[[1-[(1R)-3-(hydroxyamino)-1-(2-naphtylméthyl)-3-oxo-propyl]triazol-4-yl]méthyl]benzamide ;
2,3,4-trifluoro-N-[1-(1-hydroxycarbamoylméthyl-2-naphtalén-2-yl-éthyl)-1H-[1,2,3]triazol-4-ylméthyl]-benzamide ;
méthyl(3R)-3-[4-[[(3,4-difluorobenzoyl)amino]méthyl]triazol-1-yl]-4-(1-naphtyl)butanoate ;
N-[[1-[(1R)-1-[(4-chlorophényl)méthyl]-3-(hydroxyamino)-3-oxo-propyl]triazol-4-yl]méthyl]-3,4-difluoro-benzamide ;
N-[[1-[(1R)-3-(hydroxyamino)-1-(2-naphtylméthyl)-oxo-propyl]triazol-4-yl]méthyl]pyrimidine-4-carboxamide ;
N-[[1-[1-(4-chlorophényl)sulfanyl-3-(hydroxyamino)-3-oxo-propyl]triazol-4-yl]méthyl]-3,4-difluoro-benzamide ;
N-[[1-[(1R)-3-(hydroxyamino)-1-(2-naphtylméthyl)-oxo-propyl]triazol-4-yl]méthyl]-4-(2-méthoxy éthoxy)benzamide ;
N-[[1-[(1R)-3-(hydroxyamino)-1-(2-naphtylméthyl)-oxo-propyl]triazol-4-yl]méthyl]-1-méthyl-imidazole-4-carboxamide ;
3,4-difluoro-N-[[1-[(1R)-3-(hydroxyamino)-3-oxo-1-(2-phényléthyl)propyl]triazol-4-yl]méthyl]benzamide ;
3-[[1-[(1R)-3-(hydroxyamino)-1-(2-naphtylméthyl)-oxo-propyl]triazol-4-yl]méthyl carbamoyl]morpholine-4-carboxylate de tert-butyle ;
2-chloro-4-fluoro-N-[[1-[(1R)-3-(hydroxyamino)-1-(2-naphtylméthyl)-3-oxo-propyl]triazol-4-yl]méthyl]benzamide ;
4-hydroxy-N-[[1-[(1R)-3-(hydroxyamino)-1-(2-naphtylméthyl)-oxo-propyl]triazol-4-yl]méthyl]benzamide ;
3-{4-[(3,4-difluoro-phénylcarbamoyl)-méthyl]-[1,2,3]triazol-1-yl}-N-hydroxy-4-naphtalén-2-yl-butyramide ;
3,4-difluoro-N-[[1-[(E,1R)-1-[2-(hydroxyamino)-2-oxo-éthyl]-4-phényl-but-3-enyl]triazol-4-yl]méthyl]benzamide ;
3,4-difluoro-N-[[1-[(1R)-3-(hydroxyamino)-3-oxo-1-[[4-(trifluorométhyl)phényl]méthyl]propyl]triazol-4-yl]méthyl]benzamide ;
3,4-difluoro-N-{1-[1-(1-hydroxycarbamoylméthyl-2-naphtalén-2-yl-éthyl)-1H-[1,2,3]triazol-4-yl]-1-méthyl-éthyl}-benzamide ;
3,4-difluoro-N-[1-[1-[3-(hydroxyamino)-1-(2-naphtylméthyl)-3-oxo-propyl]triazol-4-yl]éthyl]benzamide ;
3-[4-(2-acetylamino-phénoxyméthyl)-[1,2,3]triazol-1-yl]-N-hydroxy-4-naphtalén-2-yl-butyramide ;
4-fluoro-N-[[1-[(1R)-3-(hydroxyamino)-1-(2-naphtylméthyl)-3-oxo-propyl]triazol-4-yl]méthyl]-3-méthyl-benzamide ;
N-(3,4-difluorophényl)-3-[1-[3-(hydroxyamino)-1-(2-naphtylméthyl)-3-oxo-propyl]triazol-4-yl]propanamide ;
N-[[1-[(1S)-1-(3,4-dihydro-1H-isoquinoléin-2-ylméthyl)-3-(hydroxyamino)-3-oxo-propyl]triazol-4-yl]méthyl]-3,4-difluoro-benzamide ;
3,4-difluoro-N-[[1-[(1R)-3-(hydroxyamino)-1-(1H-indol-3-ylméthyl)-3-oxo-propyl]triazol-4-yl]méthyl]benzamide ;
3,4-difluoro-N-[[1-[(1R,2S)-2-hydroxy-3-(hydroxyamino)-1-(2-naphtylméthyl)-3-oxo-propyl]triazol-4-yl]méthyl]benzamide ;
3,4-difluoro-N-{1-[1-(1(R)-hydroxycarbamoylméthyl-2-naphtalén-2-yl-éthyl)-1H-[1,2,3]triazol-4-yl]-(S)-éthyl}-benzamide ;
3,4-difluoro-N-{1-[1-(1(R)-hydroxycarbamoylméthyl-2-naphtalén-2-yl-éthyl)-1H-[1,2,3]triazol-4-yl]-(R)-éthyl}-benzamide ;
(3R)-3-[4-[[(3,4-difluorobenzoyl)-méthyl-amino]méthyl]triazol-1-yl]-4-(2-naphtyl)butanoate de méthyle ;
acide (3R)-3-[4-[(2S)-1-(3,4-difluorobenzoyl)pyrrolidin-2-yl]triazol-1-yl]-4-(1H-indol-3-yl)butanehydroxamique ;
N-[2-(4-benzyloxyphényl)-1-[1-[(1R)-3-(hydroxyamino)-1-(1H-indol-3-ylméthyl)-3-oxo-propyl]triazol-4-yl]éthyl]-3,4-difluoro-benzamide ;
acide (3R)-3-[4-[4-(3,4-difluorobenzoyl)morpholin-3-yl]triazol-1-yl]-4-(1H-indol-3-yl)butanehydroxamique ;
3,4-difluoro-N-[1-[1-[(1R)-3-(hydroxyamino)-1-(1H-indol-3-ylméthyl)-3-oxo-propyl]triazol-4-yl]éthyl]benzamide ;
3,4-difluoro-N-[(1R)-2-hydroxy-1-[1-[(1R)-3-(hydroxyamino)-1-(1H-indol-3-ylméthyl)-3-oxo-propyl]triazol-4-yl]éthyl]benzamide ;
acide (3R)-3-[4-[(2S)-1-(3,4-difluorobenzoyl)pyrrolidin-2-yl]triazol-1-yl]-4-(2-naphtyl)butanehydroxamique ;
N-[2-(4-benzyloxyphényl)-1-[1-[(1R)-3-(hydroxyamino)-1-(2-naphtylméthyl)-3-oxo-propyl]triazol-4-yl]éthyl]-3,4-difluoro-benzamide ;
acide (3R)-3-[4-[4-(3,4-difluorobenzoyl)morpholin-3-yl]triazol-1-yl]-4-(2-naphtyl)butanehydroxamique ;
3,4-difluoro-N-[(1R)-2-hydroxy-1-[1-[(1R)-3-(hydroxyamino)-1-(2-naphtylméthyl)-3-oxo-propyl]triazol-4-yl]éthyl]benzamide ;
3,4-difluoro-N-[[1-[(1R)-3-(hydroxyamino)-1-(1H-indol-3-ylméthyl)-3-oxo-propyl]triazol-4-yl]méthyl]-N-méthyl-benzamide ;
3,4-difluoro-N-[[1-[(1R)-3-(hydroxyamino)-1-(1H-indol-3-ylméthyl)-3-oxo-propyl]triazol-4-yl]méthyl]-N-isobutyl-benzamide ;
3,4-difluoro-N-[[1-[(1R)-3-(hydroxyamino)-1-(1H-indol-3-ylméthyl)-3-oxo-propyl]triazol-4-yl]méthyl]-N-isopentyl-benzamide ;
3,4-difluoro-N-[[1-[(1R)-3-(hydroxyamino)-1-(1H-indol-3-ylméthyl)-3-oxo-propyl]triazol-4-yl]méthyl]-N-[(1-méthylimidazol-2-yl)méthyl]benzamide ;
N-[1-(1-hydroxycarbamoylméthyl-2-phényl-éthyl)-1H-[1,2,3]triazol-4-ylméthyl]-4-méthyl-benzamide ;
(3R)-4-(2-naphtyl)-3-[4-[(pyridine-4-carbonylamino)méthyl]triazol-1-yl]butanoate de méthyle ;
N-[[1-[(1R)-3-(hydroxyamino)-1-(2-naphtylméthyl)-3-oxo-propyl]triazol-4-yl]méthyl]-4-(hydroxycarbamoyl)benzamide ;
3,4-difluoro-N-[[1-[(1R)-3-(hydroxyamino)-3-oxo-1-[[3-(trifluorométhyl)phényl]méthyl]propyl]triazol-4-yl]méthyl]benzamide ;
N-({1-[(2R)-1-[4-(2-tert-butyl-2H-1,2,3,4-tétrazol-5-yl)phényl]-3-(hydroxycarbamoyl)propan2-yl]-1H-1,2,3-triazol-4-yl}méthyl)-3,4-difluorobenzamide ;
(R)-3,4-difluoro-N-((1-(4-(hydroxyamino)-4-oxo-1-(quinoléin-3-yl)butan-2-yl)-1H-1,2,3-triazol-4-yl)méthyl)benzamide ;
(R)-3,4-difluoro-N-((1-(4-(hydroxyamino)-4-oxo-1-(5,6,7,8-tétrahydronaphtalén-2-yl)butan-2-yl)-1H-1,2,3-triazol-4-yl)méthyl)benzamide ;
3,4-difluoro-N-[1-[1-[(1R)-3-(hydroxyamino)-1-(1H-indol-3-ylméthyl)-3-oxo-propyl]triazol-4-yl]-2-(4-hydroxyphényl)éthyl]benzamide ;
4-fluoro-N-(1-(1-((R)-4-(hydroxyamino)-1-(naphtalén-2-yl)-4-oxobutan-2-yl)-1H-1,2,3-triazol-4-yl)éthyl)benzamide ;
(R)-4-fluoro-N-((1-(4-(hydroxyamino)-4-oxo-1-(5,6,7,8-tétrahydronaphtalén-2-yl)butan-2-yl)-1H-1,2,3-triazol-4-yl)méthyl)benzamide ;
(R)-4-fluoro-N-((1-(4-(hydroxyamino)-4-oxo-1-(5,6,7,8-tétrahydronaphtalén-2-yl)butan-2-yl)-1H-1,2,3-triazol-4-yl)méthyl)-N-méthylbenzamide ;
4-fluoro-N-(1-(1-((R)-4-(hydroxyamino)-4-oxo-1-(5,6,7,8-tétrahydronaphtalén-2-yl)butan-2-yl)-1H-1,2,3-triazol-4-yl)éthyl)benzamide ;
3,4-difluoro-N-(1-(1-((R)-4-(hydroxyamino)-4-oxo-1-(5,6,7,8-tétrahydronaphtalén-2-yl)butan-2-yl)-1H-1,2,3-triazol-4-yl)éthyl)benzamide ;
(R)-3,4-difluoro-N-((1-(4-(hydroxyamino)-4-oxo-1-(5,6,7,8-tétrahydronaphtalén-2-yl)butan-2-yl)-1H-1,2,3-triazol-4-yl)méthyl)-N-méthylbenzamide ;
(R)-3,4-difluoro-N-((1-(4-(hydroxyamino)-4-oxo-1-(quinoléin-7-yl)butan-2-yl)-1H-1,2,3-triazol-4-yl)méthyl)benzamide ;
(R)-3,4-difluoro-N-((1-(4-(hydroxyamino)-4-oxo-1-(2-oxo-1,2,3,4-tétrahydroquinoléin-6-yl)butan-2-yl)-1H-1,2,3-triazol-4-yl)méthyl)benzamide ;
4-fluoro-N-(1-(1-((R)-4-(hydroxyamino)-4-oxo-1-(5,5,8,8-tétraméthyl-5,6,7,8-tétrahydronaphtalén-2-yl)butan-2-yl)-1H-1,2,3-triazol-4-yl)éthyl)benzamide ;
(R)-N-((1-(1-(benzo[b]thiophén-5-yl)-4-(hydroxyamino)-4-oxobutan-2-yl)-1H-1,2,3-triazol-4-yl)méthyl)-4-fluorobenzamide ;
(R)-N-((1-(1-(benzo[b]thiophén-6-yl)-4-(hydroxyamino)-4-oxobutan-2-yl)-1H-1,2,3-triazol-4-yl)méthyl)-4-fluorobenzamide ;
(R)-3,4-difluoro-N-((1-(4-(hydroxyamino)-1-(1H-indol-5-yl)-4-oxobutan-2-yl)-1H-1,2,3-triazol-4-yl)méthyl)benzamide ;
(R)-4-fluoro-N-((1-(4-(hydroxyamino)-1-(1H-indol-6-yl)-4-oxobutan-2-yl)-1H-1,2,3-triazol-4-yl)méthyl)benzamide ;
(R)-4-fluoro-N-((1-(4-(hydroxyamino)-1-(1-méthyl-1H-indol-5-yl)-4-oxobutan-2-yl)-1H-1,2,3-triazol-4-yl)méthyl)benzamide ;
(R)-N-((1-(1-(benzo[d]thiazol-6-yl)-4-(hydroxyamino)-4-oxobutan-2-yl)-1H-1,2,3-triazol-4-yl)méthyl)-3,4-difluorobenzamide ;
(R)-N-((1-(1-(benzo[b]thiophén-3-yl)-4-(hydroxyamino)-4-oxobutan-2-yl)-1H-1,2,3-triazol-4-yl)méthyl)-3,4-difluorobenzamide ;
(R)-N-((1-(1-(benzo[b]thiophén-3-yl)-4-(hydroxyamino)-4-oxobutan-2-yl)-1H-1,2,3-triazol-4-yl)méthyl)-4-fluorobenzamide ;
N-(1-(1-((R)-1-(benzo[b]thiophén-3-yl)-4-(hydroxyamino)-4-oxobutan-2-yl)-1H-1,2,3-triazol-4-yl)éthyl)-4-fluorobenzamide ;
(S)-N-((1-(1-(benzo[b]thiophén-2-yl)-4-(hydroxyamino)-4-oxobutan-2-yl)-1H-1,2,3-triazol-4-yl)méthyl)-4-fluorobenzamide ;
(R)-N-((1-(1-(2,3-dihydrobenzo[b][1,4]dioxin-6-yl)-4-(hydroxyamino)-4-oxobutan-2-yl)-1H-1,2,3-triazol-4-yl)méthyl)-3,4-difluorobenzamide ;
(R)-3-fluoro-N-((1-(4-(hydroxyamino)-1-(naphtalén-2-yl)-4-oxobutan-2-yl)-1H-1,2,3-triazol-4-yl)méthyl)benzamide ;
(R)-4-fluoro-N-((1-(4-(hydroxyamino)-1-(naphtalén-2-yl)-4-oxobutan-2-yl)-1H-1,2,3-triazol-4-yl)méthyl)-N-méthylbenzamide ;
(3R)-3-(4-(3-(4-fluorobenzoyl)thiazolidin-4-yl)-1H-1,2,3-triazol-1-yl)-N-hydroxy-4-(naphtalén-2-yl)butanamide ; et
(3R)-3-(4-(1-(4-fluorobenzoyl)pipéridin-2-yl)-1H-1,2,3-triazol-1-yl)-N-hydroxy-4-(naphtalén-2-yl)butanamide.

19. Composé pour son utilisation l'une quelconque des revendications 14 à 18, dans lequel le composé est utilisé en combinaison avec une radiothérapie.
